# EUROPEAN PATENT APPLICATION

(11) **EP 2 103 603 A1**
(43) Date of publication of application: **23.09.2009**
(21) Application number: 07859751.5
(22) Date of filing: 07.12.2007
(51) Int. Cl.: C07D 263/26, A61P 3/04, A61P 3/06, A61P 3/10, C07D 401/14, C07D 403/06, C07D 403/14, C07D 409/14, C07D 417/14, A61K 31/4155, A61K 31/454, A61K 31/4545, A61K 31/496, A61K 31/497, A61K 31/506, A61K 31/541, C07C 205/51, C07D 207/06, C07D 207/26

(54) **METHOD FOR PRODUCING PYRROLIDINE COMPOUND**

(30) Priority: 07.12.2006 JP 2006331146; 21.12.2006 US 876239 P
(71) Applicant: Japan Tobacco Inc., Tokyo 105-8422 (JP)
(72) Inventor: HARADA, Kazuhito, Takatsuki-shi Osaka 569-1125 (JP); ITO, Takashi, Takatsuki-shi Osaka 569-1125 (JP)
(74) Representative: Vossius & Partner
(86) International application number: PCT/JP2007/073692
(87) International publication number: WO 2008/069313

(57) **Abstract**

Provided is a method capable of efficiently producing a compound having a superior HSD1 inhibitory action and a compound useful as a synthetic intermediate therefor, with superior achievability of asymmetric synthesis (i.e., superior selectivity), superior yield, superior safety and superior industrial workability at a low cost.

A method of producing a compound represented by the following formula [8]: 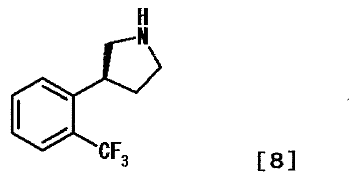 or a salt thereof, or a solvate thereof, from a compound represented by the following formula [2]: 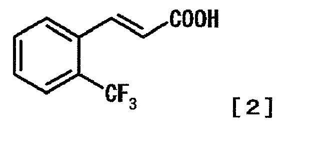 or a reactive derivative thereof, or a salt thereof, or a solvate thereof.

## Description

### Technical Field

The present invention relates to a production method of a pyrrolidine compound and an intermediate therefor.

### Background Art

As typically observed in the Cushing's syndrome, excess systemic glucocorticoid effect causes metabolic abnormalities such as accumulation of visceral fat, characteristic changes in body fat distribution, insulin resistance, diabetes, hyperlipidemia, elevation of blood pressure and the like.

It has been known for more than half a century that glucocorticoid plays a key role in diabetes and, for example, removal of hypophysis or adrenal gland from diabetic animal results in the relief of most serious conditions of diabetes and lower blood glucose concentration.

Glucocorticoid is known to (1) be essential for adipocyte differentiation program and inhibit, as a representative insulin-antagonistic hormone, glucose and lipid metabolism by insulin in various steps, (2) raise blood pressure via induction of the production of renin substrate, that is angiotensinogen, enhancement of angiotensin II receptor expression and the like, (3) cause hyperorexia and obesity as a potent leptin antagonistic hormone and the like.

In addition, it has been drawing attention that abnormally activated glucocorticoid effect in adipose tissue is involved in the pathology of metabolic syndrome clustered multiple metabolic diseases such as diabetes hyperlipidemia, hypertension, fatty liver and the like.

These facts suggest that reduction of the effect of glucocorticoid is beneficial for the prophylaxis or treatment of metabolic diseases such as diabetes, insulin resistance, diabetic complications, obesity, hyperlipidemia, hypertension, fatty liver and the like and metabolic syndrome clustered metabolic diseases, and further, fatal vascular events represented by myocardial infarction and cerebral apoplexy based on these diseases.

Active glucocorticoid (cortisol in human and corticosterone in rodents) exhibiting the glucocorticoid effect is produced from cortisone or 11-dehydrocorticosterone, both of which are inactive 11-ketometabolites, by the action of a converting enzyme, 11β-hydroxysteroid dehydrogenase type 1 (11βHSD1), in not only adrenal gland but also various tissues and cells. Since 11βHSD1 knockout mouse cannot convert an administered inactive glucocorticoid to an active type, this enzyme has been demonstrated to be the only active glucocorticoid converting enzyme in vivo. There is a report on a case of hypophyseal Cushing's disease with hyperglucocorticoidemia where the conditions of the Cushing's syndrome were not observed due to a markedly lowered 11βHSD1 activity by chance.

These results reveal that the regulation of the 11βHSD1 activity is significant for the expression of the action of glucocorticoid. In other words, regulation of the 11βHSD1 activity is beneficial for the prophylaxis or treatment of metabolic diseases such as diabetes, insulin resistance, diabetic complications, obesity, hyperlipidemia, hypertension, fatty liver and the like and metabolic syndrome clustered metabolic diseases, and further, fatal vascular events represented by myocardial infarction and cerebral apoplexy based on these diseases.

It has been clarified that transgenic mouse, which overexpresses 11βHSD1 only in the adipose tissue, also has major factors of metabolic syndromes such as visceral fat-type obesity, insulin-resistant diabetes, hyperlipidemia, hypertension, fatty liver and the like, along with increased enzyme activity comparable to that of obese individuals. In addition, while transgenic mouse exhibiting liver-specific overexpression of 11βHSD1 does not show a phenotype of visceral fat-type obesity but shows insulin resistance and the pathology of hypertension, fatty liver and the like. In contrast, 11βHSD1 knockout mouse shows resistance to the induction of hepatic gluconeogenesis enzyme (PEPCK, G6Pase etc.) due to stress-loading or high fat diet-loading, and shows clear resistance to the onset of diabetes.

Furthermore, 11βHSD1 knockout mouse shows decreased blood triglyceride level and increased blood HDL-cholesterol level because the expressions of molecular group relating to fat catabolism and PPARα that suppresses expression thereof markedly increase in the liver. It has been reported that 11βHSD1 knockout mouse shows clear attenuation of the accumulation of visceral adipose tissue and the onset of metabolic abnormalities induced by high fat diet-loading.

In fact, since insulin sensitivity is increased in 11βHSD1 deficiency, inhibition of the effect of this enzyme is expected to be useful for the prophylaxis or treatment of non-insulin dependent diabetes and obesity.

These findings support that reduction of the effect of 11βHSD1 is beneficial for the prophylaxis or treatment of metabolic diseases such as diabetes, insulin resistance, diabetic complications, obesity, hyperlipidemia, hypertension, fatty liver and the like and metabolic syndrome clustered metabolic diseases, and further, fatal vascular events represented by myocardial infarction and cerebral apoplexy based on those diseases.

Removal of adrenal gland weakens the effect of fasting which increases food intake and expression of hypothalamic neuropeptide Y. This supports the role of glucocorticoid to increase food intake and suggests that inhibition of 11βHSD1 in the brain will increase the satirety level, and therefore, decrease the food intake. That is, it is suggested that the reduction of the effect of 11βHSD1 is beneficial for the prophylaxis or treatment of hyperorexia.

Stress and glucocorticoids affect the cognitive function. In fact, 11βHSD1 knockout mouse shows marked improvement in the cognitive dysfunction caused by aging, and a recent report has documented that a 11βHSD1 non-specific inhibitor, carbenoxolone, is effective for cognitive dysfunction of elderly human. Furthermore, 11βHSD1 controls the level of action of glucocorticoid in the brain, thereby contributing to the neurotoxicity. Moreover, inhibition of 11βHSD1 in the brain results in the reduction of anxiety, which is based on the above-mentioned and known effect of glucocorticoid in the brain. Taken together, therefore, they suggest that the reduction of the 11βHSD1 effect in human brain prevents reactivation of cortisone to cortisol, and is useful for the prophylaxis or treatment of dysgnosia, neurodegenerative diseases accompanied by disappearance of nerve cells, emotional disorders such as anxiety, depression, mania and the like, schizophrenia, neurodysfunction including appetite stimulation and the like.

It is a well-known fact that glucocorticoid suppresses the immune system. Therefore, reduction of the effect of 11βHSD1 is suggested to be beneficial for the prophylaxis or treatment of diseases showing lowered immunity such as immunodeficiency and the like or for the prophylaxis or treatment of diseases requiring increase of immunity.

Recent data suggest that the levels of glucocorticoid target receptor and 11βHSD1 enzyme determine the susceptibility to glaucoma. Hence, reduction of the effect of 11βHSD1 is suggested to be beneficial for the prophylaxis or treatment of glaucoma.

Although glucocorticoid plays an essential part in the growth and function of the skeleton, excess glucocorticoid is harmful. Glucocorticoids-induced bone loss is at least partly induced via inhibition of bone formation including suppression of osteoblast growth and collagen synthesis. The negative effect on the bone nodule formation can be blocked by a non-specific 11βHSD1 inhibitor, carbenoxolone, which suggests the important role of 11βHSD1 in the glucocorticoid effect. Therefore, reduction of the effect of 11βHSD1 is suggested to be beneficial for the prophylaxis or treatment of osteoporosis.

Accordingly, it can be said that a substance having an activity to reduce the effect of 11βHSD1 is useful as a drug for the prophylaxis or treatment of the pathology involving glucocorticoid, such as
1) metabolic diseases including diabetes, insulin resistance, diabetic complications, obesity, hyperlipidemia, hypertension and fatty liver,
2) metabolic syndrome,
3) fatal vascular events represented by myocardial infarction and cerebral apoplexy based on such diseases,
4) hyperorexia,
5) diseases including dysgnosia, neurodegenerative diseases accompanied by disappearance of nerve cells, emotional disorders such as anxiety, depression, mania and the like, schizophrenia and neurodysfunction including appetite stimulation,
6) diseases requiring prophylaxis or treatment of lowered immunity or increase of immunity, such as immunodeficiency and the like,
7) glaucoma,
8) osteoporosis
and the like.

### Disclosure of the Invention

### Problems to be Solved by the Invention

A compound having an HSD1 inhibitory action is useful for the prophylaxis or treatment of metabolic diseases such as diabetes, insulin resistance, diabetic complications, obesity, hyperlipidemia, hypertension, fatty liver and the like, and the like, and the like. Therefore, provision of a compound having an HSD1 inhibitory action and superior in the yield, safety, industrial workability, degree of achievement of asymmetric synthesis (i.e., selectivity), cost and the like, and a novel production method of a synthetic intermediate therefor, as well as as a novel compound useful as a synthetic intermediate therefor has been desired.

### Means of Solving the Problems

The present inventors have conducted intensive studies and, as a result, found a compound having an HSD1 inhibitory action, which is represented by the formula [1']:

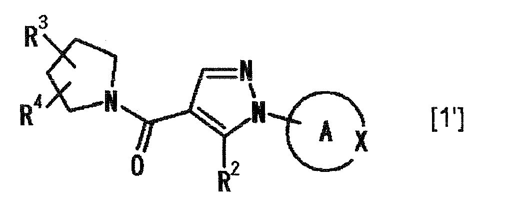

a novel production method of a synthetic intermediate therefor, as well as as a novel compound useful as a synthetic intermediate therefor, which resulted in the completion of the present invention. Accordingly, the present invention more specifically provides the following.

(1) A compound represented by the following formula [3]:

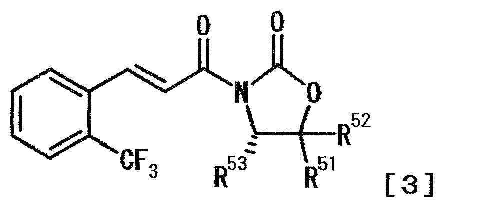

wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof.
(2) The compound of the above-mentioned (1), wherein both R⁵¹ and R⁵² are phenyl groups, or a salt thereof, or a solvate thereof.
(3) The compound of the above-mentioned (1) or (2), wherein R⁵³ is an isopropyl group, or a salt thereof, or a solvate thereof.
(4) A compound represented by the following formula [4]:

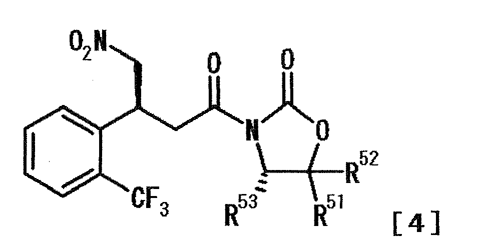

wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof.
(5) The compound of the above-mentioned (4), wherein both R⁵¹ and R⁵² are phenyl groups, or a salt thereof, or a solvate thereof.
(6) The compound of the above-mentioned (4) or (5), wherein R⁵³ is an isopropyl group, or a salt thereof, or a solvate thereof.
(7) A compound represented by the following formula [6]:

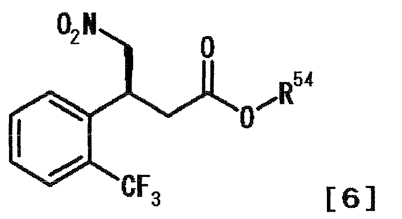

wherein R⁵⁴ is a C₁₋₆ alkyl group optionally substituted by one or more, same or different aryl groups,
or a solvate thereof.
(8) A compound represented by the following formula [7]:

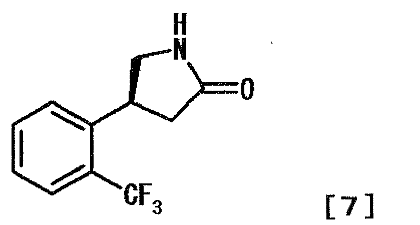

or a solvate thereof.
(9) A compound represented by the following formula [8]:

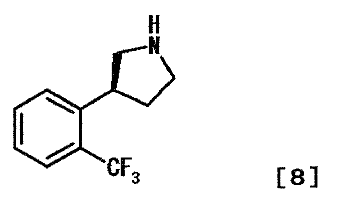

or a salt thereof, or a solvate thereof.
(10) A production method of a compound represented by the following formula [3]:

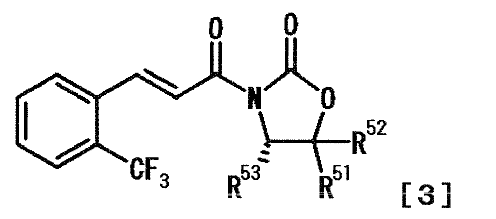

wherein R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group, or a salt thereof, or a solvate thereof, comprising reacting a compound represented by the following formula [15]:

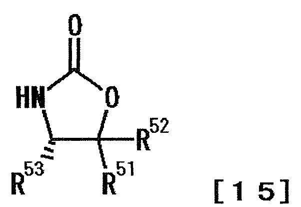

wherein R⁵¹, R⁵² and R⁵³ are as defined above, or a salt thereof, or a solvate thereof, with a compound represented by the following formula [2]:

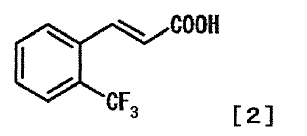

or a reactive derivative thereof, or a salt thereof, or a solvate thereof.
(11) The production method of the above-mentioned (10), wherein the reactive derivative of the compound represented by the formula [2] is a reactive derivative represented by the following formula [2a]:

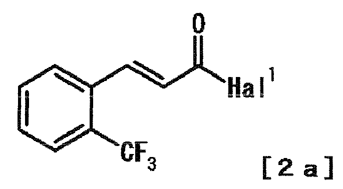

wherein Hall is a halogen atom.
(12) The production method of the above-mentioned (11), wherein Hal¹ is a chlorine atom.
(13) The production method of any one of the above-mentioned (10) to (12), wherein R⁵¹ and R⁵² are each a phenyl group.
(14) The production method of any one of the above-mentioned (10) to (13), wherein R⁵³ is an isopropyl group.
(15) The production method of any one of the above-mentioned (10) to (12), wherein the compound represented by the formula [3] is (S)-4-isopropyl-5,5-diphenyl-3-[(E)-3-(2-trifluoromethylphenyl)-acryloyl]-oxazolidin-2-one.
(16) A production method of a compound represented by the following formula [4]:

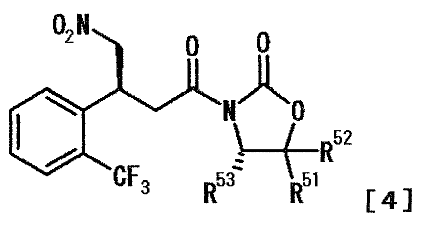

wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group, or a salt thereof, or a solvate thereof, comprising reacting a compound represented by the following formula [3]:

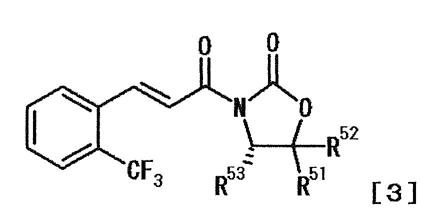

wherein R⁵¹, R⁵² and R⁵³ are as defined above,
or a salt thereof, or a solvate thereof, with nitromethane.
(17) The production method of the above-mentioned (16), wherein R⁵¹ and R⁵² are each a phenyl group.
(18) The production method of the above-mentioned (16) or (17), wherein R⁵³ is an isopropyl group.
(19) A production method of a compound represented by the following formula [6]:

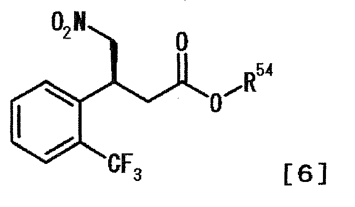

wherein R⁵⁴ is a C₁₋₆ alkyl group optionally substituted by one or more, same or different aryl groups, or a solvate thereof, comprising reacting a compound represented by the following formula [4]:

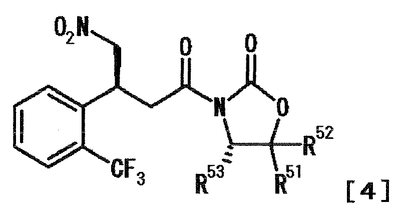

wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof, with a compound represented by the following formula [5]:

R⁵⁴-OH [5]

wherein R⁵⁴ is as defined above,
or a solvate thereof.
(20) The production method of the above-mentioned (19), wherein R⁵¹ and R⁵² are each a phenyl group.
(21) The production method of the above-mentioned (19) or (20), wherein R⁵³ is an isopropyl group.
(22) A production method of a compound represented by the following formula [7]:

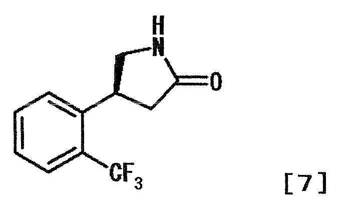

or a solvate thereof, comprising reducing a compound represented by the following formula [6]:

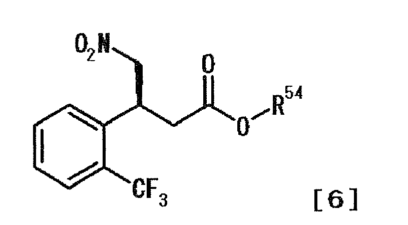

wherein R⁵⁴ is a C₁₋₆ alkyl group optionally substituted by one or more, same or different aryl groups, or a solvate thereof, and then cyclizing the compound.
(23) A production method of a compound represented by the following formula [8]:

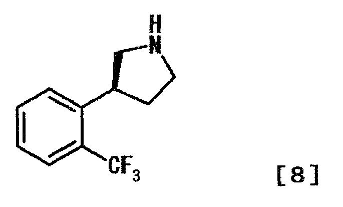

or a salt thereof, or a solvate thereof, comprising reducing a compound represented by the following formula [7]:

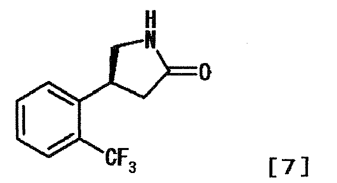

or a solvate thereof.
(24) A method of producing a compound represented by the following formula [8]:

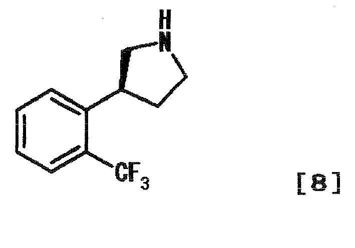

or a salt thereof, or a solvate thereof, from a compound represented by the following formula [2]:

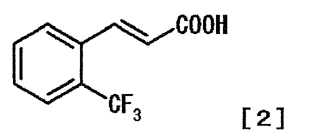

or a reactive derivative thereof, or a salt thereof, or a solvate thereof, comprising one or more steps selected from the following group A:
[group A]
(1) a step of producing a compound represented by the following formula [3]:

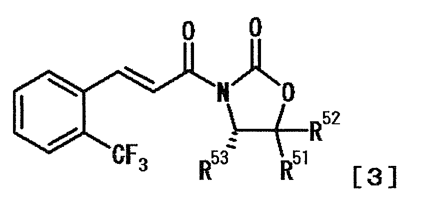

wherein R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof, comprising reacting a compound represented by the following formula [15]:

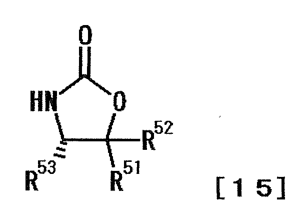

wherein R⁵¹, R⁵² and R⁵³ are as defined above, or a salt thereof, or a solvate thereof, with a compound represented by the following formula [2]:

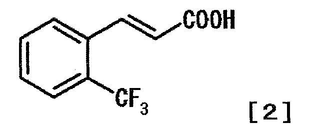

or a reactive derivative thereof, or a salt thereof, or a solvate thereof;
(2) a step of producing a compound represented by the following formula [4]:

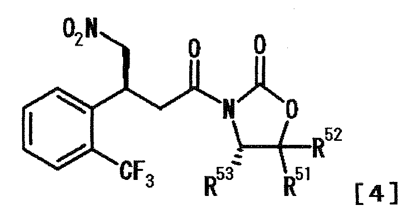

wherein R⁵¹, R⁵² and R⁵³ are as defined above,
or a salt thereof, or a solvate thereof, comprising reacting a compound represented by the above-mentioned formula [3] or a salt thereof, or a solvate thereof, with nitromethane;
(3) a step of producing a compound represented by the following formula [6]:

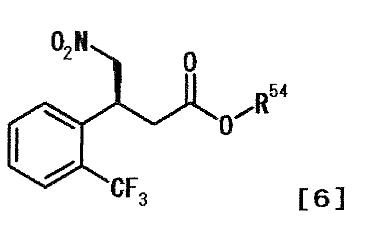

wherein R⁵⁴ is a C₁₋₆ alkyl group optionally substituted by one or more, same or different aryl groups, or a solvate thereof, comprising reacting a compound represented by the above-mentioned formula [4] or a salt thereof, or a solvate thereof, with a compound represented by the following formula [5]:

R⁵⁴-OH [5]

wherein R⁵⁴ is as defined above, or a solvate thereof;
(4) a step of producing a compound represented by the following formula [7]:

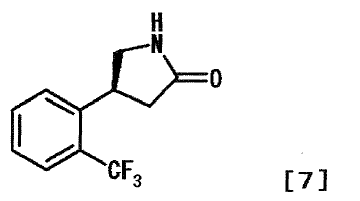

or a solvate thereof, comprising reducing a compound represented by the above-mentioned formula [6] or a solvate thereof, and then cyclizing the compound; and (5) a step of producing a compound represented by the following formula [8]:

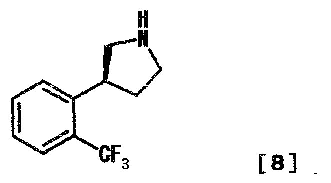

or a salt thereof, or a solvate thereof, comprising reducing a compound represented by the above-mentioned formula [7] or a solvate thereof.
(25) The production method of the above-mentioned (24), comprising all steps of group A.
(26) The production method of the above-mentioned (24) or (25), wherein R⁵¹ and R⁵² are each a phenyl group.
(27) The production method of any one of the above-mentioned (24) to (26), wherein R⁵³ is an isopropyl group.
(28) A method of producing a compound represented by the following formula [9]:

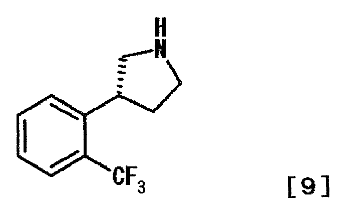

or a salt thereof, or a solvate thereof, from a compound represented by the following formula [2]:

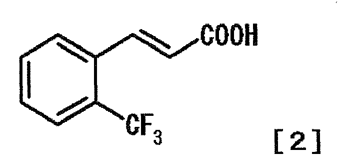

or a reactive derivative thereof, or a salt thereof, or a solvate thereof, comprising one or more steps selected from the following group B:
[group B]
(1) a step of producing a compound represented by the following formula [11]:

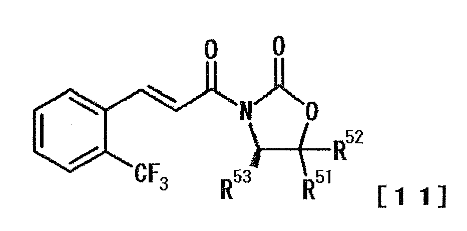

wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group, or a salt thereof, or a solvate thereof, comprising reacting a compound represented by the following formula [10]:

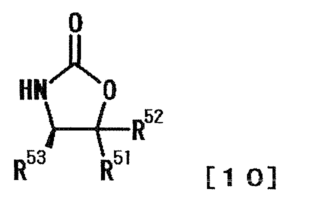

wherein R⁵¹, R⁵² and R⁵³ are as defined above, or a salt thereof, or a solvate thereof, with a compound represented by the following formula [2]:

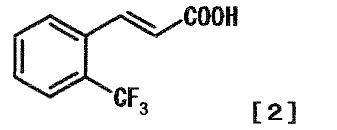

or a reactive derivative thereof, or a salt thereof, or a solvate thereof;
(2) a step of producing a compound represented by the following formula [12]:

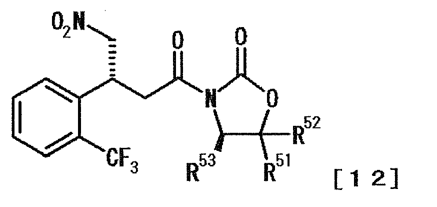

wherein R⁵¹, R⁵² and R⁵³ are as defined above, or a salt thereof, or a solvate thereof, comprising reacting a compound represented by the above-mentioned formula [11] or a salt thereof, or a solvate thereof, with nitromethane;
(3) a step of producing a compound represented by the following formula [13]:

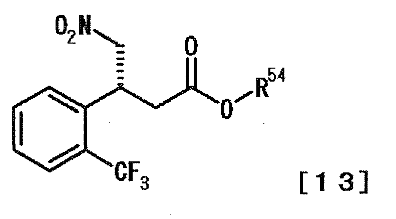

wherein R⁵⁴ is a C₁₋₆ alkyl group optionally substituted by one or more, same or different aryl groups, or a solvate thereof, comprising reacting a compound represented by the above-mentioned formula [12] or a salt thereof, or a solvate thereof, with a compound represented by the following formula [5]:

R⁵⁴-OH [5]

wherein R⁵⁴ is as defined above, or a solvate thereof;
(4) a step of producing a compound represented by the following formula [14]:

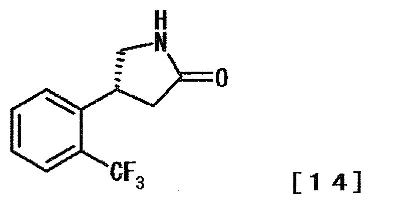

or a solvate thereof, comprising reducing a compound represented by the above-mentioned formula [13] or a solvate thereof, and then cyclizing the compound; and
(5) a step of producing a compound represented by the following formula [9]:

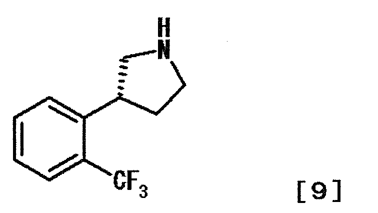

or a salt thereof, or a solvate thereof, comprising reducing a compound represented by the above-mentioned formula [14] or a solvate thereof.
(29) The production method of the above-mentioned (28), comprising all steps of group B.
(30) The production method of the above-mentioned (28) or (29), wherein R⁵¹ and R⁵² are each a phenyl group.
(31) The production method of any one of the above-mentioned (28) to (30), wherein R⁵³ is an isopropyl group.

(32) A production method of a compound represented by the following formula [17]:

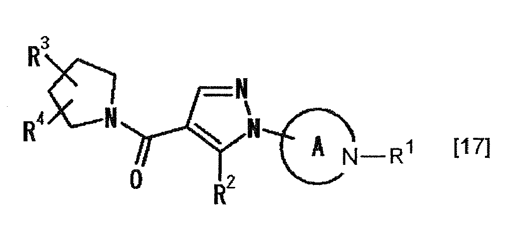

wherein each symbol is as defined below, or a salt thereof, or a solvate thereof, comprising introducing substituent R¹ (R¹ is as defined below excluding a hydrogen atom) into a nitrogen atom on ring A in
a compound represented by the following formula [16]:

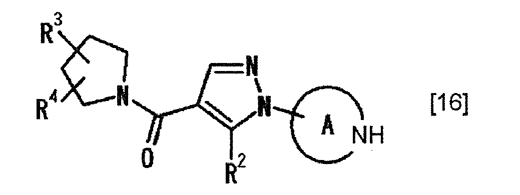

wherein
ring A is a saturated monocyclic nitrogen-containing heterocyclic group, which is optionally substituted by one or more, same or different substituent R¹,
the substituent R¹ is
1) a hydrogen atom,
2) -CONR⁵R⁶ wherein R⁵ and R⁶ are the same or different and each is
   (a) a hydrogen atom,
   (b) an aryl group (which is optionally substituted by one or more, same or different substituents selected from the following groups:
      a) a hydroxyl group,
      b) a halogen atom,
      c) a carboxyl group,
      d) a C₁₋₆ alkyl group (which is optionally substituted by one or more, same or different substituents selected from the following groups: (i) a halogen atom, (ii) a hydroxyl group and (iii) a C₁₋₆ alkoxy group), and
      e) a C₁₋₆ alkoxy group),
   (c) a cycloalkyl group (which is optionally substituted by one or more, same or different C₁₋₆ alkyl groups optionally substituted by one or more, same or different substituents selected from the following groups: (i) a hydroxyl group and (ii) a C₁₋₆ alkoxy group)),
   (d) a C₁₋₆ alkyl group (which is optionally substituted by one or more, same or different substituents selected from the following groups:
      a) a hydroxyl group,
      b) a halogen atom,
      c) an aryl group (which is optionally substituted by one or more, same or different substituents selected from the following (1) and (2): (1) a halogen atom and (2) a C₁₋₆ alkyl group optionally substituted by one or more, same or different halogen atoms),
      d) -NR²⁰R²¹ wherein R²⁰ and R²¹ are the same or different and each is a hydrogen atom, a C₁₋₆ alkyl group or a -CO-C₁₋₆ alkyl group, or R²⁰ and R²¹ optionally form, together with the nitrogen atom bonded thereto, a saturated monocyclic nitrogen-containing heterocyclic group (which is optionally substituted by one or more, same or different substituents selected from the following (i) to (iv):
         (i) a halogen atom,
         (ii) a hydroxyl group,
         (iii) an oxo group, and
         (iv) a C₁₋₆ alkoxy group)),
      e) a C₁₋₆ alkoxy group, and
      f) a carboxyl group),
   (e) -S(=O)₂-R⁹ wherein R⁹ is an aryl group (which is optionally substituted by one or more, same or different substituents selected from the following (1) and (2): (1) a halogen atom and (2) a C₁₋₆ alkyl group (which is optionally substituted by one or more, same or different halogen atoms)), or a C₁₋₆ alkyl group),
   (f) a saturated monocyclic nitrogen-containing heterocyclic group (which is optionally substituted by one or more, same or different substituents selected from the following a) to c):
      a) a C₁₋₆ alkyl group,
      b) a -CO-C₁₋₆ alkyl group and
      c) an oxo group),
   (g) a C₁₋₆ alkoxy group, or
   (h) a monocyclic nitrogen-containing unsaturated heterocyclic group (which is optionally substituted by one or more, same or different halogen atoms), or
   (i) R⁵ and R⁶ optionally form, together with the nitrogen atom bonded thereto, a saturated monocyclic nitrogen-containing heterocyclic group, or a heterocyclic group which is a fused ring of said heterocycle and a carbocycle (which heterocyclic group is optionally substituted by one or more, same or different substituents selected from the following a) to i):
      a) a halogen atom,
      b) a hydroxyl group,
      c) a C₁₋₆ alkyl group (which is optionally substituted by one or more, same or different substituents selected from the following groups: (i) a halogen atom, (ii) a hydroxyl group and (iii) a C₁₋₆ alkoxy group),
      d) a carboxyl group,
      e) a -CO-C₁₋₆ alkyl group,
      f) -CO-NR²²R²³ wherein R²² and R²³ are the same or different and each is a hydrogen atom or a C₁₋₆ alkyl group, or R²² and R²³ optionally form, together with the nitrogen atom bonded thereto, a saturated monocyclic nitrogen-containing heterocyclic group,
      g) an oxo group,
      h) -NR²⁴R²⁵ wherein R²⁴ and R²⁵ are the same or different and each is a hydrogen atom, a C₁₋₆ alkyl group or a -CO-C₁₋₆ alkyl group, or R²⁴ and R²⁵ optionally form, together with the nitrogen atom bonded thereto, a saturated monocyclic nitrogen-containing heterocyclic group, and
      i) a C₁₋₆ alkoxy group)),
3) -COOR¹⁰ wherein R¹⁰ is
   (a) a C₁₋₆ alkyl group (which is optionally substituted by one or more, same or different substituents selected from the following a) to d):
      a) a hydroxyl group,
      b) -NR²⁶R²⁷ wherein R²⁶ and R²⁷ are the same or different and each is a hydrogen atom or a C₁₋₆ alkyl group, or R²⁶ and R²⁷ optionally form, together with the nitrogen atom bonded thereto, a saturated monocyclic nitrogen-containing heterocyclic group,
      c) a C₁₋₆ alkoxy group, and
      d) an aryl group), or
   (b) a saturated monocyclic nitrogen-containing heterocyclic group (which is optionally substituted by one or more, same or different substituents selected from the following a) to c):
      a) a C₁₋₆ alkyl group,
      b) a -CO-C₁₋₆ alkyl group, and
      c) an oxo group)),
4) -COR¹¹ wherein R¹¹ is
   (a) a C₁₋₆ alkyl group (which is optionally substituted by one or more, same or different substituents selected from the following a) to h):
      a) a halogen atom,
      b) a hydroxyl group,
      c) -NR²⁸R²⁹ wherein R²⁸ and R²⁹ are the same or different and each is a hydrogen atom, a C₁₋₆ alkyl group, a -CO-C₁₋₆ alkyl group, a -CO-cycloalkyl group or a -S(=O)₂-C₁₋₆ alkyl group, or R²⁸ and R²⁹ optionally form, together with the nitrogen atom bonded thereto, a saturated monocyclic nitrogen-containing heterocyclic group,
      d) -CO-NR³⁰R³¹ wherein R³⁰ and R³¹ are the same or different and each is a hydrogen atom or a C₁₋₆ alkyl group, or R³⁰ and R³¹ optionally form, together with the nitrogen atom bonded thereto, a saturated monocyclic nitrogen-containing heterocyclic group,
      e) a C₁₋₆ alkoxy group,
      f) an aryl group (which is optionally substituted by one or more, same or different substituents selected from the following groups: (i) a halogen atom, (ii) a hydroxyl group, (iii) a C₁₋₆ alkoxy group, and (iv) a C₁₋₆ alkyl group (which is optionally substituted by one or more, same or different halogen atoms)),
      g) a carboxyl group, and
      h) an aryloxy group),
   (b) a cycloalkyl group (which is optionally substituted by one or more, same or different substituents selected from the following a) and b):
      a) an aryl group (which is optionally substituted by one or more, same or different substituents selected from the following (1) and (2): (1) a halogen atom and (2) a C₁₋₆ alkyl group (which is optionally substituted by one or more, same or different halogen atoms)), and
      b) a C₁₋₆ alkyl group (which is optionally substituted by one or more, same or different substituents selected from the following groups: (i) a hydroxyl group and (ii) a C₁₋₆ alkoxy group)),
   (c) a saturated monocyclic nitrogen-containing heterocyclic group (which is optionally substituted by one or more, same or different substituents selected from the following a) to c):
      a) a C₁₋₆ alkyl group,
      b) a -CO-C₁₋₆ alkyl group and
      c) an oxo group),
   (d) an aryl group (which is optionally substituted by one or more, same or different substituents selected from the following (1) to (3): (1) a halogen atom, (2) a C₁₋₆ alkyl group (which is optionally substituted by one or more, same or different halogen atoms) and (3) a C₁₋₆ alkoxy group), or
   (e) a carboxyl group),
5) a C₁₋₆ alkyl group (which is optionally substituted by one or more, same or different substituents selected from the following (a) to (d):
   (a) a carboxyl group,
   (b) a cycloalkyl group (which is optionally substituted by one or more, same or different -CO-NR³²R³³ wherein R³² and R³³ are the same or different and each is a hydrogen atom, a C₁₋₆ alkyl group (which is optionally substituted by one or more, same or different substituents selected from the following groups: (i) a hydroxyl group and (ii) a C₁₋₆ alkoxy group), or R³² and R³³ optionally form, together with the nitrogen atom bonded thereto, a saturated monocyclic nitrogen-containing heterocyclic group),
   (c) -CO-NR³⁴R³⁵ wherein R³⁴ and R³⁵ are the same or different and each is a hydrogen atom or a C₁₋₆ alkyl group, or R³⁴ and R³⁵ optionally form, together with the nitrogen atom bonded thereto, a saturated monocyclic nitrogen-containing heterocyclic group, and
   (d) an aryl group (which is optionally substituted by one or more, same or different substituents selected from the following groups: a) a halogen atom and b) a C₁₋₆ alkyl group (which is optionally substituted by one or more, same or different halogen atoms))),
6) a cycloalkyl group,
7) -S(=O)₂-R¹² wherein R¹² is a C₁₋₆ alkyl group (which is optionally substituted by one or more, same or different halogen atoms), or an aryl group (which is optionally substituted by one or more, same or different substituents selected from the following groups: a) a halogen atom and b) a C₁₋₆ alkyl group (which is optionally substituted by one or more, same or different halogen atoms)),
8) -C(=NCN)-R¹³ wherein R¹³ is a C₁₋₆ alkyl group,
9) -C(=NCN)NR¹⁴R¹⁵ wherein R¹⁴ and R¹⁵ are the same or different and each is a hydrogen atom or a C₁₋₆ alkyl group, or R¹⁴ and R¹⁵ optionally form, together with the nitrogen atom bonded thereto, a saturated monocyclic nitrogen-containing heterocyclic group,
10) an aryl group (which is optionally substituted by one or more, same or different substituents selected from the following (a) to (f):
   (a) a carboxyl group,
   (b) a halogen atom,
   (c) a C₁₋₆ alkyl group (which is optionally substituted by one or more, same or different substituents selected from the following groups: a) a hydroxyl group, b) a halogen atom and c) a C₁₋₆ alkoxy group),
   (d) -NR³⁸R³⁹ wherein R³⁸ and R³⁹ are the same or different and each is a hydrogen atom, a C₁₋₆ alkyl group, a -CO-C₁₋₆ alkyl group, -CO-NR⁴⁰R⁴¹ wherein R⁴⁰ and R⁴¹ are the same or different and each is a hydrogen atom, a C₁₋₆ alkyl group or a C₁₋₆ alkoxy group, or R⁴⁰ and R⁴¹ optionally form, together with the nitrogen atom bonded thereto, a saturated monocyclic nitrogen-containing heterocyclic group, or -S(=O)₂-R⁴² wherein R⁴² is a C₁₋₆ alkyl group, or R³⁸ and R³⁹ optionally form, together with the nitrogen atom bonded thereto, a saturated monocyclic nitrogen-containing heterocyclic group (which is optionally substituted by one or more oxo groups)),
   (e) -CO-NR⁴³R⁴⁴ wherein R⁴³ and R⁴⁴ are the same or different and each is a hydrogen atom or a C₁₋₆ alkyl group, or R⁴³ and R⁴⁴ optionally form, together with the nitrogen atom bonded thereto, a saturated monocyclic nitrogen-containing heterocyclic group, and
   (f) a -COO-C₁₋₆ alkyl group), or
11) an unsaturated monocyclic 5-membered or 6-membered heterocyclic group (which is optionally substituted by one or more, same or different substituents selected from the following (a) to (h):
   (a) a carboxyl group,
   (b) a C₁₋₆ alkyl group (which is optionally substituted by one or more, same or different substituents selected from the following a) to c):
      a) a halogen atom,
      b) a hydroxyl group, and
      c) a C₁₋₆ alkoxy group),
   (c) a cycloalkyl group,
   (d) a halogen atom,
   (e) -CO-NR⁴⁵R⁴⁶ wherein R⁴⁵ and R⁴⁶ are the same or different and each is a hydrogen atom or a C₁₋₆ alkyl group, or R⁴⁵ and R⁴⁶ optionally form, together with the nitrogen atom bonded thereto, a saturated monocyclic nitrogen-containing heterocyclic group,
   (f) a -COO-C₁₋₆ alkyl group,
   (g) a cyano group, and
   (h) a C₁₋₆ alkoxy group);
R² is
(1) a cycloalkyl group (which is optionally substituted by one or more, same or different substituents selected from the following groups: 1) a C₁₋₆ alkyl group (which is optionally substituted by one or more, same or different substituents selected from the following groups: (a) a hydroxyl group and (b) a C₁₋₆ alkoxy group), and 2) a C₁₋₆ alkoxy group);
R³ and R⁴ are the same or different and each is
(1) a hydrogen atom,
(2) a C₁₋₆ alkyl group (which is optionally substituted by one or more, same or different substituents selected from the following groups: 1) a halogen atom, 2) a hydroxyl group, 3) a C₁₋₆ alkoxy group and 4) a -OCO-C₁₋₆ alkyl group),
(3) an aryl group (which is optionally substituted by one or more, same or different substituents selected from the following 1) to 6):
   1) a halogen atom,
   2) a C₁₋₆ alkyl group (which is optionally substituted by one or more, same or different substituents selected from the following groups: (a) a halogen atom, (b) a hydroxyl group and (c) a C₁₋₆ alkoxy group),
   3) a C₁₋₆ alkoxy group (which is optionally substituted by one or more, same or different halogen atoms),
   4) a carboxyl group,
   5) a -COO-C₁₋₆ alkyl group, and
   6) a cyano group),
(4) an unsaturated monocyclic 5-membered or 6-membered heterocyclic group (which is optionally substituted by one or more, same or different substituents selected from the following groups: 1) a halogen atom and 2) a C₁₋₆ alkyl group (which is optionally substituted by one or more, same or different halogen atoms)),
(5) a hydroxyl group, or
(6) a C₁₋₆ alkoxy group]
or a salt thereof, or a solvate thereof.

(33) A production method of a compound represented by the following formula [19]:

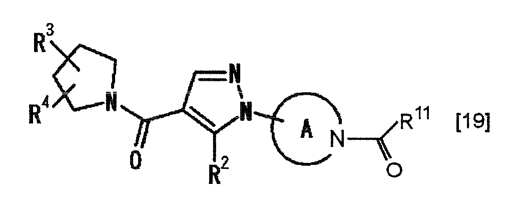

wherein each symbol is as defined above, or a salt thereof, or a solvate thereof, comprising reacting a compound represented by the following formula [16]:

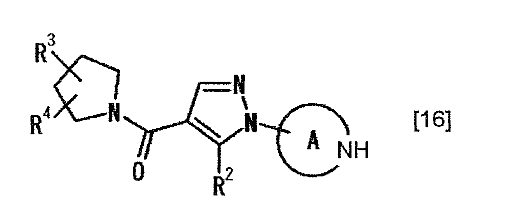

wherein each symbol is as defined above, or a salt thereof, or a solvate thereof, with a compound represented by the following formula [18]:

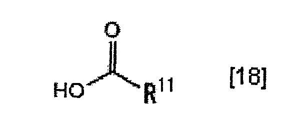

wherein R¹¹ is as defined above, or a salt thereof, or a solvate thereof.

(34) The production method of the above-mentioned (32) or (33), further comprising a step of producing a compound represented by the following formula [16]: 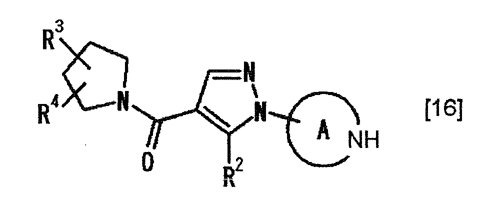
wherein each symbol is as defined above, or a salt thereof, or a solvate thereof, comprising removing an amino-protecting group R" from a compound represented by the following formula [20] :

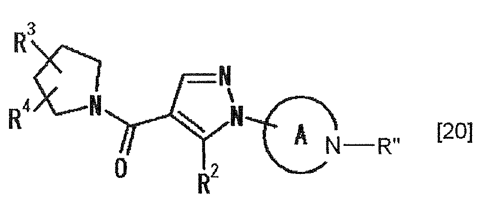

wherein each symbol is as defined above, or a salt thereof, or a solvate thereof.

(35) The production method of the above-mentioned (34), further comprising a step of producing a compound represented by the following formula [20]:

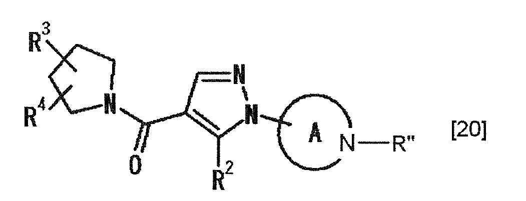

wherein each symbol is as defined above, or a salt thereof, or a solvate thereof, comprising reacting a compound represented by the following formula [21]: 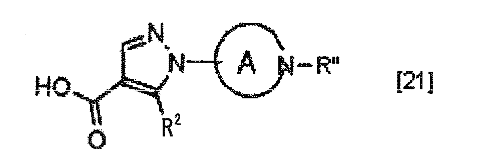 wherein each symbol is as defined above, or a salt thereof, or a solvate thereof, with a compound represented by the following formula [22]: 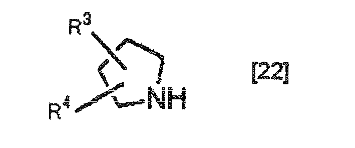 wherein each symbol is as defined above, or a salt thereof, or a solvate thereof.

(36) A production method a compound represented by the following formula [17]:

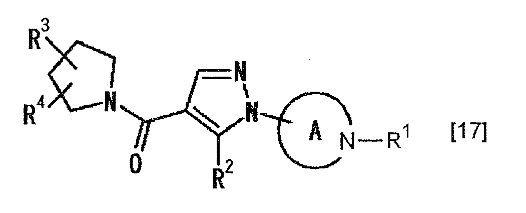

wherein each symbol is as defined above, or a salt thereof, or a solvate thereof, comprising reacting a compound represented by the following formula [23]: 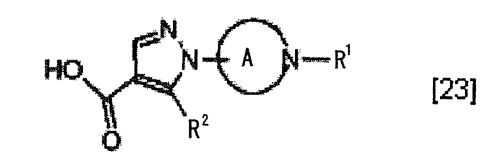 wherein each symbol is as defined above, or a salt thereof, or a solvate thereof, with a compound represented by the following formula [22]: 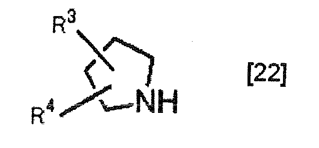 wherein each symbol is as defined above, or a salt thereof, or a solvate thereof.

(37) The production method of the above-mentioned (35) or (36), wherein the compound represented by the formula [22] is (S)-3-(2-trifluoromethylphenyl)pyrrolidine.

(38) The production method of the above-mentioned (37), further comprising the production step shown by the production method of any one of the above-mentioned (23) to (27).

(39) The production method of any one of the above-mentioned (32) to (38), wherein the compound represented by the formula [17] or a salt thereof, or a solvate thereof is selected from the following:
   (1) 1-[5-cyclopropyl-1-(piperidin-4-yl)-1H-pyrazole-4-carbonyl]-3-(pyridin-3-yl)pyrrolidine,
   (2) 1-[5-cyclopropyl-1-(piperidin-4-yl)-1H-pyrazole-4-carbonyl]-[(S)-3-(2-trifluoromethylphenyl)]pyrrolidine hydrochloride,
   (3) 1-{5-cyclopropyl-1-[1-(2-fluorophenylcarbamoyl)piperidin-4-yl]-1H-pyrazole-4-carbonyl}-3-(pyridin-3-yl)pyrrolidine,
   (4) 1-[1-(1-carbamoylpiperidin-4-yl)-5-cyclopropyl-1H-pyrazole-4-carbonyl]-[(S)-3-(2-trifluoromethylphenyl)]pyrrolidine,
   (5) 1-{1-[1-(2-carboxyphenylcarbamoyl)piperidin-4-yl]-5-cyclopropyl-1H-pyrazole-4-carbonyl}-[(S)-3-(2-trifluoromethylphenyl)]pyrrolidine,
   (6) 1-{5-cyclopropyl-1-[1-(2-hydroxymethylphenylcarbamoyl)piperidin-4-yl]-1H-pyrazole-4-carbonyl}-[(S)-3-(2-trifluoromethylphenyl)]pyrrolidine,
   (7) 1-{5-cyclopropyl-1-[1-(1-hydroxymethylcyclopropylcarbamoyl)piperidin-4-yl]-1H-pyrazole-4-carbonyl}-[(S)-3-(2-trifluoromethylphenyl)]pyrrolidine,
   (8) 1-{5-cyclopropyl-1-[1-(2-hydroxyethylcarbamoyl)piperidin-4-yl]-1H-pyrazole-4-carbonyl}-[(S)-3-(2-trifluoromethylphenyl)]pyrrolidine,
   (9) 1-{5-cyclopropyl-1-[1-(2-hydroxy-1,1-dimethylethylcarbamoyl)piperidin-4-yl]-1H-pyrazole-4-carbonyl}-[(S)-3-(2-trifluoromethylphenyl)]pyrrolidine,
   (10) 1-{1-[1-(2-acetylaminoethylcarbamoyl)piperidin-4-yl]-5-cyclopropyl-1H-pyrazole-4-carbonyl}-[(S)-3-(2-trifluoromethylphenyl)]pyrrolidine,
   (11) 1-{1-[1-(2-aminoethylcarbamoyl)piperidin-4-yl]-5-cyclopropyl-1H-pyrazole-4-carbonyl}-[(S)-3-(2-trifluoromethylphenyl)]pyrrolidine hydrochloride,
   (12) 1-{5-cyclopropyl-1-[1-(1,1-dioxothiomorpholine-4-carbonyl)piperidin-4-yl]-1H-pyrazole-4-carbonyl}-[(S)-3-(2-trifluoromethylphenyl)]pyrrolidine,
   (13) 1-{5-cyclopropyl-1-[1-(2-dimethylaminoethylcarbamoyl)piperidin-4-yl]-1H-pyrazole-4-carbonyl}-[(S)-3-(2-trifluoromethylphenyl)]pyrrolidine hydrochloride,
   (14) 1-{5-cyclopropyl-1-[1-(4-hydroxypiperidine-1-carbonyl)piperidin-4-yl]-1H-pyrazole-4-carbonyl}-[(S)-3-(2-trifluoromethylphenyl)]pyrrolidine,
   (15) 1-{1-[1-(azetidine-1-carbonyl)piperidin-4-yl]-5-cyclopropyl-1H-pyrazole-4-carbonyl}-[(S)-3-(2-trifluoromethylphenyl)]pyrrolidine,
   (16) 1-{5-cyclopropyl-1-[1-(3-hydroxypyrrolidine-1-carbonyl)piperidin-4-yl]-1H-pyrazole-4-carbonyl}-[(S)-3-(2-trifluoromethylphenyl)]pyrrolidine,
   (17) 1-{5-cyclopropyl-1-[1-(2-piperidin-1-yl-ethylcarbamoyl)piperidin-4-yl]-1H-pyrazole-4-carbonyl}-[(S)-3-(2-trifluoromethylphenyl)]pyrrolidine hydrochloride,
   (18) 1-{5-cyclopropyl-1-[1-(4,4-difluoropiperidine-1-carbonyl)piperidin-4-yl]-1H-pyrazole-4-carbonyl}-[(S)-3-(2-trifluoromethylphenyl)]pyrrolidine,
   (19) 1-{5-cyclopropyl-1-[1-(3,3-difluoropyrrolidine-1-carbonyl)piperidin-4-yl]-1H-pyrazole-4-carbonyl}-[(S)-3-(2-trifluoromethylphenyl)]pyrrolidine,
   (20) 1-{5-cyclopropyl-1-[1-(3-hydroxyazetidine-1-carbonyl)piperidin-4-yl]-1H-pyrazole-4-carbonyl}-[(S)-3-(2-trifluoromethylphenyl)]pyrrolidine,
   (21) 1-(5-cyclopropyl-1-{1-[(R)-3-hydroxypyrrolidine-1-carbonyl]piperidin-4-yl}-1H-pyrazole-4-carbonyl)-[(S)-3-(2-trifluoromethylphenyl)]pyrrolidine,
   (22) 1-(5-cyclopropyl-1-{1-[(S)-3-hydroxypyrrolidine-1-carbonyl]piperidin-4-yl}-1H-pyrazole-4-carbonyl)-[(S)-3-(2-trifluoromethylphenyl)]pyrrolidine,
   (23) 1-(5-cyclopropyl-1-{1-[(S)-2-hydroxy-1-methylethylcarbamoyl]piperidin-4-yl}-1H-pyrazole-4-carbonyl)-[(S)-3-(2-trifluoromethylphenyl)]pyrrolidine,
   (24) 1-{5-cyclopropyl-1-[1-(4-hydroxymethylpiperidine-1-carbonyl)piperidin-4-yl]-1H-pyrazole-4-carbonyl}-[(S)-3-(2-trifluoromethylphenyl)]pyrrolidine,
   (25) 1-{1-[1-(4-carboxypiperidine-1-carbonyl)piperidin-4-yl]-5-cyclopropyl-1H-pyrazole-4-carbonyl}-[(S)-3-(2-trifluoromethylphenyl)]pyrrolidine,
   (26) 1-{5-cyclopropyl-1-[1-(3-oxopiperazine-1-carbonyl)piperidin-4-yl]-1H-pyrazole-4-carbonyl}-[(S)-3-(2-trifluoromethylphenyl)]pyrrolidine,
   (27) 1-(5-cyclopropyl-1-{1-[(S)-2-hydroxymethylpyrrolidine-1-carbonyl]piperidin-4-yl}-1H-pyrazole-4-carbonyl)-[(S)-3-(2-trifluoromethylphenyl)]pyrrolidine,
   (28) 1-{5-cyclopropyl-1-[1-(3-hydroxymethylazetidine-1-carbonyl)piperidin-4-yl]-1H-pyrazole-4-carbonyl}-[(S)-3-(2-trifluoromethylphenyl)]pyrrolidine,
   (29) 1-{5-cyclopropyl-1-[1-(4-methylpiperazine-1-carbonyl)piperidin-4-yl]-1H-pyrazole-4-carbonyl}-[(S)-3-(2-trifluoromethylphenyl)]pyrrolidine hydrochloride,
   (30) 1-{5-cyclopropyl-1-[1-(4-isopropylpiperazine-1-carbonyl)piperidin-4-yl]-1H-pyrazole-4-carbonyl}-[(S)-3-(2-trifluoromethylphenyl)]pyrrolidine hydrochloride,
   (31) 1-{1-[1-(4-acetylpiperazine-1-carbonyl)piperidin-4-yl]-5-cyclopropyl-1H-pyrazole-4-carbonyl}-[(S)-3-(2-trifluoromethylphenyl)]pyrrolidine,
   (32) 1-(5-cyclopropyl-1-{1-[(R)-3-hydroxypiperidine-1-carbonyl]piperidin-4-yl}-1H-pyrazole-4-carbonyl)-[(S)-3-(2-trifluoromethylphenyl)]pyrrolidine,
   (33) 1-{1-[1-(4-carbamoylpiperidine-1-carbonyl)piperidin-4-yl]-5-cyclopropyl-1H-pyrazole-4-carbonyl}-[(S)-3-(2-trifluoromethylphenyl)]pyrrolidine,
   (34) 1-{1-[1-(3-carbamoylazetidine-1-carbonyl)piperidin-4-yl]-5-cyclopropyl-1H-pyrazole-4-carbonyl}-[(S)-3-(2-trifluoromethylphenyl)]pyrrolidine,
   (35) 1-{1-[1-(4-aminopiperidine-1-carbonyl)piperidin-4-yl]-5-cyclopropyl-1H-pyrazole-4-carbonyl}-[(S)-3-(2-trifluoromethylphenyl)]pyrrolidine hydrochloride,
   (36) 1-{1-[1-(3-aminopyrrolidine-1-carbonyl)piperidin-4-yl]-5-cyclopropyl-1H-pyrazole-4-carbonyl}-[(S)-3-(2-trifluoromethylphenyl)]pyrrolidine hydrochloride,
   (37) 1-{5-cyclopropyl-1-[1-(piperidin-4-yl-carbamoyl)piperidin-4-yl]-1H-pyrazole-4-carbonyl}-[(S)-3-(2-trifluoromethylphenyl)]pyrrolidine hydrochloride,
   (38) 1-{5-cyclopropyl-1-[1-(4-dimethylaminopiperidine-1-carbonyl)piperidin-4-yl]-1H-pyrazole-4-carbonyl}-[(S)-3-(2-trifluoromethylphenyl)]pyrrolidine hydrochloride,
   (39) 1-{1-[1-(4-acetylaminopiperidine-1-carbonyl)piperidin-4-yl]-5-cyclopropyl-1H-pyrazole-4-carbonyl}-[(S)-3-(2-trifluoromethylphenyl)]pyrrolidine,
   (40) 1-{1-[1-(3-acetylaminopyrrolidine-1-carbonyl)piperidin-4-yl]-5-cyclopropyl-1H-pyrazole-4-carbonyl}-[(S)-3-(2-trifluoromethylphenyl)]pyrrolidine,
   (41) 1-{5-cyclopropyl-1-[1-(3-dimethylaminopyrrolidine-1-carbonyl)piperidin-4-yl]-1H-pyrazole-4-carbonyl}-[(S)-3-(2-trifluoromethylphenyl)]pyrrolidine hydrochloride,
   (42) 1-{5-cyclopropyl-1-[1-(1-methylpiperidin-4-yl-carbamoyl)piperidin-4-yl]-1H-pyrazole-4-carbonyl}-[(S)-3-(2-trifluoromethylphenyl)]pyrrolidine hydrochloride,
   (43) 1-{1-[1-(1-acetylpiperidin-4-yl-carbamoyl)piperidin-4-yl]-5-cyclopropyl-1H-pyrazole-4-carbonyl}-[(S)-3-(2-trifluoromethylphenyl)]pyrrolidine,
   (44) 1-{5-cyclopropyl-1-[1-(4-oxopiperidine-1-carbonyl)piperidin-4-yl]-1H-pyrazole-4-carbonyl}-[(S)-3-(2-trifluoromethylphenyl)]pyrrolidine,
   (45) 1-{1-[1-(3-acetylaminoazetidine-1-carbonyl)piperidin-4-yl]-5-cyclopropyl-1H-pyrazole-4-carbonyl}-[(S)-3-(2-trifluoromethylphenyl)]pyrrolidine,
   (46) 1-{5-cyclopropyl-1-[1-(3-oxopyrrolidine-1-carbonyl)piperidin-4-yl]-1H-pyrazole-4-carbonyl}-[(S)-3-(2-trifluoromethylphenyl)]pyrrolidine,
   (47) 1-{5-cyclopropyl-1-[1-(2,2,2-trifluoroethylcarbamoyl)piperidin-4-yl]-1H-pyrazole-4-carbonyl}-[(S)-3-(2-trifluoromethylphenyl)]pyrrolidine,
   (48) 1-{5-cyclopropyl-1-[1-(3,3,4,4-tetrafluoropyrrolidine-1-carbonyl)piperidin-4-yl]-1H-pyrazole-4-carbonyl}-[(S)-3-(2-trifluoromethylphenyl)]pyrrolidine,
   (49) 1-(5-cyclopropyl-1-{1-[(S)-1-hydroxymethyl-2-methylpropylcarbamoyl]piperidin-4-yl}-1H-pyrazole-4-carbonyl)-[(S)-3-(2-trifluoromethylphenyl)]pyrrolidine,
   (50) 1-(1-{1-[(S)-1-benzyl-2-hydroxyethylcarbamoyl]piperidin-4-yl}-5-cyclopropyl-1H-pyrazole-4-carbonyl)-[(S)-3-(2-trifluoromethylphenyl)]pyrrolidine,
   (51) 1-(5-cyclopropyl-1-{1-[(S)-2-hydroxy-1-phenylethylcarbamoyl]piperidin-4-yl}-1H-pyrazole-4-carbonyl)-[(S)-3-(2-trifluoromethylphenyl)]pyrrolidine,
   (52) 1-(5-cyclopropyl-1-{1-[(S)-1-hydroxymethyl-3-methylbutylcarbamoyl]piperidin-4-yl}-1H-pyrazole-4-carbonyl)-[(S)-3-(2-trifluoromethylphenyl)]pyrrolidine,
   (53) 1-{5-cyclopropyl-1-[1-(2-hydroxyphenylcarbamoyl)piperidin-4-yl]-1H-pyrazole-4-carbonyl}-[(S)-3-(2-trifluoromethylphenyl)]pyrrolidine,
   (54) 1-{5-cyclopropyl-1-[1-(1-hydroxymethylcyclopentylcarbamoyl)piperidin-4-yl]-1H-pyrazole-4-carbonyl}-[(S)-3-(2-trifluoromethylphenyl)]pyrrolidine,
   (55) 1-[1-(1-benzenesulfonylaminocarbonylpiperidin-4-yl)-5-cyclopropyl-1H-pyrazole-4-carbonyl]-[(S)-3-(2-trifluoromethylphenyl)]pyrrolidine,
   (56) 1-[5-cyclopropyl-1-(1-methanesulfonylaminocarbonylpiperidin-4-yl)-1H-pyrazole-4-carbonyl]-[(S)-3-(2-trifluoromethylphenyl)]pyrrolidine,
   (57) 1-[5-cyclopropyl-1-(1-methoxycarbonylpiperidin-4-yl)-1H-pyrazole-4-carbonyl]-[(S)-3-(2-trifluoromethylphenyl)]pyrrolidine,
   (58) 1-{5-cyclopropyl-1-[1-(2-hydroxyethoxycarbonyl)piperidin-4-yl]-1H-pyrazole-4-carbonyl}-[(S)-3-(2-trifluoromethylphenyl)]pyrrolidine,
   (59) 1-{5-cyclopropyl-1-[1-(2-dimethylaminoethoxycarbonyl)piperidin-4-yl]-1H-pyrazole-4-carbonyl}-[(S)-3-(2-trifluoromethylphenyl)]pyrrolidine hydrochloride,
   (60) 1-{5-cyclopropyl-1-[1-(2-piperidin-1-yl-ethoxycarbonyl)piperidin-4-yl]-1H-pyrazole-4-carbonyl}-[(S)-3-(2-trifluoromethylphenyl)]pyrrolidine hydrochloride,
   (61) 1-{5-cyclopropyl-1-[1-(piperidin-4-yl-oxycarbonyl)piperidin-4-yl]-1H-pyrazole-4-carbonyl}-[(S)-3-(2-trifluoromethylphenyl)]pyrrolidine hydrochloride,
   (62) 1-{5-cyclopropyl-1-[1-(1-methylpiperidin-4-yl-oxycarbonyl)piperidin-4-yl]-1H-pyrazole-4-carbonyl}-[(S)-3-(2-trifluoromethylphenyl)]pyrrolidine hydrochloride,
   (63) 1-{1-[1-(1-acetylpiperidin-4-yl-oxycarbonyl)piperidin-4-yl]-5-cyclopropyl-1H-pyrazole-4-carbonyl}-[(S)-3-(2-trifluoromethylphenyl)]pyrrolidine,
   (64) 1-[1-(1-cyclopropanecarbonylpiperidin-4-yl)-5-cyclopropyl-1H-pyrazole-4-carbonyl]-[(S)-3-(2-trifluoromethylphenyl)]pyrrolidine,
   (65) 1-{5-cyclopropyl-1-[1-(2-hydroxyacetyl)piperidin-4-yl]-1H-pyrazole-4-carbonyl}-[(S)-3-(2-trifluoromethylphenyl)]pyrrolidine,
   (66) 1-(5-cyclopropyl-1-{1-[1-(4-fluorophenyl)cyclopropanecarbonyl]piperidin-4-yl}-1H-pyrazole-4-carbonyl)-[(S)-3-(2-trifluoromethylphenyl)]pyrrolidine,
   (67) 1-{5-cyclopropyl-1-[1-(2-dimethylaminoacetyl)piperidin-4-yl]-1H-pyrazole-4-carbonyl}-[(S)-3-(2-trifluoromethylphenyl)]pyrrolidine hydrochloride,
   (68) 1-{1-[1-(2-acetylaminoacetyl)piperidin-4-yl]-5-cyclopropyl-1H-pyrazole-4-carbonyl}-[(S)-3-(2-trifluoromethylphenyl)]pyrrolidine,
   (69) 1-{5-cyclopropyl-1-[1-(1-methylcyclopropanecarbonyl)piperidin-4-yl]-1H-pyrazole-4-carbonyl}-[(S)-3-(2-trifluoromethylphenyl)]pyrrolidine,
   (70) 1-{1-[1-(2-acetylamino-2-methylpropionyl)piperidin-4-yl]-5-cyclopropyl-1H-pyrazole-4-carbonyl}-[(S)-3-(2-trifluoromethylphenyl)]pyrrolidine,
   (71) 1-(1-{1-[(S)-2-acetylaminopropionyl]piperidin-4-yl}-5-cyclopropyl-1H-pyrazole-4-carbonyl)-[(S)-3-(2-trifluoromethylphenyl)]pyrrolidine,
   (72) 1-(1-{1-[(S)-2-acetylamino-3-methylbutyryl]piperidin-4-yl}-5-cyclopropyl-1H-pyrazole-4-carbonyl)-[(S)-3-(2-trifluoromethylphenyl)]pyrrolidine,
   (73) 1-{5-cyclopropyl-1-[1-(3,3,3-trifluoropropionyl)piperidin-4-yl]-1H-pyrazole-4-carbonyl}-[(S)-3-(2-trifluoromethylphenyl)]pyrrolidine,
   (74) 1-(5-cyclopropyl-1-{1-[(S)-5-oxopyrrolidine-2-carbonyl]piperidin-4-yl}-1H-pyrazole-4-carbonyl)-[(S)-3-(2-trifluoromethylphenyl)]pyrrolidine,
   (75) 1-{1-[1-(3-acetylaminopropionyl)piperidin-4-yl]-5-cyclopropyl-1H-pyrazole-4-carbonyl}-[(S)-3-(2-trifluoromethylphenyl)]pyrrolidine,
   (76) 1-{5-cyclopropyl-1-[1-(3-hydroxy-2,2-dimethylpropionyl)piperidin-4-yl]-1H-pyrazole-4-carbonyl}-[(S)-3-(2-trifluoromethylphenyl)]pyrrolidine,
   (77) 1-{1-[1-(2-aminoacetyl)piperidin-4-yl]-5-cyclopropyl-1H-pyrazole-4-carbonyl}-[(S)-3-(2-trifluoromethylphenyl)]pyrrolidine hydrochloride,
   (78) 1-{5-cyclopropyl-1-[1-(1-hydroxymethylcyclopropanecarbonyl)piperidin-4-yl]-1H-pyrazole-4-carbonyl}-[(S)-3-(2-trifluoromethylphenyl)]pyrrolidine,
   (79) 1-{1-[1-(2-amino-2-methylpropionyl)piperidin-4-yl]-5-cyclopropyl-1H-pyrazole-4-carbonyl}-[(S)-3-(2-trifluoromethylphenyl)]pyrrolidine hydrochloride,
   (80) 1-{5-cyclopropyl-1-[1-(4-hydroxybutyryl)piperidin-4-yl]-1H-pyrazole-4-carbonyl}-[(S)-3-(2-trifluoromethylphenyl)]pyrrolidine,
   (81) 1-(5-cyclopropyl-1-{1-[(S)-pyrrolidine-2-carbonyl]piperidin-4-yl}-1H-pyrazole-4-carbonyl)-[(S)-3-(2-trifluoromethylphenyl)]pyrrolidine hydrochloride,
   (82) 1-(5-cyclopropyl-1-{1-[(S)-1-methylpyrrolidine-2-carbonyl]piperidin-4-yl}-1H-pyrazole-4-carbonyl)-[(S)-3-(2-trifluoromethylphenyl)]pyrrolidine hydrochloride,
   (83) 1-{1-[1-(3-aminopropionyl)piperidin-4-yl]-5-cyclopropyl-1H-pyrazole-4-carbonyl}-[(S)-3-(2-trifluoromethylphenyl)]pyrrolidine hydrochloride,
   (84) 1-(1-{1-[(S)-2-amino-3-methylbutyryl]piperidin-4-yl}-5-cyclopropyl-1H-pyrazole-4-carbonyl)-[(S)-3-(2-trifluoromethylphenyl)]pyrrolidine hydrochloride,
   (85) 1-{5-cyclopropyl-1-[1-(2-methylaminoacetyl)piperidin-4-yl]-1H-pyrazole-4-carbonyl}-[(S)-3-(2-trifluoromethylphenyl)]pyrrolidine hydrochloride,
   (86) 1-{5-cyclopropyl-1-[1-(piperidine-4-carbonyl)piperidin-4-yl]-1H-pyrazole-4-carbonyl}-[(S)-3-(2-trifluoromethylphenyl)]pyrrolidine hydrochloride,
   (87) 1-{5-cyclopropyl-1-[1-(2-isobutyrylaminoacetyl)piperidin-4-yl]-1H-pyrazole-4-carbonyl}-[(S)-3-(2-trifluoromethylphenyl)]pyrrolidine,
   (88) 1-{1-[1-(2-cyclopropanecarbonylaminoacetyl)piperidin-4-yl]-5-cyclopropyl-1H-pyrazole-4-carbonyl}-[(S)-3-(2-trifluoromethylphenyl)]pyrrolidine,
   (89) 1-(1-{1-[(S)-1-acetylpyrrolidine-2-carbonyl]piperidin-4-yl}-5-cyclopropyl-1H-pyrazole-4-carbonyl)-[(S)-3-(2-trifluoromethylphenyl)]pyrrolidine,
   (90) 1-{5-cyclopropyl-1-[1-(2-methanesulfonylaminoacetyl)piperidin-4-yl]-1H-pyrazole-4-carbonyl}-[(S)-3-(2-trifluoromethylphenyl)]pyrrolidine,
   (91) 1-{1-[1-(1-acetylpiperidine-4-carbonyl)piperidin-4-yl]-5-cyclopropyl-1H-pyrazole-4-carbonyl}-[(S)-3-(2-trifluoromethylphenyl)]pyrrolidine,
   (92) 1-{5-cyclopropyl-1-[1-(1-methylpiperidine-4-carbonyl)piperidin-4-yl]-1H-pyrazole-4-carbonyl}-[(S)-3-(2-trifluoromethylphenyl)]pyrrolidine hydrochloride,
   (93) 1-{1-[1-(3-carbamoylpropionyl)piperidin-4-yl]-5-cyclopropyl-1H-pyrazole-4-carbonyl}-[(S)-3-(2-trifluoromethylphenyl)]pyrrolidine,
   (94) 1-[1-(1-carbamoylmethylpiperidin-4-yl)-5-cyclopropyl-1H-pyrazole-4-carbonyl]-[(S)-3-(2-trifluoromethylphenyl)]pyrrolidine hydrochloride,
   (95) 1-[5-cyclopropyl-1-(1-methylcarbamoylmethylpiperidin-4-yl)-1H-pyrazole-4-carbonyl]-[(S)-3-(2-trifluoromethylphenyl)]pyrrolidine hydrochloride,
   (96) 1-{1-[1-(1-carbamoyl-1-methylethyl)piperidin-4-yl]-5-cyclopropyl-1H-pyrazole-4-carbonyl}-[(S)-3-(2-trifluoromethylphenyl)]pyrrolidine hydrochloride,
   (97) 1-{1-[1-(2-carbamoylethyl)piperidin-4-yl]-5-cyclopropyl-1H-pyrazole-4-carbonyl}-[(S)-3-(2-trifluoromethylphenyl)]pyrrolidine hydrochloride,
   (98) 1-[5-cyclopropyl-1-(1-cyclopropylmethylpiperidin-4-yl)-1H-pyrazole-4-carbonyl]-[(S)-3-(2-trifluoromethylphenyl)]pyrrolidine hydrochloride,
   (99) 1-[5-cyclopropyl-1-(1-cyclopropylpiperidin-4-yl)-1H-pyrazole-4-carbonyl]-[(S)-3-(2-trifluoromethylphenyl)]pyrrolidine hydrochloride,
   (100) 1-[5-cyclopropyl-1-(1-dimethylcarbamoylmethylpiperidin-4-yl)-1H-pyrazole-4-carbonyl]-[(S)-3-(2-trifluoromethylphenyl)]pyrrolidine hydrochloride,
   (101) 1-[1-(1-carboxymethylpiperidin-4-yl)-5-cyclopropyl-1H-pyrazole-4-carbonyl]-[(S)-3-(2-trifluoromethylphenyl)]pyrrolidine hydrochloride,
   (102) 1-[1-(1-carboxyethylpiperidin-4-yl)-5-cyclopropyl-1H-pyrazole-4-carbonyl]-[(S)-3-(2-trifluoromethylphenyl)]pyrrolidine hydrochloride,
   (103) 1-{1-[1-(1-carbamoylethyl)piperidin-4-yl]-5-cyclopropyl-1H-pyrazole-4-carbonyl}-[(S)-3-(2-trifluoromethylphenyl)]pyrrolidine hydrochloride,
   (104) 1-{1-[1-(2-carboxy-2-methylpropyl)piperidin-4-yl]-5-cyclopropyl-1H-pyrazole-4-carbonyl}-[(S)-3-(2-trifluoromethylphenyl)]pyrrolidine hydrochloride,
   (105) 1-{1-[1-(2-carbamoyl-2-methylpropyl)piperidin-4-yl]-5-cyclopropyl-1H-pyrazole-4-carbonyl}-[(S)-3-(2-trifluoromethylphenyl)]pyrrolidine hydrochloride,
   (106) 1-{1-[1-(1-carbamoylcyclopropylmethyl)piperidin-4-yl]-5-cyclopropyl-1H-pyrazole-4-carbonyl}-[(S)-3-(2-trifluoromethylphenyl)]pyrrolidine hydrochloride,
   (107) 1-(5-cyclopropyl-1-{1-[1-(2-hydroxyethylcarbamoyl)cyclopropylmethyl]piperidin-4-yl}-1H-pyrazole-4-carbonyl)-[(S)-3-(2-trifluoromethylphenyl)]pyrrolidine hydrochloride,
   (108) 1-[5-cyclopropyl-1-(1-trifluoromethanesulfonylpiperidin-4-yl)-1H-pyrazole-4-carbonyl]-[(S)-3-(2-trifluoromethylphenyl)]pyrrolidine,
   (109) 1-{5-cyclopropyl-1-[1-(2,2,2-trifluoroethanesulfonyl)piperidin-4-yl]-1H-pyrazole-4-carbonyl}-[(S)-3-(2-trifluoromethylphenyl)]pyrrolidine,
   (110) 1-{1-[1-(1-cyanoiminoethyl)piperidin-4-yl]-5-cyclopropyl-1H-pyrazole-4-carbonyl}-[(S)-3-(2-trifluoromethylphenyl)]pyrrolidine,
   (111) 1-(1-{1-[cyanoimino(methylamino)methyl]piperidin-4-yl}-5-cyclopropyl-1H-pyrazole-4-carbonyl)-[(S)-3-(2-trifluoromethylphenyl)]pyrrolidine,
   (112) 1-{1-[1-(N-cyanocarbamimidoyl)piperidin-4-yl]-5-cyclopropyl-1H-pyrazole-4-carbonyl}-[(S)-3-(2-trifluoromethylphenyl)]pyrrolidine,
   (113) 4-(4-{5-cyclopropyl-4-[3-(2-trifluoromethylphenyl)pyrrolidine-1-carbonyl]pyrazol-1-yllpiperidin-1-yl)benzoic acid,
   (114) 3-(4-{5-cyclopropyl-4-[3-(2-trifluoromethylphenyl)pyrrolidine-1-carbonyl]pyrazol-1-yl}piperidin-1-yl)benzoic acid,
   (115) 5-(4-{5-cyclopropyl-4-[3-(2-trifluoromethylphenyl)pyrrolidine-1-carbonyl]pyrazol-1-yl}piperidin-1-yl)thiophene-2-carboxylic acid,
   (116) 2-(4-{5-cyclopropyl-4-[3-(2-trifluoromethylphenyl)pyrrolidine-1-carbonyl]pyrazol-1-yl}piperidin-1-yl)thiazole-4-carboxylic acid,
   (117) 1-{5-cyclopropyl-1-[1-(5-methyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (118) 1-{5-cyclopropyl-1-[1-(5-cyclopropyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (119) 1-{5-cyclopropyl-1-[1-(5-hydroxymethyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (120) 1-{5-cyclopropyl-1-[1-(5-trifluoromethyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (121) 1-{5-cyclopropyl-1-[1-(2-fluorophenylcarbamoyl)piperidin-4-yl]-1H-pyrazole-4-carbonyl}-[(R)-3-(2-trifluoromethylphenyl)]pyrrolidine,
   (122) 1-{5-cyclopropyl-1-[1-(1-methylcyclopropylcarbamoyl)piperidin-4-yl]-1H-pyrazole-4-carbonyl}-[(R)-3-(2-trifluoromethylphenyl)]pyrrolidine,
   (123) 1-{5-cyclopropyl-1-[1-(1-isopropylcarbamoyl)piperidin-4-yl]-1H-pyrazole-4-carbonyl}-[(R)-3-(2-trifluoromethylphenyl)]pyrrolidine,
   (124) 1-{5-cyclopropyl-1-[1-(2-fluorophenylcarbamoyl)piperidin-4-yl]-1H-pyrazole-4-carbonyl}-[(S)-3-(2-trifluoromethylphenyl)]pyrrolidine,
   (125) 1-{5-cyclopropyl-1-[1-(1-isopropylcarbamoyl)piperidin-4-yl]-1H-pyrazole-4-carbonyl}-[(S)-3-(2-trifluoromethylphenyl)]pyrrolidine,
   (126) 1-{5-cyclopropyl-1-[1-(1-methylcyclopropylcarbamoyl)piperidin-4-yl]-1H-pyrazole-4-carbonyl}-[(S)-3-(2-trifluoromethylphenyl)]pyrrolidine,
   (127) (-)-3-(4-fluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride,
   (128) (+)-3-(4-fluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride,
   (129) 1-[5-cyclopropyl-1-(1-pyrazin-2-ylpiperidin-4-yl)-1H-pyrazole-4-carbonyl]-[(S)-3-(2-trifluoromethylphenyl)]pyrrolidine hydrochloride,
   (130) 1-[1-(trans-4-carbamoylcyclohexyl)-5-cyclopropyl-1H-pyrazole-4-carbonyl]-[(S)-3-(2-trifluoromethylphenyl)]pyrrolidine,
   (131) 1-[5-cyclopropyl-1-(trans-4-ureidocyclohexyl)-1H-pyrazole-4-carbonyl]-[(S)-3-(2-trifluoromethylphenyl)]pyrrolidine,
   (132) 1-{5-cyclopropyl-1-[trans-4-(1H-tetrazol-5-yl)cyclohexyl]-1H-pyrazole-4-carbonyl}-[(S)-3-(2-trifluoromethylphenyl)]pyrrolidine,
   (133) 1-{5-(1-methylcyclopropyl)-1-[1-(4-trifluoromethylphenylcarbamoyl)piperidin-4-yl]-1H-pyrazole-4-carbonyl}-3-(pyridin-3-yl)pyrrolidine,
   (134) 1-{1-[1-(2-fluorophenylcarbamoyl)piperidin-4-yl]-5-(1-methoxycyclopropyl)-1H-pyrazole-4-carbonyl}-3-(2-trifluoromethylphenyl)pyrrolidine,
   (135) 1-{1-[1-(2-fluorophenylcarbamoyl)piperidin-4-yl]-5-(1-methylcyclopropyl)-1H-pyrazole-4-carbonyl}-3-phenyl-3-trifluoromethylpyrrolidine,
   (136) 3-(2-chlorophenyl)-1-{5-cyclopropyl-1-[1-(2,4-difluorophenylcarbamoyl)piperidin-4-yl]-1H-pyrazole-4-carbonyl}pyrrolidine,
   (137) 3-(2-chloropyridin-3-yl)-1-{5-cyclopropyl-1-[1-(2-fluorophenylcarbamoyl)piperidin-4-yl]-1H-pyrazole-4-carbonyl}pyrrolidine,
   (138) 1-{1-[1-(2-fluorophenylcarbamoyl)piperidin-4-yl]-5-(2,2,3,3-tetramethylcyclopropyl)-1H-pyrazole-4-carbonyl}-3-(pyridin-3-yl)pyrrolidine,
   (139) 1-{5-cyclohexyl-1-[1-(2-fluorophenylcarbamoyl)piperidin-4-yl]-1H-pyrazole-4-carbonyl}-3-(pyridin-3-yl)pyrrolidine,
   (140) 1-{5-cyclopropyl-1-[1-(2-fluorophenylcarbamoyl)piperidin-4-yl]-1H-pyrazole-4-carbonyl}-3-(2-trifluoromethoxyphenyl)pyrrolidine,
   (141) 1-{5-cyclopropyl-1-[1-(4-trifluoromethylphenylcarbamoyl)piperidin-4-yl]-1H-pyrazole-4-carbonyl}-3-(2-trifluoromethylphenyl)pyrrolidine,
   (142) 1-(5-cyclopropyl-1-{1-[((S)-1-carboxy-2-methylpropyl)methylcarbamoyl]piperidin-4-yl}-1H-pyrazole-4-carbonyl)-3-(2-trifluoromethylphenyl)pyrrolidine,
   (143) 1-{5-cyclopropyl-1-[1-(2-fluorophenylcarbamoyl)piperidin-4-yl]-1H-pyrazole-4-carbonyl}-3-methoxy-3-(2-trifluoromethylphenyl)pyrrolidine,
   (144) 1-{1-[1-(2-fluorophenylcarbamoyl)piperidin-4-yl]-5-(1-hydroxymethylcyclopropyl)-1H-pyrazole-4-carbonyl}-3-(2-trifluoromethylphenyl)pyrrolidine,
   (145) 1-{5-cyclopropyl-1-[1-(thiazol-2-ylcarbamoyl)piperidin-4-yl]-1H-pyrazole-4-carbonyl}-3-(2-trifluoromethylphenyl)pyrrolidine,
   (146) 1-{1-[1-(isopropoxycarbamoyl)piperidin-4-yl]-5-(1-methylcyclopropyl)-1H-pyrazole-4-carbonyl}-3-(2-trifluoromethylphenyl)pyrrolidine,
   (147) 1-{5-cyclopropyl-1-[1-(4-fluorobenzylcarbamoyl)piperidin-4-yl]-1H-pyrazole-4-carbonyl}-3-(2-trifluoromethylphenyl)pyrrolidine,
   (148) 1-{1-[1-(2,3-dihydroindole-1-carbonyl)piperidin-4-yl]-5-(1-methylcyclopropyl)-1H-pyrazole-4-carbonyl}-3-(pyridin-3-yl)pyrrolidine,
   (149) 1-{1-[1-(2,3-dihydro[1,4]oxazine-4-carbonyl)piperidin-4-yl]-5-(1-methylcyclopropyl)-1H-pyrazole-4-carbonyl}-3-(pyridin-3-yl)pyrrolidine,
   (150) 1-{5-cyclopropyl-1-[1-(2,6-dichloropyridin-3-ylcarbamoyl)piperidin-4-yl]-1H-pyrazole-4-carbonyl}-3-(2-trifluoromethylphenyl)pyrrolidine,
   (151) 1-{5-(1-methylcyclopropyl)-1-[1-(2,3,4,5-tetrahydrobenzo[b]azepine-1-carbonyl)piperidin-4-yl]-1H-pyrazole-4-carbonyl}-3-(pyridin-3-yl)pyrrolidine,
   (152) ethyl 4-(4-{5-cyclopropyl-4-[3-(2-trifluoromethylphenyl)pyrrolidine-1-carbonyl]pyrazol-1-yl}piperidin-1-yl)benzoate,
   (153) 1-[1-(1-benzyloxycarbonylpiperidin-4-yl)-5-cyclopropyl-1H-pyrazole-4-carbonyl]-3-(pyridin-3-yl)pyrrolidine,
   (154) 1-{1-[1-(3-carboxy-3-methyl-butyryl)piperidin-4-yl]-5-cyclopropyl-1H-pyrazole-4-carbonyl}-3-(2-trifluoromethylphenyl)pyrrolidine,
   (155) 1-{5-cyclopropyl-1-[1-(2-phenoxyacetyl)piperidin-4-yl]-1H-pyrazole-4-carbonyl}-3-(pyridin-3-yl)pyrrolidine,
   (156) 1-{1-[1-(3-chlorobenzoyl)piperidin-4-yl]-5-cyclopropyl-1H-pyrazole-4-carbonyl}-3-(pyridin-3-yl)pyrrolidine,
   (157) 1-{1-[1-(2-chlorobenzoyl)piperidin-4-yl]-5-cyclopropyl-1H-pyrazole-4-carbonyl}-3-(pyridin-3-yl)pyrrolidine,
   (158) 1-[5-cyclopropyl-1-(1-oxalylpiperidin-4-yl)-1H-pyrazole-4-carbonyl]-3-(2-trifluoromethylphenyl)pyrrolidine,
   (159) 1-{1-[1-(4-chlorobenzoyl)piperidin-4-yl]-5-cyclopropyl 1H-pyrazole-4-carbonyl}-3-(pyridin-3-yl)pyrrolidine,
   (160) 1-(5-cyclopropyl-1-{1-[2-(4-fluorophenyl)acetyl]piperidin-4-yl}-1H-pyrazole-4-carbonyl)-3-(pyridin-3-yl)pyrrolidine,
   (161) 1-{5-cyclopropyl-1-[1-(3-trifluoromethylbenzoyl)piperidin-4-yl]-1H-pyrazole-4-carbonyl}-3-(pyridin-3-yl)pyrrolidine,
   (162) 1-{5-cyclopropyl-1-[1-(3-methoxybenzoyl)piperidin-4-yl]-1H-pyrazole-4-carbonyl}-3-(pyridin-3-yl)pyrrolidine,
   (163) 1-{1-[1-(4-fluorobenzyl)piperidin-4-yl]-5-(1-methylcyclopropyl)-1H-pyrazole-4-carbonyl}-3-(2-trifluoromethylphenyl)pyrrolidine hydrochloride,
   (164) 1-[1-(1-benzenesulfonylpiperidin-4-yl)-5-cyclopropyl-1H-pyrazole-4-carbonyl]-3-(pyridin-3-yl)pyrrolidine,
   (165) 1-{1-[1-(4-carbamoylphenyl)piperidin-4-yl]-5-cyclopropyl-1H-pyrazole-4-carbonyl}-3-(2-trifluoromethylphenyl)pyrrolidine,
   (166) 1-{5-cyclopropyl-1-[1-(4-hydroxymethylphenyl)piperidin-4-yl]-1H-pyrazole-4-carbonyl}-3-(2-trifluoromethylphenyl)pyrrolidine,
   (167) 1-{1-[1-(5-carbamoyl-pyridin-2-yl)piperidin-4-yl]-5-cyclopropyl-1H-pyrazole-4-carbonyl}-3-(2-trifluoromethylphenyl)pyrrolidine,
   (168) 1-{1-[1-(4-aminophenyl)piperidin-4-yl]-5-cyclopropyl-1H-pyrazole-4-carbonyl}-3-(2-trifluoromethylphenyl)pyrrolidine,
   (169) 1-(5-cyclopropyl-1-{1-[4-(2-oxooxazolidin-3-yl)phenyl]piperidin-4-yl}-1H-pyrazole-4-carbonyl)-3-(2-trifluoromethylphenyl)pyrrolidine,
   (170) 1-(5-cyclopropyl-1-{1-[4-(3-methoxyureido)phenyl]piperidin-4-yl}-1H-pyrazole-4-carbonyl)-3-(2-trifluoromethylphenyl)pyrrolidine,
   (171) 1-{5-cyclopropyl-1-[1-(4-methanesulfonylaminophenyl)piperidin-4-yl]-1H-pyrazole-4-carbonyl}-3-(2-trifluoromethylphenyl)pyrrolidine,
   (172) 1-[1-(1-ethoxycarbamoylpiperidin-4-yl)-5-(1-methylcyclopropyl)-1H-pyrazole-4-carbonyl]-3-(2-trifluoromethylphenyl)pyrrolidine,
   (173) 1-{1-[1-(5-chloropyridin-2-yl)azetidin-3-yl]-5-cyclopropyl-1H-pyrazole-4-carbonyl}-3-(2-trifluoromethylphenyl)pyrrolidine,
   (174) 1-{1-[1-(5-chloropyridin-2-yl)piperidin-4-yl]-5-(1-methylcyclopropyl)-1H-pyrazole-4-carbonyl}-3-methoxymethyl-3-phenylpyrrolidine,
   (175) 1-{1-[1-(5-cyanopyridin-2-yl)piperidin-4-yl]-5-cyclopropyl-1H-pyrazole-4-carbonyl}-3-(2-trifluoromethylphenyl)pyrrolidine,
   (176) 3-(2-acetoxyethyl)-3-(4-fluorophenyl)-1-{5-(1-methylcyclopropyl)-1-[1-(pyrimidin-2-yl)piperidin-4-yl]-1H-pyrazole-4-carbonyl}pyrrolidine hydrochloride,
   (177) (3S*,4R*)-3-methyl-1-{5-(1-methylcyclopropyl)-1-[1-(pyrimidin-2-yl)piperidin-4-yl]-1H-pyrazole-4-carbonyl}-4-phenylpyrrolidine hydrochloride,
   (178) methyl 6-(4-{5-cyclopropyl-4-[3-(2-trifluoromethylphenyl)pyrrolidine-1-carbonyl]pyrazol-1-yl}piperidin-1-yl)nicotinate,
   (179) methyl 2-{1-[5-(1-methylcyclopropyl)-1-(1-pyrimidin-2-ylpiperidin-4-yl)-1H-pyrazole-4-carbonyl]pyrrolidin-3-yl}benzoate,
   (180) 3-(2-hydroxymethylphenyl)-1-{5-(1-methylcyclopropyl)-1-[1-(pyrimidin-2-yl)piperidin-4-yl]-1H-pyrazole-4-carbonyl}pyrrolidine hydrochloride,
   (181) 1-[5-cyclopropyl-1-(1-pyridin-2-ylpiperidin-4-yl)-1H-pyrazole-4-carbonyl]-3-hydroxy-3-(pyridin-3-yl)pyrrolidine,
   (182) 1-{1-[1-(4-acetylaminophenyl)piperidin-4-yl]-5-cyclopropyl-1H-pyrazole-4-carbonyl}-3-(2-trifluoromethylphenyl)pyrrolidine,
   (183) sodium 4-(4-{5-cyclopropyl-4-[3-(2-trifluoromethylphenyl)pyrrolidine-1-carbonyl]pyrazol-1-yl}piperidin-1-yl)-3-fluorobenzoate,
   (184) 3-(2-cyanophenyl)-1-{5-cyclopropyl-1-[1-(2-fluorophenylcarbamoyl)piperidin-4-yl]-1H-pyrazole-4-carbonyl}pyrrolidine,
   (185) 3-hydroxymethyl-1-{1-[1-(4-methoxypyrimidin-2-yl)piperidin-4-yl]-5-(1-methylcyclopropyl)-1H-pyrazole-4-carbonyl}-3-phenylpyrrolidine hydrochloride,
   (186) 3-(3,5-difluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride,
   (187) 1-{1-[1-(3-chloropyridin-2-yl)piperidin-4-yl]-5-cyclopropyl-1H-pyrazole-4-carbonyl}-3-(pyridin-3-yl)pyrrolidine,
   (188) 1-{5-cyclopropyl-1-[1-(2-fluorophenylcarbamoyl)piperidin-4-yl]-1H-pyrazole-4-carbonyl}-3-(thiazol-2-yl)pyrrolidine,
   (189) (S)-2-[(trans-4-{5-cyclopropyl-4-[3-(2-trifluoromethylphenyl)pyrrolidine-1-carbonyl]pyrazol-1-yl}cyclohexanecarbonyl)methylamino]-3-methylbutyric acid,
   (190) 1-{5-(1-methylcyclopropyl)-1-[trans-4-(2-fluorophenylcarbamoyl)cyclohexyl]-1H-pyrazole-4-carbonyl}-3-(2-trifluoromethylphenyl)pyrrolidine, and
   (191) cis-4-{5-(1-methylcyclopropyl)-4-[3-(2-trifluoromethylphenyl)pyrrolidine-1-carbonyl]pyrazol-1-yl}cyclohexanecarboxylic acid.

In addition, the compound of the present invention is useful as a synthetic intermediate for producing the following compound.
1. A compound represented by the following formula [1'] or a salt thereof:

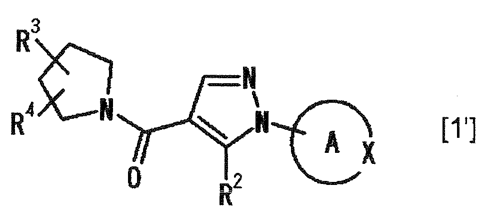

wherein
ring A is
(1) a saturated monocyclic nitrogen-containing heterocyclic group, or
(2) a cycloalkyl group,
which is optionally substituted by one or more, same or different substituents R¹,
the substituent R¹ is
1) a hydrogen atom,
2) -CONR⁵R⁶ wherein R⁵ and R⁶ are the same or different and each is
   (a) a hydrogen atom,
   (b) an aryl group (which is optionally substituted by one or more, same or different substituents selected from the following groups:
      a) a hydroxyl group,
      b) a halogen atom,
      c) a carboxyl group,
      d) a C₁₋₆ alkyl group (which is optionally substituted by one or more, same or different substituents selected from the following groups: (i) a halogen atom, (ii) a hydroxyl group and (iii) a C₁₋₆ alkoxy group), and
      e) a C₁₋₆ alkoxy group),
   (c) a cycloalkyl group (which is optionally substituted by one or more, same or different C₁₋₆ alkyl groups (which are optionally substituted by one or more, same or different substituents selected from the following groups: (i) a hydroxyl group and (ii) a C₁₋₆ alkoxy group)),
   (d) a C₁₋₆ alkyl group (which is optionally substituted by one or more, same or different substituents selected from the following groups:
      a) a hydroxyl group,
      b) a halogen atom,
      c) an aryl group (which is optionally substituted by one or more, same or different substituents selected from the following (1) and (2): (1) a halogen atom and (2) a C₁₋₆ alkyl group (which is optionally substituted by one or more, same or different halogen atoms)),
      d) -NR²⁰R²¹ wherein R²⁰ and R²¹ are the same or different and each is a hydrogen atom, a C₁₋₆ alkyl group or a -CO-C₁₋₆ alkyl group, or R²⁰ and R²¹ optionally form, together with the nitrogen atom bonded thereto, a saturated monocyclic nitrogen-containing heterocyclic group (which is optionally substituted by one or more, same or different substituents selected from the following (i) to (iv):
         (i) a halogen atom,
         (ii) a hydroxyl group,
         (iii) an oxo group, and
         (iv) a C₁₋₆ alkoxy group)),
      e) a C₁₋₆ alkoxy group, and
      f) a carboxyl group),
   (e) -S(=O)₂-R⁹ wherein R⁹ is an aryl group (which is optionally substituted by one or more, same or different substituents selected from the following (1) and (2): (1) a halogen atom and (2) a C₁₋₆ alkyl group (which is optionally substituted by one or more, same or different halogen atoms)) or a C₁₋₆ alkyl group,
   (f) a saturated monocyclic nitrogen-containing heterocyclic group (which is optionally substituted by one or more, same or different substituents selected from the following a) to c):
      a) a C₁₋₆ alkyl group,
      b) a -CO-C₁₋₆ alkyl group, and
      c) an oxo group),
   (g) a C₁₋₆ alkoxy group, or
   (h) a monocyclic nitrogen-containing unsaturated heterocyclic group (which is optionally substituted by one or more, same or different halogen atoms),
      or
   (i) R⁵ and R⁶ optionally form, together with the nitrogen atom bonded thereto, a saturated monocyclic nitrogen-containing heterocyclic group, or a heterocyclic group which is a fused ring of said heterocycle and a carbocycle (which heterocyclic group is optionally substituted by one or more, same or different substituents selected from the following a) to i):
      a) a halogen atom,
      b) a hydroxyl group,
      c) a C₁₋₆ alkyl group (which is optionally substituted by one or more, same or different substituents selected from the following groups: (i) a halogen atom, (ii) a hydroxyl group and (iii) a C₁₋₆ alkoxy group),
      d) a carboxyl group,
      e) a -CO-C₁₋₆ alkyl group,
      f) -CO-NR²²R²³ wherein R²² and R²³ are the same or different and each is a hydrogen atom or a C₁₋₆ alkyl group, or R²² and R²³ optionally form, together with the nitrogen atom bonded thereto, a saturated monocyclic nitrogen-containing heterocyclic group,
      g) an oxo group,
      h) -NR²⁴R²⁵ wherein R²⁴ and R²⁵ are the same or different and each is a hydrogen atom, a C₁₋₆ alkyl group or a -CO-C₁₋₆ alkyl group, or R²⁴ and R²⁵ optionally form, together with the nitrogen atom bonded thereto, a saturated monocyclic nitrogen-containing heterocyclic group, and
      i) a C₁₋₆ alkoxy group)),
3) -COOR¹⁰ wherein R¹⁰ is
   (a) a C₁₋₆ alkyl group (which is optionally substituted by one or more, same or different substituents selected from the following a) to d):
      a) a hydroxyl group,
      b) -NR²⁶R²⁷ wherein R²⁶ and R²⁷ are the same or different and each is a hydrogen atom or a C₁₋₆ alkyl group, or R²⁶ and R²⁷ optionally form, together with the nitrogen atom bonded thereto, a saturated monocyclic nitrogen-containing heterocyclic group,
      c) a C₁₋₆ alkoxy group, and
      d) an aryl group), or
   (b) a saturated monocyclic nitrogen-containing heterocyclic group (which is optionally substituted by one or more, same or different substituents selected from the following a) to c):
      a) a C₁₋₆ alkyl group,
      b) a -CO-C₁₋₆ alkyl group, and
      c) an oxo group)),
4) -COR¹¹ wherein R¹¹ is
   (a) a C₁₋₆ alkyl group (which is optionally substituted by one or more, same or different substituents selected from the following a) to h):
      a) a halogen atom,
      b) a hydroxyl group,
      c) -NR²⁸R²⁹ wherein R²⁸ and R²⁹ are the same or different and each is a hydrogen atom, a C₁₋₆ alkyl group, a -CO-C₁₋₆ alkyl group, a -CO-cycloalkyl group or a -S(=O)₂-C₁₋₆ alkyl group, or R²⁸ and R²⁹ optionally form, together with the nitrogen atom bonded thereto, a saturated monocyclic nitrogen-containing heterocyclic group,
      d) -CO-NR³⁰R³¹ wherein R³⁰ and R³¹ are the same or different and each is a hydrogen atom or a C₁₋₆ alkyl group, or R³⁰ and R³¹ optionally form, together with the nitrogen atom bonded thereto, a saturated monocyclic nitrogen-containing heterocyclic group,
      e) a C₁₋₆ alkoxy group,
      f) an aryl group (which is optionally substituted by one or more, same or different substituents selected from the following groups: (i) a halogen atom, (ii) a hydroxyl group, (iii) a C₁₋₆ alkoxy group, and (iv) a C₁₋₆ alkyl group (which is optionally substituted by one or more, same or different halogen atoms)),
      g) a carboxyl group, and
      h) an aryloxy group),
   (b) a cycloalkyl group (which is optionally substituted by one or more, same or different substituents selected from the following a) and b):
      a) an aryl group (which is optionally substituted by one or more, same or different substituents selected from the following (1) and (2): (1) a halogen atom and (2) a C₁₋₆ alkyl group (which is optionally substituted by one or more, same or different halogen atoms)), and
      b) a C₁₋₆ alkyl group (which is optionally substituted by one or more, same or different substituents selected from the following groups: (i) a hydroxyl group and (ii) a C₁₋₆ alkoxy group)),
   (c) a saturated monocyclic nitrogen-containing heterocyclic group (which is optionally substituted by one or more, same or different substituents selected from the following a) to c):
      a) a C₁₋₆ alkyl group,
      b) a -CO-C₁₋₆ alkyl group, and
      c) an oxo group),
   (d) an aryl group (which is optionally substituted by one or more, same or different substituents selected from the following (1) to (3): (1) a halogen atom, (2) a C₁₋₆ alkyl group (which is optionally substituted by one or more, same or different halogen atoms) and (3) a C₁₋₆ alkoxy group), or
   (e) a carboxyl group,
5) a C₁₋₆ alkyl group (which is optionally substituted by one or more, same or different substituents selected from the following (a) to (d):
   (a) a carboxyl group,
   (b) a cycloalkyl group (which is optionally substituted by one or more, same or different -CO-NR³²R³³ wherein R³² and R³³ are the same or different and each is a hydrogen atom, a C₁₋₆ alkyl group (which is optionally substituted by one or more, same or different substituents selected from the following groups: (i) a hydroxyl group and (ii) a C₁₋₆ alkoxy group, or R³² and R³³ optionally form, together with the nitrogen atom bonded thereto, a saturated monocyclic nitrogen-containing heterocyclic group)),
   (c) -CO-NR³⁴R³⁵ wherein R³⁴ and R³⁵ are the same or different and each is a hydrogen atom or a C₁₋₆ alkyl group, or R³⁴ and R³⁵ optionally form, together with the nitrogen atom bonded thereto, a saturated monocyclic nitrogen-containing heterocyclic group, and
   (d) an aryl group (which is optionally substituted by one or more, same or different substituents selected from the following groups: a) a halogen atom and b) a C₁₋₆ alkyl group (which is optionally substituted by one or more, same or different halogen atoms))),
6) a cycloalkyl group,
7) -S(=O)₂-R¹² wherein R¹² is a C₁₋₆ alkyl group (which is optionally substituted by one or more, same or different halogen atoms), or an aryl group (which is optionally substituted by one or more, same or different substituents selected from the following groups: a) a halogen atom and b) a C₁₋₆ alkyl group (which is optionally substituted by one or more, same or different halogen atoms)),
8) -C(=NCN)-R¹³ wherein R¹³ is a C₁₋₆ alkyl group,
9) -C(=NCN)NR¹⁴R¹⁵ wherein R¹⁴ and R¹⁵ are the same or different and each is a hydrogen atom or a C₁₋₆ alkyl group, or R¹⁴ and R¹⁵ optionally form, together with the nitrogen atom bonded thereto, a saturated monocyclic nitrogen-containing heterocyclic group,
10) an aryl group (which is optionally substituted by one or more, same or different substituents selected from the following (a) to (f):
   (a) a carboxyl group,
   (b) a halogen atom,
   (c) a C₁₋₆ alkyl group (which is optionally substituted by one or more, same or different substituents selected from the following groups: a) a hydroxyl group, b) a halogen atom and c) a C₁₋₆ alkoxy group),
   (d) -NR³⁸R³⁹ wherein R³⁸ and R³⁹ are the same or different and each is a hydrogen atom, a C₁₋₆ alkyl group, a -CO-C₁₋₆ alkyl group, -CO-NR⁴⁰R⁴¹ wherein R⁴⁰ and R⁴¹ are the same or different and each is a hydrogen atom, a C₁₋₆ alkyl group or a C₁₋₆ alkoxy group, or R⁴⁰ and R⁴¹ optionally form, together with the nitrogen atom bonded thereto, a saturated monocyclic nitrogen-containing heterocyclic group, or -S(=O)₂-R⁴² wherein R⁴² is a C₁₋₆ alkyl group, or R³⁸ and R³⁹ optionally form, together with the nitrogen atom bonded thereto, a saturated monocyclic nitrogen-containing heterocyclic group (which is optionally substituted by one or more oxo groups)),
   (e) -CO-NR⁴³R⁴⁴ wherein R⁴³ and R⁴⁴ are the same or different and each is a hydrogen atom or a C₁₋₆ alkyl group, or R⁴³ and R⁴⁴ optionally form, together with the nitrogen atom bonded thereto, a saturated monocyclic nitrogen-containing heterocyclic group, and
   (f) a -COO-C₁₋₆ alkyl group), or
11) an unsaturated monocyclic 5-membered or 6-membered heterocyclic group (which is optionally substituted by one or more, same or different substituents selected from the following (a) to (h):
   (a) a carboxyl group,
   (b) a C₁₋₆ alkyl group (which is optionally substituted by one or more, same or different substituents selected from the following a) to c):
      a) a halogen atom,
      b) a hydroxyl group, and
      c) a C₁₋₆ alkoxy group),
   (c) a cycloalkyl group,
   (d) a halogen atom,
   (e) -CO-NR⁴⁵R⁴⁶ wherein R⁴⁵ and R⁴⁶ are the same or different and each is a hydrogen atom or a C₁₋₆ alkyl group, or R⁴⁵ and R⁴⁶ optionally form, together with the nitrogen atom bonded thereto, a saturated monocyclic nitrogen-containing heterocyclic group,
   (f) a -COO-C₁₋₆ alkyl group,
   (g) a cyano group, and
   (h) a C₁₋₆ alkoxy group;
-X- is
(1) -N(R¹)- wherein R¹ is as defined above, or
(2) -C(R⁷R⁸)- wherein R⁷ and R⁸ are the same or different and each is
   1) a hydrogen atom,
   2) -NR¹⁶R¹⁷ wherein R¹⁶ and R¹⁷ are the same or different and each is a hydrogen atom, a C₁₋₆ alkyl group or -CO-NR³⁶R³⁷ wherein R³⁶ and R³⁷ are the same or different and each is a hydrogen atom or a C₁₋₆ alkyl group, or R³⁶ and R³⁷ optionally form, together with the nitrogen atom bonded thereto, a saturated monocyclic nitrogen-containing heterocyclic group, or R¹⁶ and R¹⁷ optionally form, together with the nitrogen atom bonded thereto, a saturated monocyclic nitrogen-containing heterocyclic group,
   3) -CONR¹⁸R¹⁹ wherein R¹⁸ and R¹⁹ are the same or different and each is a hydrogen atom, a C₁₋₆ alkyl group (which is optionally substituted by one or more carboxyl groups, or an aryl group (which is optionally substituted by one or more, same or different substituents selected from the following groups: (a) a halogen atom and (b) a C₁₋₆ alkyl group (which is optionally substituted by one or more, same or different halogen atoms)), or R¹⁸ and R¹⁹ optionally form, together with the nitrogen atom bonded thereto, a saturated monocyclic nitrogen-containing heterocyclic group),
   4) an unsaturated monocyclic 5-membered or 6-membered heterocyclic group, or
   5) a carboxyl group;
R² is
(1) a cycloalkyl group (which is optionally substituted by one or more, same or different substituents selected from the following groups: 1) a C₁₋₆ alkyl group (which is optionally substituted by one or more, same or different substituents selected from the following groups: (a) a hydroxyl group and (b) a C₁₋₆ alkoxy group), and 2) a C₁₋₆ alkoxy group; R³ and R⁴ are the same or different and each is

(1) a hydrogen atom,
(2) a C₁₋₆ alkyl group (which is optionally substituted by one or more, same or different substituents selected from the following groups: 1) a halogen atom, 2) a hydroxyl group, 3) a C₁₋₆ alkoxy group and 4) a -OCO-C₁₋₆ alkyl group),
(3) an aryl group (which is optionally substituted by one or more, same or different substituents selected from the following 1) to 6):
   1) a halogen atom,
   2) a C₁₋₆ alkyl group (which is optionally substituted by one or more, same or different substituents selected from the following groups: (a) a halogen atom, (b) a hydroxyl group and (c) a C₁₋₆ alkoxy group),
   3) a C₁₋₆ alkoxy group (which is optionally substituted by one or more, same or different halogen atoms),
   4) a carboxyl group,
   5) a -COO-C₁₋₆ alkyl group, and
   6) a cyano group),
(4) an unsaturated monocyclic 5-membered or 6-membered heterocyclic group (which is optionally substituted by one or more, same or different substituents selected from the following groups: 1) a halogen atom and 2) a C₁₋₆ alkyl group (which is optionally substituted by one or more, same or different halogen atoms)),
(5) a hydroxyl group, or
(6) a C₁₋₆ alkoxy group.

2. A compound represented by the following formula [1] or a salt thereof:

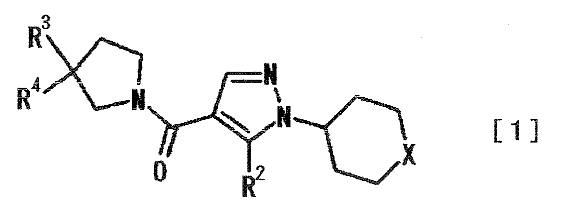

wherein
-X- is
(1) -N(R¹)- wherein R¹ is
   1) a hydrogen atom,
   2) -CONR⁵R⁶ wherein R⁵ and R⁶ are the same or different and each is
      (a) a hydrogen atom,
      (b) an aryl group (which is optionally substituted by one or more, same or different substituents selected from the following groups:
         a) a hydroxyl group,
         b) a halogen atom,
         c) a carboxyl group,
         d) a C₁₋₆ alkyl group (which is optionally substituted by one or more, same or different substituents selected from the following groups: (i) a halogen atom, (ii) a hydroxyl group and (iii) a C₁₋₆ alkoxy group), and
         e) a C₁₋₆ alkoxy group),
      (c) a cycloalkyl group (which is optionally substituted by one or more, same or different C₁₋₆ alkyl groups optionally substituted by one or more, same or different substituents selected from the following groups: (i) a hydroxyl group and (ii) a C₁₋₆ alkoxy group),
      (d) a C₁₋₆ alkyl group (which is optionally substituted by one or more, same or different substituents selected from the following groups:
         a) a hydroxyl group,
         b) a halogen atom,
         c) an aryl group (which is optionally substituted by one or more, same or different substituents selected from the following (1) and (2): (1) a halogen atom and (2) a C₁₋₆ alkyl group (which is optionally substituted by one or more, same or different halogen atoms)),
         d) -NR²⁰R²¹ wherein R²⁰ and R²¹ are the same or different and each is a hydrogen atom, a C₁₋₆ alkyl group or a -CO-C₁₋₆ alkyl group, or R²⁰ and R²¹ optionally form, together with the nitrogen atom bonded thereto, a saturated monocyclic nitrogen-containing heterocyclic group (which is optionally substituted by one or more, same or different substituents selected from the following (i) to (iv):
            (i) a halogen atom,
            (ii) a hydroxyl group,
            (iii) an oxo group, and
            (iv) a C₁₋₆ alkoxy group)),
         e) a C₁₋₆ alkoxy group, and
         f) a carboxyl group),
      (e) -S(=O)₂-R⁹ wherein R⁹ is an aryl group (which is optionally substituted by one or more, same or different substituents selected from the following (1) and (2): (1) a halogen atom and (2) a C₁₋₆ alkyl group (which is optionally substituted by one or more, same or different halogen atoms)) or a C₁₋₆ alkyl group,
      (f) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (g) a C₁₋₆ alkoxy group, or
      (h) a monocyclic nitrogen-containing unsaturated heterocyclic group (which is optionally substituted by one or more, same or different halogen atoms),
         or
      (i) R⁵ and R⁶ optionally form, together with the nitrogen atom bonded thereto, a saturated monocyclic nitrogen-containing heterocyclic group, or a heterocyclic group which is a fused ring of said heterocycle and a carbocycle (which heterocyclic group is optionally substituted by one or more, same or different substituents selected from the following a) to i):
         a) a halogen atom,
         b) a hydroxyl group,
         c) a C₁₋₆ alkyl group (which is optionally substituted by one or more, same or different substituents selected from the following groups: (i) a halogen atom, (ii) a hydroxyl group and (iii) a C₁₋₆ alkoxy group),
         d) a carboxyl group,
         e) a -CO-C₁₋₆ alkyl group,
         f) -CO-NR²²R²³ wherein R²² and R²³ are the same or different and each is a hydrogen atom or a C₁₋₆ alkyl group, or R²² and R²³ optionally form, together with the nitrogen atom bonded thereto, a saturated monocyclic nitrogen-containing heterocyclic group,
         g) an oxo group,
         h) -NR²⁴R²⁵ wherein R²⁴ and R²⁵ are the same or different and each is a hydrogen atom, a C₁₋₆ alkyl group or a -CO-C₁₋₆ alkyl group, or R²⁴ and R²⁵ optionally form, together with the nitrogen atom bonded thereto, a saturated monocyclic nitrogen-containing heterocyclic group, and
         i) a C₁₋₆ alkoxy group)),
   3) -COOR¹⁰ wherein R¹⁰ is
      (a) a C₁₋₆ alkyl group (which is optionally substituted by one or more, same or different substituents selected from the following a) to d):
         a) a hydroxyl group,
         b) -NR²⁶R²⁷ wherein R²⁶ and R²⁷ are the same or different and each is a hydrogen atom or a C₁₋₆ alkyl group, or R²⁶ and R²⁷ optionally form, together with the nitrogen atom bonded thereto, a saturated monocyclic nitrogen-containing heterocyclic group,
         c) a C₁₋₆ alkoxy group, and
         d) an aryl group), or
      (b) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
   4) -COR¹¹ wherein R¹¹ is
      (a) a C₁₋₆ alkyl group (which is optionally substituted by one or more, same or different substituents selected from the following a) to h):
         a) a halogen atom,
         b) a hydroxyl group,
         c) -NR²⁸R²⁹ wherein R²⁸ and R²⁹ are the same or different and each is a hydrogen atom, a C₁₋₆ alkyl group, a -CO-C₁₋₆ alkyl group, a -CO-cycloalkyl group or an -S(=O)₂-C₁₋₆ alkyl group, or R²⁸ and R²⁹ optionally form, together with the nitrogen atom bonded thereto, a saturated monocyclic nitrogen-containing heterocyclic group,
         d) -CO-NR³⁰R³¹ wherein R³⁰ and R³¹ are the same or different and each is a hydrogen atom or a C₁₋₆ alkyl group, or R³⁰ and R³¹ optionally form, together with the nitrogen atom bonded thereto, a saturated monocyclic nitrogen-containing heterocyclic group,
         e) a C₁₋₆ alkoxy group,
         f) an aryl group (which is optionally substituted by one or more, same or different substituents selected from the following groups: (i) a halogen atom, (ii) a hydroxyl group, (iii) a C₁₋₆ alkoxy group and (iv) a C₁₋₆ alkyl group (which is optionally substituted by one or more, same or different halogen atoms)),
         g) a carboxyl group, and
         h) an aryloxy group),
      (b) a cycloalkyl group (which is optionally substituted by one or more, same or different substituents selected from the following a) and b):
         a) an aryl group (which is optionally substituted by one or more, same or different substituents selected from the following (1) and (2): (1) a halogen atom and (2) a C₁₋₆ alkyl group (which is optionally substituted by one or more, same or different halogen atoms)), and
         b) a C₁₋₆ alkyl group (which is optionally substituted by one or more, same or different substituents selected from the following groups: (i) a hydroxyl group and (ii) a C₁-₆ alkoxy group)),
      (c) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (d) an aryl group (which is optionally substituted by one or more, same or different substituents selected from the following (1) to (3): (1) a halogen atom, (2) a C₁₋₆ alkyl group (which is optionally substituted by one or more, same or different halogen atoms) and (3) a C₁₋₆ alkoxy group), or
      (e) a carboxyl group,
   5) a C₁₋₆ alkyl group (which is optionally substituted by one or more, same or different substituents selected from the following (a) to (d):
      (a) a carboxyl group,
      (b) a cycloalkyl group (which is optionally substituted by one or more, same or different -CO-NR³²R³³ wherein R³² and R³³ are the same or different and each is a hydrogen atom, a C₁₋₆ alkyl group (which is optionally substituted by one or more, same or different substituents selected from the following groups: (i) a hydroxyl group and (ii) a C₁₋₆ alkoxy group), or R³² and R³³ optionally form, together with the nitrogen atom bonded thereto, a saturated monocyclic nitrogen-containing heterocyclic group),
      (c) -CO-NR³⁴R³⁵ wherein R³⁴ and R³⁵ are the same or different and each is a hydrogen atom or a C₁₋₆ alkyl group, or R³⁴ and R³⁵ optionally form, together with the nitrogen atom bonded thereto, a saturated monocyclic nitrogen-containing heterocyclic group, and
      (d) an aryl group (which is optionally substituted by one or more, same or different substituents selected from the following groups: a) a halogen atom and b) a C₁₋₆ alkyl group (which is optionally substituted by one or more, same or different halogen atoms))),
   6) a cycloalkyl group,
   7) -S(=O)₂-R¹² wherein R¹² is a C₁₋₆ alkyl group (which is optionally substituted by one or more, same or different halogen atoms), or an aryl group (which is optionally substituted by one or more, same or different substituents selected from the following groups: a) a halogen atom and b) a C₁₋₆ alkyl group (which is optionally substituted by one or more, same or different halogen atoms)),
   8) -C(=NCN)-R¹³ wherein R¹³ is a C₁₋₆ alkyl group,
   9) -C(=NCN)NR¹⁴R¹⁵ wherein R¹⁴ and R¹⁵ are the same or different and each is a hydrogen atom or a C₁₋₆ alkyl group, or R¹⁴ and R¹⁵ optionally form, together with the nitrogen atom bonded thereto, a saturated monocyclic nitrogen-containing heterocyclic group,
   10) an aryl group (which is optionally substituted by one or more, same or different substituents selected from the following (a) to (f):
      (a) a carboxyl group,
      (b) a halogen atom,
      (c) a C₁₋₆ alkyl group (which is optionally substituted by one or more, same or different substituents selected from the following groups: a) a hydroxyl group, b) a halogen atom and c) a C₁₋₆ alkoxy group),
      (d) -NR³⁸R³⁹ wherein R³⁸ and R³⁹ are the same or different and each is a hydrogen atom, a C₁₋₆ alkyl group, a -CO-C₁₋₆ alkyl group, -CO-NR⁴⁰R⁴¹ wherein R⁴⁰ and R⁴¹ are the same or different and each is a hydrogen atom, a C₁₋₆ alkyl group or a C₁₋₆ alkoxy group, or R⁴⁰ and R⁴¹ optionally form, together with the nitrogen atom bonded thereto, a saturated monocyclic nitrogen-containing heterocyclic group, or -S(=O)₂-R⁴² wherein R⁴² is a C₁₋₆ alkyl group, or R^{3B} and R³⁹ optionally form, together with the nitrogen atom bonded thereto, a saturated monocyclic nitrogen-containing heterocyclic group (which is optionally substituted by one or more oxo groups),
      (e) -CO-NR⁴³R⁴⁴ wherein R⁴³ and R⁴⁴ are the same or different and each is a hydrogen atom or a C₁₋₆ alkyl group, or R⁴³ and R⁴⁴ optionally form, together with the nitrogen atom bonded thereto, a saturated monocyclic nitrogen-containing heterocyclic group, and
      (f) a -COO-C₁₋₆ alkyl group), or
   11) an unsaturated monocyclic 5-membered or 6-membered heterocyclic group (which is optionally substituted by one or more, same or different substituents selected from the following (a) to (h):
      (a) a carboxyl group,
      (b) a C₁₋₆ alkyl group (which is optionally substituted by one or more, same or different substituents selected from the following a) to c):
         a) a halogen atom,
         b) a hydroxyl group, and
         c) a C₁₋₆ alkoxy group),
      (c) a cycloalkyl group,
      (d) a halogen atom,
      (e) -CO-NR⁴⁵R⁴⁶ wherein R⁴⁵ and R⁴⁶ are the same or different and each is a hydrogen atom or a C₁₋₆ alkyl group, or R⁴⁵ and R⁴⁶ optionally form, together with the nitrogen atom bonded thereto, a saturated monocyclic nitrogen-containing heterocyclic group,
      (f) a -COO-C₁₋₆alkyl group,
      (g) a cyano group, and
      (h) a C₁₋₆ alkoxy group), or,
(2) -C(R⁷R^{B})- wherein R⁷ and R⁸ are the same or different and each is
   1) a hydrogen atom,
   2) -NR¹⁶R¹⁷ wherein R¹⁶ and R¹⁷ are the same or different and each is a hydrogen atom, a C₁₋₆ alkyl group or -CO-NR³⁶R³⁷ wherein R³⁶ and R³⁷ are the same or different and each is a hydrogen atom or a C₁₋₆ alkyl group, or R³⁶ and R³⁷ optionally form, together with the nitrogen atom bonded thereto, a saturated monocyclic nitrogen-containing heterocyclic group, or R¹⁶ and R¹⁷ optionally form, together with the nitrogen atom bonded thereto, a saturated monocyclic nitrogen-containing heterocyclic group,
   3) -CONR¹⁸R¹⁹ wherein R¹⁸ and R¹⁹ are the same or different and each is a hydrogen atom, a C₁₋₆ alkyl group (which is optionally substituted by one or more carboxyl groups, or an aryl group (which is optionally substituted by one or more, same or different substituents selected from the following groups: (a) a halogen atom and (b) a C₁₋₆ alkyl group (which is optionally substituted by one or more, same or different halogen atoms)), or R¹⁸ and R¹⁹ optionally form, together with the nitrogen atom bonded thereto, a saturated monocyclic nitrogen-containing heterocyclic group,
   4) an unsaturated monocyclic 5-membered or 6-membered heterocyclic group, or
   5) a carboxyl group;
R² is
(1) a cycloalkyl group (which is optionally substituted by one or more, same or different substituents selected from the following groups: 1) a C₁₋₆ alkyl group (which is optionally substituted by one or more, same or different substituents selected from the following groups: (a) a hydroxyl group and (b) a C₁₋₆ alkoxy group), and 2) a C₁₋₆ alkoxy group); R³ and R⁴ are the same or different and each is

(1) a hydrogen atom,
(2) a C₁₋₆ alkyl group (which is optionally substituted by one or more, same or different substituents selected from the following groups: 1) a halogen atom, 2) a hydroxyl group, 3) a C₁₋₆ alkoxy group and 4) a -OCO-C₁₋₆ alkyl group),
(3) an aryl group (which is optionally substituted by one or more, same or different substituents selected from the following 1) to 6):
   1) a halogen atom,
   2) a C₁₋₆ alkyl group (which is optionally substituted by one or more, same or different substituents selected from the following groups: (a) a halogen atom, (b) a hydroxyl group and (c) a C₁₋₆ alkoxy group),
   3) a C₁₋₆ alkoxy group (which is optionally substituted by one or more, same or different halogen atoms),
   4) a carboxyl group,
   5) a -COO-C₁₋₆ alkyl group, and
   6) a cyano group),
(4) an unsaturated monocyclic 5-membered or 6-membered heterocyclic group (which is optionally substituted by one or more, same or different substituents selected from the following groups: 1) a halogen atom and 2) a C₁₋₆ alkyl group (which is optionally substituted by one or more, same or different halogen atoms)),
(5) a hydroxyl group, or
(6) a C₁₋₆ alkoxy group.

3. The compound of any one of the above-mentioned 1. and 2., wherein -X- is -C(R⁷R⁸)- wherein R⁷ and R⁸ are as defined for above-mentioned 1., or a salt thereof.

4. The compound of any one of the above-mentioned 1. to 3., wherein R⁸ is

1) -NR¹⁶R¹⁷ wherein R¹⁶ and R¹⁷ are the same or different and each is a hydrogen atom or a C₁₋₆ alkyl group, or R¹⁶ and R¹⁷ optionally form, together with the nitrogen atom bonded thereto, a saturated monocyclic nitrogen-containing heterocyclic group,
2) -CONR¹⁸R¹⁹ wherein R¹⁸ and R¹⁹ are the same or different and each is a hydrogen atom or a C₁₋₆ alkyl group, or R¹⁸ and R¹⁹ optionally form, together with the nitrogen atom bonded thereto, a saturated monocyclic nitrogen-containing heterocyclic group, or
3) an unsaturated monocyclic 5-membered or 6-membered heterocyclic group,
or a salt thereof.

5. The compound of any one of the above-mentioned 1. and 2., wherein -X- is -N(R¹)- wherein R¹ is as defined for the above-mentioned 1., or a salt thereof.

6. The compound of any one of the above-mentioned 1., 2. and 5., wherein R¹ is
   1) a hydrogen atom,
   2) -CONR⁵R⁶ wherein R⁵ and R⁶ are the same or different and each is
      (a) a hydrogen atom,
      (b) an aryl group (which is substituted by one or more, same or different substituents selected from the following groups:
         a) a hydroxyl group,
         b) a halogen atom,
         c) a carboxyl group,
         d) a C₁₋₆ alkyl group (which is optionally substituted by one or more, same or different substituents selected from the following groups: (i) a halogen atom, (ii) a hydroxyl group and (iii) a C₁₋₆ alkoxy group) and
         e) a C₁₋₆ alkoxy group),
      (c) a cycloalkyl group (which is optionally substituted by one or more, same or different C₁₋₆ alkyl groups (which is optionally substituted by one or more, same or different substituents selected from the following groups: (i) a hydroxyl group and (ii) a C₁₋₆ alkoxy group)),
      (d) a C₁₋₆ alkyl group (which is substituted by one or more, same or different substituents selected from the following groups:
         a) a hydroxyl group,
         b) a halogen atom,
         c) an aryl group (which is optionally substituted by one or more, same or different substituents selected from the following (1) and (2): (1) a halogen atom and (2) a C₁₋₆ alkyl group (which is optionally substituted by one or more, same or different halogen atoms)),
         d) -NR²⁰R²¹ wherein R²⁰ and R²¹ are the same or different and each is a hydrogen atom, a C₁₋₆ alkyl group or a -CO-C₁₋₆ alkyl group, or R²⁰ and R²¹ optionally form, together with the nitrogen atom bonded thereto, a saturated monocyclic nitrogen-containing heterocyclic group (which is optionally substituted by one or more, same or different substituents selected from the following (i) to (iv):
            (i) a halogen atom,
            (ii) a hydroxyl group,
            (iii) an oxo group, and
            (iv) a C₁₋₆ alkoxy group)),
         e) a C₁₋₆ alkoxy group, and
         f) a carboxyl group),
      (e) -S(=O)₂-R⁹ wherein R⁹ is an aryl group (which is optionally substituted by one or more, same or different substituents selected from the following (1) and (2): (1) a halogen atom and (2) a C₁₋₆ alkyl group (which is optionally substituted by one or more, same or different halogen atoms)), or a C₁₋₆ alkyl group, or
      (f) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group), or
      (g) a monocyclic nitrogen-containing unsaturated heterocyclic group (which is optionally substituted by one or more, same or different halogen atoms),
         or
      (h) R⁵ and R⁶ optionally form, together with the nitrogen atom bonded thereto, a saturated monocyclic nitrogen-containing heterocyclic group (which is optionally substituted by one or more, same or different substituents selected from the following a) to i):
         a) a halogen atom,
         b) a hydroxyl group,
         c) a C₁₋₆ alkyl group (which is optionally substituted by one or more, same or different substituents selected from the following groups: (i) a hydroxyl group and (ii) a C₁₋₆ alkoxy group),
         d) a carboxyl group,
         e) a -CO-C₁₋₆ alkyl group,
         f) -CO-NR²²R²³ wherein R²² and R²³ are the same or different and each is a hydrogen atom or a C₁₋₆ alkyl group, or R²² and R²³ optionally form, together with the nitrogen atom bonded thereto, a saturated monocyclic nitrogen-containing heterocyclic group,
         g) an oxo group,
         h) -NR²⁴R²⁵ wherein R²⁴ and R²⁵ are the same or different and each is a hydrogen atom, a C₁₋₆ alkyl group or a -CO-C₁₋₆ alkyl group, or R²⁴ and R²⁵ optionally form, together with the nitrogen atom bonded thereto, a saturated monocyclic nitrogen-containing heterocyclic group, and
         i) a C₁₋₆ alkoxy group) (provided that R⁵ and R⁶ are not hydrogen atoms at the same time)),
   3) -COOR¹⁰ wherein R¹⁰ is,
      (a) a C₁₋₆ alkyl group (which is optionally substituted by one or more, same or different substituents selected from the following a) to d):
         a) a hydroxyl group,
         b) -NR²⁶R²⁷ wherein R²⁶ and R²⁷ are the same or different and each is a hydrogen atom or a C₁₋₆ alkyl group, or R²⁶ and R²⁷ optionally form, together with the nitrogen atom bonded thereto, a saturated monocyclic nitrogen-containing heterocyclic group,
         c) a C₁₋₆ alkoxy group, and
         d) an aryl group), or
      (b) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
   4) -COR¹¹ wherein R¹¹ is
      (a) a C₁₋₆ alkyl group (which is optionally substituted by one or more, same or different substituents selected from the following a) to h):
         a) a halogen atom,
         b) a hydroxyl group,
         c) -NR²⁸R²⁹ wherein R²⁸ and R²⁹ are the same or different and each is a hydrogen atom, a C₁₋₆ alkyl group, a -CO-C₁₋₆ alkyl group, a -CO-cycloalkyl group or a -S(=O)₂-C₁₋₆ alkyl group, or R²⁸ and R²⁹ optionally form, together with the nitrogen atom bonded thereto, a saturated monocyclic nitrogen-containing heterocyclic group,
         d) -CO-NR³⁰R³¹ wherein R³⁰ and R³¹ are the same or different and each is a hydrogen atom or a C₁₋₆ alkyl group, or R³⁰ and R³¹ optionally form, together with the nitrogen atom bonded thereto, a saturated monocyclic nitrogen-containing heterocyclic group,
         e) a C₁₋₆ alkoxy group,
         f) an aryl group which is optionally substituted by one or more, same or different substituents selected from the following groups: (i) a halogen atom, (ii) a hydroxyl group, (iii) a C₁₋₆ alkoxy group and (iv) a C₁₋₆ alkyl group (which is optionally substituted by one or more, same or different halogen atoms)),
         g) a carboxyl group, and
         h) an aryloxy group),
      (b) a cycloalkyl group (which is optionally substituted by one or more, same or different substituents selected from the following a) and b):
         a) an aryl group (which is optionally substituted by one or more, same or different substituents selected from the following (1) and (2): (1) a halogen atom and (2) a C₁₋₆ alkyl group (which is optionally substituted by one or more, same or different halogen atoms)), and
         b) a C₁₋₆ alkyl group (which is optionally substituted by one or more, same or different substituents selected from the following groups: (i) a hydroxyl group and (ii) a C₁₋₆ alkoxy group)), or
      (c) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (d) an aryl group (which is optionally substituted by one or more, same or different substituents selected from the following (1) to (3): (1) a halogen atom, (2) a C₁₋₆ alkyl group (which is optionally substituted by one or more, same or different halogen atoms), and (3) a C₁₋₆ alkoxy group), or
      (e) a carboxyl group,
   5) a C₁₋₆ alkyl group (which is optionally substituted by one or more, same or different substituents selected from the following (a) to (d):
      (a) a carboxyl group,
      (b) a cycloalkyl group (which is optionally substituted by one or more, same or different -CO-NR³²R³³ wherein R³² and R³³ are the same or different and each is a hydrogen atom, a C₁₋₆ alkyl group (which is optionally substituted by one or more, same or different substituents selected from the following groups: (i) a hydroxyl group and (ii) a C₁₋₆ alkoxy group), or R³² and R³³ optionally form, together with the nitrogen atom bonded thereto, a saturated monocyclic nitrogen-containing heterocyclic group)),
      (c) -CO-NR³⁴R³⁵ wherein R³⁴ and R³⁵ are the same or different and each is a hydrogen atom or a C₁₋₆ alkyl group, or R³⁴ and R³⁵ optionally form, together with the nitrogen atom bonded thereto, a saturated monocyclic nitrogen-containing heterocyclic group, and
      (d) an aryl group (which is optionally substituted by one or more, same or different substituents selected from the following groups: a) a halogen atom and b) a C₁₋₆ alkyl group (which is optionally substituted by one or more, same or different halogen atoms))),
   6) a cycloalkyl group,
   7) -S(=O)₂-R¹² wherein R¹² is a C₁₋₆ alkyl group (which is optionally substituted by one or more, same or different halogen atoms), or an aryl group (which is optionally substituted by one or more, same or different substituents selected from the following groups: a) a halogen atom and b) a C₁₋₆ alkyl group (which is optionally substituted by one or more, same or different halogen atoms)),
   8) -C(=NCN)-R¹³ wherein R¹³ is a C₁₋₆ alkyl group,
   9) -C(=NCN)NR¹⁴R¹⁵ wherein R¹⁴ and R¹⁵ are the same or different and each is a hydrogen atom or a C₁₋₆ alkyl group, or R¹⁴ and R¹⁵ optionally form, together with the nitrogen atom bonded thereto, a saturated monocyclic nitrogen-containing heterocyclic group,
   10) an aryl group (which is optionally substituted by one or more, same or different substituents selected from the following (a) to (f):
      (a) a carboxyl group,
      (b) a halogen atom,
      (c) a C₁₋₆ alkyl group (which is optionally substituted by one or more, same or different substituents selected from the following groups: a) a hydroxyl group, b) a halogen atom and c) a C₁₋₆ alkoxy group),
      (d) -NR³⁸R³⁹ wherein R³⁸ and R³⁹ are the same or different and each is a hydrogen atom, a C₁₋₆ alkyl group, a -CO-C₁₋₆ alkyl group, -CO-NR⁴⁰R⁴¹ wherein R⁴⁰ and R⁴¹ are the same or different and each is a hydrogen atom, a C₁₋₆ alkyl group, or a C₁₋₆ alkoxy group, or R⁴⁰ and R⁴¹ optionally form, together with the nitrogen atom bonded thereto, a saturated monocyclic nitrogen-containing heterocyclic group, or -S(=O)₂-R⁴² wherein R⁴² is a C₁₋₆ alkyl group, or R³⁸ and R³⁹ optionally form, together with the nitrogen atom bonded thereto, a saturated monocyclic nitrogen-containing heterocyclic group (which is optionally substituted by one or more oxo groups),
      (e) -CO-NR⁴³R⁴⁴ wherein R⁴³ and R⁴⁴ are the same or different and each is a hydrogen atom or a C₁₋₆ alkyl group, or R⁴³ and R⁴⁴ optionally form, together with the nitrogen atom bonded thereto, a saturated monocyclic nitrogen-containing heterocyclic group, and
      (f) a -COO-C₁₋₆ alkyl group), or
   11) an unsaturated monocyclic 5-membered or 6-membered heterocyclic group (which is optionally substituted by one or more, same or different substituents selected from the following (a) to (h):
      (a) a carboxyl group,
      (b) a C₁₋₆ alkyl group (which is optionally substituted by one or more, same or different substituents selected from the following a) to c):
         a) a halogen atom,
         b) a hydroxyl group, and
         c) a C₁₋₆ alkoxy group),
      (c) a cycloalkyl group,
      (d) a halogen atom,
      (e) -CO-NR⁴⁵R⁴⁶ wherein R⁴⁵ and R⁴⁶ are the same or different and each is a hydrogen atom or a C₁₋₆ alkyl group, or R⁴⁵ and R⁴⁶ optionally form, together with the nitrogen atom bonded thereto, a saturated monocyclic nitrogen-containing heterocyclic group,
      (f) a -COO-C₁₋₆ alkyl group,
      (g) a cyano group, and
      (h) a C₁₋₆ alkoxy group,
or a salt thereof.

7. The compound of any one of the above-mentioned 1., 2. and 5., wherein R¹ is
   1) -CONR⁵R⁶ wherein R⁵ and R⁶ are the same or different and each is
      (a) a hydrogen atom,
      (b) an aryl group (which is optionally substituted by one or more, same or different substituents selected from the following groups:
         a) a hydroxyl group,
         b) a halogen atom,
         c) a carboxyl group,
         d) a C₁₋₆ alkyl group (which is optionally substituted by one or more, same or different substituents selected from the following groups: (i) a halogen atom, (ii) a hydroxyl group and (iii) a C₁₋₆ alkoxy group), and
         e) a C₁₋₆ alkoxy group),
      (c) a cycloalkyl group (which is optionally substituted by one or more, same or different C₁₋₆ alkyl groups optionally substituted by one or more, same or different substituents selected from the following groups: (i) a hydroxyl group and (ii) a C₁₋₆ alkoxy group),
      (d) a C₁₋₆ alkyl group (which is optionally substituted by one or more, same or different substituents selected from the following groups:
         a) a hydroxyl group,
         b) a halogen atom,
         c) an aryl group (which is optionally substituted by one or more, same or different substituents selected from the following (1) and (2): (1) a halogen atom and (2) a C₁₋₆ alkyl group (which is optionally substituted by one or more, same or different halogen atoms)),
         d) -NR²⁰R²¹ wherein R²⁰ and R²¹ are the same or different and each is a hydrogen atom, a C₁₋₆ alkyl group or a -CO-C₁₋₆ alkyl group, or R²⁰ and R²¹ optionally form, together with the nitrogen atom bonded thereto, a saturated monocyclic nitrogen-containing heterocyclic group (which is optionally substituted by one or more, same or different substituents selected from the following (i) to (iv):
            (i) a halogen atom,
            (ii) a hydroxyl group,
            (iii) an oxo group, and
            (iv) a C₁₋₆ alkoxy group),
         e) a C₁₋₆ alkoxy group, and
         f) a carboxyl group),
      (e) -S(=O)₂-R⁹ wherein R⁹ is an aryl group (which is optionally substituted by one or more, same or different substituents selected from the following (1) and (2): (1) a halogen atom and (2) a C₁₋₆ alkyl group (which is optionally substituted by one or more, same or different halogen atoms)), or a C₁₋₆ alkyl group, or
      (f) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (g) a C₁₋₆ alkoxy group, or
      (h) a monocyclic nitrogen-containing unsaturated heterocyclic group (which is optionally substituted by one or more, same or different halogen atoms),
         or
         (i) R⁵ and R⁶ optionally form, together with the nitrogen atom bonded thereto, a saturated monocyclic nitrogen-containing heterocyclic group (which is optionally substituted by one or more, same or different substituents selected from the following a) to i):
            a) a halogen atom,
            b) a hydroxyl group,
            c) a C₁₋₆ alkyl group (which is optionally substituted by one or more, same or different substituents selected from the following groups: (i) a hydroxyl group and (ii) a C₁₋₆ alkoxy group),
            d) a carboxyl group,
            e) a -CO-C₁₋₆ alkyl group,
            f) -CO-NR²²R²³ wherein R²² and R²³ are the same or different and each is a hydrogen atom or a C₁₋₆ alkyl group, or R²² and R²³ optionally form, together with the nitrogen atom bonded thereto, a saturated monocyclic nitrogen-containing heterocyclic group,
            g) an oxo group,
            h) -NR²⁴R²⁵ wherein R²⁴ and R²⁵ are the same or different and each is a hydrogen atom, a C₁₋₆ alkyl group or a -CO-C₁₋₆ alkyl group, or R²⁴ and R²⁵ optionally form, together with the nitrogen atom bonded thereto, a saturated monocyclic nitrogen-containing heterocyclic group, and
            i) a C₁₋₆ alkoxy group) (provided that R⁵ and R⁶ do not form a combination of substituents selected from the following groups: (i) a hydrogen atom and (ii) an unsubstituted C₁₋₆ alkyl group)),
   2) -COOR¹⁰ wherein R¹⁰ is
      (a) a C₁₋₆ alkyl group (which is substituted by one or more, same or different substituents selected from the following a) to d) :
         a) a hydroxyl group,
         b) -NR²⁶R²⁷ wherein R²⁶ and R²⁷ are the same or different and each is a hydrogen atom or a C₁₋₆ alkyl group, or R²⁶ and R²⁷ optionally form, together with the nitrogen atom bonded thereto, a saturated monocyclic nitrogen-containing heterocyclic group,
         c) a C₁₋₆ alkoxy group, and
         d) an aryl group), or
      (b) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
   3) -COR¹¹ wherein R¹¹ is
      (a) a C₁₋₆ alkyl group (which is substituted by one or more, same or different substituents selected from the following a) to h):
         a) a halogen atom,
         b) a hydroxyl group,
         c) -NR²⁸R²⁹ wherein R²⁸ and R²⁹ are the same or different and each is a hydrogen atom, a C₁₋₆ alkyl group, a -CO-C₁₋₆ alkyl group, a -CO-cycloalkyl group or a -S(=O)₂-C₁₋₆ alkyl group, or R²⁸ and R²⁹ optionally form, together with the nitrogen atom bonded thereto, a saturated monocyclic nitrogen-containing heterocyclic group,
         d) -CO-NR³⁰R³¹ wherein R³⁰ and R³¹ are the same or different and each is a hydrogen atom or a C₁₋₆ alkyl group, or R³⁰ and R³¹ optionally form, together with the nitrogen atom bonded thereto, a saturated monocyclic nitrogen-containing heterocyclic group,
         e) a C₁₋₆ alkoxy group,
         f) an aryl group (which is optionally substituted by one or more, same or different substituents selected from the following groups: (i) a halogen atom, (ii) a hydroxyl group, (iii) a C₁₋₆ alkoxy group and (iv) a C₁₋₆ alkyl group (which is optionally substituted by one or more, same or different halogen atoms)),
         g) a carboxyl group, and
         h) an aryloxy group),
      (b) a cycloalkyl group (which is optionally substituted by one or more, same or different substituents selected from the following a) and b):
         a) an aryl group (which is optionally substituted by one or more, same or different substituents selected from the following (1) and (2): (1) a halogen atom and (2) a C₁₋₆ alkyl group (which is optionally substituted by one or more, same or different halogen atoms)), and
         b) a C₁₋₆ alkyl group (which is optionally substituted by one or more, same or different substituents selected from the following groups: (i) a hydroxyl group and (ii) a C₁₋₆ alkoxy group)), or
      (c) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (d) an acryl group (which is optionally substituted by one or more, same or different substituents selected from the following (1) to (3): (1) a halogen atom, (2) a C₁₋₆ alkyl group (which is optionally substituted by one or more, same or different halogen atoms) and (3) a C₁₋₆ alkoxy group), or
      (e) a carboxyl group,
   4) a C₁₋₆ alkyl group (which is substituted by one or more, same or different substituents selected from the following (a) to (d):
      (a) a carboxyl group,
      (b) a cycloalkyl group (which is optionally substituted by one or more, same or different -CO-NR³²R³³ wherein R³² and R³³ are the same or different and each is a hydrogen atom, a C₁₋₆ alkyl group (which is optionally substituted by one or more, same or different substituents selected from the following groups: (i) a hydroxyl group and (ii) a C₁₋₆ alkoxy group), or R³² and R³³ optionally form, together with the nitrogen atom bonded thereto, a saturated monocyclic nitrogen-containing heterocyclic group),
      (c) -CO-NR³⁴R³⁵ wherein R³⁴ and R³⁵ are the same or different and each is a hydrogen atom or a C₁₋₆ alkyl group, or R³⁴ and R³⁵ optionally form, together with the nitrogen atom bonded thereto, a saturated monocyclic nitrogen-containing heterocyclic group, and
      (d) an aryl group (which is optionally substituted by one or more, same or different substituents selected from the following groups: a) a halogen atom and b) a C₁₋₆ alkyl group (which is optionally substituted by one or more, same or different halogen atoms))),
   5) a cycloalkyl group,
   6) -S(=O)₂-R¹² wherein R¹² is a C₁₋₆ alkyl group (which is substituted by one or more, same or different halogen atoms), or an aryl group (which is optionally substituted by one or more, same or different substituents selected from the following groups: a) a halogen atom and b) a C₁₋₆ alkyl group (which is optionally substituted by one or more, same or different halogen atoms)),
   7) -C(=NCN)-R¹³ wherein R¹³ is a C₁₋₆ alkyl group,
   8) -C(=NCN)NR¹⁴R¹⁵ wherein R¹⁴ and R¹⁵ are the same or different and each is a hydrogen atom or a C₁₋₆ alkyl group, or R¹⁴ and R¹⁵ optionally form, together with the nitrogen atom bonded thereto, a saturated monocyclic nitrogen-containing heterocyclic group, or
   9) an unsaturated monocyclic 5-membered or 6-membered heterocyclic group (which is substituted by one or more, same or different substituents selected from the following (a) to (g)
      (a) a C₁₋₆ alkyl group (which is substituted by one or more, same or different substituents selected from the following a) to c):
         a) a halogen atom,
         b) a hydroxyl group, and
         c) a C₁₋₆ alkoxy group),
      (b) a cycloalkyl group,
      (c) a halogen atom,
      (d) -CO-NR⁴⁵R⁴⁶ wherein R⁴⁵ and R⁴⁶ are the same or different and each is a hydrogen atom or a C₁₋₆ alkyl group, or R⁴⁵ and R⁴⁶ optionally form, together with the nitrogen atom bonded thereto, a saturated monocyclic nitrogen-containing heterocyclic group,
      (e) a -COO-C₁₋₆ alkyl group,
      (f) a cyano group, and
      (g) a C₁₋₆ alkoxy group,
or a salt thereof.

8. The compound of any one of the above-mentioned 1., 2. and 5., wherein R¹ is
   1) -CONR⁵R⁶ wherein R⁵ and R⁶ are the same or different and each is
      (a) a hydrogen atom,
      (b) an aryl group (which is substituted by one or more, same or different substituents selected from the following groups:
         a) a hydroxyl group,
         b) a halogen atom,
         c) a carboxyl group,
         d) a C₁₋₆ alkyl group (which is optionally substituted by one or more, same or different substituents selected from the following groups: (i) a halogen atom, (ii) a hydroxyl group and (iii) a C₁₋₆ alkoxy group), and
         e) a C₁₋₆ alkoxy group),
      (c) a cycloalkyl group (which is optionally substituted by one or more, same or different C₁₋₆ alkyl groups optionally substituted by one or more, same or different substituents selected from the following groups: (i) a hydroxyl group and (ii) a C₁₋₆ alkoxy group),
      (d) a C₁₋₆ alkyl group (which is substituted by one or more, same or different substituents selected from the following groups:
         a) a hydroxyl group,
         b) a halogen atom,
         c) an aryl group (which is optionally substituted by one or more, same or different substituents selected from the following (1) and (2): (1) a halogen atom and (2) a C₁₋₆ alkyl group (which is optionally substituted by one or more, same or different halogen atoms)),
         d) -NR²⁰R²¹ wherein R²⁰ and R²¹ are the same or different and each is a hydrogen atom, a C₁₋₆ alkyl group or a -CO-C₁₋₆ alkyl group, or R²⁰ and R²¹ optionally form, together with the nitrogen atom bonded thereto, a saturated monocyclic nitrogen-containing heterocyclic group (which is optionally substituted by one or more, same or different substituents selected from the following (i) to (iv):
            (i) a halogen atom,
            (ii) a hydroxyl group,
            (iii) an oxo group, and
            (iv) a C₁₋₆ alkoxy group)),
         e) a C₁₋₆ alkoxy group, and
         f) a carboxyl group),
      (e) -S(=O)₂-R⁹ wherein R⁹ is an aryl group (which is optionally substituted by one or more, same or different substituents selected from the following (1) and (2): (1) a halogen atom and (2) a C₁₋₆ alkyl group (which is optionally substituted by one or more, same or different halogen atoms)), or a C₁₋₆ alkyl group), or
      (f) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group), or
      (g) a monocyclic nitrogen-containing unsaturated heterocyclic group (which is optionally substituted by one or more, same or different halogen atoms),
         or
      (h) R⁵ and R⁶ optionally form, together with the nitrogen atom bonded thereto, a saturated monocyclic nitrogen-containing heterocyclic group (which is optionally substituted by one or more, same or different substituents selected from the following a) to i):
         a) a halogen atom,
         b) a hydroxyl group,
         c) a C₁₋₆ alkyl group (which is optionally substituted by one or more, same or different substituents selected from the following groups: (i) a hydroxyl group and (ii) a C₁₋₆ alkoxy group),
         d) a carboxyl group,
         e) a -CO-C₁₋₆ alkyl group,
         f) -CO-NR²²R²³ wherein R²² and R²³ are the same or different and each is a hydrogen atom or a C₁₋₆ alkyl group, or R²² and R²³ optionally form, together with the nitrogen atom bonded thereto, a saturated monocyclic nitrogen-containing heterocyclic group,
         g) an oxo group,
         h) -NR²⁴R²⁵ wherein R²⁴ and R²⁵ are the same or different and each is a hydrogen atom, a C₁₋₆ alkyl group or a -CO-C₁₋₆ alkyl group, or R²⁴ and R²⁵ optionally form, together with the nitrogen atom bonded thereto, a saturated monocyclic nitrogen-containing heterocyclic group, and
         i) a C₁₋₆ alkoxy group) (provided that R⁵ and R⁶ are not hydrogen atoms at the same time)),
   2) -COOR¹⁰ wherein R¹⁰ is
      (a) a C₁₋₆ alkyl group (which is substituted by one or more, same or different substituents selected from the following a) to d):
         a) a hydroxyl group,
         b) -NR²⁶R²⁷ wherein R²⁶ and R²⁷ are the same or different and each is a hydrogen atom or a C₁₋₆ alkyl group, or R²⁶ and R²⁷ optionally form, together with the nitrogen atom bonded thereto, a saturated monocyclic nitrogen-containing heterocyclic group,
         c) a C₁₋₆ alkoxy group, and
         d) an aryl group), or
      (b) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
   3) -COR¹¹ wherein R¹¹ is
      (a) a C₁₋₆ alkyl group (which is substituted by one or more, same or different substituents selected from the following a) to h):
         a) a halogen atom,
         b) a hydroxyl group,
         c) -NR²⁸R²⁹ wherein R²⁸ and R²⁹ are the same or different and each is a hydrogen atom, a C₁₋₆ alkyl group, a -CO-C₁₋₆ alkyl group, a -CO-cycloalkyl group or a -S(=O)₂-C₁₋₆ alkyl group, or R²⁸ and R²⁹ optionally form, together with the nitrogen atom bonded thereto, a saturated monocyclic nitrogen-containing heterocyclic group,
         d) -CO-NR³⁰R³¹ wherein R³⁰ and R³¹ are the same or different and each is a hydrogen atom or a C₁₋₆ alkyl group, or R³⁰ and R³¹ optionally form, together with the nitrogen atom bonded thereto, a saturated monocyclic nitrogen-containing heterocyclic group,
         e) a C₁₋₆ alkoxy group,
         f) an aryl group (which is optionally substituted by one or more, same or different substituents selected from the following groups: (i) a halogen atom, (ii) a hydroxyl group, (iii) a C₁₋₆ alkoxy group and (iv) a C₁₋₆ alkyl group (which is optionally substituted by one or more, same or different halogen atoms)),
         g) a carboxyl group, and
         h) an aryloxy group),
      (b) a cycloalkyl group (which is optionally substituted by one or more, same or different substituents selected from the following a) and b):
         a) an aryl group (which is optionally substituted by one or more, same or different substituents selected from the following (1) and (2): (1) a halogen atom and (2) a C₁₋₆ alkyl group (which is optionally substituted by one or more, same or different halogen atoms)), and
         b) a C₁₋₆ alkyl group (which is optionally substituted by one or more, same or different substituents selected from the following groups: (i) a hydroxyl group and (ii) a C₁₋₆ alkoxy group)), or
      (c) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (d) an aryl group (which is optionally substituted by one or more, same or different substituents selected from the following (1) to (3): (1) a halogen atom, (2) a C₁₋₆ alkyl group (which is optionally substituted by one or more, same or different halogen atoms), and (3) a C₁₋₆ alkoxy group), or
      (e) a carboxyl group,
   4) a C₁₋₆ alkyl group (which is substituted by one or more, same or different substituents selected from the following (a) to (d):
      (a) a carboxyl group,
      (b) a cycloalkyl group (which is optionally substituted by one or more, same or different -CO-NR³²R³³ wherein R³² and R³³ are the same or different and each is a hydrogen atom, a C₁₋₆ alkyl group (which is optionally substituted by one or more, same or different substituents selected from the following groups: (i) a hydroxyl group and (ii) a C₁₋₆ alkoxy group), or R³² and R³³ optionally form, together with the nitrogen atom bonded thereto, a saturated monocyclic nitrogen-containing heterocyclic group),
      (c) -CO-NR³⁴R³⁵ wherein R³⁴ and R³⁵ are the same or different and each is a hydrogen atom or a C₁₋₆ alkyl group, or R³⁴ and R³⁵ optionally form, together with the nitrogen atom bonded thereto, a saturated monocyclic nitrogen-containing heterocyclic group, and
      (d) an aryl group (which is optionally substituted by one or more, same or different substituents selected from the following groups: a) a halogen atom and b) a C₁₋₆ alkyl group (which is optionally substituted by one or more, same or different halogen atoms))),
   5) a cycloalkyl group,
   6) -S(=O)₂-R¹² wherein R¹² is a C₁₋₆ alkyl group (which is substituted by one or more, same or different halogen atoms), or an aryl group (which is optionally substituted by one or more, same or different substituents selected from the following groups: a) a halogen atom and b) a C₁₋₆ alkyl group (which is optionally substituted by one or more, same or different halogen atoms)),
   7) -C(=NCN)-R¹³ wherein R¹³ is a C₁₋₆ alkyl group,
   8) -C(=NCN)NR¹⁴R¹⁵ wherein R¹⁴ and R¹⁵ are the same or different and each is a hydrogen atom or a C₁₋₆ alkyl group, or R¹⁴ and R¹⁵ optionally form, together with the nitrogen atom bonded thereto, a saturated monocyclic nitrogen-containing heterocyclic group, or
   9) an unsaturated monocyclic 5-membered or 6-membered heterocyclic group (which is substituted by one or more, same or different substituents selected from the following (a) to (g):
      (a) a C₁₋₆ alkyl group (which is substituted by one or more, same or different substituents selected from the following a) to c):
         a) a halogen atom,
         b) a hydroxyl group, and
         c) a C₁₋₆ alkoxy group),
      (b) a cycloalkyl group,
      (c) a halogen atom,
      (d) -CO-NR⁴⁵R⁴⁶ wherein R⁴⁵ and R⁴⁶ are the same or different and each is a hydrogen atom or a C₁₋₆ alkyl group, or R⁴⁵ and R⁴⁶ optionally form, together with the nitrogen atom bonded thereto, a saturated monocyclic nitrogen-containing heterocyclic group,
      (e) a -COO-C₁₋₆ alkyl group,
      (f) a cyano group, and
      (g) a C₁₋₆ alkoxy group,
or a salt thereof.

9. The compound of any one of the above-mentioned 1. to 8., wherein R² is a cyclopropyl group or a 1-methylcyclopropyl group, or a salt thereof.

10. The compound of any one of the above-mentioned 1. to 9., wherein
R³ and R⁴ are the same or different and each is
(1) a hydrogen atom,
(2) a C₁₋₆ alkyl group (which is substituted by one or more, same or different substituents selected from the following group: 1) a halogen atom, and 2) a -OCO-C₁₋₆ alkyl group),
(3) an aryl group (which is substituted by one or more, same or different substituents selected from the following 1) to 5) :
   1) a C₁₋₆ alkyl group (which is substituted by one or more, same or different substituents selected from the following group: (a) a halogen atom, and (b) a hydroxyl group),
   2) a C₁₋₆ alkoxy group (which is substituted by one or more, same or different halogen atoms),
   3) a carboxyl group,
   4) a -COO-C₁₋₆ alkyl group, and
   5) a cyano group), or
(4) an unsaturated monocyclic 5-membered or 6-membered heterocyclic group (which is substituted by one or more, same or different substituents selected from the following group: 1) a halogen atom, and 2) a C₁₋₆ alkyl group (which is optionally substituted by one or more, same or different halogen atoms)),
(provided that R³ and R⁴ are not hydrogen atoms at the same time),
or a salt thereof.

11. The compound of the above-mentioned 1., wherein R¹ is
   1) -CONR⁵R⁶ wherein R⁵ and R⁶ are the same or different and each is
      (a) a hydrogen atom,
      (b) an aryl group (which is substituted by one or more, same or different substituents selected from the following group:
         a) a hydroxyl group,
         b) a halogen atom,
         c) a carboxyl group,
         d) a C₁₋₆ alkyl group (which is optionally substituted by one or more, same or different substituents selected from the following groups: (i) a halogen atom, (ii) a hydroxyl group, and (iii) a C₁₋₆ alkoxy group), and
         e) a C₁₋₆ alkoxy group),
      (c) a cycloalkyl group (which is optionally substituted by one or more, same or different C₁₋₆ alkyl groups optionally substituted by one or more, same or different substituents selected from the following groups: (i) a hydroxyl group and (ii) a C₁₋₆ alkoxy group),
      (d) a C₁₋₆ alkyl group (which is substituted by one or more, same or different substituents selected from the following groups:
         a) a hydroxyl group,
         b) a halogen atom,
         c) an aryl group (which is optionally substituted by one or more, same or different substituents selected from the following (1) and (2): (1) a halogen atom, and (2) a C₁₋₆ alkyl group (which is optionally substituted by one or more, same or different halogen atoms)),
         d) -NR²⁰R²¹ wherein R²⁰ and R²¹ are the same or different and each is a hydrogen atom, a C₁₋₆ alkyl group, or a -CO-C₁₋₆ alkyl group, or R²⁰ and R²¹ optionally form, together with the nitrogen atom bonded thereto, a saturated monocyclic nitrogen-containing heterocyclic group (which is optionally substituted by one or more, same or different substituents selected from the following (i) to (iv):
            (i) a halogen atom,
            (ii) a hydroxyl group,
            (iii) an oxo group, and
            (iv) a C₁₋₆ alkoxy group)),
         e) a C₁₋₆ alkoxy group, and
         f) a carboxyl group),
      (e) -S(=O)₂-R⁹ wherein R⁹ is an aryl group (which is optionally substituted by one or more, same or different substituents selected from the following (1) and (2): (1) a halogen atom, and (2) a C₁₋₆ alkyl group optionally substituted by one or more, same or different halogen atoms), or a C₁₋₆ alkyl group), or
      (f) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (g) a C₁₋₆ alkoxy group, or
      (h) s monocyclic nitrogen-containing unsaturated heterocyclic group (which is optionally substituted by one or more, same or different halogen atoms), or
      (i) R⁵ and R⁶ optionally form, together with the nitrogen atom bonded thereto, a saturated monocyclic nitrogen-containing heterocyclic group (which is optionally substituted by one or more, same or different substituents selected from the following a) to i):
         a) a halogen atom,
         b) a hydroxyl group,
         c) a C₁₋₆ alkyl group (which is optionally substituted by one or more, same or different substituents selected from the following group: (i) a halogen atom, (ii) a hydroxyl group, and (iii) a C₁₋₆ alkoxy group),
         d) a carboxyl group,
         e) a -CO-C₁₋₆ alkyl group,
         f) -CO-NR²²R²³ wherein R²² and R²³ are the same or different and each is a hydrogen atom, or a C₁₋₆ alkyl group, or R²² and R²³ optionally form, together with the nitrogen atom bonded thereto, a saturated monocyclic nitrogen-containing heterocyclic group),
         g) an oxo group,
         h) -NR²⁴R²⁵ wherein R²⁴ and R²⁵ are the same or different and each is a hydrogen atom, a C₁₋₆ alkyl group, or a -CO-C₁₋₆ alkyl group, or R²⁴ and R²⁵ optionally form, together with the nitrogen atom bonded thereto, a saturated monocyclic nitrogen-containing heterocyclic group), and
         i) a C₁₋₆ alkoxy group) (provided that R⁵ and R⁶ are not hydrogen atoms at the same time)),
   2) -COOR¹⁰ wherein R¹⁰ is
      (a) a C₁₋₆ alkyl group (which is substituted by one or more, same or different substituents selected from the following a) to d):
         a) a hydroxyl group,
         b) -NR²⁶R²⁷ wherein R²⁶ and R²⁷ are the same or different and each is a hydrogen atom, or a C₁₋₆ alkyl group, or R²⁶ and R²⁷ optionally form, together with the nitrogen atom bonded thereto, a saturated monocyclic nitrogen-containing heterocyclic group),
         c) a C₁₋₆ alkoxy group, and
         d) an aryl group), or
      (b) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group)),
   3) -COR¹¹ wherein R¹¹ is
      (a) a C₁₋₆ alkyl group (which is substituted by one or more, same or different substituents selected from the following a) to h):
         a) a halogen atom,
         b) a hydroxyl group,
         c) -NR²⁸R²⁹ wherein R²⁸ and R²⁹ are the same or different and each is a hydrogen atom, a C₁₋₆ alkyl group, a -CO-C₁₋₆ alkyl group, a -CO-cycloalkyl group, or an -S(=O)₂-C₁₋₆ alkyl group, or R²⁸ and R²⁹ optionally form, together with the nitrogen atom bonded thereto, a saturated monocyclic nitrogen-containing heterocyclic group),
         d) -CO-NR³⁰R³¹ wherein R³⁰ and R³¹ are the same or different and each is a hydrogen atom, or a C₁₋₆ alkyl group, or R³⁰ and R³¹ optionally form, together with the nitrogen atom bonded thereto, a saturated monocyclic nitrogen-containing heterocyclic group),
         e) a C₁₋₆ alkoxy group,
         f) an aryl group (which is optionally substituted by one or more, same or different substituents selected from the following groups: (i) a halogen atom, (ii) a hydroxyl group, (iii) a C₁₋₆ alkoxy group, and (iv) a C₁₋₆ alkyl group (which is optionally substituted by one or more, same or different halogen atoms)),
         g) a carboxyl group, and
         h) an aryloxy group),
      (b) a cycloalkyl group (which is optionally substituted by one or more, same or different substituents selected from the following a) and b):
         a) an aryl group (which is optionally substituted by one or more, same or different substituents selected from the following (1) and (2): (1) a halogen atom, and (2) a C₁₋₆ alkyl group (which is optionally substituted by one or more, same or different halogen atoms)), and
         b) a C₁₋₆ alkyl group (which is optionally substituted by one or more, same or different substituents selected from the following group: (i) a hydroxyl group, and (ii) a C₁₋₆ alkoxy group)), or
      (c) a saturated monocyclic nitrogen-containing heterocyclic group (which is optionally substituted by one or more, same or different substituents selected from the following a) to c):
         a) a C₁₋₆ alkyl group,
         b) a -CO-C₁₋₆ alkyl group, and
         c) an oxo group),
      (d) an aryl group (which is optionally substituted by one or more, same or different substituents selected from the following (1) to (3): (1) a halogen atom, (2) a C₁₋₆ alkyl group (which is optionally substituted by one or more, same or different halogen atoms), and (3) a C₁₋₆ alkoxy group), or
      (e) a carboxyl group),
   4) a C₁₋₆ alkyl group (which is substituted by one or more, same or different substituents selected from the following (a) to (d):
      (a) a carboxyl group,
      (b) a cycloalkyl group (which is optionally substituted by one or more, same or different -CO-NR³²R³³ wherein R³² and R³³ are the same or different and each is a hydrogen atom, a C₁₋₆ alkyl group (which is optionally substituted by one or more, same or different substituents selected from the following groups: (i) a hydroxyl group, and (ii) a C₁₋₆ alkoxy group), or R³² and R³³ optionally form, together with the nitrogen atom bonded thereto, a saturated monocyclic nitrogen-containing heterocyclic group)),
      (c) -CO-NR³⁴R³⁵ wherein R³⁴ and R³⁵ are the same or different and each is a hydrogen atom, or a C₁₋₆ alkyl group, or R³⁴ and R³⁵ optionally form, together with the nitrogen atom bonded thereto, a saturated monocyclic nitrogen-containing heterocyclic group), and
      (d) an aryl group (which is optionally substituted by one or more, same or different substituents selected from the following groups: a) a halogen atom, and b) a C₁₋₆ alkyl group (which is optionally substituted by one or more, same or different halogen atoms))),
   5) a cycloalkyl group,
   6) -S(=O)₂-R¹² wherein R¹² is a C₁₋₆ alkyl group (which is substituted by one or more, same or different halogen atoms), or an aryl group (which is optionally substituted by one or more, same or different substituents selected from the following groups: a) a halogen atom, and b) a C₁₋₆ alkyl group (which is optionally substituted by one or more, same or different halogen atoms))),
   7) -C(=NCN)-R¹³ wherein R¹³ is a C₁₋₆ alkyl group),
   8) -C(=NCN)NR¹⁴R¹⁵ wherein R¹⁴ and R¹⁵ are the same or different and each is a hydrogen atom, or a C₁₋₆ alkyl group, or R¹⁴ and R¹⁵ optionally form, together with the nitrogen atom bonded thereto, a saturated monocyclic nitrogen-containing heterocyclic group),
   9) an aryl group (which is optionally substituted by one or more, same or different substituents selected from the following (a) to (f):
      (a) a carboxyl group,
      (b) a halogen atom,
      (c) a C₁₋₆ alkyl group (which is optionally substituted by one or more, same or different substituents selected from the following groups: a) a hydroxyl group, b) a halogen atom, and c) a C₁₋₆ alkoxy group),
      (d) -NR³⁸R³⁹ wherein R³⁸ and R³⁹ are the same or different and each is a hydrogen atom, a C₁₋₆ alkyl group, a -CO-C₁₋₆ alkyl group, -CO-NR⁴⁰R⁴¹ wherein R⁴⁰ and R⁴¹ are the same or different and each is a hydrogen atom, a C₁₋₆ alkyl group, or a C₁₋₆ alkoxy group, or R⁴⁰ and R⁴¹ optionally form, together with the nitrogen atom bonded thereto, a saturated monocyclic nitrogen-containing heterocyclic group), or -S(=O)₂-R⁴² wherein R⁴² is a C₁₋₆ alkyl group, or R³⁸ and R³⁹ optionally form, together with the nitrogen atom bonded thereto, a saturated monocyclic nitrogen-containing heterocyclic group (which is optionally substituted by one or more oxo groups),
      (e) -CO-NR⁴³R⁴⁴ wherein R⁴³ and R⁴⁴ are the same or different and each is a hydrogen atom or a C₁₋₆ alkyl group, or R⁴³ and R⁴⁴ optionally form, together with the nitrogen atom bonded thereto, a saturated monocyclic nitrogen-containing heterocyclic group, and
      (f) a -COO-C₁₋₆ alkyl group), or
   10) an unsaturated monocyclic 5-membered or 6-membered heterocyclic group (which is substituted by one or more, same or different substituents selected from the following (a) to (h):
      (a) a carboxyl group,
      (b) a C₁₋₆ alkyl group (which is optionally substituted by one or more, same or different substituents selected from the following a) to c):
         a) a halogen atom,
         b) a hydroxyl group, and
         c) a C₁₋₆ alkoxy group),
      (c) a cycloalkyl group,
      (d) a halogen atom,
      (e) -CO-NR⁴⁵R⁴⁶ wherein R⁴⁵ and R⁴⁶ are the same or different and each is a hydrogen atom or a C₁₋₆ alkyl group, or R⁴⁵ and R⁴⁶ optionally form, together with the nitrogen atom bonded thereto, a saturated monocyclic nitrogen-containing heterocyclic group,
      (f) a -COO-C₁₋₆ alkyl group,
      (g) a cyano group, and
      (h) a C₁₋₆ alkoxy group),
   or a salt thereof.

12. The compound of the above-mentioned 1. or a salt thereof, which is selected from the following:
   (1) 1-[5-cyclopropyl-1-(piperidin-4-yl)-1H-pyrazole-4-carbonyl]-3-(pyridin-3-yl)pyrrolidine,
   (2) 1-[5-cyclopropyl-1-(piperidin-4-yl)-1H-pyrazole-4-carbonyl]-[(S)-3-(2-trifluoromethylphenyl)]pyrrolidine hydrochloride,
   (3) 1-{5-cyclopropyl-1-[1-(2-fluorophenylcarbamoyl)piperidin-4-yl]-1H-pyrazole-4-carbonyl}-3-(pyridin-3-yl)pyrrolidine,
   (4) 1-[1-(1-carbamoylpiperidin-4-yl)-5-cyclopropyl-1H-pyrazole-4-carbonyl]-[(S)-3-(2-trifluoromethylphenyl)]pyrrolidine,
   (5) 1-{1-[1-(2-carboxyphenylcarbamoyl)piperidin-4-yl]-5-cyclopropyl-1H-pyrazole-4-carbonyl}-[(S)-3-(2-trifluoromethylphenyl)]pyrrolidine,
   (6) 1-{5-cyclopropyl-1-[1-(2-hydroxymethylphenylcarbamoyl)piperidin-4-yl]-1H-pyrazole-4-carbonyl}-[(S)-3-(2-trifluoromethylphenyl)]pyrrolidine,
   (7) 1-{5-cyclopropyl-1-[1-(1-hydroxymethylcyclopropylcarbamoyl)piperidin-4-yl]-1H-pyrazole-4-carbonyl}-[(S)-3-(2-trifluoromethylphenyl)]pyrrolidine,
   (8) 1-{5-cyclopropyl-1-[1-(2-hydroxyethylcarbamoyl)piperidin-4-yl]-1H-pyrazole-4-carbonyl}-[(S)-3-(2-trifluoromethylphenyl)]pyrrolidine,
   (9) 1-{5-cyclopropyl-1-[1-(2-hydroxy-1,1-dimethylethylcarbamoyl)piperidin-4-yl]-1H-pyrazole-4-carbonyl}-[(S)-3-(2-trifluoromethylphenyl)]pyrrolidine,
   (10) 1-{1-[1-(2-acetylaminoethylcarbamoyl)piperidin-4-yl]-5-cyclopropyl-1H-pyrazole-4-carbonyl}-[(S)-3-(2-trifluoromethylphenyl)]pyrrolidine,
   (11) 1-{1-[1-(2-aminoethylcarbamoyl)piperidin-4-yl]-5-cyclopropyl-1H-pyrazole-4-carbonyl}-[(S)-3-(2-trifluoromethylphenyl)]pyrrolidine hydrochloride,
   (12) 1-{5-cyclopropyl-1-[1-(1,1-dioxothiomorpholine-4-carbonyl)piperidin-4-yl]-1H-pyrazole-4-carbonyl}-[(S)-3-(2-trifluoromethylphenyl)]pyrrolidine,
   (13) 1-{5-cyclopropyl-1-[1-(2-dimethylaminoethylcarbamoyl)piperidin-4-yl]-1H-pyrazole-4-carbonyl}-[(S)-3-(2-trifluoromethylphenyl)]pyrrolidine hydrochloride,
   (14) 1-{5-cyclopropyl-1-[1-(4-hydroxypiperidine-1-carbonyl)piperidin-4-y1]-1H-pyrazole-4-carbonyl}-[(S)-3-(2-trifluoromethylphenyl)]pyrrolidine,
   (15) 1-{1-[1-(azetidine-1-carbonyl)piperidin-4-yl]-5-cyclopropyl-1H-pyrazole-4-carbonyl}-[(S)-3-(2-trifluoromethylphenyl)]pyrrolidine,
   (16) 1-{5-cyclopropyl-1-[1-(3-hydroxypyrrolidine-1-carbonyl)piperidin-4-yl]-1H-pyrazole-4-carbonyl}-[(S)-3-(2-trifluoromethylphenyl)]pyrrolidine,
   (17) 1-{5-cyclopropyl-1-[1-(2-piperidin-1-yl-ethylcarbamoyl)piperidin-4-yl]-1H-pyrazole-4-carbonyl}-[(S)-3-(2-trifluoromethylphenyl)]pyrrolidine hydrochloride,
   (18) 1-{5-cyclopropyl-1-[1-(4,4-difluoropiperidine-1-carbonyl)piperidin-4-yl]-1H-pyrazole-4-carbonyl}-[(S)-3-(2-trifluoromethylphenyl)]pyrrolidine,
   (19) 1-{5-cyclopropyl-1-[1-(3,3-difluoropyrrolidine-1-carbonyl)piperidin-4-yl]-1H-pyrazole-4-carbonyl}-[(S)-3-(2-trifluoromethylphenyl)]pyrrolidine,
   (20) 1-{5-cyclopropyl-1-[1-(3-hydroxyazetidine-1-carbonyl)piperidin-4-yl]-1H-pyrazole-4-carbonyl}-[(S)-3-(2-trifluoromethylphenyl)]pyrrolidine,
   (21) 1-(5-cyclopropyl-1-{1-[(R)-3-hydroxypyrrolidine-1-carbonyl]piperidin-4-yl}-1H-pyrazole-4-carbonyl)-[(S)-3-(2-trifluoromethylphenyl)]pyrrolidine,
   (22) 1-(5-cyclopropyl-1-{1-[(S)-3-hydroxypyrrolidine-1-carbonyl]piperidin-4-yl}-1H-pyrazole-4-carbonyl)-[(S)-3-(2-trifluoromethylphenyl)]pyrrolidine,
   (23) 1-(5-cyclopropyl-1-{1-[(S)-2-hydroxy-1-methylethylcarbamoyl]piperidin-4-yl}-1H-pyrazole-4-carbonyl)-[(S)-3-(2-trifluoromethylphenyl)]pyrrolidine,
   (24) 1-{5-cyclopropyl-1-[1-(4-hydroxymethylpiperidine-1-carbonyl)piperidin-4-yl]-1H-pyrazole-4-carbonyl}-[(S)-3-(2-trifluoromethylphenyl)]pyrrolidine,
   (25) 1-{1-[1-(4-carboxypiperidine-1-carbonyl)piperidin-4-yl]-5-cyclopropyl-1H-pyrazole-4-carbonyl}-[(S)-3-(2-trifluoromethylphenyl)]pyrrolidine,
   (26) 1-{5-cyclopropyl-1-[1-(3-oxopiperazine-1-carbonyl)piperidin-4-yl]-1H-pyrazole-4-carbonyl}-[(S)-3-(2-trifluoromethylphenyl)]pyrrolidine,
   (27) 1-(5-cyclopropyl-1-{1-[(S)-2-hydroxymethylpyrrolidine-1-carbonyl]piperidin-4-yl}-1H-pyrazole-4-carbonyl)-[(S)-3-(2-trifluoromethylphenyl)]pyrrolidine,
   (28) 1-{5-cyclopropyl-1-[1-(3-hydroxymethylazetidine-1-carbonyl)piperidin-4-yl]-1H-pyrazole-4-carbonyl}-[(S)-3-(2-trifluoromethylphenyl)]pyrrolidine,
   (29) 1-{5-cyclopropyl-1-[1-(4-methylpiperazine-1-carbonyl)piperidin-4-yl]-1H-pyrazole-4-carbonyl}-[(S)-3-(2-trifluoromethylphenyl)]pyrrolidine hydrochloride,
   (30) 1-{5-cyclopropyl-1-[1-(4-isopropylpiperazine-1-carbonyl)piperidin-4-yl]-1H-pyrazole-4-carbonyl}-[(S)-3-(2-trifluoromethylphenyl)]pyrrolidine hydrochloride,
   (31) 1-{1-[1-(4-acetylpiperazine-1-carbonyl)piperidin-4-yl]-5-cyclopropyl-1H-pyrazole-4-carbonyl}-[(S)-3-(2-trifluoromethylphenyl)]pyrrolidine,
   (32) 1-(5-cyclopropyl-1-{1-[(R)-3-hydroxypiperidine-1-carbonyl]piperidin-4-yl}-1H-pyrazole-4-carbonyl)-[(S)-3-(2-trifluoromethylphenyl)]pyrrolidine;
   (33) 1-{1-[1-(4-carbamoylpiperidine-1-carbonyl)piperidin-4-yl]-5-cyclopropyl-1H-pyrazole-4-carbonyl}-[(S)-3-(2-trifluoromethylphenyl)]pyrrolidine,
   (34) 1-{1-[1-(3-carbamoylazetidine-1-carbonyl)piperidin-4-yl]-5-cyclopropyl-1H-pyrazole-4-carbonyl}-[(S)-3-(2-trifluoromethylphenyl)]pyrrolidine,
   (35) 1-{1-[1-(4-aminopiperidine-1-carbonyl)piperidin-4-yl]-5-cyclopropyl-1H-pyrazole-4-carbonyl}-[(S)-3-(2-trifluoromethylphenyl)]pyrrolidine hydrochloride,
   (36) 1-{1-[1-(3-aminopyrrolidine-1-carbonyl)piperidin-4-yl]-5-cyclopropyl-1H-pyrazole-4-carbonyl}-[(S)-3-(2-trifluoromethylphenyl)]pyrrolidine hydrochloride,
   (37) 1-{5-cyclopropyl-1-[1-(piperidin-4-yl-carbamoyl)piperidin-4-yl]-1H-pyrazole-4-carbonyl}-[(S)-3-(2-trifluoromethylphenyl)]pyrrolidine hydrochloride,
   (38) 1-{5-cyclopropyl-1-[1-(4-dimethylaminopiperidine-1-carbonyl)piperidin-4-yl]-1H-pyrazole-4-carbonyl}-[(S)-3-(2-trifluoromethylphenyl)]pyrrolidine hydrochloride,
   (39) 1-{1-[1-(4-acetylaminopiperidine-1-carbonyl)piperidin-4-yl]-5-cyclopropyl-1H-pyrazole-4-carbonyl}-[(S)-3-(2-trifluoromethylphenyl)]pyrrolidine,
   (40) 1-{1-[1-(3-acetylaminopyrrolidine-1-carbonyl)piperidin-4-yl]-5-cyclopropyl-1H-pyrazole-4-carbonyl}-[(S)-3-(2-trifluoromethylphenyl)]pyrrolidine,
   (41) 1-{5-cyclopropyl-1-[1-(3-dimethylaminopyrrolidine-1-carbonyl)piperidin-4-yl]-1H-pyrazole-4-carbonyl}-[(S)-3-(2-trifluoromethylphenyl)]pyrrolidine hydrochloride,
   (42) 1-{5-cyclopropyl-1-[1-(1-methylpiperidin-4-yl-carbamoyl)piperidin-4-yl]-1H-pyrazole-4-carbonyl}-[(S)-3-(2-trifluoromethylphenyl)]pyrrolidine hydrochloride,
   (43) 1-{1-[1-(1-acetylpiperidin-4-yl-carbamoyl)piperidin-4-yl]-5-cyclopropyl-1H-pyrazole-4-carbonyl}-[(S)-3-(2-trifluoromethylphenyl)]pyrrolidine,
   (44) 1-{5-cyclopropyl-1-[1-(4-oxopiperidine-1-carbonyl)piperidin-4-yl]-1H-pyrazole-4-carbonyl}-[(S)-3-(2-trifluoromethylphenyl)]pyrrolidine,
   (45) 1-{1-[1-(3-acetylaminoazetidine-1-carbonyl)piperidin-4-yl]-5-cyclopropyl-1H-pyrazole-4-carbonyl}-[(S)-3-(2-trifluoromethylphenyl)]pyrrolidine,
   (46) 1-{5-cyclopropyl-1-[1-(3-oxopyrrolidine-1-carbonyl)piperidin-4-yl]-1H-pyrazole-4-carbonyl}-[(S)-3-(2-trifluoromethylphenyl)]pyrrolidine,
   (47) 1-{5-cyclopropyl-1-[1-(2,2,2-trifluoroethylcarbamoyl)piperidin-4-yl]-1H-pyrazole-4-carbonyl}-[(S)-3-(2-trifluoromethylphenyl)]pyrrolidine,
   (48) 1-{5-cyclopropyl-1-[1-(3,3,4,4-tetrafluoropyrrolidine-1-carbonyl)piperidin-4-yl]-1H-pyrazole-4-carbonyl}-[(S)-3-(2-trifiuoromethylphenyl)]pyrrolidine,
   (49) 1-(5-cyclopropyl-1-{1-[(S)-1-hydroxymethyl-2-methylpropylcarbamoyl]piperidin-4-yl}-1H-pyrazole-4-carbonyl)-[(S)-3-(2-trzfluoromethylphenyl)]pyrrolidine,
   (50) 1-(1-{1-[(S)-1-benzyl-2-hydroxyethylcarbamoyl]piperidin-4-yl}-5-cyclopropyl-1H-pyrazole-4-carbonyl)-[(S)-3-(2-trifluoromethylphenyl)]pyrrolidine,
   (51) 1-(5-cyclopropyl-1-{1-[(S)-2-hydroxy-1-phenylethylcarbamoyl]piperidin-4-yl}-1H-pyrazole-4-carbonyl)-[(S)-3-(2-trifluoromethylphenyl)]pyrrolidine,
   (52) 1-(5-cyclopropyl-1-{1-[(S)-1-hydroxymethyl-3-methylbutylcarbamoyl]piperidin-4-yl}-1H-pyrazole-4-carbonyl)-[(S)-3-(2-trifluoromethylphenyl)]pyrrolidine,
   (53) 1-{5-cyclopropyl-1-[1-(2-hydroxyphenylcarbamoyl)piperidin-4-yl]-1H-pyrazole-4-carbonyl}-[(S)-3-(2-trifluoromethylphenyl)]pyrrolidine,
   (54) 1-{5-cyclopropyl-1-[1-(1-hydroxymethylcyclopentylcarbamoyl)piperidin-4-yl]-1H-pyrazole-4-carbonyl}-[(S)-3-(2-trifluoromethylphenyl)]pyrrolidine,
   (55) 1-[1-(1-benzenesulfonylaminocarbonylpiperidin-4-yl)-5-cyclopropyl-1H-pyrazole-4-carbonyl]-[(S)-3-(2-trifluoromethylphenyl)]pyrrolidine,
   (56) 1-[5-cyclopropyl-1-(1-methanesulfonylaminocarbonylpiperidin-4-yl)-1H-pyrazole-4-carbonyl]-[(S)-3-(2-trifluoromethylphenyl)]pyrrolidine,
   (57) 1-[5-cyclopropyl-1-(1-methoxycarbonylpiperidin-4-yl)-1H-pyrazole-4-carbonyl]-[(S)-3-(2-trifluoromethylphenyl)]pyrrolidine,
   (58) 1-{5-cyclopropyl-1-[1-(2-hydroxyethoxycarbonyl)piperidin-4-yl]-1H-pyrazole-4-carbonyl}-[(S)-3-(2-trifluoromethylphenyl)]pyrrolidine,
   (59) 1-{5-cyclopropyl-1-[1-(2-dimethylaminoethoxycarbonyl)piperidin-4-yl]-1H-pyrazole-4-carbonyl}-[(S)-3-(2-trifluoromethylphenyl)]pyrrolidine hydrochloride,
   (60) 1-{5-cyclopropyl-1-[1-(2-piperidin-1-yl-ethoxycarbonyl)piperidin-4-yl]-1H-pyrazole-4-carbonyl}-[(S)-3-(2-trifluoromethylphenyl)]pyrrolidine hydrochloride,
   (61) 1-{5-cyclopropyl-1-[1-(piperidin-4-yl-oxycarbonyl)piperidin-4-yl]-1H-pyrazole-4-carbonyl}-[(S)-3-(2-trifluoromethylphenyl)]pyrrolidine hydrochloride,
   (62) 1-{5-cyclopropyl-1-[1-(1-methylpiperidin-4-yl-oxycarbonyl)piperidin-4-yl]-1H-pyrazole-4-carbonyl}-[(S)-3-(2-trifluoromethylphenyl)]pyrrolidine hydrochloride,
   (63) 1-{1-[1-(1-acetylpiperidin-4-yl-oxycarbonyl)piperidin-4-yl]-5-cyclopropyl-1H-pyrazole-4-carbonyl}-[(S)-3-(2-trifluoromethylphenyl)]pyrrolidine,
   (64) 1-[1-(1-cyclopropanecarbonylpiperidin-4-yl)-5-cyclopropyl-1H-pyrazole-4-carbonyl]-[(S)-3-(2-trifluoromethylphenyl)]pyrrolidine,
   (65) 1-{5-cyclopropyl-1-[1-(2-hydroxyacetyl)piperidin-4-yl]-1H-pyrazole-4-carbonyl}-[(S)-3-(2-trifluoromethylphenyl)]pyrrolidine,
   (66) 1-(5-cyclopropyl-1-{1-[1-(4-fluorophenyl)cyclopropanecarbonyl]piperidin-4-yl}-1H-pyrazole-4-carbonyl)-[(S)-3-(2-trifluoromethylphenyl)]pyrrolidine,
   (67) 1-{5-cyclopropyl-1-[1-(2-dimethylaminoacetyl)piperidin-4-yl]-1H-pyrazole-4-carbonyl}-[(S)-3-(2-trifluoromethylphenyl)]pyrrolidine hydrochloride,
   (68) 1-{2-[1-(2-acetylaminoacetyl)piperidin-4-yl]-5-cyclopropyl-1H-pyrazole-4-carbonyl}-[(S)-3-(2-trifluoromethylphenyl)]pyrrolidine,
   (69) 1-{5-cyclopropyl-1-[1-(1-methylcyclopropanecarbonyl)piperidin-4-yl]-1H-pyrazole-4-carbonyl}-[(S)-3-(2-trifluoromethylphenyl)]pyrrolidine,
   (70) 1-{1-[1-(2-acetylamino-2-methylpropionyl)piperidin-4-yl]-5-cyclopropyl-1H-pyrazole-4-carbonyl}-[(S)-3-(2-trifluoromethylphenyl)]pyrrolidine,
   (71) 1-(1-{1-[(S)-2-acetylaminopropionyl]piperidin-4-yl}-5-cyclopropyl-1H-pyrazole-4-carbonyl)-[(S)-3-(2-trifluoromethylphenyl)]pyrrolidine,
   (72) 1-(1-{1-[(S)-2-acetylamino-3-methylbutyryl]piperidin-4-yl}-5-cyclopropyl-1H-pyrazole-4-carbonyl)-[(S)-3-(2-trifluoromethylphenyl)]pyrrolidine,
   (73) 2-{5-cyclopropyl-1-[1-(3,3,3-trifluoropropionyl)piperidin-4-yl]-1H-pyrazole-4-carbonyl}-[(S)-3-(2-trifluoromethylphenyl)]pyrrolidine,
   (74) 1-(5-cyclopropyl-1-{1-[(S)-5-oxopyrrolidine-2-carbonyl]piperidin-4-yl}-1H-pyrazole-4--carbonyl)-[(S)-3-(2-trifluoromethylphenyl)]pyrrolidine,
   (75) 1-{1-[1-(3-acetylaminopropionyl)piperidin-4-yl]-5-cyclopropyl-1H-pyrazole-4-carbonyl}-[(S)-3-(2-trifluoromethylphenyl)]pyrrolidine,
   (76) 1-{5-cyclopropyl-1-[1-(3-hydroxy-2,2-dimethylpropionyl)piperidin-4-yl]-1H-pyrazole-4-carbonyl}-[(S)-3-(2-trifluoromethylphenyl)]pyrrolidine,
   (77) 1-{1-[1-(2-aminoacetyl)piperidin-4-yl]-5-cyclopropyl-1H-pyrazole-4-carbonyl}-[(S)-3-(2-trifluoromethylphenyl)]pyrrolidine hydrochloride,
   (78) 1-{5-cyclopropyl-1-[1-(1-hydroxymethylcyclopropanecarbonyl)piperidin-4-yl]-1H-pyrazole-4-carbonyl}-[(S)-3-(2-trifluoromethylphenyl)]pyrrolidine,
   (79) 1-{1-[1-(2-amino-2-methylpropionyl)piperidin-4-yl]-5-cyclopropyl-1H-pyrazole-4-carbonyl}-[(S)-3-(2-trifluoromethylphenyl)]pyrrolidine hydrochloride,
   (80) 1-{5-cyclopropyl-1-[1-(4-hydroxybutyryl)piperidin-4-yl]-1H-pyrazole-4-carbonyl}-[(S)-3-(2-trifluoromethylphenyl)]pyrrolidine,
   (81) 1-(5-cyclopropyl-1-{1-[(S)-pyrrolidine-2-carbonyl]piperidin-4-yl}-1H-pyrazole-4-carbonyl)-[(S)-3-(2-trifluoromethylphenyl)]pyrrolidine hydrochloride,
   (82) 1-(5-cyclopropyl-1-{1-[(S)-1-methylpyrrolidine-2-carbonyl]piperidin-4-yl}-1H-pyrazole-4-carbonyl)-[(S)-3-(2-trifluoromethylphenyl)]pyrrolidine hydrochloride,
   (83) 1-{1-[1-(3-aminopropionyl)piperidin-4-yl]-5-cyclopropyl-1H-pyrazole-4-carbonyl}-[(S)-3-(2-trifluoromethylphenyl)]pyrrolidine hydrochloride,
   (84) 1-(1-{1-[(S)-2-amino-3-methylbutyryl]piperidin-4-yl}-5-cyclopropyl-1H-pyrazole-4-carbonyl)-[(S)-3-(2-trifluoromethylphenyl)]pyrrolidine hydrochloride,
   (85) 1-{5-cyclopropyl-1-[1-(2-methylaminoacetyl)piperidin-4-yl]-1H-pyrazole-4-carbonyl}-[(S)-3-(2-trifluoromethylphenyl)]pyrrolidine hydrochloride,
   (86) 1-{5-cyclopropyl-1-[1-(piperidine-4-carbonyl)piperidin-4-y1]-1H-pyrazole-4-carbonyl}-[(S)-3-(2-trifluoromethylphenyl)]pyrrolidine hydrochloride,
   (87) 1-{5-cyclopropyl-1-[1-(2-isobutyrylaminoacetyl)piperidin-4-yl]-1H-pyrazole-4-carbonyl}-[(S)-3-(2-trifluoromethylphenyl)]pyrrolidine,
   (88) 1-{1-[1-(2-cyclopropanecarbonylaminoacetyl)piperidin-4-yl]-5-cyclopropyl-1H-pyrazole-4-carbonyl}-[(S)-3-(2-trifluoromethylphenyl)]pyrrolidine,
   (89) 1-(1-{1-[(S)-1-acetylpyrrolidine-2-carbonyl]piperidin-4-yl}-5-cyclopropyl-1H-pyrazole-4-carbonyl)-[(S)-3-(2-trifluoromethylphenyl)]pyrrolidine,
   (90) 1-{5-cyclopropyl-1-[1-(2-methanesulfonylaminoacetyl)piperidin-4-yl]-1H-pyrazole-4-carbonyl}-[(S)-3-(2-trifluoromethylphenyl)]pyrrolidine,
   (91) 1-{1-[1-(1-acetylpiperidine-4-carbonyl)piperidin-4-yl]-5-cyclopropyl-1H-pyrazole-4-carbonyl}-[(S)-3-(2-trifluoromethylphenyl)]pyrrolidine,
   (92) 1-{5-cyclopropyl-1-[1-(1-methylpiperidine-4-carbonyl)piperidin-4-yl]-1H-pyrazole-4-carbonyl}-[(S)-3-(2-trifluoromethylphenyl)]pyrrolidine hydrochloride,
   (93) 1-{1-[1-(3-carbamoylpropionyl)piperidin-4-yl]-5-cyclopropyl-1H-pyrazole-4-carbonyl}-[(S)-3-(2-trifluoromethylphenyl)]pyrrolidine,
   (94) 1-[1-(1-carbamoylmethylpiperidin-4-yl)-5-cyclopropyl-1H-pyrazole-4-carbonyl]-[(S)-3-(2-trifluoromethylphenyl)]pyrrolidine hydrochloride,
   (95) 1-[5-cyclpropyl-1-(1-methylcarbamoylmethylpiperidin-4-yl)-1H-pyrazole-4-carbonyl]-[(S)-3-(2-trifluoromethylphenyl)]pyrrolidine hydrochloride,
   (96) 1-{1-[1-(1-carbamoyl-1-methylethyl)piperidin-4-yl]-5-cyclopropyl-1H-pyrazole-4-carbonyl}-[(S)-3-(2-trifluoromethylphenyl)]pyrrolidine hydrochloride,
   (97) 1-{1-[1-(2-carbamoylethyl)piperidin-4-yl]-5-cyclopropyl-1H-pyrazole-4-carbonyl}-[(S)-3-(2-trifluoromethylphenyl)]pyrrolidine hydrochloride,
   (98) 1-[5-cyclopropyl-1-(1-cyclopropylmethylpiperidin-4-yl)-1H-pyrazole-4-carbonyl]-[(S)-3-(2-trifluoromethylphenyl)]pyrrolidine hydrochloride,
   (99) 1-[5-cyclopropyl-1-(1-cyclopropylpiperidin-4-yl)-1H-pyrazole-4-carbonyl]-[(S)-3-(2-trifluoromethylphenyl)]pyrrolidine hydrochloride,
   (100) 1-[5-cyclopropyl-1-(1-dimethylcarbamoylmethylpiperidin-4-yl)-1H-pyrazole-4-carbonyl]-[(S)-3-(2-trifluoromethylphenyl)]pyrrolidine hydrochloride,
   (101) 1-[1-(1-carboxymethylpiperidin-4-yl)-5-cyclopropyl-1H-pyrazole-4-carbonyl]-[(S)-3-(2-trifluoromethylphenyl)]pyrrolidine hydrochloride,
   (102) 1-[1-(1-carboxyethylpiperidin-4-yl)-5-cyclopropyl-1H-pyrazole-4-carbonyl]-[(S)-3-(2-trifluoromethylphenyl)]pyrrolidine hydrochloride,
   (103) 1-{1-[1-(1-carbamoylethyl)piperidin-4-yl]-5-cyclopropyl-1H-pyrazole-4-carbonyl}-[(S)-3-(2-trifluoromethylphenyl)]pyrrolidine hydrochloride,
   (104) 1-{1-[1-(2-carboxy-2-methylpropyl)piperidin-4-yl]-5-cyclopropyl-1H-pyrazole-4-carbonyl}-[(S)-3-(2-trifluoromethylphenyl)]pyrrolidine hydrochloride,
   (105) 1-{1-[1-(2-carbamoyl-2-methylpropyl)piperidin-4-yl]-5-cyclopropyl-1H-pyrazole-4-carbonyl}-[(S)-3-(2-trifluoromethylphenyl)]pyrrolidine hydrochloride,
   (106) 1-{1-[1-(1-carbamoylcyclopropylmethyl)piperidin-4-yl]-5-cyclopropyl-1H-pyrazole-4-carbonyl}-[(S)-3-(2-trifluoromethylphenyl)]pyrrolidine hydrochloride,
   (107) 1-(5-cyclopropyl-1-{1-[1-(2-hydroxyethylcarbamoyl)cyclopropylmethyl]piperidin-4-yl}-1H-pyrazole-4-carbonyl)-[(S)-3-(2-trifluoromethylphenyl)]pyrrolidine hydrochloride,
   (108) 1-[5-cyclopropyl-1-(1-trifluoromethanesulfonylpiperidin-4-yl)-1H-pyrazole-4-carbonyl]-[(S)-3-(2-trifluoromethylphenyl)]pyrrolidine,
   (109) 1-{5-cyclopropyl-1-[1-(2,2,2-trifluoroethanesulfonyl)piperidin-4-yl]-1H-pyrazole-4-carbonyl}-[(S)-3-(2-trifluoromethylphenyl)]pyrrolidine,
   (110) 1-{1-[1-(1-cyanoiminoethyl)piperidin-4-yl]-5-cyclopropyl-1H-pyrazole-4-carbonyl}-[(S)-3-(2-trifluoromethylphenyl)]pyrrolidine,
   (111) 1-(1-{1-[cyanoimino(methylamino)methyl]piperidin-4-yl}-5-cyclopropyl-1H-pyrazole-4-carbonyl)-[(S)-3-(2-trifluoromethylphenyl)]pyrrolidine,
   (112) 1-{1-[1-(N-cyanocarbamimidoyl)piperidin-4-yl]-5-cyclopropyl-1H-pyrazole-4-carbonyl}-[(S)-3-(2-trifluoromethylphenyl)]pyrrolidine,
   (113) 4-(4-{5-cyclopropyl-4-[3-(2-trifluoromethylphenyl)pyrrolidine-1-carbonyl]pyrazol-1-yl}piperidin-1-yl)benzoic acid,
   (114) 3-(4-{5-cyclopropyl-4-[3-(2-trifluoromethylphenyl)pyrrolidine-1-carbonyl]pyrazol-1-yl}piperidin-1-yl)benzoic acid,
   (115) 5-(4-{5-cyclopropyl-4-[3-(2-trifluoromethylphenyl)pyrrolidine-1-carbonyl]pyrazol-1-yl}piperidin-1-yl)thiophene-2-carboxylic acid,
   (116) 2-(4-{5-cyclopropyl-4-[3-(2-trifluoromethylphenyl)pyrrolidine-1-carbonyl]pyrazol-1-yl}piperidin-1-yl)thiazole-4-carboxylic acid,
   (117) 1-{5-cyclopropyl-1-[1-(5-methyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (118) 1-{5-cyclopropyl-1-[1-(5-cyclopropyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (119) 1-{5-cyclopropyl-1-[1-(5-hydroxymethyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (120) 1-{5-cyclopropyl-1-[1-(5-trifluoromethyl-4H-[1,2,4]triazol-3-yl)piperidin-4-yl]-1H-pyrazole-4-carbonyl}-[(S)-3-(2-trifluoromethylphenyl)]pyrrolidine hydrochloride,
   (121) 1-{5-cyclopropyl-1-[1-(2-fluorophenylcarbamoyl)piperidin-4-yl]-1H-pyrazole-4-carbonyl}-[(R)-3-(2-trifluoromethylphenyl)]pyrrolidine,
   (122) 1-{5-cyclopropyl-1-[1-(1-methylcyclopropylcarbamoyl)piperidin-4-yl]-1H-pyrazole-4-carbonyl}-[(R)-3-(2-trifluoromethylphenyl)]pyrrolidine,
   (123) 1-{5-cyclopropyl-1-[1-(1-isopropylcarbamoyl)piperidin-4-yl]-1H-pyrazole-4-carbonyl}-[(R)-3-(2-trifluoromethylphenyl)]pyrrolidine,
   (124) 1-{5-cyclopropyl-1-[1-(2-fluorophenylcarbamoyl)piperidin-4-yl]-1H-pyrazole-4-carbonyl}-[(S)-3-(2-trifluoromethylphenyl)]pyrrolidine,
   (125) 1-{5-cyclopropyl-1-[1-(1-isopropylcarbamoyl)piperidin-4-yl]-1H-pyrazole-4-carbonyl}-[(S)-3-(2-trifluoromethylphenyl)]pyrrolidine,
   (126) 1-{5-cyclopropyl-1-[1-(1-methylcyclopropylcarbamoyl)piperidin-4-yl]-1H-pyrazole-4-carbonyl}-[(S)-3-(2-trifluoromethylphenyl)]pyrrolidine,
   (127) (-)-3-(4-fluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride,
   (128) (+)-3-(4-fluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride,
   (129) 1-[5-cyclopropyl-1-(1-pyrazin-2-ylpiperidin-4-yl)-1H-pyrazole-4-carbonyl]-[(S)-3-(2-trifluoromethylphenyl)]pyrrolidine hydrochloride,
   (130) 1-[1-(trans-4-carbamoylcyclohexyl)-5-cyclopropyl-1H-pyrazole-4-carbonyl]-[(S)-3-(2-trifluoromethylphenyl)]pyrrolidine,
   (131) 1-[5-cyclopropyl-1-(trans-4-ureidocyclohexyl)-1H-pyrazole-4-carbonyl]-[(S)-3-(2-trifluoromethylphenyl)]pyrrolidine,
   (132) 1-{5-cyclopropyl-1-[trans-4-(1H-tetrazol-5-yl)cyclohexyl]-1H-pyrazole-4-carbonyl}-[(S)-3-(2-trifluoromethylphenyl)]pyrrolidine,
   (133) 1-{5-(1-methylcyclopropyl)-1-[1-(4-trifluoromethylphenylcarbamoyl)piperidin-4-yl]-1H-pyrazole-4-carbonyl}-3-(pyridin-3-yl)pyrrolidine,
   (134) 1-{1-[1-(2-fluorophenylcarbamoyl)piperidin-4-yl]-5-(1-methoxycyclopropyl)-1H-pyrazole-4-carbonyl}-3-(2-trifluoromethylphenyl)pyrrolidine,
   (135) 1-{1-[1-(2-fluorophenylcarbamoyl)piperidin-4-yl]-5-(1-methylcyclopropyl)-1H-pyrazole-4-carbonyl}-3-phenyl-3-trifluoromethylpyrrolidine,
   (136) 3-(2-chlorophenyl)-1-{5-cyclopropyl-1-[1-(2,4-difluorophenylcarbamoyl)piperidin-4-yl]-1H-pyrazole-4-carbonyl}pyrrolidine,
   (137) 3-(2-chloropyridin-3-yl)-1-{5-cyclopropyl-1-[1-(2-fluorophenylcarbamoyl)piperidin-4-yl]-1H-pyrazole-4-carbonyl}pyrrolidine,
   (138) 1-{1-[1-(2-fluorophenylcarbamoyl)piperidin-4-yl]-5-(2,2,3,3-tetramethylcyclopropyl)-1H-pyrazole-4-carbonyl}-3-(pyridin-3-yl)pyrrolidine,
   (139) 1-{5-cyclohexyl-1-[1-(2-fluorophenylcarbamoyl)piperidin-4-yl]-1H-pyrazole-4-carbonyl}-3-(pyridin--3-yl)pyrrolidine,
   (140) 1-{5-cyclopropyl-1-[1-(2-fluorophenylcarbamoyl)piperidin-4-yl]-1H-pyrazole-4-carbonyl}-3-(2-trifluoromethoxyphenyl)pyrrolidine,
   (141) 1-{5-cyclopropyl-1-[1-(4-trifluoromethylphenylcarbamoyl)piperidin-4-yl]-1H-pyrazole-4-carbonyl}-3-(2-trifluoromethylphenyl)pyrrolidine,
   (142) 1-(5-cyclopropyl-1-{1-[((S)-1-carboxy-2-methylpropyl)methylcarbamoyl]piperidin-4-yl}-1H-pyrazole-4-carbonyl)-3-(2-trifluoromethylphenyl)pyrrolidine,
   (143) 1-{5-cyclopropyl-1-[1-(2-fluorophenylcarbamoyl)piperidin-4-yl]-1H-pyrazole-4-carbonyl}-3-methoxy-3-(2-trifluoromethylphenyl)pyrrolidine,
   (144) 1-{1-[1-(2-fluorophenylcarbamoyl)piperidin-4-yl]-5-(1-hydroxymethylcyclopropyl)-1H-pyrazole-4-carbonyl}-3-(2-trifluoromethylphenyl)pyrrolidine,
   (145) 1-{5-cyclopropyl-1-[1-(thiazol-2-ylcarbamoyl)piperidin-4-yl]-1H-pyrazole-4-carbonyl}-3-(2-trifluoromethylphenyl)pyrrolidine,
   (146) 1-{1-[1-(isopropoxycarbamoyl)piperidin-4-yl]-5-(1-methylcyclopropyl)-1H-pyrazole-4-carbonyl}-3-(2-trifluoromethylphenyl)pyrrolidine,
   (147) 1-{5-cyclopropyl-1-[1-(4-fluorobenzylcarbamoyl)piperidin-4-yl]-1H-pyrazole-4-carbonyl}-3-(2-trifluoromethylphenyl)pyrrolidine,
   (148) 1-{1-[1-(2,3-dihydroindole-1-carbonyl)piperidin-4-yl]-5-(1-methylcyclopropyl)-1H-pyrazole-4-carbonyl}-3-(pyridin-3-yl)pyrrolidine,
   (149) 1-{1-[1-(2,3-dihydro[1,4]oxazine-4-carbonyl)piperidin-4-yl]-5-(1-methylcyclopropyl)-1H-pyrazole-4-carbonyl}-3-(pyridin-3-yl)pyrrolidine,
   (150) 1-{5-cyclopropyl-1-[1-(2,6-dichloropyridin-3-ylcarbamoyl)piperidin-4-yl]-1H-pyrazole-4-carbonyl}-3-(2-trifluoromethylphenyl)pyrrolidine,
   (151) 1-{5-(1-methylcyclopropyl)-1-[1-(2,3,4,5-tetrahydrobenzo[b]azepine-1-carbonyl)piperidin-4-yl]-1H-pyrazole-4-carbonyl}-3-(pyridin-3-yl)pyrrolidine,
   (152) ethyl 4-(4-{5-cyclopropyl-4-[3-(2-trifluoromethylphenyl)pyrrolidine-1-carbonyl]pyrazol-1-yl}piperidin-1-yl)benzoate,
   (153) 1-[1-(1-benzyloxycarbonylpiperidin-4-yl)-5-cyclopropyl-1H-pyrazole-4-carbonyl]-3-(pyridin-3-yl)pyrrolidine,
   (154) 1-{1-[1-(3-carboxy-3-methyl-butyryl)piperidin-4-yl]-5-cyclopropyl-1H-pyrazole-4-carbonyl}-3-(2-trifluoromethylphenyl)pyrrolidine,
   (155) 1-{5-cyclopropyl-1-[1-(2-phenoxyacetyl)piperidin-4-yl]-1H-pyrazole-4-carbonyl}-3-(pyridin-3-yl)pyrrolidine,
   (156) 1-{1-[1-(3-chlorobenzoyl)piperidin-4-yl]-5-cyclopropyl-1H-pyrazole-4-carbonyl}-3-(pyridin-3-yl)pyrrolidine,
   (157) 1-{1-[1-(2-chlorobenzoyl)piperidin-4-yl]-5-cyclopropyl-1H-pyrazole-4-carbonyl}-3-(pyridin-3-yl)pyrrolidine,
   (158) 1-[5-cyclopropyl-1-(1-oxalylpiperidin-4-yl)-1H-pyrazole-4-carbonyl]-3-(2-trifluoromethylphenyl)pyrrolidine,
   (159) 1-{1-[1-(4-chlorobenzoyl)piperidin-4-yl]-5-cyclopropyl-1H-pyrazole-4-carbonyl}-3-(pyridin-3-yl)pyrrolidine,
   (160) 1-(5-cyclopropyl-1-{1-[2-(4-fluorophenyl)acetyl]piperidin-4-yl}-1H-pyrazole-4-carbonyl)-3-(pyridin-3-yl)pyrrolidine,
   (161) 1-{5-cyclopropyl-1-[1-(3-trifluoromethylbenzoyl)piperidin-4-yl]-1H-pyrazole-4-carbonyl}-3-(pyridin-3-yl)pyrrolidine,
   (162) 1-{5-cyclopropyl-1-[1-(3-methoxybenzoyl)piperidin-4-yl]-1H-pyrazole-4-carbonyl}-3-(pyridin-3-yl)pyrrolidine,
   (163) 1-{1-[1-(4-fluorobenzyl)piperidin-4-yl]-5-(1-methylcyclopropyl)-1H-pyrazole-4-carbonyl}-3-(2-trifluoromethylphenyl)pyrrolidine hydrochloride,
   (164) 1-[1-(1-benzenesulfonylpiperidin-4-yl)-5-cyclopropyl-1H-pyrazole-4-carbonyl]-3-(pyridin-3-yl)pyrrolidine,
   (165) 1-{1-[1-(4-carbamoylphenyl)piperidin-4-yl]-5-cyclopropyl-1H-pyrazole-4-carbonyl}-3-(2-trifluoromethylphenyl)pyrrolidine,
   (166) 1-{5-cyclopropyl-1-[1-(4-hydroxymethylphenyl)piperidin-4-yl]-1H-pyrazole-4-carbonyl}-3-(2-trifluoromethylphenyl)pyrrolidine,
   (167) 1-{1-[1-(5-carbamoyl-pyridin-2-yl)piperidin-4-yl]-5-cyclopropyl-1H-pyrazole-4-carbonyl}-3-(2-trifluoromethylphenyl)pyrrolidine,
   (168) 1-{1-[1-(4-aminophenyl)piperidin-4-yl]-5-cyclopropyl-1H-pyrazole-4-carbonyl}-3-(2-trifluoromethylphenyl)pyrrolidine,
   (169) 1-(5-cyclopropyl-1-{1-[4-(2-oxooxazolidin-3-yl)phenyl]piperidin-4-yl}-1H-pyrazole-4-carbonyl)-3-(2-trifluoromethylphenyl)pyrrolidine,
   (170) 1-(5-cyclopropyl-1-{1-[4-(3-methoxyureido)phenyl]piperidin-4-yl}-1H-pyrazole-4-carbonyl)-3-(2-trifluoromethylphenyl)pyrrolidine,
   (171) 1-{5-cyclopropyl-1-[1-(4-methanesulfonylaminophenyl)piperidin-4-yl]-1H-pyrazole-4-carbonyl}-3-(2-trifluoromethylphenyl)pyrrolidine,
   (172) 1-[1-(1-ethoxycarbamoylpiperidin-4-yl)-5-(1-methylcyclopropyl)-1H-pyrazole-4-carbonyl]-3-(2-trifluoromethylphenyl)pyrrolidine,
   (173) 1-{1-[1-(5-chloropyridin-2-yl)azetidin-3-yl]-5-cyclopropyl-1H-pyrazole-4-carbonyl}-3-(2-trifluoromethylphenyl)pyrrolidine,
   (174) 1-{1-[1-(5-chloropyridin-2-yl)piperidin-4-yl]-5-(1-methylcyclopropyl)-1H-pyrazole-4-carbonyl}-3-methoxymethyl-3-phenylpyrrolidine,
   (175) 1-{1-[1-(5-cyanopyridin-2-yl)piperidin-4-yl]-5-cyclopropyl-1H-pyrazole-4-carbonyl}-3-(2-trifluoromethylphenyl)pyrrolidine,
   (176) 3-(2-acetoxyethyl)-3-(4-fluorophenyl)-1-{5-(1-methylcyclopropyl)-1-[1-(pyrimidin-2-yl)piperidin-4-yl]-1H-pyrazole-4-carbonyl}pyrrolidine hydrochloride,
   (177)(3S*,4R*)-3-methyl-1-{5-(1-methylcyclopropyl)-1-[1-(pyrimidin-2-yl)piperidin-4-yl]-1H-pyrazole-4-carbonyl}-4-phenylpyrrolidine hydrochloride,
   (178) methyl 6-(4-{5-cyclopropyl-4-[3-(2-trifluoromethylphenyl)pyrrolidine-1-carbonyl]pyrazol-1-yl}piperidin-1-yl)nicotinate,
   (179) methyl 2-{1-[5-(1-methylcyclopropyl)-1-(1-pyrimidin-2-ylpiperidin-4-yl)-1H-pyrazole-4-carbonyl]pyrrolidin-3-yl}benzoate,
   (180) 3-(2-hydroxymethylphenyl)-1-{5-(1-methylcyclapropyl)-1-[1-(pyrimidin-2-yl)piperidin-4-yl]-1H-pyrazole-4-carbonyl}pyrrolidine hydrochloride,
   (181) 1-[5-cyclopropyl-1-(1-pyridin-2-ylpiperidin-4-yl)-1H-pyrazole-4-carbonyl]-3-hydroxy-3-(pyridin-3-yl)pyrrolidine,
   (182) 1-{1-[1-(4-acetylaminophenyl)piperidin-4-yl]-5-cyclopropyl-1H-pyrazole-4-carbonyl}-3-(2-trifluoromethylphenyl)pyrrolidine,
   (183) sodium 4-(4-{5-cyclopropyl-4-[3-(2-trifluoromethylphenyl)pyrrolidine-1-carbonyl]pyrazol-1-yl}piperidin-1-yl)-3-fluorobenzoate,
   (184) 3-(2-cyanophenyl)-1-{5-cyclopropyl-1-[1-(2-fluorophenylcarbamoyl)piperidin-4-yl]-1H-pyrazole carbonyl}pyrrolidine,
   (185) 3-hydroxymethyl-1-{1-[1-(4-methoxypyrimidin-2-yl)piperidin-4-yl]-5-(1-methylcyclopropyl)-1H-pyrazole-4-carbonyl}-3-phenylpyrrolidine hydrochloride,
   (186) 3-(3,5-difluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride,
   (187) 1-{1-[1-(3-chloropyridin-2-yl)piperidin-4-yl]-5-cyclopropyl-1H-pyrazole-4-carbonyl}-3-(pyridin-3-yl)pyrrolidine,
   (188) 1-{5-cyclopropyl-1-[1-(2-fluorophenylcarbamoyl)piperidin-4-yl]-1H-pyrazole-4-carbonyl}-3-(thiazol-2-yl)pyrrolidine,
   (189) (S)-2-[(trans-4-{5-cyclopropyl-4-[3-(2-trifluoromethylphenyl)pyrrolidine-1-carbonyl]pyrazol-1-yl}cyclohexanecarbonyl)methylamino]-3-methylbutyric acid,
   (190) 1-{5-(1-methylcyclopropyl)-1-[trans-4-(2-fluorophenylcarbamoyl)cyclohexyl]-1H-pyrazole-4-carbonyl}-3-(2-trifluoromethylphenyl)pyrrolidine, and
   (191) cis-4-{5-(1-methylcyclopropyl)-4-[3-(2-trifluoromethylphenyl)pyrrolidine-1-carbonyl]pyrazol-1-yl}cyclohexanecarboxylic acid.

### Effect of the Invention

According to the present invention, methods capable of efficiently producing a compound represented by the above-mentioned formula [1'] useful as a prophylactic or therapeutic agent for metabolic diseases such as diabetes, insulin resistance, diabetic complications, obesity, hyperlipidemia, hypertension, fatty liver and the like and the like, and a compound useful as a synthetic intermediate therefor, particularly, a synthetic intermediate for a compound represented by the formula [1'] and having a stronger HSD1 inhibitory action, can be provided with superior achievability of asymmetric synthesis (i.e., superior selectivity), superior yield, superior safety and superior industrial workability at a low cost.
According to the present invention, moreover, a compound which can be advantageously used as a synthetic intermediate for a compound represented by the above-mentioned formula [1'] can be provided.

### Best Mode for Carrying out the Invention

In the compound of the present invention and a compound represented by the formula [1'], the definitions of the substituents to be used are as follows.

The "C₁₋₆ alkyl group" is a linear or branched chain alkyl group having 1 to 6 carbon atoms and, for example, methyl group, ethyl group, propyl group, isopropyl group, butyl group, isobutyl group, sec-butyl group, tert-butyl group, pentyl group, isopentyl group, neopentyl group, tert-pentyl group, 1,2-dimethyl-propyl group, hexyl group, 1,3-dimethyl-butyl group and the like can be mentioned, with preference given to a linear or branched chain alkyl group having 1 to 4 carbon atoms.
For R⁵³, preferable "C₁₋₆ alkyl group" is an isopropyl group.
For R⁵⁴, preferable "C₁₋₆ alkyl group" is a methyl group.
For R¹, preferable "C₁₋₆ alkyl group" is a methyl group, an ethyl group, an isopropyl group or an isobutyl group.
For R³ or R⁴, preferable "C₁₋₆ alkyl group" is a methyl group.
For R⁵ or R⁶, preferable "C₁₋₆ alkyl group" is an ethyl group, an isopropyl group, a tert-butyl group, a 1,2-dimethyl-propyl group or a 1,3-dimethyl-butyl group.
For R⁹, preferable "C₁₋₆ alkyl group" is a methyl group.
For R¹⁰, preferable "C₁₋₆ alkyl group" is a methyl group or an ethyl group.
For R¹¹, preferable "C₁₋₆ alkyl group" is a methyl group, an ethyl group, a propyl group, an isopropyl group, an isobutyl group or a tert-butyl group.
For R¹², preferable "C₁₋₆ alkyl group" is a methyl group or an ethyl group.
For R¹³, preferable "C₁₋₆ alkyl group" is a methyl group.
For R¹⁴ or R¹⁵, preferable "C₁₋₆ alkyl group" is a methyl group.

The "C₂₋₆ alkenyl group" is a linear or branched chain alkenyl group having 2 to 6 carbon atoms and, for example, vinyl group, n-propenyl group, isopropenyl group, n-butenyl group, isobutenyl group, sec-butenyl group, tert-butenyl group, n-pentenyl group, isopentenyl group, neopentenyl group, 1-methylpropenyl group, n-hexenyl group, isohexenyl group, 1,1-dimethylbutenyl group, 2,2-dimethylbutenyl group, 3,3-dimethylbutenyl group, 3,3-dimethylpropenyl group, 2-ethylbutenyl group and the like can be mentioned.

The "C₁₋₆ alkoxy group" is an alkoxy group wherein the alkyl moiety is the "C₁₋₆ alkyl group" defined above and, for example, methoxy group, ethoxy group, propoxy group, isopropyloxy group, butoxy group, isobutyloxy group, tert-butyloxy group, pentyloxy group, hexyloxy group and the like can be mentioned.

The "halogen atom" is fluorine atom, chlorine atom, bromine atom or iodine atom. Preferred is fluorine atom or chlorine atom and more preferred is fluorine atom.

The "aryl group" is an aromatic hydrocarbon group having 6 to 14 carbon atoms and, for example, phenyl group, naphthyl group, anthryl group, azulenyl group, phenanthryl group and the like can be mentioned. Preferred is phenyl group.
For R⁵¹ or R⁵², preferable "aryl group" is a phenyl group.

The "aryloxy group" is an aryloxy group wherein the aryl moiety is the "aryl group" defined above and, for example, phenoxy group, naphthyloxy group and the like can be mentioned.

The "cycloalkyl group" is a cycloalkyl group having 3 to 8 carbon atoms and, for example, cyclopropyl group, cyclobutyl group, cyclopentyl group, cyclohexyl group, cycloheptyl group, cyclooctyl group and the like can be mentioned. Preferred is cycloalkyl group having 3 to 6 carbon atoms, such as cyclopropyl group, cyclobutyl group, cyclopentyl group and cyclohexyl group. Particularly preferred are cyclopropyl group and cyclopentyl group.

The "5-membered or 6-membered unsaturated monocyclic heterocyclic group" is a 5-membered or 6-membered unsaturated monocyclic heterocyclic group having, besides carbon atom, at least one, preferably 1 to 4, hetero atoms selected from nitrogen atom, oxygen atom and sulfur atom. The unsaturated includes partial unsaturation and complete unsaturation. For example, a 5-membered monocyclic aromatic heterocyclic group such as furyl group, thienyl group, pyrrolyl group, pyrazolyl group, imidazolyl group, oxazolyl group, isoxazolyl group, thiazolyl group, isothiazolyl group, triazolyl group, oxadiazolyl group, tetrazolyl group, thiadiazolyl group and the like, and a 6-membered monocyclic aromatic heterocyclic group such as pyridyl group, pyridazinyl group, pyrimidinyl group, pyrazinyl group, triazinyl group, thiazinyl group, oxadiazinyl group, pyranyl group, thiopyranyl group and the like can be mentioned. As the 5-membered monocyclic aromatic heterocyclic group, preferred is thienyl group or thiazolyl group. As the 6-membered monocyclic aromatic heterocyclic group, preferred is pyridyl group or pyrimidinyl group.
For R¹, preferable "5-membered or 6-membered unsaturated monocyclic heterocyclic group" is pyridyl group, thienyl group, thiazolyl group, triazolyl group (particularly preferably 1,2,4-triazolyl group), tetrazolyl group, pyrimidinyl group or pyrazinyl group.
For R³ or R⁴, preferable "5-membered or 6-membered unsaturated monocyclic heterocyclic group" is pyridyl group or thiazolyl group. More preferred is pyridyl group.

The "nitrogen-containing saturated monocyclic heterocyclic group" is, for example, a 4-membered to 6-membered saturated monocyclic heterocycle having at least one nitrogen atom, such as azetidinyl group, pyrrolidinyl group, imidazolidinyl group, pyrazolidinyl group, oxazolidinyl group, 2-oxopyrrolidinyl group, thiazolidinyl group, isothiazolidinyl group, piperidyl group, piperazinyl group, morpholinyl group, thiomorpholinyl group, 2-oxopiperidinyl group, 4-oxopiperidinyl group, 2,6-dioxopiperidinyl group and the like. Preferable "nitrogen-containing saturated monocyclic heterocyclic group" is azetidinyl group, pyrrolidinyl group, piperidyl group, piperazinyl group or thiomorpholinyl group.
For -NR⁵R⁶, preferable "nitrogen-containing saturated monocyclic heterocyclic group" is azetidinyl group, pyrrolidinyl group, piperidyl group, piperazinyl group or thiomorpholinyl group (said heterocyclic group is optionally substituted by one or more, same or different substituents selected from the following groups:
halogen atom,
hydroxyl group,
C₁₋₆ alkyl group optionally substituted by one or more hydroxyl groups,
carboxyl group,
acetyl group,
carbamoyl group,
amino group optionally substituted by one or more, same or different substituents selected from C₁₋₆ alkyl group and acetyl group, and oxo group).
For -NR²⁰R²¹, preferable "nitrogen-containing saturated monocyclic heterocyclic group" is azetidinyl group, pyrrolidinyl group or piperidyl group (said heterocyclic group is optionally substituted by one or more, same or different substituents selected from halogen atom and hydroxyl group).
For -NR²⁶R²⁷, preferable "nitrogen-containing saturated monocyclic heterocyclic group" is piperidyl group or pyrrolidinyl group, more preferably piperidyl group.
For R¹⁰, preferable "nitrogen-containing saturated monocyclic heterocyclic group" is piperidyl group.
For R¹¹, preferable "nitrogen-containing saturated monocyclic heterocyclic group" is pyrrolidinyl group or piperidyl group (said heterocyclic group is optionally substituted by one or more, same or different substituents selected from C₁₋₆ alkyl group, -CO-C₁₋₆ alkyl group and oxo group).

Being "optionally substituted by one or more substituents" means being unsubstituted or optionally substituted by at least one to the acceptable maximum number of substituents. For example, in the case of methyl group, it means being optionally substituted by 1 to 3 substituents, and in the case of ethyl group, it means being optionally substituted by 1 to 5 substituents. The substituents in the case of substitution with two or more substituents may be the same or different and the positions of the substituents are not particularly limited and may be any.
The "C₁₋₆ alkyl group optionally substituted by one or more, same or different substituents" is preferably C₁₋₆ alkyl group optionally substituted by the same or different 1 to 3 substituents. Particularly, "C₁₋₆ alkyl group optionally substituted by one or more, same or different halogen atom" is preferably C₁₋₆ alkyl group optionally substituted by 1 to 3 halogen atoms, more preferably C₁₋₆ alkyl group optionally substituted by three halogen atoms, and more preferably trifluoromethyl group or 2,2,2-trifluoroethyl group.

A preferable group for each group in the present invention is as follows.
R⁵¹ and R⁵² are each preferably an aryl group, more preferably a phenyl group.
R⁵³ is preferably a C₁₋₆ alkyl group, more preferably an isopropyl group.
R⁵⁴ is preferably an unsubstituted alkyl group, more preferably a methyl group.
Hal¹ is preferably a chlorine atom.

A preferable group for each group in a compound represented by the formula [1'] is as follows.
[Preferable ring A]
Ring A is
(1) a nitrogen-containing saturated monocyclic heterocyclic group (i.e., -X- is -N(R¹)-), or
(2) a cycloalkyl group (i.e., -X- is -C(R⁷R⁸)-), and preferably

(1) an azetidinyl group or a piperidyl group, or
(2) a cyclohexyl group.

[Preferable R²]
R² is preferably a cyclopropyl group or a cyclohexyl group optionally substituted by one or more, same or different substituents selected from a C₁₋₆ alkyl group (said C₁₋₆ alkyl group is optionally substituted by a hydroxyl group) and a C₁₋₆ alkoxy group. More preferably, it is a cyclopropyl group or a cyclohexyl group optionally substituted by one or more methyl groups (said methyl group is optionally substituted by a hydroxyl group) or methoxy groups. Particularly preferably, it is cyclopropyl group, methylcyclopropyl group, methoxycyclopropyl group, tetramethylcyclopropyl group, hydroxymethylcyclopropyl group or cyclohexyl group.

[Preferable R³ and R⁴]
R³ and R⁴ are preferably not hydrogen atoms at the same time.
More preferably, one of R³ and R⁴ is a hydrogen atom, a hydroxyl group, a C₁₋₆ alkyl group (said C₁₋₆ alkyl group is optionally substituted by one or more, same or different substituents selected from halogen atom, hydroxyl group, methoxy group and acetyloxy group) or a C₁₋₆ alkoxy group, and the other is not a hydrogen atom.
Further preferably, one of R³ and R⁴ is a hydrogen atom, a hydroxyl group, a C₁₋₆ alkyl group (said C₁₋₆ alkyl group is optionally substituted by one or more, same or different substituents selected from halogen atom, hydroxyl group, methoxy group and acetyloxy group) or a C₁₋₆ alkoxy group, and the other is a substituent selected from the following group, which is optionally substituted by one or more, same or different substituents selected from halogen atom, C₁₋₆ alkyl group (said C₁₋₆ alkyl group is optionally substituted by one or more, same or different halogen atoms or hydroxyl group), C₁₋₆ alkoxy group (said C₁₋₆ alkoxy group is optionally substituted by one or more, same or different halogen atoms), C₁₋₆ alkoxycarbonyl group and cyano group:
·phenyl group,
·pyridyl group, and
·thiazolyl group.
Particularly preferably, one of R³ and R⁴ is a hydrogen atom, a hydroxyl group, a C₁₋₆ alkyl group (said C₁₋₆ alkyl group is substituted by one or more, same or different substituents selected from halogen atom, hydroxyl group, methoxy group and acetyloxy group) or a methoxy group, and the other is a substituent selected from the following group, which is optionally substituted by one or more, same or different substituents selected from halogen atom, trifluoromethyl group, trifluoromethoxy group, methoxycarbonyl group, hydroxymethyl group and cyano group:
·phenyl group,
·pyridyl group, and
·thiazolyl group.

[Preferable R⁵ and R⁶]
R⁵ and R⁶ are preferably the same or different and each is
· a hydrogen atom,
· a phenyl group optionally substituted by one or more, same or different substituents selected from hydroxyl group, halogen atom, carboxyl group and C₁₋₆ alkyl group (said C₁₋₆ alkyl group is optionally substituted by one or more hydroxyl groups or halogen atoms),
· a cycloalkyl group having 3 to 6 carbon atoms (said cycloalkyl group is optionally substituted by C₁₋₆ alkyl group optionally substituted by one or more hydroxyl groups),
· a C₁₋₆ alkyl group optionally substituted by one or more, same or different substituents selected from hydroxyl group, halogen atom, phenyl group (said phenyl group is optionally substituted by halogen atom), carboxyl group and -NR²⁰R²¹ (wherein R²⁰ and R²¹ are as defined for the formula [1]),
· -S(=O)₂-R⁹ wherein R⁹ is phenyl group or C₁₋₆ alkyl group,
· optionally substituted C₁₋₆ alkoxy group,
· a pyridinyl group optionally substituted by halogen atom at the 2-position and the 6-position,
· an optionally substituted nitrogen-containing unsaturated heterocyclic group, or
· a nitrogen-containing saturated monocyclic heterocyclic group optionally substituted by one or more, same or different substituents selected from C₁₋₆ alkyl group and -CO-C₁₋₆ alkyl group, or
· R⁵ and R⁶ may form, together with the nitrogen atom bonded thereto, a nitrogen-containing saturated monocyclic heterocyclic group selected from azetidinyl group, pyrrolidinyl group, piperidyl group, piperazinyl group, and thiomorpholinyl group, or a heterocyclic group selected from dihydroindolyl group, dihydro[1,4]oxazinyl group, and tetrahydrobenzo[b]azepinyl group, which is a fused ring of said heterocycle and a carbocycle (both said heterocyclic groups are optionally substituted by one or more, same or different substituents selected from the following groups: halogen atom,
hydroxyl group,
C₁₋₆ alkyl group optionally substituted by one or more hydroxyl groups,
carboxyl group,
acetyl group,
carbamoyl group,
amino group optionally substituted by one or more, same or different substituents selected from C₁₋₆ alkyl group and acetyl group, and
oxo group).

More preferably, R⁵ and R⁶ are the same or different and each is
· a hydrogen atom,
· a phenyl group optionally substituted at the ortho-position and/or the para-position by substituents selected from hydroxyl group, halogen atom, carboxyl group and C₁₋₆ alkyl group (said C₁₋₆ alkyl group is optionally substituted by one or more hydroxyl groups or halogen atoms),
· a cyclopropyl group or a cyclopentyl group optionally substituted by C₁₋₆ alkyl group optionally substituted by one or more hydroxyl groups,
· a C₁₋₆ alkyl group substituted by one or more, same or different substituent selected from hydroxyl group, halogen atom, phenyl group (said phenyl group is optionally substituted by halogen atom), carboxyl group and -NR²⁰R²¹ (wherein R²⁰ and R²¹ are as defined for the formula [1]),
· -S(=O)₂-R⁹ wherein R⁹ is a phenyl group or a methyl group,
· a C₁₋₆ alkoxy group,
· a pyridinyl group substituted by halogen atom at the 2-position and the 6-position,
· an optionally substituted thiazolyl group, or
· a piperidin-4-yl group optionally substituted at the 1-position by one substituent selected from C₁₋₆ alkyl group and - CO-C₁₋₆ alkyl group, or
· R⁵ and R⁶ may form, together with the nitrogen atom bonded thereto, a nitrogen-containing saturated monocyclic heterocyclic group selected from azetidin-1-yl group, pyrrolidin-1-yl group, piperidin-1-yl group, piperazin-1-yl group, and thiomorpholin-4-yl group, or a heterocyclic group selected from dihydroindol-1-yl group, dihydro[1,4]oxazin-1-yl group, and tetrahydrobenzo[b]azepin-1-yl group, which is a fused ring of said heterocycle and a carbocycle (both said heterocyclic groups are optionally substituted by one or more, same or different substituents selected from the following groups:
   halogen atom,
   hydroxyl group,
   C₁₋₆ alkyl group optionally substituted by one or more hydroxyl groups,
   carboxyl group,
   acetyl group,
   carbamoyl group,
   amino group optionally substituted by one or more, same or different substituents selected from C₁₋₆ alkyl group and acetyl group, and
   oxo group).

Further preferably, R⁵ and R⁶ are the same or different and each is
- a hydrogen atom,
- a phenyl group substituted at the ortho-position and/or the para-position by substituents selected from hydroxyl group, halogen atom, carboxyl group and C₁₋₆ alkyl group (said C₁₋₆ alkyl group is optionally substituted by one or more hydroxyl groups or halogen atoms),
- a cyclopropyl group or a cyclopentyl group substituted by C₁₋₆ alkyl group substituted by one or more hydroxyl groups,
- a C₁₋₆ alkyl group substituted by one substituent selected from hydroxyl group, halogen atom, phenyl group (said phenyl group is optionally substituted by halogen atom), carboxyl group and -NR²⁰R²¹ (wherein R²⁰ and R²¹ are as defined for the formula [1]),
- -S(=O)₂-R⁹ wherein R⁹ is a phenyl group or a methyl group,
- a C₁₋₆ alkoxy group,
- a pyridin-3-yl group substituted by halogen atom at the 2-position and the 6-position,
- a thiazolyl group, or
- a piperidin-4-yl group optionally substituted at the 1-position by -CO-C₁₋₆ alkyl group, or
- R⁵ and R⁶ may form, together with the nitrogen atom bonded thereto, a nitrogen-containing saturated monocyclic heterocyclic group selected from azetidin-1-yl group, pyrrolidin-1-yl group, piperidin-1-yl group, piperazin-1-yl group, and thiomorpholin-4-yl group, or a heterocyclic group selected from dihydroindol-1-yl group, dihydro[1,4]oxazin-1-yl group, and tetrahydrobenzo[b]azepin-1-yl group, which is a fused ring of said heterocycle and a carbocycle (both said heterocyclic groups are optionally substituted by one or more, same or different substituents selected from the following groups:
   halogen atom,
   hydroxyl group,
   C₁₋₆ alkyl group optionally substituted by one or more hydroxyl groups,
   carboxyl group,
   acetyl group,
   carbamoyl group,
   amino group optionally substituted by one or more, same or different substituents selected from C₁₋₆ alkyl group and acetyl group, and
   oxo group).

Particularly preferably, R⁵ and R⁶ are the same or different and each is
- hydrogen atom,
- 2-fluorophenyl group,
- 2-carboxyphenyl group,
- 2-hydroxymethylphenyl group,
- 1-hydroxymethylcyclopropyl group,
- 2-hydroxyethyl group,
- 2-hydroxy-l,1-dimethylethyl group,
- 2-acetylaminoethyl group,
- 2-aminoethyl group,
- 2-dimethylaminoethyl group,
- 2-piperidin-1-yl-ethyl group,
- 2-hydroxy-1-methylethyl group,
- piperidin-4-yl group,
- 1-methyl-piperidin-4-yl group,
- 1-acetyl-piperidin-4-yl group,
- 2,2,2-trifluoroethyl group,
- 1-hydroxymethyl-2-methylpropyl group,
- 1-hydroxymethyl-2-phenylethyl group,
- 2-hydroxy-1-phenylethyl group,
- 1-hydroxymethyl-3-methylbutyl group,
- 2-hydroxyphenyl group,
- 1-hydroxymethylcyclopentyl group,
- benzenesulfonyl group,
- 4-trifluoromethylphenyl group,
- 2,4-difluorophenyl group,
- 1-carboxy-2-methylpropyl group,
- thiazol-2-yl group,
- isopropoxy group,
- 4-fluorobenzyl group,
- 2,6-dichloropyridin-3-yl group,
- ethoxy group, or
- mesyl group, or
- R⁵ and R⁶ optionally form, together with the nitrogen atom bonded thereto, a nitrogen-containing saturated monocyclic heterocyclic group or a heterocyclic group which is a fused ring of said heterocycle and a carbocycle, which is selected from the following groups:
   azetidin-1-yl group,
   3-hydroxyazetidin-1-yl group,
   3-hydroxymethylazetidin-1-yl group,
   3-acetylazetidin-1-yl group,
   3-acetylaminoazetidin-1-yl group,
   3-hydroxypyrrolidin-1-yl group,
   3,3-difluoropyrrolidin-1-yl group,
   3,3,4,4-tetrafluoropyrrolidin-1-yl group,
   2-hydroxymethylpyrrolidin-1-yl group,
   2-aminopyrrolidin-1-yl group,
   2-acetylaminopyrrolidin-1-yl group,
   2-dimethylaminopyrrolidin-1-yl group,
   3-oxopyrrolidin-1-yl group,
   4-hydroxypiperidin-1-yl group,
   4,4-difluoropiperidin-1-yl group,
   4-hydroxymethylpiperidin-1-yl group,
   4-carboxypiperidin-1-yl group,
   3-hydroxypiperidin-1-yl group,
   4-carbamoylpiperidin-1-yl group,
   4-aminopiperidin-1-yl group,
   4-dimethylaminopiperidin-1-yl group,
   4-acetylaminopiperidin-1-yl group,
   4-methylpiperazin-1-yl group,
   4-isopropylpiperazin-1-yl group,
   4-acetylpiperazin-1-yl group,
   1,1-dioxothiomorpholin-4-yl group,
   3-oxopiperidin-1-yl group,
   4-oxopiperidin-1-yl group,
   2,3-dihydroindol-1-yl group,
   2,3-dihydro[1,4]oxazin-1-yl group, and
   2,3,4,5-tetrahydrobenzo[b]azepin-1-yl group.

Further preferably, R⁵ and R⁶ are not hydrogen atoms at the same time. More preferably, one of R⁵ and R⁶ is a hydrogen atom and the other is not a hydrogen atom.

[Preferable R¹⁰]
R¹⁰ is preferably
- a C₁₋₆ alkyl group (said C₁₋₆ alkyl group is optionally substituted by one or more, same or different substituents selected from hydroxyl group, phenyl group and -NR²⁶R²⁷ wherein R²⁶ and R²⁷ are the same or different and each is a C₁₋₆ alkyl group, or R²⁶ and R²⁷ optionally form a piperidyl group together with the nitrogen atom bonded thereto), or
- a piperidyl group (said piperidyl group is optionally substituted by one or more, same or different substituents selected from C₁₋₆ alkyl group and -CO-C₁₋₆ alkyl group).

R¹⁰ is more preferably
- a C₁₋₆ alkyl group (said C₁₋₆ alkyl group is optionally substituted by one or more, same or different substituents selected from hydroxyl group, phenyl group and -NR²⁶R²⁷ wherein R²⁶ and R²⁷ are the same or different and each is C₁₋₆ alkyl group, or R²⁶ and R²⁷ optionally form a piperidyl group together with the nitrogen atom bonded thereto), or
- a piperidin-4-yl group (said piperidin-4-yl group is optionally substituted by one or more, same or different substituents selected from C₁₋₆ alkyl group and -CO-C₁₋₆ alkyl group).

R¹⁰ is further preferably
- a C₁₋₆ alkyl group (said C₁₋₆ alkyl group is substituted by one or more, same or different substituents selected from hydroxyl group, phenyl group and -NR²⁶R²⁷ wherein R²⁶ and R²⁷ are the same or different and each is C₁₋₆ alkyl group, or R²⁶ and R²⁷ optionally form a piperidyl group together with the nitrogen atom bonded thereto), or
- a piperidin-4-yl group (said piperidin-4-yl group is optionally substituted by one or more, same or different substituents selected from C₁₋₆ alkyl group and -CO-C₁₋₆ alkyl group).

R¹⁰ is particularly preferably
- methyl group,
- 2-hydroxyethyl group,
- 2-dimethylaminoethyl group,
- 2-piperidin-1-ylethyl group,
- piperidin-4-yl group,
- 1-methylpiperidin-4-yl group,
- benzyl group, or
- 1-acetylpiperidin-4-yl group.

[Preferable R¹¹]
R¹¹ is preferably
- a C₁₋₆ alkyl group (said C₁₋₆ alkyl group is optionally substituted by one or more, same or different substituents selected from the following a) to g):
   a) a halogen atom,
   b) a hydroxyl group,
   c) -NR²⁸R²⁹ wherein R²⁸ and R²⁹ are the same or different and each is a hydrogen atom, a C₁₋₆ alkyl group, a -CO-C₁₋₆ alkyl group, -CO-cycloalkyl group, or -S(=O)₂-C₁₋₆ alkyl group,
   d) a carbamoyl group,
   e) a carboxyl group,
   f) a phenoxy group, and
   g) a fluorophenyl group),
- a cycloalkyl group (said cycloalkyl group is optionally substituted by one or more, same or different substituents selected from the following a) and b):
   a) an aryl group optionally substituted by one or more, same or different halogen atoms, and
   b) a C₁₋₆ alkyl group optionally substituted by one or more hydroxyl groups),
- an aryl group (said aryl group is optionally substituted by one or more, same or different substituents selected from the following a) to c):
   a) one or more, same or different halogen atoms,
   b) a C₁₋₆ alkyl group optionally substituted by one or more, same or different halogen atoms, and
   c) a C₁₋₆ alkoxy group),
- a carboxyl group, or
- a nitrogen-containing saturated monocyclic heterocyclic group (said heterocyclic group is optionally substituted by one or more, same or different substituents selected from the following a) to c):
   a) a C₁₋₆ alkyl group,
   b) a -CO-C₁₋₆ alkyl group, and
   c) an oxo group).

R¹¹ is more preferably
- a C₁₋₆ alkyl group (said C₁₋₆ alkyl group is substituted by one or more, same or different substituents selected from the following a) to g):
   a) a halogen atom,
   b) a hydroxyl group,
   c) -NR²⁸R²⁹ wherein R²⁸ and R²⁹ are the same or different and each is a hydrogen atom, a C₁₋₆ alkyl group, a -CO-C₁₋₆ alkyl group, -CO-cycloalkyl group, or -S(=O)₂-C₁₋₆ alkyl group,
   d) a carbamoyl group,
   e) a carboxyl group,
   f) a phenoxy group, and
   g) a fluorophenyl group),
- a cyclopropyl group (said cyclopropyl group is optionally substituted by one or more, same or different substituents selected from the following a) and b):
   a) an aryl group optionally substituted by one or more, same or different halogen atoms, and
   b) a C₁₋₆ alkyl group optionally substituted by one or more hydroxyl groups),
- a phenyl group (said phenyl group is optionally substituted by one or more, same or different substituents selected from the following a) to c):
   a) one or more, same or different halogen atoms,
   b) a C₁₋₆ alkyl group optionally substituted by one or more, same or different halogen atoms, and
   c) a C₁₋₆ alkoxy group),
- a carboxyl group, or
- a nitrogen-containing saturated monocyclic heterocyclic group selected from a pyrrolidinyl group and a piperidyl group (said heterocyclic group is optionally substituted by one or more, same or different substituents selected from the following a) to C) :
   a) a C₁₋₆ alkyl group,
   b) a -CO-C₁₋₆ alkyl group, and
   c) an oxo group).

R¹¹ is further preferably
- a C₁₋₆ alkyl group (said C₁₋₆ alkyl group is substituted by one or more, same or different substituents selected from the following a) to g):
   a) a halogen atom,
   b) a hydroxyl group,
   c) -NR²⁸R²⁹ wherein R²⁸ and R²⁹ are the same or different and each is a hydrogen atom, a C₁₋₆ alkyl group, a -CO-C₁₋₆ alkyl group, -CO-cycloalkyl group, or -S(=O)₂-C₁₋₆ alkyl group,
   d) a carbamoyl group,
   e) a carboxyl group,
   f) a phenoxy group, and
   g) a fluorophenyl group),
- a cyclopropyl group (said cyclopropyl group is optionally substituted by one or more, same or different substituents selected from the following a) and b):
   a) an aryl group optionally substituted by one or more, same or different halogen atoms, and
   b) a C₁₋₆ alkyl group optionally substituted by one or more hydroxyl groups),
- a phenyl group (said phenyl group is substituted by one or more, same or different substituents selected from the following a) to c):
   a) one or more, same or different halogen atoms,
   b) a C₁₋₆ alkyl group optionally substituted by one or more, same or different halogen atoms, and
   c) a C₁₋₆ alkoxy group),
- a carboxyl group, or
- a nitrogen-containing saturated monocyclic heterocyclic group selected from a pyrrolidin-2-yl group and a piperidin-4-yl group (said heterocyclic group is optionally substituted by one or more, same or different substituents selected from the following a) to c):
   a) a C₁₋₆ alkyl group,
   b) a -CO-C₁₋₆ alkyl group, and
   c) an oxo group).

R¹¹ is particularly preferably
- 2,2,2-trifluoroethyl group,
- hydroxymethyl group,
- 3-hydroxypropyl group,
- 2-hydroxy-1,1-dimethylethyl group,
- aminomethyl group,
- 2-aminoethyl group,
- 1-amino-1-methylethyl group,
- 1-amino-2-methylpropyl group,
- methylaminomethyl group,
- dimethylaminomethyl group,
- acetylaminomethyl group,
- 1-acetylaminoethyl group,
- 2-acetylaminoethyl group,
- 1-acetylamino-1-methylethyl group,
- 1-acetylamino-2-methylpropyl group,
- isobutyrylaminomethyl group,
- cyclopropanecarbonylaminomethyl group,
- methanesulfonylaminomethyl group,
- 2-carbamoylethyl group,
- cyclopropyl group,
- 1-(4-fluorophenyl)cyclopropyl group,
- 1-methylcyclopropyl group,
- 1-hydroxymethylcyclopropyl group,
- pyrrolidin-1-yl group,
- pyrrolidin-2-yl group,
- piperidin-4-yl group,
- 1-methylpyrrolidin-2-yl group,
- 1-methylpiperidin-4-yl group,
- 1-acetylpyrrolidin-2-yl group,
- 1-acetylpiperidin-4-yl group,
- 2-carboxy-2-methylpropyl group,
- phenoxymethyl group,
- 2-chlorophenyl group,
- 3-chlorophenyl group,
- 4-chlorophenyl group,
- carboxyl group,
- 4-fluorobenzyl group,
- 3-trifluoromethylphenyl group,
- 3-methoxyphenyl group, or
- 5-oxopyrrolidin-2-yl group.

As a salt of the compound of the present invention is preferably a pharmaceutically acceptable slat. As a salt of a compound represented by the formula [1'] is preferably a pharmaceutically acceptable salt. A salt of a compound can be produced according to a conventional method. A salt of a compound can be obtained by reacting the compound with, for example, inorganic acids such as hydrochloric acid, sulfuric acid, phosphoric acid, hydrobromic acid and the like; organic acids such as oxalic acid, malonic acid, citric acid, fumaric acid, lactic acid, malic acid, succinic acid, tartaric acid, acetic acid, trifluoroacetic acid, gluconic acid, ascorbic acid, methanesulfonic acid, benzenesulfonic acid and the like; inorganic bases such as sodium hydroxide, potassium hydroxide, calcium hydroxide, magnesium hydroxide, ammonium hydroxide and the like; organic bases such as methylamine, diethylamine, triethylamine, triethanolamine, ethylenediamine, tris(hydroxymethyl)methylamine, guanidine, choline, cinchonine and the like; amino acids such as lysine, arginine, alanine and the like, and the like.

The compound of the present invention or a salt thereof may include various solvates (e.g., semihydrate, monohydrate, dihydrate). Such solvates are encompassed in the present invention. The solvates can be obtained according to a method known per se.

In addition, the compound represented by the formula [1'] has various isomers. For example, E form and Z form can be present as geometric isomers, when an asymmetric carbon atom is present, enantiomer and diastereomer are present as stereoisomers based thereon, and tautomers can also be present.

Now the production methods of the compound of the present invention are specifically explained.
Even in the absence of description in the production method, efficient production can be afforded by designs such as introducing, where necessary, a protecting group into a functional group followed by deprotection in a subsequent step; exchanging the order of respective steps; and the like. The work-up treatment in each step can be applied by a typical method, wherein isolation and purification is performed by selecting or combining conventional methods, as necessary, such as crystallization, recrystallization, silica gel chromatography, preparative HPLC and the like.

### Production Methods

### Step 1: acylation step

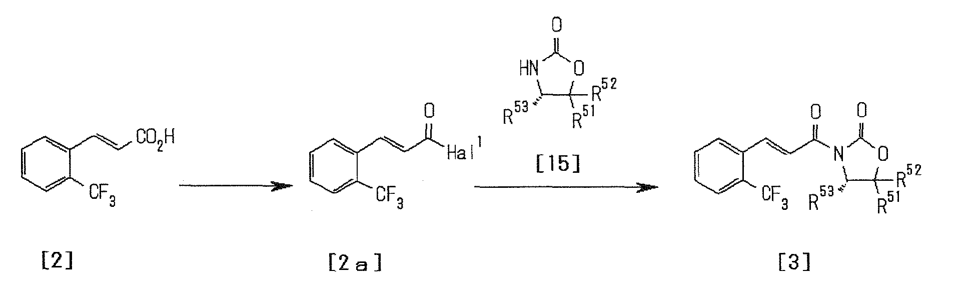

wherein each symbol is as defined above.
A compound represented by the formula [3] is obtained by inducing a reactive derivative (acid halide) represented by the formula [2a] from a compound represented by the formula [2] using a halogenating agent, and reacting the derivative with an asymmetric auxiliary group represented by the formula [15] in the presence of a base and an additive in a solvent. As the halogenating agent, thionyl chloride, oxalyl chloride, phosphorus oxychloride, phosphorus oxybromide and the like can be mentioned, with preference given to thionyl chloride. As the base, triethylamine, N,N-diisopropylethylamine, pyridine, 4-dimethylaminopyridine, n-butyllithium and the like can be mentioned, with preference given to triethylamine. As the additive, lithium chloride, lithium bromide, 4-dimethylaminopyridine and the like can be mentioned, with preference given to lithium chloride. As the solvent, N,N-dimethylformamide, N,N-dimethylacetamide, acetonitrile, tetrahydrofuran, 1,4-dioxane, dichloromethane, chloroform, a mixed solvent thereof and the like can be mentioned, with preference given to tetrahydrofuran. The reaction temperature in the step for inducing the acid halide is generally room temperature to 120°C, preferably about 60°C to 90°C, and that in the step of reacting the asymmetric auxiliary group represented by the formula [15] is generally about -20°C to room temperature, preferably about 0°C to room temperature. The reaction time for both steps is generally about 1 hr to 24 hr, preferably about 1 hr to 3 hr. The amount of the halogenating agent to be used is preferably about 1 - 8 mol, more preferably about 1 - 2 mol, per 1 mol of the compound represented by the formula [2]. In addition, the amount of the compound represented by the formula [2] to be used is preferably about 1 - 2 mol, more preferably about 1 - 1.2 mol, per 1 mol of the asymmetric auxiliary group represented by the formula [15]. The amount of the base and additive to be used is each preferably about 1 - 3 mol, more preferably about 1 - 1.3 mol, per 1 mol of the asymmetric auxiliary group represented by the formula [15]. The amount of the solvent to be used is not particularly limited, and can be appropriately determined according to the kind and amount of use of the reaction substrate, reaction temperature, reaction time and the like (same applies to the amount of the solvent to be used in steps 2 - 5 explained in the following).

As the compound represented by the formula [2] and the asymmetric auxiliary group represented by the formula [15], commercially available products may be directly used, or they may be synthesized according to a method known per se.

### Step 2: Michael addition reaction step

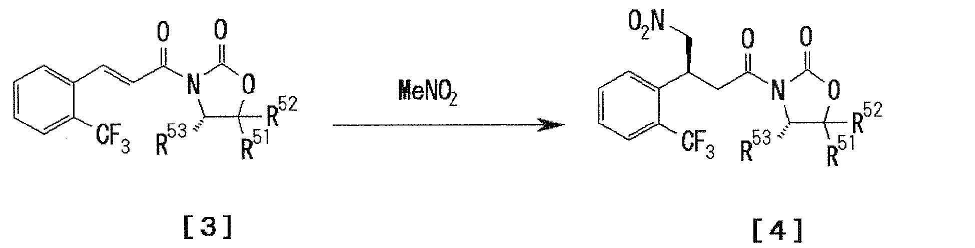

wherein each symbol is as defined above.
A compound represented by the formula [4] is obtained by reacting a compound represented by the formula [3] with nitromethane in the presence of an additive in a solvent or without solvent. As the additive, 1,8-diazabicyclo[5,4,0]undec-7-ene (DBU), 1,1,3,3-tetramethylguanidine, potassium fluoride, cesium fluoride, tetrabutylammonium fluoride and the like can be mentioned, with preference given to 1,8-diazabicyclo[5,4,0]undec-7-ene (DBU). As the solvent, N,N-dimethylformamide, N,N-dimethylacetamide, dimethyl sulfoxide, N-methylpyrrolidone, 1,3-dimethyl-3,4,5,6-tetrahydro-2(1H)-pyrimidinone (DMPU), 1,3-dimethyl-2-imidazolidinone (DMI), acetone, acetonitrile, a mixed solvent thereof and the like can be mentioned, with preference given to N,N-dimethylformamide, N,N-dimethylacetamide and N-methylpyrrolidone. The reaction temperature is generally about -20°C to 60°C, preferably about 0°C to room temperature. The reaction time is generally about 30 min to 24 hr, preferably about 1 hr to 5 hr. The amount of nitromethane to be used is preferably about 1 mol - excess amount, more preferably about 1 - 2 mol, per 1 mol of the compound represented by the formula [3]. The amount of the additive to be used is preferably about 0.05 - 2 mol, more preferably about 0.1 - 0.5 mol, per 1 mol of the compound represented by the formula [3].

### step 3: alcoholysis (elimination of asymmetric auxiliary group) step

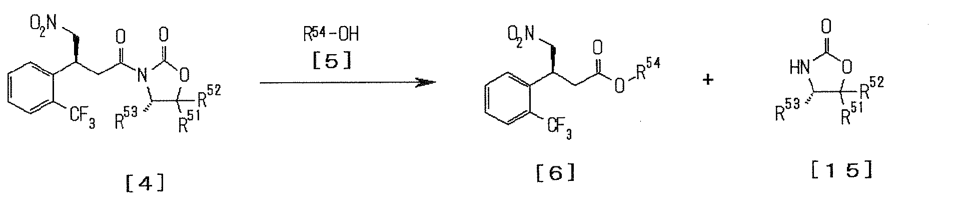

wherein each symbol is as defined above.
A compound represented by the formula [6] and an asymmetric auxiliary group represented by the formula [15] are obtained by reacting a compound represented by the formula [4] with a compound represented by the formula [5] in the presence of a base in a solvent. The compound represented by the formula [5] is an alcohol, and, for example, methanol, ethanol, 1-propanol, 1-butanol, benzyl alcohol and the like can be mentioned, with preference given to methanol. As the solvent, diethyl ether, tetrahydrofuran, 1,4-dioxane etc., or a compound represented by the formula [5] (alcohol) can be used alone as a solvent or a mixed solvent thereof may be used. A compound represented by the formula [5] itself is preferably a solvent. As the base, sodium methoxide, potassium methoxide, sodium ethoxide, potassium ethoxide, sodium t-butoxide, potassium t-butoxide, lithium hydride, n-butyllithium, sodium hydride, potassium hydride, 1,8-diazabicyclo[5,4,0]undec-7-ene (DBU) and the like can be mentioned, with preference given to sodium methoxide and 1,8-diazabicyclo[5,4,0]undec-7-ene (DBU). When 1,8-diazabicyclo[5,4,0]undec-7-ene (DBU) is used as a base, the reaction may be performed using lithium chloride, lithium bromide and the like as an additive. The amount of the additive to be used is preferably about 1 - 5 mol, more preferably about 1 - 3 mol, per 1 mol of the compound represented by the formula [4]. The reaction temperature is generally about -20°C to 50°C, preferably about 0°C to room temperature. The reaction time is generally about 1 hr to 24 hr, preferably about 1 hr to 4 hr. The amount of the base to be used is preferably about 1 - 5 mol, more preferably about 1 - 3 mol, per 1 mol of the compound represented by the formula [4].

### step 4: lactamization step

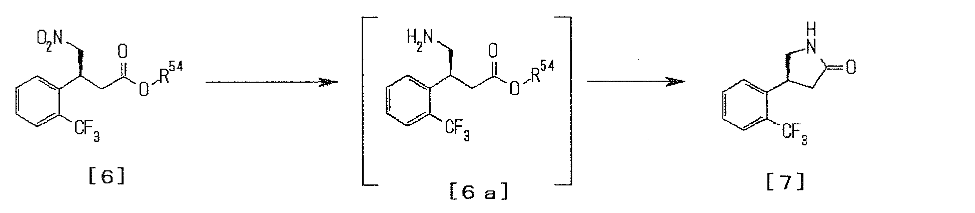

wherein each symbol is as defined above.
A lactam represented by the formula [7] is obtained via a compound represented by the formula [6a] by subjecting a compound represented by the formula [6] to a catalytic hydrogenation reaction under a hydrogen atmosphere in the presence of a catalyst in a solvent, or other general reduction reaction of nitro group. As the catalyst to be used for the catalytic hydrogenation reaction, Raney nickel, palladium-carbon, palladium hydroxide, platinum oxide and the like can be mentioned, with preference given to Raney nickel and palladium-carbon. When palladium-carbon is used as the catalyst, the reaction may be performed using hydrochloric acid, acetic acid, methanesulfonic acid and the like as an additive, which is preferably methanesulfonic acid. The amount of the additive to be used is preferably about 0.5 - 3 mol, more preferably about 1 - 2 mol, per 1 mol of the compound represented by the formula [6]. As the solvent, methanol, ethanol, 2-propanol, tetrahydrofuran, 1,4-dioxane, acetic acid, methyl acetate, ethyl acetate, butyl acetate and the like can be mentioned, which may be used alone or in a mixture of two or more kinds thereof, with preference given to methanol and tetrahydrofuran. The reaction temperature is generally about 0°C to 100°C, preferably room temperature to 60°C. The reaction time is generally about 1 hr to 48 hr, preferably about 1 hr to 24 hr. The hydrogen pressure is generally about 0.1 - 1.0 MPa, preferably about 0.3 - 0.5 MPa. The amount of the catalyst to be used is preferably about 0.05 g - 2.0 g, more preferably about 0.05 g - 0.5 g, per 1 g of the compound represented by the formula [6]. As other general reduction reaction of the nitro group, a reduction reaction by tin, zinc or iron in the presence of an inorganic acid such as hydrochloric acid and the like, a reduction reaction by sodium borohydride in the presence of Lewis acid such as cobalt chloride, nickel chloride and the like, a reduction reaction by lithium aluminum hydride, a reduction reaction by iron chloride in the presence of hydrazine, a reduction reaction by iron in the presence of ammonium chloride, a reduction reaction by tin chloride and the like can be mentioned.

When this reaction stops at a compound represented by the formula [6a], a lactam represented by the formula [7] can be induced therefrom by further reacting, after removal of the catalyst, the compound in the presence of a base (the amount of the base to be used is preferably about 0.2 - 3 mol, more preferably about 0.5 - 1 mol, per 1 mol of the compound represented by the formula [6]). As the base, 1,8-diazabicyclo[5,4,0]undec-7-ene (DBU), 1,1,3,3-tetramethylguanidine, triethylamine, pyridine, 4-dimethylaminopyridine, N-methylmorpholine, sodium carbonate, potassium carbonate, sodium hydrogen carbonate, potassium hydrogen carbonate and the like can be mentioned, with preference given to 1,8-diazabicyclo[5,4,0]undec-7-ene (DBU) and triethylamine.

### step 5:lactam reduction step

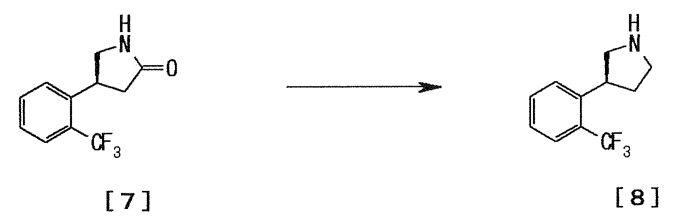

wherein each symbol is as defined above.
An amine represented by the formula [8] is obtained by reacting a lactam represented by the formula [7] with a reducing agent in a solvent. As the solvent, diethyl ether, tetrahydrofuran, 1,4-dioxane, 1,2-dimethoxyethane, benzene, toluene, xylene and the like can be mentioned, which may be used alone or in a mixture of two or more kinds thereof, with preference given to tetrahydrofuran. As the reducing agent, lithium aluminum hydride, sodium borohydride, lithium tetrahydroborate, diisobutylaluminum hydride, sodium bis(2-methoxyethoxy)aluminum hydride, borane complex and the like can be mentioned, with preference given to lithium aluminum hydride and borane complex. The reaction temperature is generally about 0°C to 150°C, preferably about 0°C to 90°C. The reaction time is generally 30 min to 24 hr, preferably 1 hr to 3 hr. The amount of reducing agent to be used is preferably about 1 - 5 mol, more preferably about 1 - 2 mol, per 1 mol of the lactam represented by the formula [7].

The obtained amine represented by the formula [8] can be obtained as a salt by reaction in a solvent with an inorganic acid such as hydrochloric acid, sulfuric acid, phosphoric acid, hydrobromic acid and the like, an organic acid such as oxalic acid, malonic acid, citric acid, fumaric acid, lactic acid, malic acid, tartaric acid, succinic acid, acetic acid, trifluoroacetic acid, gluconic acid, ascorbic acid, methylsulfonic acid, benzenesulfonic acid, (-)-10-camphorsulfonic acid and the like and the like, with preference given to a salt with hydrochloric acid or (-)-10-camphorsulfonic acid. As the solvent, diethyl ether, tetrahydrofuran, 1,4-dioxane, 1,2-dimethoxyethane, benzene, toluene, xylene, methyl acetate, ethyl acetate, butyl acetate, acetone, methanol, ethanol, 2-propanol, a mixed solvent thereof and the like can be mentioned, with preference given to diethyl ether and ethyl acetate. The amount of each of the inorganic acid and organic acid to be used is preferably about 0.5 - 5 mol, more preferably about 0.5 - 2 mol, per 1 mol of the amine represented by the formula [8].

Now the production methods of a compound represented by the formula [1'] are specifically explained. However, the production methods of the compound are not limited thereto. Even in the absence of description in the production method, efficient production can be afforded by designs such as introducing, where necessary, a protecting group into a functional group followed by deprotection in a subsequent step; exchanging the order of respective steps; and the like. Examples of the protecting group include carboxy-protecting group (here, the carboxy-protecting group means one generally used in the field of organic synthesis chemistry, for example, methyl group, ethyl group, propyl group, tert-butyl group, benzyl group, p-methoxybenzyl group and the like, which forms an ester that can be easily induced to carboxylic acid by hydrolysis, catalytic hydrogenation reaction and the like), amino-protecting group (here, the amino-protecting group means one generally used in the field of organic synthesis chemistry, for example, tert-butoxycarbonyl group, benzyloxycarbonyl group, benzyl group and the like, which permits easy induction to amino group by hydrolysis, catalytic hydrogenation reaction and the like), hydroxyl-protecting group (here, the hydroxyl-protecting group means one generally used in the field of organic synthesis chemistry, for example, tetrahydropyranyl group, methoxymethyl group, methoxyethoxymethyl group, benzyloxymethyl group, benzyl group, p-methoxybenzyl group, p-nitrobenzyl group, trimethylsilyl group, t-butyldimethylsilyl group, acetyl group, benzoyl group, allyl group, t-butyl group and the like, which permits easy induction to hydroxyl group by hydrolysis, catalytic hydrogenation reaction and the like) and the like. In each step, moreover, work-up after reaction can be performed according to a general method, and isolation and purification can be performed by a conventional method such as crystallization, recrystallization, silica gel column chromatography, preparative HPLC and the like, which can be selected appropriately or used in combination.

### Production Method 1

In the compound represented by the formula [1'], a compound wherein -X- is -NH- can be produced by the following Steps.

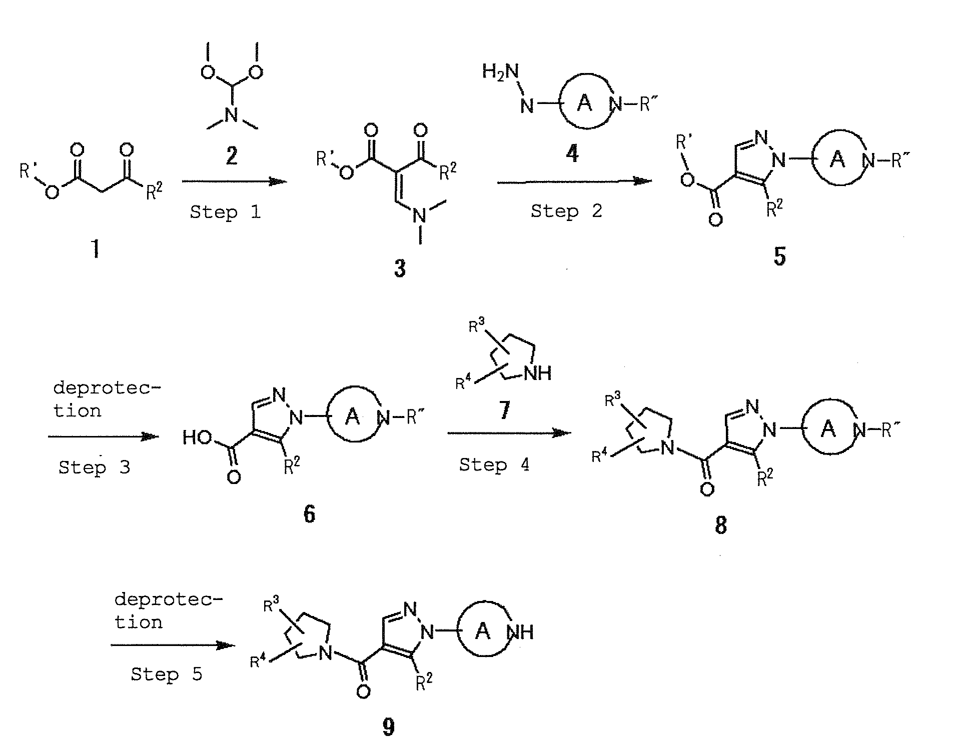

wherein R' is a carboxy-protecting group (here, the carboxy-protecting group means those generally used in the field of organic synthesis chemistry, such as methyl group, ethyl group, propyl group, tert-butyl group, benzyl group, p-methoxybenzyl group and the like), which is in the form of esters capable of being easily led to carboxylic acid by hydrolysis, catalytic hydrogenation and the like, R" is an amino protecting group (here, the amino-protecting group means those generally used in the field of organic synthesis chemistry, such as tert-butoxycarbonyl group, benzyloxycarbonyl group, benzyl group and the like) which can be easily removed by hydrolysis, catalytic hydrogenation and the like, and other symbols are as defined above.

### Step 1

Compound (3) can be obtained by reacting β-ketoester (1) synthesized by a known method with dimethylformamide dimethylacetal (2) in a solvent or in the absence of a solvent. As the solvent, methanol, ethanol, benzene, toluene, xylene, tetrahydrofuran, 1,4-dioxane, N,N-dimethylformamide, a mixed solvent thereof and the like can be mentioned. The reaction temperature is generally 20°C to 250°C. The reaction time is generally 1 hr to 24 hr. The amount of dimethylformamide dimethylacetal (2) to be used is generally about 1.5 - about 3 mol, per 1 mol of β-ketoester (1). The amount of the solvent to be used is not particularly limited and can be appropriately determined depending on the kind and the amount of use of the reaction substrate (β-ketoester (1) and dimethylformamide dimethylacetal (2)), reaction temperature, reaction time and the like (the same applies to the amount of the solvent to be used in each Step to be explained below).
As dimethylformamide dimethylacetal (2), a commercially available product may be directly used or it may be synthesized according to a method known per se.

### Step 2: Step for constructing pyrazole ring

Compound (5) is obtained by reacting hydrazine (4) synthesized by a known method or a salt thereof with compound (3) in a solvent. As the solvent, methanol, ethanol, n-propanol, n-butanol, isopropanol, acetonitrile, diethyl ether, tetrahydrofuran, 1,4-dioxane, N,N-dimethylformamide, dichloromethane, chloroform, benzene, toluene, xylene, a mixed solvent thereof and the like can be mentioned. The reaction temperature is generally 20°C to 250°C. The reaction time is generally 1 hr to 24 hr. The amount of hydrazine (4) to be used is generally about 1 - about 2 mol, per 1 mol of compound (3). When hydrazine (4) is a salt, the reaction is carried out in the presence of a base (the amount of the base to be used is generally about 1 - about 2 mol, per 1 mol of compound (3)), such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like.

### Step 3: Step for removing carboxyl-protecting group

Compound (6) is obtained by eliminating the carboxyl-protecting group R' of compound (5) by a known method.

### Step 4: Amidation step

Compound (8) is obtained by reacting compound (6) with amine (7) or a salt thereof (as amine (7), preferred is amine represented by the above-mentioned formula [8], which is the compound of the present invention, hereinafter the same) in the presence of a condensing agent and an additive in a solvent. As the condensing agent, dicyclohexylcarbodiimide (DCC), 1-ethyl-3-(3-dimethylaminopropyl)carbodiimide hydrochloride (WSC.HCl), diisopropylcarbodiimide, 1,1'-carbonyldiimidazole (CDI), diphenylphosphoryl azide (DPPA) and the like can be mentioned. As the additive, 1-hydroxybenzotriazole (HOBT), N-hydroxysuccinimide (HOSu), 4-dimethylaminopyridine (DMAP) and the like can be mentioned. As the solvent, dichloromethane, chloroform, acetonitrile, tetrahydrofuran, 1,4-dioxane, N,N-dimethylformamide, a mixed solvent thereof and the like can be mentioned. The reaction temperature is generally 20°C to 100°C. The reaction time is generally 1 hr to 24 hr. The amount of amine (7) to be used is generally about 1 - about 1.5 mol, per 1 mol of compound (6). The amount of each of the condensing agent and additive to be used is generally about 1 - about 1.5 mol and about 1 - about 1.5 mol, per 1 mol of compound (6). When amine (7) is a salt, the reaction is performed in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like. Alternatively, compound (6) (carboxylic acid) may be induced to an acid halide or a mixed acid anhydride, and reacted with amine (7) in the presence of a base. The amount of the base to be used is generally about 1 - about 1.5 mol, per 1 mol of compound (6).

### Step 5: Step for removing amino-protecting group

Compound (9) is obtained by removing amino-protecting group R" from compound (8) by a known method. Where necessary, a salt may be formed by a known method.

### Production Method 2

In the compound represented by the formula [1'], a compound wherein -X- is -N(R¹)- and R¹ is -CONR⁵R⁶ wherein R⁵ is a hydrogen atom can be produced by the following Steps.

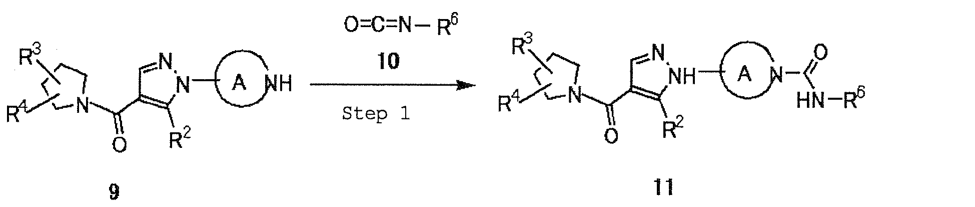

wherein each symbol is as defined above.

### Step 1: Ureation step

### 1) When R⁶ is other than hydrogen

Compound (11) is obtained by reacting compound (9) obtained in Production Method 1 or a salt thereof with isocyanate (10) in a solvent. As the solvent, acetonitrile, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 1 hr to 24 hr. The amount of isocyanate (10) to be used is generally about 1 - about 2 mol, per 1 mol of compound (9). When compound (9) is a salt, the reaction may be performer in the presence of a base such as triethylamine, N,N-diisopropylethylamine, pyridine and the like (the amount of the base to be used is generally about 1 - about 2 mol, per 1 mol of compound (9)). In addition, compound (9) may be reacted with 1,1'-carbonyldiimidazole (CDI) (the amount of CDI to be used is generally about 1 - about 3 mol, per 1 mol of compound (9)), and then with amine represented by H₂N-R⁶.
As isocyanate (10), a commercially available product may be directly used, or it may be synthesized according to a method known per se.
When R⁶ is alkyl group, cycloalkyl group, aryl group, heterocyclic group etc. having a functional group, it is appropriately protected with a protecting group and, after ureation, deprotected. When the alkyl group, cycloalkyl group, aryl group, heterocyclic group and the like have substituent(s), they can be converted to other functional groups after ureation by a known method.
For example, conversion from hydroxyl group to alkoxy group or ketone, from amino group to alkylamino group, alkylcarbonylamino group or alkylsulfonylamino group, from alkoxycarbonyl group to carboxyl group, aminocarbonyl group, alkylaminocarbonyl group or hydroxymethyl group, and from sulfide to sulfone or sulfoxide can be mentioned.

### 2) When R⁶ is hydrogen

Compound (11) is obtained by reacting compound (9) or a salt thereof with cyanate such as sodium cyanate and the like in a solvent. As the solvent, acetonitrile, diethyl ether, tetrahydrofuran, 1,4-dioxane, N,N-dimethylformamide, water, acetic acid, a mixed solvent thereof and the like can be mentioned. The reaction temperature is generally 0°C to 100°C. The reaction time is generally 1 hr to 24 hr. The amount of the cyanate to be used is generally about 1 - about 10 mol, per 1 mol of compound (9). When compound (9) is a salt, the reaction may be performed in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like (the amount of the base to be used is generally about 1 - about 2 mol, per 1 mol of compound (9)).

### Production Method 3

In the compound represented by the formula [1'], a compound wherein -X- is -N(R¹) - and R¹ is -CONR⁵R⁶ can be produced by the following Steps.

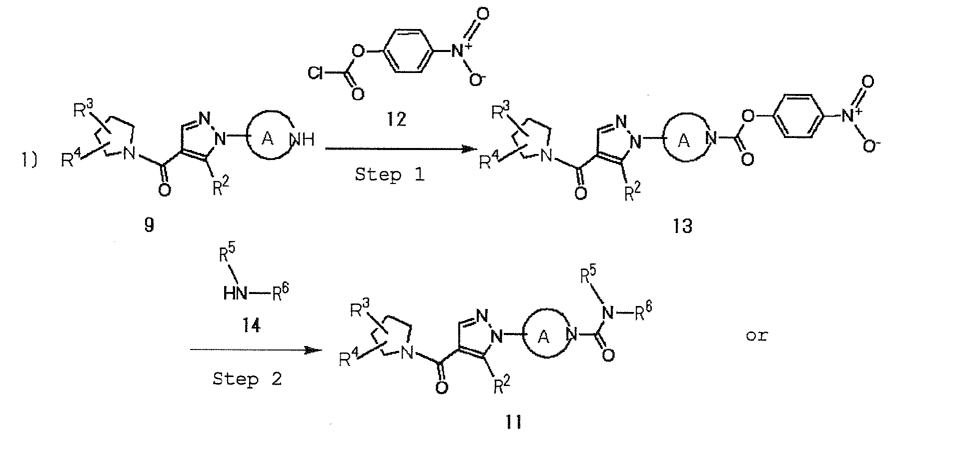

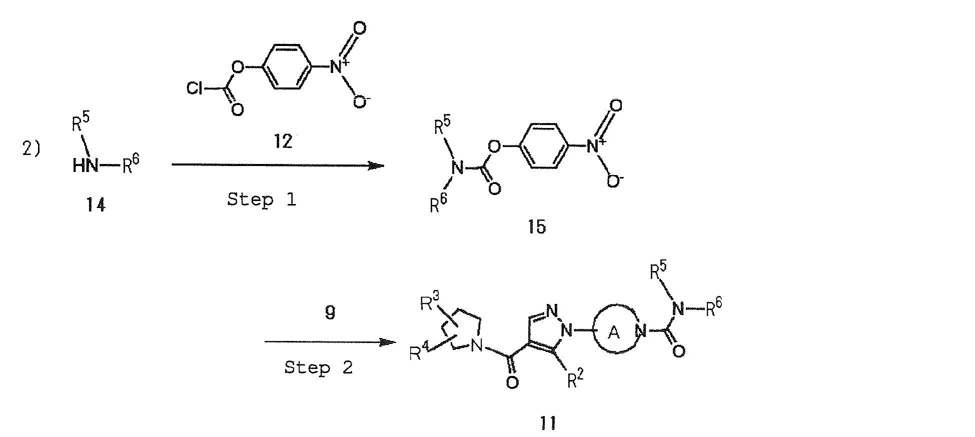

wherein each symbol is as defined above.

### Step 1 of the formula 1): Phenylcarbamation step

Compound (13) is obtained by reacting compound (9) obtained in Production Method 1 or a salt thereof with 4-nitrophenyl chloroformate (12) in the presence of a base in a solvent. As the solvent, acetonitrile, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. As the base, triethylamine, N,N-diisopropylethylamine, pyridine and the like can be mentioned. The reaction temperature is generally 0°C to 100°C. The reaction time is generally 1 hr to 24 hr. The amount of 4-nitrophenyl chloroformate (12) to be used is generally about 1 - about 2 mol, per 1 mol of compound (9). The amount of the base to be used is generally about 1 - about 2 mol, per 1 mol of compound (9). When compound (9) is a salt, the reaction may be performed using about 2 - about 4 mol of a base per 1 mol of compound (9).
As the 4-nitrophenyl chloroformate (12), a commercially available product may be directly used, or it may be synthesized according to a method known per se.

### Step 2 of the formula 1): Ureation step

Compound (11) is obtained by reacting compound (13) (phenylcarbamate intermediate) with amino (14) or a salt thereof in a solvent. As the solvent, acetonitrile, diethyl ether, tetrahydrofuran, 1,4-dioxane, N,N-dimethylformamide, N-methylpyrrolidone, dimethyl sulfoxide, dichloromethane, chloroform, benzene, toluene, xylene, a mixed solvent thereof and the like can be mentioned. The reaction temperature is generally 0°C to 150°C. The reaction time is generally 1 hr to 24 hr. The amount of amine (14) to be used is generally about 1 - about 3 mol, per 1 mol of compound (9). When amine (14) is a salt, the reaction may be performed in the presence of a base such as triethylamine, N,N-diisopropylethylamine, pyridine and the like (the amount of the base to be used is generally about 1 - about 3 mol, per 1 mol of compound (13)).
As amine (14), a commercially available product may be directly used, or it may be synthesized according to a method known per se.

As an alternative method, as shown in the formula 2), compound (15) is obtained by reacting amine (14) with 4-nitrophenyl chloroformate (12) in a solvent in Step 1, then in Step 2, compound (15) is reacted with compound (9) obtained in Production Method 1, whereby compound (11) can be obtained.
In addition, other chloroformate, 1,1'-carbonyldiimidazole (CDI), may be used instead of 4-nitrophenyl chloroformate (12).
When R⁵ and/or R⁶ are/is alkyl group, cycloalkyl group, aryl group, heterocyclic group etc. each having a functional group, they are appropriately protected with a protecting group, and after ureation, deprotected. When alkyl group, cycloalkyl group, aryl group, heterocyclic group and the like have a substituent, they can also be converted, after ureation, to other functional groups by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 4

In the compound represented by the formula [1'], a compound wherein -X- is -N(R¹)-, and R¹ is -CONR⁵R⁶ wherein R⁵ is a hydrogen atom and R⁶ is -S(=O)₂R⁹), can be produced by the following Steps.

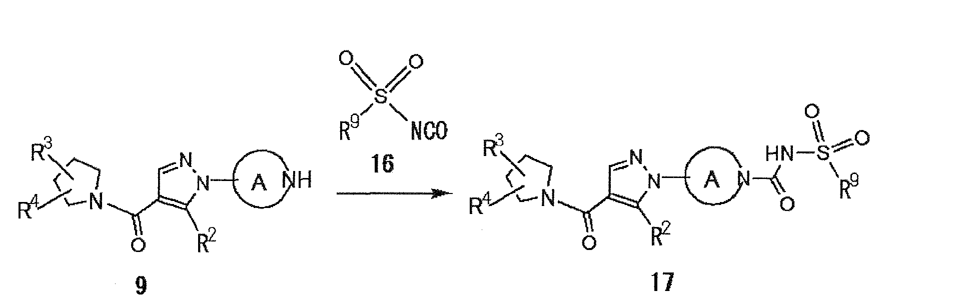

wherein each symbol is as defined above.

### Step 1: Sulfonylureation step

Compound (17) is obtained by reacting compound (9) obtained in Production Method 1 or a salt thereof with sulfonylisocyanate (16) in a solvent. As the solvent, acetonitrile, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 1 hr to 24 hr. The amount of sulfonylisocyanate (16) to be used is generally about 1 - about 2 mol, per 1 mol of compound (9). When compound (9) is a salt, the reaction may be performed in the presence of a base such as triethylamine, N,N-diisopropylethylamine, pyridine and the like (the amount of the base to be used is generally about 1 - about 2 mol, per 1 mol of compound (9)).
As sulfonylisocyanate (16), a commercially available product may be directly used, or it may be synthesized according to a method known per se.

### Production Method 5

In the compound represented by the formula [1'], a compound wherein -X- is -N(R¹)- and R¹ is -COOR¹⁰ can be produced by the following Steps.

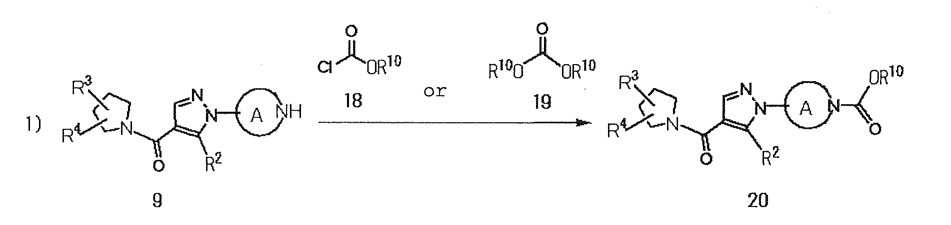

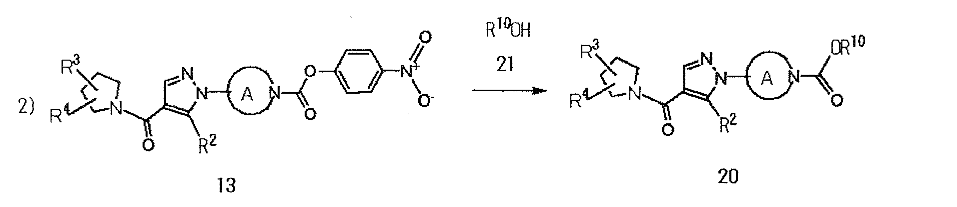

wherein each symbol is as defined above.

### Step 1 of the formula 1): Carbamation step

Compound (20) is obtained by reacting compound (9) obtained in Production Method 1 or a salt thereof with chlorocarbonate (18) or carbonate (19) in the presence of a base in a solvent or without solvent. As the solvent, acetonitrile, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. As the base, triethylamine, N,N-diisopropylethylamine, pyridine and the like can be mentioned. The reaction temperature is generally 0°C to 150°C. The reaction time is generally 1 hr to 24 hr. The amount of chlorocarbonate (18) or carbonate (19) to be used is generally about 1 - about 2 mol, per 1 mol of compound (9). The amount of the base to be used is generally about 1 - about 2 mol, per 1 mol of compound (9). When compound (9) is a salt, the reaction may be performed using about 2 - about 4 mol of a base, per 1 mol of compound (9).
As chlorocarbonate (18) and carbonate (19), commercially available products may be directly used, or they may be synthesized according to a method known per se.
When R¹⁰ is alkyl group, cycloalkyl group, aryl group, heterocyclic group etc. each having a functional group, they are appropriately protected with a protecting group, and deprotected after carbamation. When the alkyl group, cycloalkyl group, aryl group, heterocyclic group and the like have a substituent, they can also be converted, after carbamation, to other functional groups by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

As an alternative method, as shown in the formula 2), compound (20) is obtained by reacting phenylcarbamate intermediate (13) produced in Production Method 3, Step 1 of the formula 1) with alcohol (21) in the presence of a base in a solvent. As the solvent, acetonitrile, diethyl ether, tetrahydrofuran, 1,4-dioxane, N,N-dimethylformamide, N-methylpyrrolidone, dimethyl sulfoxide, dichloromethane, chloroform, benzene, toluene, xylene, a mixed solvent thereof and the like can be mentioned. As the base, sodium hydride, potassium hydride, sodium carbonate, potassium carbonate, sodium hydroxide, potassium hydroxide, lithium hydroxide, triethylamine, N,N-diisopropylethylamine, pyridine and the like can be mentioned. The reaction temperature is generally 0°C to 150°C. The reaction time is generally 1 hr to 24 hr. The amount of alcohol (21) to be used is generally about 1 - about 3 mol, per 1 mol of phenylcarbamate intermediate (13). The amount of the base to be used is generally about 1 - about 2 mol, per 1 mol of phenylcarbamate intermediate (13).
When R¹⁰ is alkyl group, cycloalkyl group, aryl group, heterocyclic group etc. each having a functional group, they are appropriately protected with a protecting group, and deprotected after carbamation. When alkyl group, cycloalkyl group, aryl group, heterocyclic group and the like have a substituent, they can also be converted, after carbamation, to other functional groups by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 6

In the compound represented by the formula [1'], a compound wherein -X- is -N(R¹)- and R¹ is -COR¹¹ can be produced by the following Steps.

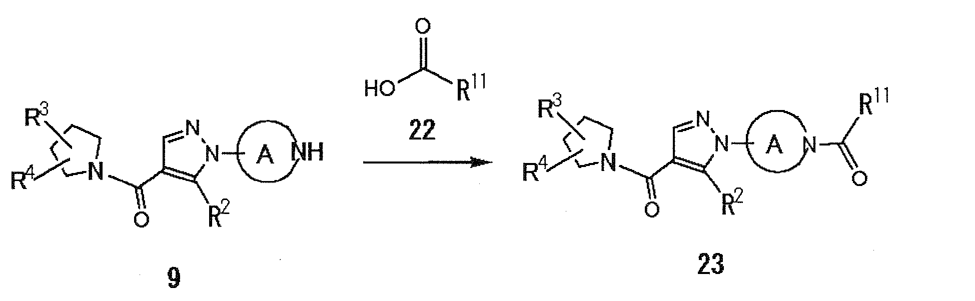

wherein each symbol is as defined above.

### Step 1: Amidation step

Compound (23) is obtained by reacting compound (9) obtained in Production Method 1 or a salt thereof with carboxylic acid (22) in the presence of a condensing agent and an additive in a solvent. As the condensing agent, dicyclohexylcarbodiimide (DCC), 1-ethyl-3-(3-dimethylaminopropyl)carbodiimide hydrochloride (WSC_{·}HCl), diisopropylcarbodiimide, 1,1'-carbonyldiimidazole (CDI), diphenylphosphoryl azide (DPPA) and the like can be mentioned. As the additive, 1-hydroxybenzotriazole (HOBT), N-hydroxysuccinimide (HOSu), 4-dimethylaminopyridine (MAP) and the like can be mentioned. As the solvent, dichloromethane, chloroform, acetonitrile, tetrahydrofuran, 1,4-dioxane, N,N-dimethylformamide, a mixed solvent thereof and the like can be mentioned. The reaction temperature is generally 20°C to 100°C. The reaction time is generally 1 hr to 24 hr. The amount of carboxylic acid (22) to be used is generally about 1 - about 1.5 mol, per 1 mol of compound (9). The amount of each of the condensing agent and additive to be used is generally about 1 - about 1.5 mol and about 1 - about 1.5 mol, per 1 mol of compound (9). When compound (9) is a salt, the reaction may be performed in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like. In addition, carboxylic acid (22) may be induced to acid halide or mixed acid anhydride, and reacted with compound (9) in the presence of a base. The amount of the base to be used is generally about 1 - about 1.5 mol, per 1 mol of compound (9).
As carboxylic acid (22), a commercially available product may be directly used, or it may be synthesized according to a method known per se.

When R¹¹ is alkyl group, cycloalkyl group, aryl group, heterocyclic group etc. each having a functional group, they are appropriately protected with a protecting group, and deprotected after amidation. When alkyl group, cycloalkyl group, aryl group, heterocyclic group and the like have a substituent, they can also be converted, after amidation, to other functional groups by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 7

In the compound represented by the formula [1'], a compound wherein -X- is -N(R¹)- and R¹ is C₁₋₆ alkyl group or cycloalkyl group can be produced by the following Steps.

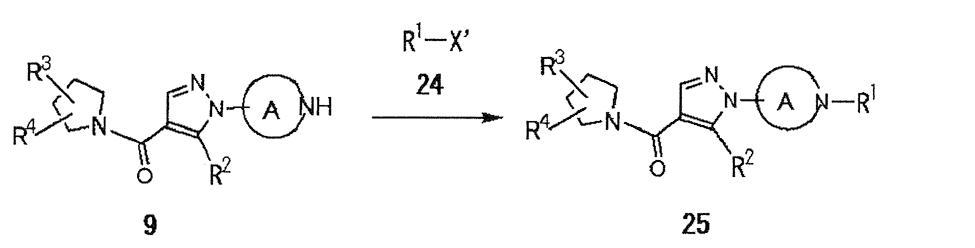

wherein X' is a halogen atom and other symbols are as defined above.

### Step 1: N-alkylation step.

Compound (25) is obtained by reacting compound (9) obtained in Production Method 1 or a salt thereof with alkyl halide or cycloalkyl halide (24) in the presence of a base in a solvent. As the base, sodium carbonate, potassium carbonate, sodium hydrogen carbonate, potassium hydrogen carbonate, sodium acetate, potassium acetate, sodium hydroxide, potassium hydroxide, lithium hydroxide, sodium hydride, potassium hydride, triethylamine, N,N-diisopropylethylamine, pyridine and the like can be mentioned. As the solvent, acetonitrile, acetone, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 30 min to 24 hr. The amount of alkyl halide or cycloalkyl halide (24) to be used is generally about 1 - about 2 mol, per 1 mol of compound (9). The amount of the base to be used is generally about 1 - about 2 mol, per 1 mol of compound (9). When compound (9) is a salt, the reaction may be performed using about 2 - about 4 mol of a base, per 1 mol of compound (9). In addition, the reductive amination may be performed using compound (9) and aldehyde, ketone, or an equivalent thereof corresponding to alkyl halide or cycloalkyl halide (24).
As alkyl halide and cycloalkyl halide (24), commercially available products may be directly used, or they may be synthesized according to a method known per se.

When R¹ is an alkyl group or cycloalkyl group having a functional group, it is appropriately protected with a protecting group, and deprotected after N-alkylation or N-cycloalkylation. When the alkyl group and cycloalkyl group have a substituent, they can also be converted, after N-alkylation or N-cycloalkylation, to other functional groups by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 8

In the compound represented by the formula [1'], a compound wherein -X- is -N(R¹)- and R¹ is -S(=O)₂R¹² can be produced by the following Steps.

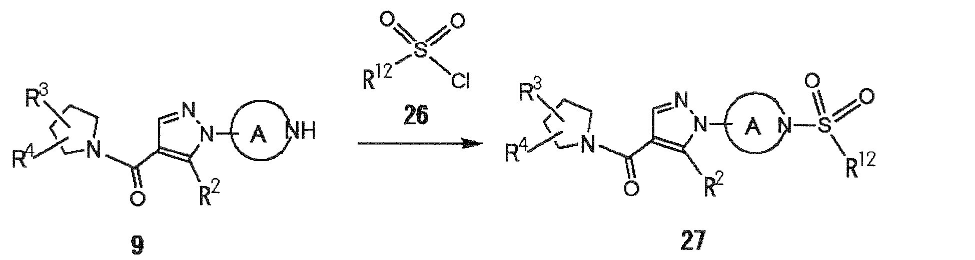

wherein each symbol is as defined above.

### Step 1: Sulfonylation step

compound (27)is obtained by reacting compound (9) obtained in Production Method 1 or a salt thereof with sulfonyl chloride (26) in the presence of a base in a solvent. As the base, sodium carbonate, potassium carbonate, sodium hydrogen carbonate, potassium hydrogen carbonate, sodium acetate, potassium acetate, sodium hydroxide, potassium hydroxide, lithium hydroxide, sodium hydride, potassium hydride, triethylamine, N,N-diisopropylethylamine, pyridine and the like can be mentioned. As the solvent, acetonitrile, acetone, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 30 min to 24 hr. The amount of sulfonyl chloride (26) to be used is generally about 1 - about 2 mol, per 1 mol of compound (9). The amount of the base to be used is generally about 1 - about 2 mol, per 1 mol of compound (9). When compound (9) is a salt, the reaction may be performed using about 2 - about 4 mol of a base, per 1 mol of compound (9). In addition, compound (9) may be reacted with a sulfonic acid anhydride corresponding to sulfonyl chloride (26).
As the sulfonyl chloride (26), a commercially available product may be directly used, or it may be synthesized according to a method known per se.

When R¹² is an alkyl group having a functional group, it is appropriately protected with a protecting group, and deprotected after sulfonylation. When the alkyl group has a substituent, it can also be converted, after sulfonylation, to other functional group by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 9

In the compound represented by the formula [1'], a compound wherein -X- is -N(R¹)- and R¹ is -C(=NCN)R¹³ can be produced by the following Steps.

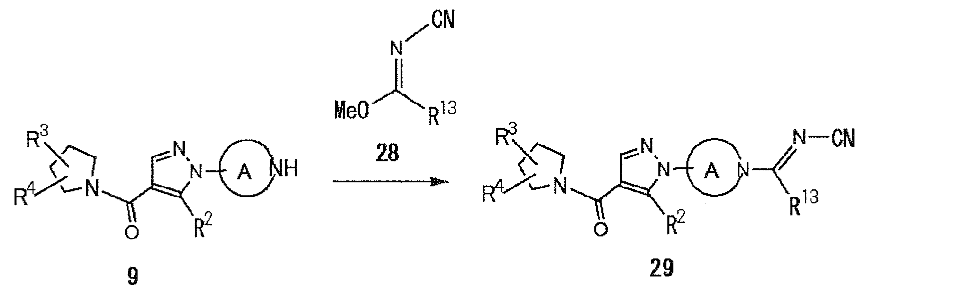

wherein each symbol is as defined above.

### Step 1: Step for producing N-cyanoamidine

Compound (29) is obtained by reacting compound (9) obtained in Production Method 1 or a salt thereof with N-cyanoimidoacetate (28) in a solvent. As the solvent, acetonitrile, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 1 hr to 24 hr. The amount of N-cyanoimidoacetate (28) to be used is generally about 1 - about 2 mol, per 1 mol of compound (9). When compound (9) is a salt, the reaction may be performed in the presence of a base such as triethylamine, N,N-diisopropylethylamine, pyridine and the like (the amount of the base to be used is generally about 1 - about 2 mol, per 1 mol of compound (9)).
As N-cyanoimidoacetate (28), a commercially available product may be directly used, or it may be synthesized according to a method known per se.

### Production Method 10

In the compound represented by the formula [1'], a compound wherein -X- is -N(R¹)- and R¹ is -C(=NCN)NR¹⁴R¹⁵ can be produced by the following Steps.

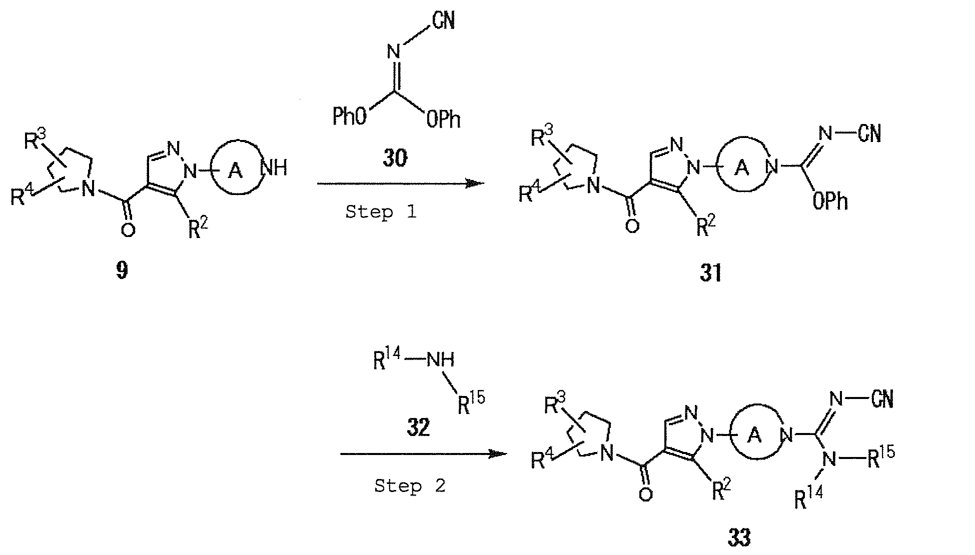

wherein Ph is a phenyl group and other symbols are as defined above.

### Step 1

Compound (31) is obtained by reacting compound (9) obtained in Production Method 1 or a salt thereof with diphenyl N-cyanocarbonimidate (30) in a solvent. As the solvent, acetonitrile, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 1 hr to 24 hr. The amount of diphenyl N-cyanocarbonimidate (30) to be used is generally about 1 - about 2 mol, per 1 mol of compound (9). When compound (9) is a salt, the reaction may be performed in the presence of a base such as triethylamine, N,N-diisopropylethylamine, pyridine and the like (the amount of the base to be used is generally about 1 - about 2 mol, per 1 mol of compound (9)).
As diphenyl N-cyanocarbonimidate (30), a commercially available product may be directly used, or it may be synthesized according to a method known per se.

### Step 2: Step for producing N-cyanoguanidine

Compound (33) is obtained by reacting compound (31) with amine (32) or a salt thereof in a solvent. As the solvent, acetonitrile, diethyl ether, tetrahydrofuran, 1,4-dioxane, N,N-dimethylformamide, N-methylpyrrolidone, dimethyl sulfoxide, dichloromethane, chloroform, benzene, toluene, xylene, a mixed solvent thereof and the like can be mentioned. The reaction temperature is generally 0°C to 150°C. The reaction time is generally 1 hr to 24 hr. The amount of amine (32) to be used is generally about 1 - about 5 mol, per 1 mol of compound (31). When amine (32) is a salt, the reaction may be performed in the presence of a base such as triethylamine, N,N-diisopropylethylamine, pyridine and the like (the amount of the base to be used is generally about 1 - about 20 mol, per 1 mol of compound (31)).
As amine (32), a commercially available product may be directly used, or it may be synthesized according to a method known per se.

### Production Method 11

In the compound represented by the formula [1'], a compound wherein -X- is -N(R¹)- and R¹ is an aryl group or unsaturated monocyclic 5-membered or 6-membered heterocyclic group, can be produced by the following Steps.

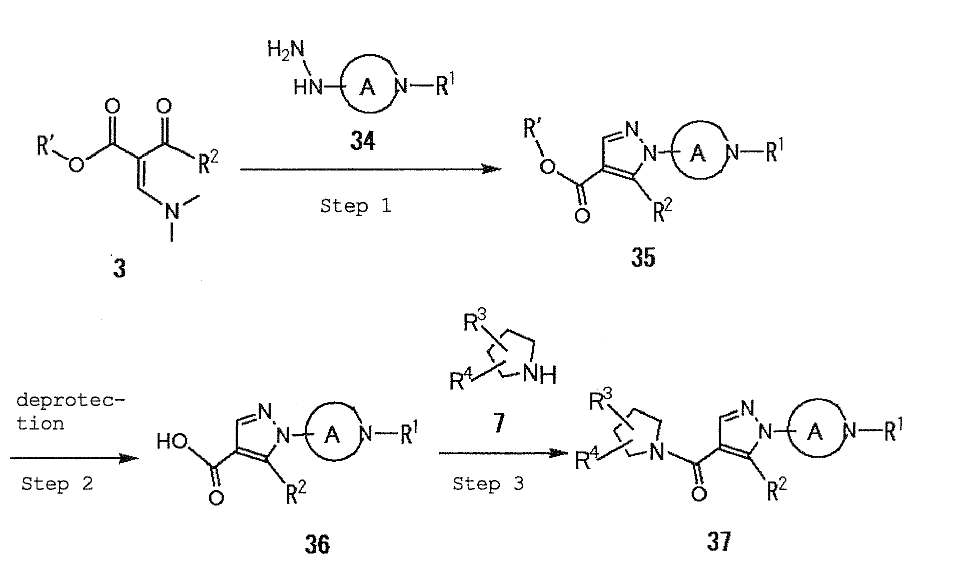

wherein each symbol is as defined above.

### Step 1: Step for constructing pyrazole ring from hydrazine (34) and compound (3)

Compound (35) is obtained by reacting hydrazine (34) synthesized by a known method or a salt thereof with compound (3) obtained in Production Method 1, Step 1 in a solvent. As the solvent, methanol, ethanol, n-propanol, n-butanol, isopropanol, acetonitrile, diethyl ether, tetrahydrofuran, 1,4-dioxane, N,N-dimethylformamide, dichloromethane, chloroform, benzene, toluene, xylene, a mixed solvent thereof and the like can be mentioned. The reaction temperature is generally 20°C to 250°C. The reaction time is generally 1 hr to 24 hr. The amount of hydrazine (34) to be used is generally about 1.5 - about 3 mol, per 1 mol of compound (3). When hydrazine (34) is a salt, the reaction may be performed in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like (the amount of the base to be used is generally about 1.5 - about 3 mol, per 1 mol of compound (3)).

### Step 2: Step for removing carboxyl-protecting group R'

Compound (36) is obtained by removing carboxyl-protecting group R' by a known method.

### Step 3: Amidation step

Compound (37) is obtained by reacting compound (36) with amine (7) or a salt thereof in the presence of a condensing agent and an additive in a solvent. As the condensing agent, dicyclohexylcarbodiimide (DCC), 1-ethyl-3-(3-dimethylaminopropyl)carbodiimide hydrochloride (WSC·HCl), diisopropylcarbodiimide, 1,1'-carbonyldiimidazole (CDI), diphenylphosphoryl azide (DPPA) and the like can be mentioned. As the additive, 1-hydroxybenzotriazole (HOBT), N-hydroxysuccinimide (HOSu), 4-dimethylaminopyridine and the like can be mentioned. As the solvent, dichloromethane, chloroform, acetonitrile, tetrahydrofuran, 1,4-dioxane, N,N-dimethylformamide, a mixed solvent thereof and the like can be mentioned. The reaction temperature is generally 20°C to 100°C. The reaction time is generally 1 hr to 24 hr. The amount of amine (7) to be used is generally about 1 - about 1.5 mol, per 1 mol of compound (36). In addition, the amount of the condensing agent and additive to be used is generally about 1 - about 1.5 mol and about 1 - about 1.5 mol, respectively, per 1 mol of compound (36). When amine (7) is a salt, the reaction may be performed in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like. In addition, compound (36) (carboxylic acid) may be induced to acid halide, and reacted with amine (7) in the presence of a base. The amount of the base to be used is generally about 1 - about 1.5 mol, per 1 mol of compound (36).

When R¹ is an aryl group or unsaturated monocyclic 5-membered or 6-membered heterocyclic group, having a functional group, it is appropriately protected with a protecting group, and deprotected after this step. When the aryl group or unsaturated monocyclic 5-membered or 6-membered heterocyclic group has a substituent, it can also be converted, after this step, to other functional group by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 12

In the compound represented by the formula [1'], a compound wherein -X- is -N(R¹)- and R¹ is an aryl group or unsaturated monocyclic 5-membered or 6-membered heterocyclic group, can be produced by the following Steps.

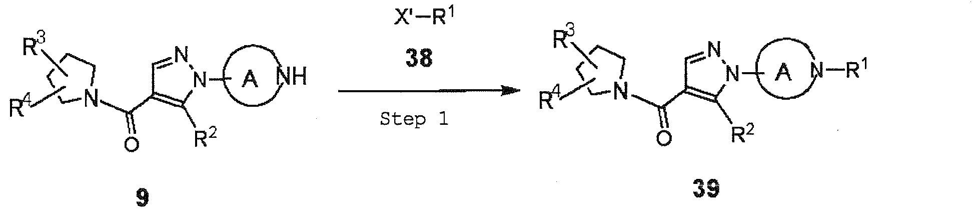

wherein each symbol is as defined above.

### Step 1: Step for palladium catalyzed Buchwald/Hartwig type amination

Compound (39) is obtained by reacting compound (9) obtained in Production Method 1 with aromatic halide or heteroaromatic halide (38) in the presence of a palladium catalyst and a base in a solvent. As the palladium catalyst, a mixture of palladium acetate and 2,2'-bis(diphenylphosphino)-1,1'-binaphthyl, bis(diphenylphosphino)ferrocene palladium(II) chloride, tris(dibenzylideneacetone)dipalladium and the like can be mentioned. As the base, tripotassium phosphate (K₃PO₄), sodium carbonate, potassium carbonate, cesium carbonate, sodium hydrogen carbonate, potassium hydrogen carbonate, potassium tert-butoxide and the like can be mentioned. As the solvent, 1,2-dimethoxyethane, tetrahydrofuran, 1,4-dioxane, benzene, toluene, xylene, tert-butanol, a mixed solvent thereof and the like can be mentioned. The reaction temperature is generally 20°C to 250°C. The reaction time is generally 1 hr to 24 hr. The amount of aromatic halide or heteroaromatic halide (38) to be used is generally about 1 - about 1.5 mol, per 1 mol of compound (9). In addition, the amount of the palladium catalyst and base to be used is generally about 0.01 - about 0.1 mol and about 1 - about 2 mol, respectively, per 1 mol of compound (9).
As aromatic halide or heteroaromatic halide (38), a commercially available product may be directly used, or it may be synthesized according to a method known per se.

When R¹ is an aryl group or unsaturated monocyclic 5-membered or 6-membered heterocyclic group having a functional group, it is appropriately protected with a protecting group, and deprotected after this step. When the aryl group or unsaturated monocyclic 5-membered or 6-membered heterocycle has a substituent, it can also be converted, after this step, to other functional group by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 13

In the compound represented by the formula [1'], a compound wherein -X- is -N(R¹)- and R¹ is a thiazolyl group can be produced by the following Steps.

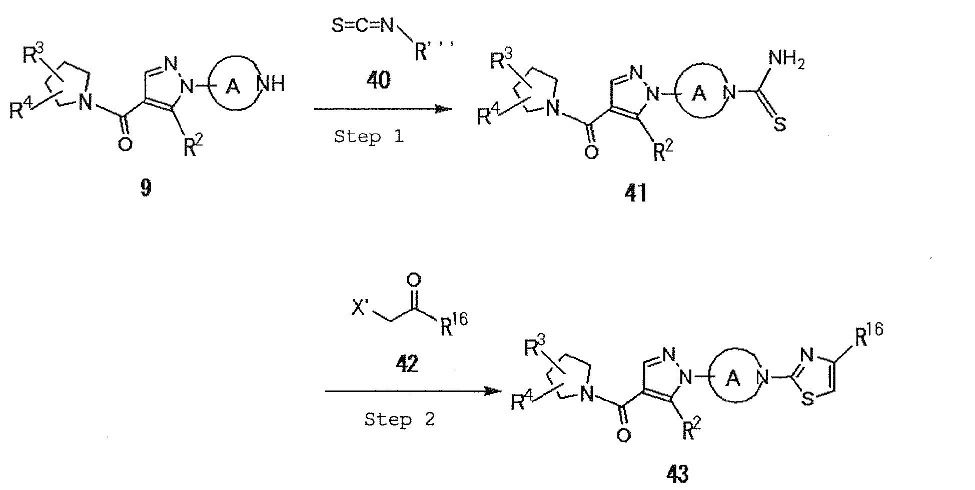

wherein X' is a halogen atom, R"' is a substituent (e.g., ethoxycarbonyl group, benzoyl group, 9-fluorenylmethoxycarbonyl group and the like) on the nitrogen atom, and other symbols are as defined above.

### Step 1: Thioureation step

Compound (41) is obtained by reacting compound (9) obtained in Production Method 1 with isothiocyanate (40) in a solvent and removing the substituent (R"') on the nitrogen atom by a known method. As isothiocyanate (40), ethoxycarbonylisothiocyanate, benzoylisothiocyanate, 9-fluorenylmethoxycarbonylisothiocyanate and the like can be mentioned. As the solvent, acetonitrile, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 1 hr to 24 hr. The amount of isothiocyanate (40) to be used is generally about 1 - about 2 mol, per 1 mol of compound (9). In addition, compound (9) may be treated with thiophosgene (the amount of thiophosgene to be used is generally about 1 - about 1.5 mol, per 1 mol of compound (9)) and then with ammonia.
As isothiocyanate (40), a commercially available product may be directly used, or it may be synthesized according to a method known per se.

### Step 2: Step for constructing thiazole ring

Compound (43) is obtained by reacting compound (41) with halomethylketone (42) in a solvent. As the solvent, methanol, ethanol, n-propanol, n-butanol, isopropanol, acetonitrile, diethyl ether, tetrahydrofuran, 1,4-dioxane, N,N-dimethylformamide, dichloromethane, chloroform, benzene, toluene, xylene, a mixed solvent thereof and the like can be mentioned. The reaction temperature is generally 20°C to 250°C. The reaction time is generally 1 hr to 24 hr. The amount of halomethylketone (42) to be used is generally about 1 - about 1.5 mol, per 1 mol of compound (41).
As halomethylketone (42), a commercially available product may be directly used, or it may be synthesized according to a method known per se.

A thiazolyl group having a functional group is appropriately protected with a protecting group, and deprotected after this step. When the thiazolyl group has a substituent, it can also be converted, after this step, to other functional groups by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 14

In the compound represented by the formula [1'], a compound wherein -X- is -N(R¹)- and R¹ is a triazolyl group can be produced by the following Steps.

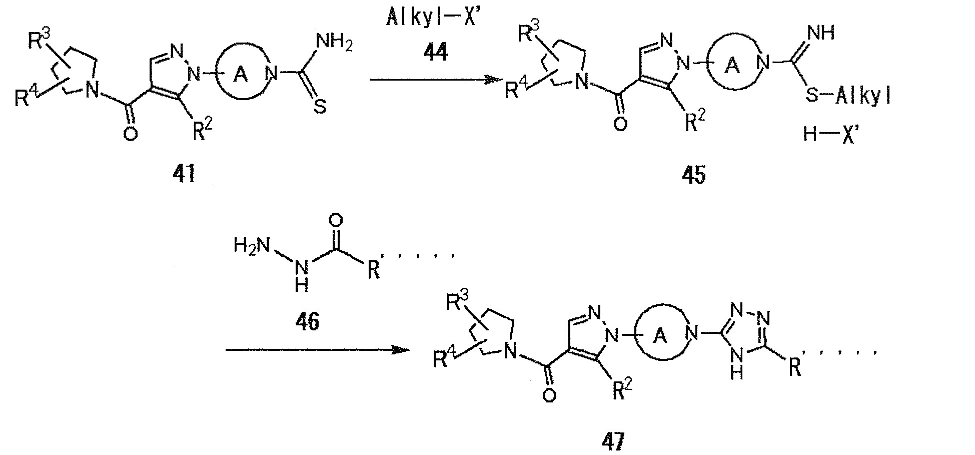

wherein X' is a halogen atom, Alkyl is a C₁₋₆ alkyl group, R""' is a carboxyl group, a C₁₋₆ alkyl group (which is optionally substituted by one or more, same or different substituents selected from a halogen atom and a hydroxyl group) or a cycloalkyl group, and other symbols are as defined above.

### Step 1

Isothiourea·hydrohalic acid salt (45) is obtained by reacting compound (41) obtained in Production Method 13, Step 1 with alkylhalide (44) in a solvent. As the solvent, acetonitrile, acetone, diethyl ether, tetrahydrofuran, 1,4-dioxane, N,N-dimethylformamide, dimethyl sulfoxide, dichloromethane, chloroform, benzene, toluene, xylene, a mixed solvent thereof and the like can be mentioned. The reaction temperature is generally 0°C to 100°C. The reaction time is generally 30 min to 24 hr. The amount of alkylhalide (44) to be used is generally about 1 - about 2 mol, per 1 mol of compound (41).
As alkylhalide (44), a commercially available product may be directly used, or it may be synthesized according to a method known per se.

### Step 2: Step for constructing triazole ring from isothiourea and hydrazide

Compound (47) is obtained by reacting isothiourea· hydrohalic acid salt (45) with hydrazide (46) synthesized by a known method or a salt thereof in the presence of a base in a solvent. As the solvent, methanol, ethanol, n-propanol, isopropanol, n-butanol, acetonitrile, diethyl ether, tetrahydrofuran, 1,4-dioxane, N,N-dimethylformamide, dimethyl sulfoxide, dichloromethane, 1,2-dichloroethane, chloroform, benzene, chlorobenzene, o-dichlorobenzene, toluene, xylene, pyridine, 2,6-lutidine, 2,4,6-collidine, acetic acid, water, a mixed solvent thereof and the like can be mentioned. As the base, sodium hydroxide, potassium hydroxide, lithium hydroxide, sodium carbonate, potassium carbonate, sodium hydrogen carbonate, potassium hydrogen carbonate, sodium acetate, potassium acetate, sodium hydride, potassium hydride, triethylamine, N,N-diisopropylethylamine, pyridine and the like can be mentioned. The reaction temperature is generally 20°C to 250°C. The reaction time is generally 30 min to 24 hr. The amount of hydrazide (46) to be used is generally about 1 - about 2 mol, per 1 mol of isothiourea·hydrohalic acid salt (45). The amount of the base to be used is generally about 1 - about 2 mol, per 1 mol of isothiourea·hydrohalic acid salt (45). When hydrazide (46) is a salt, the reaction may be performed using about 2 - about 4 mol of a base, per 1 mol of isothiourea·hydrohalic acid salt (45).

A triazolyl group having a functional group is appropriately protected with a protecting group, and deprotected after this step. When the triazolyl group has a substituent, it can also be converted, after this step, to other functional groups by a known method. Examples thereof include conversion of hydroxyl group to alkoxy group or ketone, conversion of amino group to alkylamino group, alkylcarbonylamino group, alkylsulfonylamino group, conversion of alkoxycarbonyl group to carboxyl group, aminocarbonyl group, alkylaminocarbonyl group, hydroxymethyl group, and conversion of sulfide to sulfone, sulfoxide.

### Production Method 15

In the compound represented by the formula [1'], a compound wherein -X- is -N(R¹)- can be produced by the following Steps.

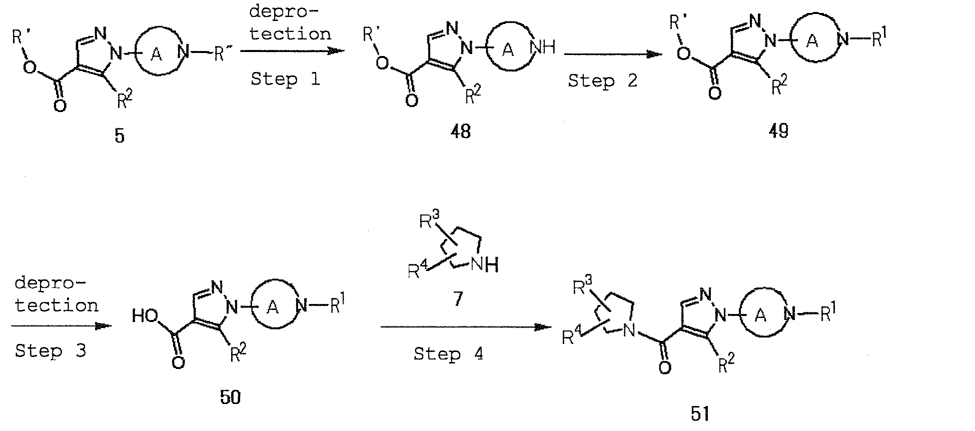

wherein each symbol is as defined above.

### Step 1: Step for removing amino protecting group

Compound (48) is obtained by eliminating the amino protecting group R" of compound (5) obtained in Production Method 1, Step 2.

### Step 2: Introduction of R¹

Compound (49) is obtained by introducing R¹ onto the nitrogen atom in ring A of compound (48) by a method similar to Production Methods 2 to 10, and Production Methods 12 to 14.

### Step 3: Step for removing carboxyl-protecting group

Compound (50) is obtained by eliminating the carboxyl-protecting group R' of compound (49) by a known method.

### Step 4: Amidation step

Compound (51) is obtained by reacting compound (50) with amine (7) or a salt thereof in a solvent in the presence of a condensing agent and an additive. As the condensing agent, dicyclohexylcarbodiimide (DCC), 1-ethyl-3-(3-dimethylaminopropyl)carbodiimide hydrochloride (WSC.HCl), diisopropylcarbodiimide, 1,1'-carbonyldiimidazole (CDI), diphenylphosphoryl azide (DPPA) and the like can be mentioned. As the additive, 1-hydroxybenzotriazole (HOBT), N-hydroxysuccinimide (HOSu), 4-dimethylaminopyridine (DMAP) and the like can be mentioned. As the solvent, dichloromethane, chloroform, tetrahydrofuran, acetonitrile, 1,4-dioxane, N,N-dimethylformamide, a mixed solvent thereof and the like can be mentioned. The reaction temperature is generally 20°C to 100°C. The reaction time is generally 1 hr to 24 hr. The amount of amine (7) to be used is generally about 1 - about 1.5 mol, per 1 mol of compound (50). In addition, the amount of the condensing agent and the additive to be used is generally about 1- about 1.5 mol and about 1- about 1.5 mol, respectively, per 1 mol of compound (50). When amine (7) is a salt, the reaction is carried out in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like (the amount of the base to be used is generally about 1 - about 1.5 mol, per 1 mol of compound (50)).

R¹ having a functional group is appropriately protected with a protecting group and thereafter deprotected. When R¹ has substituent(s), it can be converted to other functional group after this step by a known method. For example, conversion from hydroxyl group to alkoxy group or ketone, from amino group to alkylamino group, alkylcarbonylamino group or alkylsulfonylamino group, from alkoxycarbonyl group to carboxyl group, aminocarbonyl group, alkylaminocarbonyl group or hydroxymethyl group, and from sulfide to sulfone or sulfoxide can be mentioned.

### Production Method 16

In the compound represented by the formula [1'], a compound wherein -X- is -C(R⁷R⁸)- wherein R⁷ is a hydrogen atom and R⁸ is -CONR¹⁸R¹⁹) can be produced by the following Steps.

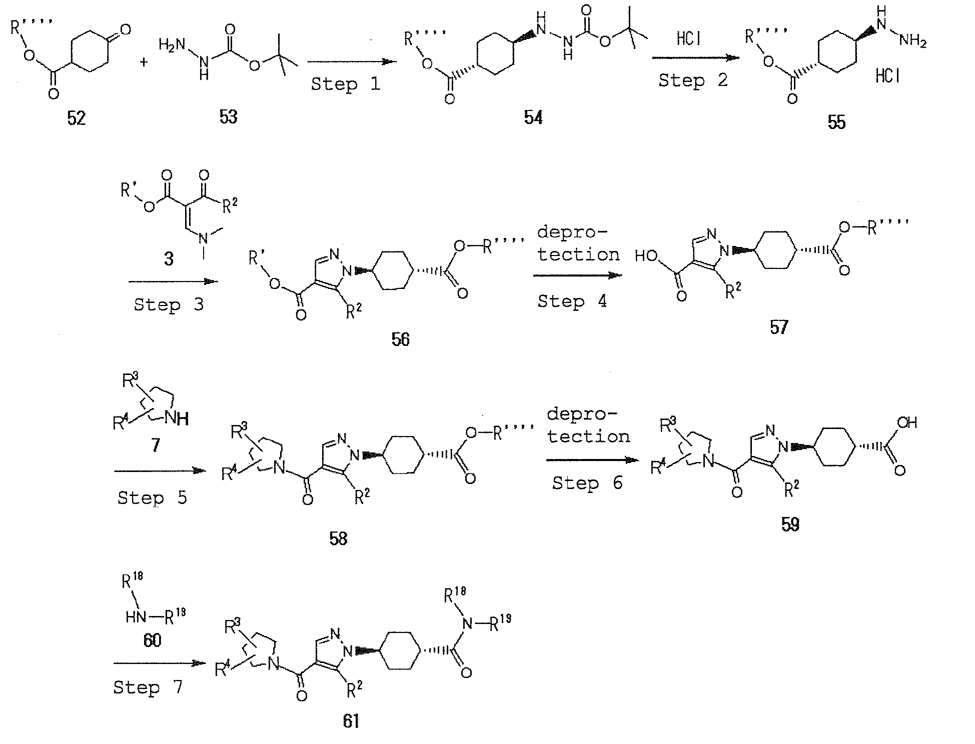

wherein each symbol is as defined above where R"" is a carboxy-protecting group (here, the carboxy-protecting group means those generally used in the field of organic synthesis chemistry, such as methyl group, ethyl group, propyl group, tert-butyl group, benzyl group, p-methoxybenzyl group and the like, which is in the form of esters capable of being easily led to carboxylic acid by hydrolysis, catalytic hydrogenation reaction and the like).

### Step 1: Reductive alkylation

Compound (54) is obtained by reacting ketone (52) synthesized by a conventional method with tert-butyl carbazate (53) in a solvent in the presence of a reducing agent. As the reducing agent, sodium borohydride, sodium cyanoborohydride, sodium triacetoxyborohydride, borane complex and the like can be mentioned. As the solvent, methanol, ethanol, n-propanol, n-butanol, isopropanol, dichloromethane, chloroform, diethyl ether, tetrahydrofuran, 1,4-dioxane, N,N-dimethylformamide, benzene, toluene, xylene, acetic acid, a mixed solvent thereof and the like can be mentioned. The reaction temperature is generally 0°C to 100°C. The reaction time is generally 1 hr to 24 hr. The amount of tert-butyl carbazate (53) to be used is generally about 1 - about 1.5 mol, per 1 mol of ketone (52). The amount of reducing agent to be used is generally about 2 - about 5 mol, per 1 mol of ketone (52).
As tert-butyl carbazate (53), a commercially available product may be directly used, or it may be synthesized according to a method known per se.

### Step 2: elimination of BOC (elimination of tert-butoxycarbonyl group)

Hydrazine hydrochloride (55) is obtained by reacting compound (54) with a hydrogen chloride ethyl acetate solution, or hydrogen chloride 1,4-dioxane solution. The reaction temperature is generally 0°C to 100°C. The reaction time is generally 30 min to 24 hr.
In addition, compound (54) may be reacted with trifluoroacetic acid in a solvent to allow elimination of BOC, after which it is converted to hydrazine hydrochloride (55). The reaction temperature is generally 0°C to 100°C. The reaction time is generally 30 min to 24 hr. The amount of trifluoroacetic acid to be used is generally about 5 - about 100 mol, per 1 mol of compound (54). As the solvent, dichloromethane, chloroform and the like can be mentioned.

### Step 3: Step for constructing pyrazole ring from hydrazine hydrochloride (55) and compound (3)

Compound (56) is obtained by reacting hydrazine hydrochloride (55) with compound (3) obtained in Production Method 1, Step 1 in the presence of a base in a solvent. As the solvent, methanol, ethanol, n-propanol, n-butanol, isopropanol, acetonitrile, diethyl ether, tetrahydrofuran, 1,4-dioxane, N,N-dimethylformamide, dichloromethane, chloroform, benzene, toluene, xylene, a mixed solvent thereof and the like can be mentioned. As the base, sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like can be mentioned. The reaction temperature is generally 20°C to 250°C. The reaction time is generally 1 hr to 24 hr. The amount of hydrazine hydrochloride (55) to be used is generally about 1 - about 2 mol, per 1 mol of compound (3). The amount of the base to be used is generally about 1 - about 2 mol, per 1 mol of compound (3).

### Step 4: Deprotection step of carboxyl-protecting group

Compound (57) is obtained by eliminating carboxyl-protecting group R' of compound (56) by a known method.

### Step 5: Amidation step

Compound (58) is obtained by reacting compound (57) with amine (7) or a salt thereof in a solvent in the presence of a condensing agent and an additive. As the condensing agent, dicyclohexylcarbodiimide (DCC), 1-ethyl-3-(3-dimethylaminopropyl)carbodiimide hydrochloride (WSC.HCl), diisopropylcarbodiimide, 1,1'-carbonyldiimidazole (CDI), diphenylphosphoryl azide (DPPA) and the like can be mentioned. As the additive, 1-hydroxybenzotriazole (HOBT), N-hydroxysuccinimide (HOSu), 4-dimethylaminopyridine (DMAP) and the like can be mentioned. As the solvent, dichloromethane, chloroform, acetonitrile, tetrahydrofuran, 1,4-dioxane, N,N-dimethylformamide, a mixed solvent thereof and the like can be mentioned. The reaction temperature is generally 20°C to 100°C. The reaction time is generally 1 hr to 24 hr. The amount of amine (7) to be used is generally about 1 - about 1.5 mol, per 1 mol of compound (57). In addition, the amount of the condensing agent and additive to be used is generally about 1 - about 1.5 mol and about 1 - about 1.5 mol, respectively, per 1 mol of compound (57). When amine (7) is a salt, the reaction may be performed in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like. In addition, compound (57) (carboxylic acid) may be induced to acid halide or mixed acid anhydride, and reacted with amine (7) in the presence of a base. The amount of the base to be used is generally about 1 - about 1.5 mol, per 1 mol of compound (57).

### Step 6: Step for removing carboxyl-protecting group

Carboxylic acid (59) is obtained by removing the carboxyl-protecting group R"" of compound (58) by a known method.

### Step 7: Amidation step

Compound (61) (amide) is obtained by reacting carboxylic acid (59) with amine (60) or a salt thereof in the presence of a condensing agent and an additive in a solvent. As the condensing agent, dicyclohexylcarbodiimide (DCC), 1-ethyl-3-(3-dimethylaminopropyl)carbodiimide hydrochloride (WSC.HCl) diisopropylcarbodiimide, 1,1'-carbonyldiimidazole (CDI), diphenylphosphoryl azide (DPPA) and the like can be mentioned. As the additive, 1-hydroxybenzotriazole (HOBT), N-hydroxysuccinimide (HOSu), 4-dimethylaminopyridine (DMAP) and the like can be mentioned. As the solvent, dichloromethane, chloroform, acetonitrile, tetrahydrofuran, 1,4-dioxane, N,N-dimethylformamide, a mixed solvent thereof and the like can be mentioned. The reaction temperature is generally 20°C to 100°C. The reaction time is generally 1 hr to 24 hr. The amount of amine (60) to be used is generally about 1 - about 1.5 mol, per 1 mol of carboxylic acid (59). In addition, the amount of the condensing agent and additive to be used is generally about 1 - about 1.5 mol and about 1 - about 1.5 mol, respectively, per 1 mol of compound (59). When amine (60) is a salt, the reaction may be performed in the presence of a base such as sodium carbonate, potassium carbonate, sodium hydrogen carbonate, potassium hydrogen carbonate, sodium acetate, potassium acetate, sodium hydroxide, potassium hydroxide, lithium hydroxide, triethylamine, N,N-diisopropylethylamine, pyridine and the like. In addition, carboxylic acid (59) may be induced to acid halide or mixed acid anhydride, and then reacted with amine (60) in the presence of a base. The amount of the base to be used is generally about 1 - about 1.5 mol, per 1 mol of carboxylic acid (59).
As amine (60), a commercially available product may be directly used, or it may be synthesized according to a method known per se.

### Production Method 17

In the compound represented by the formula [1'], a compound -X- is -C(R⁷R⁸)-wherein R⁷ is a hydrogen atom, R⁸ is - NR¹⁶R¹⁷ wherein R¹⁶ is a hydrogen atom, and R¹⁷ is -CO-NR³⁶R³⁷ can be produced by the following Steps.

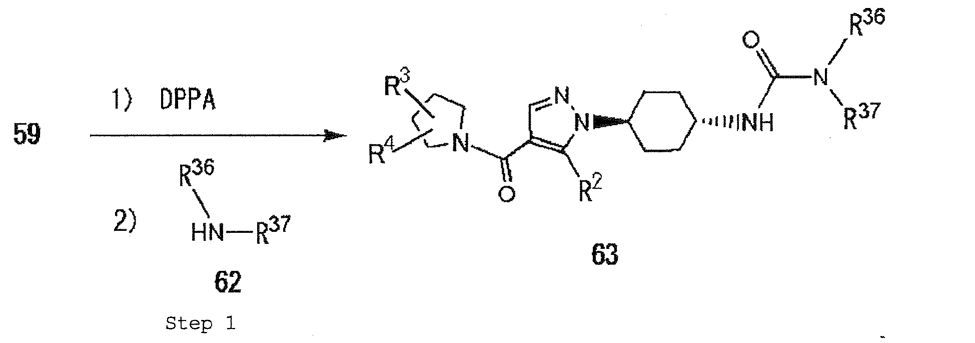

wherein each symbol is as defined above.

### Step 1: Ureation step

Urea (63) is obtained by reacting carboxylic acid (59) obtained in Production Method 16, step 6 with diphenylphosphoryl azide (DPPA) in the presence of a base in a solvent to induce same to isocyanate, and reacting the isocyanate with amine (62). As the solvent, dichloromethane, chloroform, tetrahydrofuran, 1,4-dioxane, N,N-dimethylformamide, benzene, toluene, xylene and the like can be mentioned. As the base, triethylamine, N,N-diisopropylethylamine, pyridine and the like can be mentioned. The reaction temperature of the step for inducing to isocyanate is generally 20°C to 150°C, and that of the step for inducing to urea is generally 0°C to 100°C. The reaction time is generally 1 hr to 24 hr for both steps. The amount of DPPA to be used is generally about 1 - about 2 mol, per 1 mol of carboxylic acid (59). The amount of the base to be used is generally about 1 - about 2 mol, per 1 mol of carboxylic acid (59). The amount of amine (62) to be used is generally about 1 - about 5 mol, per 1 mol of carboxylic acid (59).
As amine (62), a commercially available product may be directly used, or it may be synthesized according to a method known per se.

### Production Method 18

In the compound represented by the formula [1'], a compound wherein -X- is -C(R⁷R⁸)- wherein R⁷ is a hydrogen atom and R⁸ is a tetrazolyl group can be produced by the following Steps.

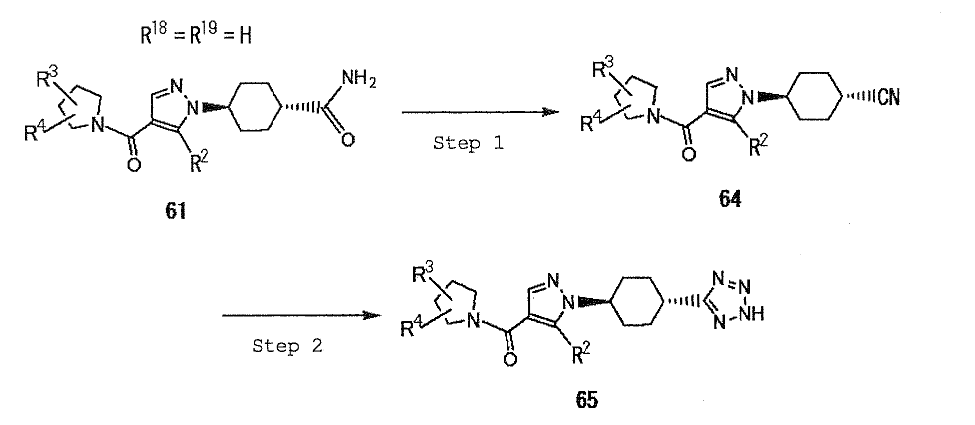

wherein each symbol is as defined above.

### Step 1: Cyanation step

Nitrile (64) is obtained by reacting amide (61) obtained in Production Method 16, step 7 with a dehydrating agent in the presence of a base in a solvent. As the dehydrating agent, trifluoromethanesulfonic acid anhydride, p-toluenesulfonyl chloride, trichloroacetyl chloride, trifluoroacetic acid anhydride, phosphorus oxychloride, thionyl chloride and the like can be mentioned. As the base, triethylamine, N,N-diisopropylethylamine, pyridine and the like can be mentioned. As the solvent, dichloromethane, chloroform, tetrahydrofuran, 1,4-dioxane, N,N-dimethylformamide, pyridine, a mixed solvent thereof and the like can be mentioned. The reaction temperature is generally 20°C to 100°C. The reaction time is generally 1 hr to 24 hr. The amount of dehydrating agent to be used is generally about 1 - about 2 mol, per 1 mol of amide (61). The amount of the base to be used is generally about 1 - about 2 mol, per 1 mol of amide (61).

### Step 2: Step for constructing tetrazole ring

Nitrile (64) is reacted in a solvent in the presence of an azidating reagent to give tetrazole (65). As the azidating reagent, sodium azide, azidotrimethyltin, azidotributyltin, azidotrimethylsilane and the like can be mentioned. As the solvent, dichloromethane, chloroform, tetrahydrofuran, 1,2-dimethoxyethane, 1,4-dioxane, N,N-dimethylformamide, benzene, toluene, xylene, a mixed solvent thereof and the like can be mentioned. The reaction temperature is generally 20°C to 150°C. The reaction time is generally 1 hr to 24 hr. The amount of the azidating reagent to be used is generally about 1 - about 2 mol, per 1 mol of nitrile (64).

The production methods described in this specification are examples of the production methods of the compound represented by the formula [1'], and compounds other than those explained above can be produced by combining the production methods described in the present specification with known conventional methods in the field of organic synthesis chemistry.

### Examples

The compound of the present invention, the compound represented by the formula [1'] and production methods thereof are explained in detail in the following by referring to Examples, which are not to be construed as limitative.
¹H-NMR spectrum was measured in CDCl₃ or DMSO-d₆ with tetramethylsilane as an internal standard, and all δ values are shown in ppm.
The symbols in the Tables mean the following.
s: singlet
d: doublet
t: triplet
dd: double doublet
ddd: double double doublet
brs: broad singlet
m: multiplet
J: coupling constant
[α]²⁵D is specific optical rotation measured using sodium D line (589 nm) at 25°C, and c is the compound (g) in 100 ml of a solution.

### Example 1

### Production of 1-[5-cyclopropyl-1-(piperidin-4-yl)-1H-pyrazole-4-carbonyl]-3-(pyridin-3-yl)pyrrolidine

### Step 1

### Production of methyl 2-cyclopropylcarbonyl-3-dimethylaminoacrylate

Methyl 3-cyclopropyl-3-oxopropionate (2.0 g) was dissolved in toluene (20 ml). To the obtained solution was added dimethylformamide dimethylacetal (3.0 ml) and the mixture was refluxed for 3 hr. The reaction mixture was allowed to cool and concentrated under reduced pressure, and the obtained residue was purified by silica gel column chromatography (n-hexane:acetone=2:1) to give the title compound as a yellow oil (2.61 g).

### Step 2

### Production of methyl 1-(1-benzyloxycarbonylpiperidin-4-yl)-5-cyclopropyl-1H-pyrazole-4-carboxylate

Methyl 2-cyclopropylcarbonyl-3-dimethylaminoacrylate (1.0 g) produced in the previous step and benzyl 4-hydrazinopiperidine-1-carboxylate hydrochloride (1.49 g) synthesized by a known method were suspended in ethanol. To the obtained suspension was added triethylamine (0.78 ml) and the mixture was refluxed for 3.5 hr. Water was added to the reaction mixture and the mixture was extracted with diethyl ether. The diethyl ether layer was washed with water and brine, dried over anhydrous magnesium sulfate and, after filtration, the filtrate was concentrated under reduced pressure. The obtained residue was purified by silica gel column chromatography (n-hexane:ethyl acetate=2.5:1) to give the title compound as a yellow oil (1.58 g).

### Step 3

### Production of 1-(1-benzyloxycarbonylpiperidin-4-yl)-5-cyclopropyl-1H-pyrazole-4-carboxylic acid

Methyl 1-(1-benzyloxycarbonylpiperidin-4-yl)-5-cyclopropyl-1H-pyrazole-4-carboxylate (1.58 g) produced in the previous step was dissolved in tetrahydrofuran (5 ml), methanol (5 ml) and water (10 ml), lithium hydroxide monohydrate (834 mg) was added and the mixture was stirred at 50°C for 6 hr. The reaction mixture was concentrated under reduced pressure, water was added to the obtained residue, and the mixture was acidified with 2N hydrochloric acid and extracted with ethyl acetate. The ethyl acetate layer was washed with brine, dried over anhydrous magnesium sulfate and, after filtration, the filtrate was concentrated under reduced pressure. To the obtained residue were added diethyl ether and n-hexane, and the precipitated solid was collected by filtration, and dried to give the title compound as a white amorphous solid (1.27 g).

### Step 4

### Production of 1-[1-(1-benzyloxycarbonylpiperidin-4-yl)-5-cyclopropyl-1H-pyrazole-4-carbonyl]-3-(pyridin-3-yl)pyrrolidine

1-(1-Benzyloxycarbonylpiperidin-4-yl)-5-cyclopropyl-1H-pyrazole-4-carboxylic acid (800 mg) produced in the previous step, 1-hydroxybenzotriazole (332 mg) and 1-ethyl-3-(3-dimethylaminopropyl)-carbodiimide hydrochloride (498 mg) were dissolved in N,N-dimethylformamide (10 ml), and the mixture was stirred for 15 min. To the obtained solution were added 3-(pyridin-3-yl)pyrrolidine (330 mg) and 4-dimethylaminopyridine (catalytic amount) and the mixture was stirred overnight at room temperature. The reaction mixture was concentrated under reduced pressure, water was added to the obtained residue, and the mixture was extracted with ethyl acetate. The ethyl acetate layer was washed with brine, dried over anhydrous sodium sulfate and, after filtration, the filtrate was concentrated under reduced pressure. The obtained residue was purified by silica gel column chromatography (chloroform:methanol=11:1) to give the title compound as a white amorphous solid (1.06 g).

### Step 5

### Production of 1-[5-cyclopropyl-1-(piperidin-4-yl)-1H-pyrazole-4-carbonyl]-3-(pyridin-3-yl)pyrrolidine

1-[1-(1-Benzyloxycarbonylpiperidin-4-yl)-5-cyclopropyl-1H-pyrazole-4-carbonyl]-3-(pyridin-3-yl)pyrrolidine (970 mg) produced in the previous step was dissolved in methanol (12 ml), 10% palladium carbon (50% water content) (200 mg) was added, and the mixture was stirred under a hydrogen atmosphere for 2.5 hr. The catalyst was filtered off, and the filtrate was concentrated under reduced pressure. The obtained residue was purified by chromatography (neutral alumina) (chloroform:methanol=7:1) to give the title compound as a white amorphous solid (718 mg).

### Example 2

### Production of 1-[5-cyclopropyl-1-(piperidin-4-yl)-1H-pyrazole-4-carbonyl]-[(S)-3-(2-trifluoromethylphenyl)]pyrrolidine hydrochloride

### Step 1

### Production of (S)-3-acetyl-4-isopropyl-5,5-diphenyloxazolidin-2-one

Under an argon atmosphere, (S)-4-isopropyl-5,5-diphenyloxazolidin-2-one (1.56 g) was suspended in tetrahydrofuran (22 ml), and 1.6M n-butyllithium/n-hexane solution (3.64 ml) was added under ice-cooling. After 10 min, acetyl chloride (0.47 ml) was added, and the mixture was stirred overnight while allowing the mixture to gradually return to room temperature. Saturated aqueous ammonium chloride solution was added to the reaction mixture, and the mixture was concentrated under reduced pressure. Water was added to the obtained residue and the mixture was extracted with ethyl acetate. The ethyl acetate layer was washed with 1N hydrochloric acid, water, saturated aqueous sodium hydrogen carbonate solution and water, and dried over anhydrous magnesium sulfate. After filtration, the filtrate was concentrated under reduced pressure, and the obtained residue was purified by silica gel column chromatography (n-hexane:ethyl acetate=4:1) to give the title compound as white crystals (1.69 g).

### Step 2

### Production of (S)-4-isopropyl-3-[(S)-4-nitro-3-(2-trifluoromethylphenyl)butyryl]-5,5-diphenyloxazolidin-2-one

(S)-3-Acetyl-4-isopropyl-5,5-diphenyloxazolidin-2-one (4.74 g) produced in the previous step was dissolved in dichloromethane (73 ml) under an argon atmosphere, and titanium tetrachloride (3.17 ml) and N,N-diisopropylethylamine (3.06 ml) were added at -78°C. The mixture was gradually warmed to 0°C over 30 min, again cooled to -78°C and a solution of 1-[(E)-2-nitrovinyl]-2-trifluoromethylbenzene (3.50 g) in dichloromethane (27 ml) was added. Titanium tetrachloride (3.17 ml) was added and, 2 hr later, saturated aqueous ammonium chloride solution was added to the reaction mixture. The mixture was extracted with ethyl acetate, and the ethyl acetate layer was washed with 1N hydrochloric acid, water, saturated aqueous sodium hydrogen carbonate solution and water, and dried over anhydrous magnesium sulfate. After filtration, the filtrate was concentrated under reduced pressure, and the obtained residue was purified by silica gel column chromatography (n-hexane:ethyl acetate=5:1) and crystallized from diethyl ether:n-pentane to give the title compound as white crystals (2.62 g).

### Step 3

### Production of (S)-4-(2-trifluoromethylphenyl)pyrrolidin-2-one

To (S)-4-isopropyl-3-[(S)-4-nitro-3-(2-trifluoromethylphenyl)butyryl]-5,5-diphenyloxazolidin-2-one (2.51 g) produced in the previous step were added ethanol (35 ml) and ethyl acetate (35 ml), and Raney nickel (50% in water) (2.8 g) was further added. After stirring overnight under a hydrogen atmosphere, the insoluble material was filtered through celite, and the filtrate was concentrated under reduced pressure. The obtained residue was purified by silica gel column chromatography (chloroform:methanol=20:1) to give the title compound as white crystals (850 mg).

### Step 4

### Production of (S)-3-(2-trifluoromethylphenyl)pyrrolidine

To (S)-4-(2-trifluoromethylphenyl)pyrrolidin-2-one (803 mg) produced in the previous step was added tetrahydrofuran (7 ml), and lithium aluminum hydride (200 mg) was further added. After heating under reflux for 1.5 hr, water (0.2 ml), 4N aqueous sodium hydroxide solution (0.2 ml) and water (0.4 ml) were added under ice-cooling. Anhydrous magnesium sulfate was added, the insoluble material was filtered through celite, and the filtrate was concentrated under reduced pressure to give the title compound as a pale-yellow oil (722 mg).
[α]²⁵D=+23.9 (c=0.504, EtOH)

### Step 5

### Production of 1-[1-(1-benzyloxycarbonylpiperidin-4-yl)-5-cyclopropyl-1H-pyrazole-4-carbonyl]-[(S)-3-(2-trifluoromethylphenyl)]pyrrolidine

1-(1-Benzyloxycarbonylpiperidin-4-yl)-5-cyclopropyl-1H-pyrazole-4-carboxylic acid (235 mg) produced in Example 1, Step 3, 1-hydroxybenzotriazole (127 mg) and 1-ethyl-3-(3-dimethylaminopropyl)-carbodiimide hydrochloride (159 mg) were dissolved in N,N-dimethylformamide (6 ml), and the mixture was stirred for 15 min. To the obtained solution were added (S)-3-(2-trifluoromethylphenyl)pyrrolidine (151 mg) produced in the previous step and 4-dimethylaminopyridine (78 mg), and the mixture was stirred overnight at room temperature. The reaction mixture was concentrated under reduced pressure, water was added to the obtained residue, and the mixture was extracted with ethyl acetate. The ethyl acetate layer was washed with water, 1N aqueous potassium hydrogen sulfate solution, saturated aqueous sodium hydrogen carbonate solution and brine, dried over anhydrous sodium sulfate and, after filtration, the filtrate was concentrated under reduced pressure. The obtained residue was purified by silica gel column chromatography (ethyl acetate:methanol=40:1) to give the title compound as a white amorphous solid (287 mg).

### Step 6

### Production of 1-[5-cyclopropyl-1-(piperidin-4-yl)-1H-pyrazole-4-carbonyl]-[(S)-3-(2-trifluoromethylphenyl)]pyrrolidine hydrochloride

1-[1-(1-Benzyloxycarbonylpiperidin-4-yl)-5-cyclopropyl-1H-pyrazole-4-carbonyl]-[(S)-3-(2-trifluoromethylphenyl)]pyrrolidine (2.0 g) produced in the previous step was dissolved in methanol (14 ml), palladium hydroxide carbon (0.2 g) was added, and the mixture was stirred under a hydrogen atmosphere for 15 hr. The catalyst was filtered off through celite, and the filtrate was concentrated under reduced pressure. The residue was dissolved in methanol (6.4 ml), and 4N hydrogen chloride ethyl acetate solution (1.32 ml) was added. The mixture was concentrated under reduced pressure, and the obtained residue was crystallized from ethyl acetate+diisopropyl ether to give the title compound as white crystals (1.45 g).

### Step 7

(S)-3-(2-trifluoromethylphenyl)pyrrolidine produced in the above-mentioned Step 4 and a hydrochloride thereof can also be produced according to the following Step.

### Step 7-1

### Production of dimethyl 2-[(S)-2-nitro-1-(2-trifluoromethylphenyl)ethyl]malonate

Toluene (40 ml) was added to 1-((E)-2-nitrovinyl)-2-trifluoromethylbenzene (4.34 g), and dimethyl malonate (3.4 ml) and 1-(3,5-bis-trifluoromethylphenyl)-3-((1S,2S)-2-dimethylaminocyclohexyl)-thiourea (413 mg) disclosed in WO2005/000803 and J. Am. Chem. Soc., 2005, 127, 119-125 were added under ice-cooling. The mixture was stirred under ice-cooling for 24 hr. The reaction mixture was concentrated under reduced pressure, and the obtained residue was crystallized from 2-propanol to give the title compound (5.31 g) as white crystals.

This reaction can also be carried out using catalysts disclosed in J. Am. Chem. Soc., 1999, 121, 10215-10216, J. Am. Chem. Soc., 2005, 127, 9958-9959, J. Am. Chem. Soc., 2006, 128, 1454-1455 and the like, instead of 1-(3,5-bistrifluoromethylphenyl)-3-((1S,2S)-2-dimethylaminocyclohexyl)-thiourea.

### Step 7-2

### Production of methyl (S)-2-oxo-4-(2-trifluoromethylphenyl)pyrrolidine-3-carboxylate

Dimethyl 2-[(S)-2-nitro-1-(2-trifluoromethylphenyl)ethyl]malonate (4.0 g) produced in the previous Step was added to a suspension of Raney nickel (2.0 g) in tetrahydrofuran (10 ml)/methanol (10 ml), and the mixture was stirred under 3.5 atm hydrogen atmosphere at room temperature for 24 hr. Insoluble materials were filtered off through celite and washed with tetrahydrofuran·methanol mixed solvent. The filtrate and washing were combined and concentrated under reduced pressure. Toluene was added to the residue, and the mixture was washed with 10% aqueous potassium carbonate solution and water, and the organic layer was concentrated under reduced pressure. The residue was crystallized from toluene+n-hexane to give the title compound (2.38 g) as white crystals.
This reaction can also be carried out using palladium carbon instead of Raney nickel with an acid such as acetic acid, tosylic acid, mesylic acid and the like.

### Step 7-3

### Production of (S)-4-(2-trifluoromethylphenyl)pyrrolidin-2-one

Methanol (3.8 ml) was added to methyl (S)-2-oxo-4-(2-trifluoromethylphenyl)pyrrolidine-3-carboxylate (1.0 g) produced in the previous Step, 1N-aqueous sodium hydroxide solution (3.8 ml) was further added, and the mixture was stirred at 65°C for 1 hr. The reaction mixture was concentrated under reduced pressure, and the obtained residue was acidified with 1N-hydrochloric acid under ice-cooling and extracted twice with butyl acetate. The organic layers were combined, washed with brine, dried over anhydrous magnesium sulfate, and concentrated under reduced pressure. Butyl acetate (10 ml) was newly added to the residue, and the mixture was stirred overnight at 100°C. The reaction mixture was concentrated under reduced pressure, and the residue was crystallized from n-hexane to give the title compound (667 mg) as white crystals.

### Step 7-4

### Production of (S)-3-(2- trifluoromethylphenyl)pyrrolidine hydrochloride

To a solution of (S)-4-(2-trifluoromethylphenyl)pyrrolidin-2-one (22.9 g) produced in the previous Step in THF (100 ml) was added dropwise 1M lithium aluminum hydride THF solution (100 ml) under an argon atmosphere at 85°C over 1 hr, and the mixture was further stirred at said temperature for 1 hr. After allowing to cool, 50% aqueous Rochelle salt solution was added under ice-cooling, and the mixture was stirred and partitioned between water and ethyl acetate. The organic layer was separated and concentrated under reduced pressure. The residue was partitioned between ethyl acetate and water, and the organic layer was dried over anhydrous magnesium sulfate and, after filtration, the filtrate was concentrated under reduced pressure. Ethyl acetate (60 ml) was added to the residue, 4N hydrogen chloride ethyl acetate solution (37.5 ml) was further added under ice-cooling, and the mixture was stirred at room temperature. The precipitated crystals were collected by filtration to give the title compound (20.6 g) as white crystals.
[α]²⁵D = +10.3 (c = 0.55, MeOH), >99%ee

### Example 2-1

### Production of (S)-3-(2-trifluoromethylphenyl)pyrrolidine

(S)-3-(2-Trifluoromethylphenyl)pyrrolidine produced in the above-mentioned Example 2, step 4 and a salt thereof can also be produced according to the following Steps.

### Step 1

### Production of (S)-4-isopropyl-5,5-diphenyl-3-[(E)-3-(2-trifluoromethylphenyl)-acryloyl]-oxazolidin-2-one

Under an argon atmosphere, to a suspension of 2-(trifluoromethyl)cinnamic acid (10.0 g) in toluene (40 ml) were added N,N-dimethylformamide (0.02 ml) and thionyl chloride (5.06 ml) at room temperature, and the mixture was stirred at 90°C for 2.5 hr. The reaction mixture was concentrated under reduced pressure, azeotroped with toluene, and the obtained residue was dissolved in tetrahydrofuran (24 ml).
To a suspension of (S)-4-isopropyl-5,5-diphenyloxazolidin-2-one (11.8 g), lithium chloride (1.96 g) and triethylamine (7.03 ml) in tetrahydrofuran (94.6 ml) was added dropwise a solution of acid chloride produced above in tetrahydrofuran under an argon atmosphere under ice-cooling over 20 min, and the mixture was stirred at room temperature for 1 hr. The insoluble material was filtered through celite, and the filtrate was concentrated under reduced pressure. The obtained residue was slurry washed with an ethanol-water (1:1) (140 ml) mixed solvent at 110°C to give the title compound (19.8 g: yield 99%) as a white solid.
[α]²⁵D=-118. 8 (c=1.04, CHCl₃)

### Step 2

### Production of (S)-4-isopropyl-3-[(S)-4-nitro-3-(2-trifluoromethylphenyl)butyryl]-5,5-diphenyloxazolidin-2-one

(S)-4-Isopropyl-5,5-diphenyl-3-[(E)-3-(2-trifluoromethylphenyl)-acryloyl]-oxazolidin-2-one (76.4 g) synthesized in the previous step was dissolved in N,N-dimethylformamide (530 ml) under a nitrogen atmosphere, and 1,8-diazabicyclo[5,4,0]undec-7-en (4.8 ml) was added at room temperature. Under ice-cooling, nitromethane (11.2 ml) was added, and the mixture was stirred for 3 hr. 1N Hydrochloric acid was added to the reaction mixture, and ethyl acetate-heptane (1:1) mixed solvent and water were added thereto to separate the layers. The aqueous layer was extracted with an ethyl acetate-heptane (1:1) mixed solvent, and the combined organic layer was washed twice with water, and concentrated under reduced pressure. The obtained residue was recrystallized from 2-propanol to give the title compound (64.2 g: yield 74%) as a white solid.
[α]²⁵D=-117.9 (c=1.02, CHCl₃), 98%de

### Step 3

### Production of methyl (S)-4-nitro-3-(2-trifluoromethylphenyl)butyrate

Under an argon atmosphere, (S)-4-isopropyl-3-[(S)-4-nitro-3-(2-trifluoromethylphenyl)butyryl]-5,5-diphenyloxazolidin-2-one (62.9 g) synthesized in the previous step was suspended in methanol (440 ml). Under ice-cooling, a mixed solution of 28% sodium methoxide methanol solution (44.9 ml) and methanol (63 ml) was added dropwise, and the mixture was stirred at room temperature for 2.5 hr. 1N Hydrochloric acid was added to the reaction mixture, and the mixture was stirred overnight. The precipitate was collected by filtration, and washed with methanol. The obtained white crystals were (S)-4-isopropyl-5,5-diphenyloxazolidin-2-one (31.9 g: yield 98%) used in Step 1. The filtrate was concentrated under reduced pressure, and extracted twice with ethyl acetate. The combined organic layer was washed with water, concentrated under reduced pressure, and azeotroped with toluene. The resulting insoluble material was filtered off, and the filtrate was concentrated under reduced pressure to give the title compound (35.6 g: yield quantitative) as a pale-yellow oil.
[α]²⁵D=-10.6 (c=1.02, CHCl₃), 98%ee

### Step 4

### Production of (S)-4-(2-trifluoromethylphenyl)pyrrolidin-2-one

Methyl (S)-4-nitro-3-(2-trifluoromethylphenyl)butyrate (24.0 g) produced in the previous step was dissolved in methanol (90 ml) and tetrahydrofuran (90 ml), and Raney nickel (50% water content) (12.0 g) was added. Under hydrogen pressurization (0.3 - 0.4 Mpa), the mixture was stirred overnight, the insoluble material was filtered through celite, 1,8-diazabicyclo[5,4,0]undec-7-en (6.2 ml) was added to the filtrate, and the mixture was stirred at room temperature for 1 hr and further at 50°C for 1 hr. The reaction mixture was concentrated under reduced pressure, ethyl acetate was added, and the mixture was washed with 1N hydrochloric acid and water. The organic layer was concentrated under reduced pressure. The obtained residue was azeotroped with butyl acetate, crystallized from butyl acetate and heptane, and the crystals were collected by filtration under ice-cooling to give the title compound (12.08 g: yield 64%) as white crystals.
[α]²⁵D=+45.4 (c=1.02, CHCl₃), >99%ee

### Step 5

### Production of (S)-3-(2-trifluoromethylphenyl)pyrrolidine(+)-10-camphorsulfonate

To a solution of (S)-4-(2-trifluoromethylphenyl)pyrrolidin-2-one (11.0 g) produced in the previous step in tetrahydrofuran (48 ml) was added dropwise a solution (48 ml) of 1M lithium aluminum hydride in tetrahydrofuran over 1 hr under an argon atmosphere at 85°C, and the mixture was further stirred for 1 hr. Under ice-cooling, 50% aqueous Rochelle salt solution was added and the mixture was stirred. The organic layer was separated, and concentrated under reduced pressure. The obtained residue was partitioned between toluene and water, and the organic layer was concentrated under reduced pressure to give (S)-3-(2-trifluoromethylphenyl)pyrrolidine (10.0 g) as a pale-brown oil. Ethyl acetate (200 ml) was added, (+)-camphor-10-sulfonic acid (10.79 g) was added and the mixture was stirred at room temperature. The precipitated crystals were collected by filtration to give the title compound (16.43 g: yield 79%) as white crystals.

(R)-3-(2-Trifluoromethylphenyl)pyrrolidine can be produced in the same manner as in Example 2, Steps 1 - 4 and using (R)-4-isopropyl-5,5-diphenyloxazolidin-2-one instead of (S)-4-isopropyl-5,5-diphenyloxazolidin-2-one.

### Example 3

### Production of 1-{5-cyclopropyl-1-[1-(2-fluorophenylcarbamoyl)piperidin-4-yl]-1H-pyrazole-4-carbonyl}-3-(pyridin-3-yl)pyrrolidine

1-[5-Cyclopropyl-1-(piperidin-4-yl)-1H-pyrazole-4-carbonyl]-3-(pyridin-3-yl)pyrrolidine (100 mg) produced in Example 1, Step 5 was dissolved in chloroform (2 ml), triethylamine (38 µl) and 2-fluorophenylisocyanate (34 µl) were added, and the mixture was stirred at room temperature for 3 hr. The reaction mixture was concentrated under reduced pressure, and the obtained residue was purified by silica gel column chromatography (chloroform:methanol=11:1) to give the title compound as a white amorphous solid (111 mg).

### Example 4

### Production of 1-[1-(1-carbamoylpiperidin-4-yl)-5-cyclopropyl-1H-pyrazole-4-carbonyl]-[(S)-3-(2-trifluoromethylphenyl)]pyrrolidine

1-[5-cyclopropyl-1-(piperidin-4-yl)-1H-pyrazole-4-carbonyl]-[(S)-3-(2-trifluoromethylphenyl)]pyrrolidine (1.0 g) produced by a method similar to that of Example 1, Step 5 was dissolved in acetic acid (3 ml) and water (6 ml), and an aqueous solution (6 ml) of sodium cyanate (300 mg) was added dropwise at room temperature. The mixture was stirred at room temperature for 6 hr, during which sodium cyanate (750 mg total) was further added. The mixture was extracted with ethyl acetate, and the ethyl acetate layer was washed with 1N hydrochloric acid, water, saturated aqueous sodium hydrogen carbonate solution, water and brine, and dried over anhydrous magnesium sulfate. The mixture was concentrated under reduced pressure, and the obtained residue was purified by silica gel column chromatography (chloroform:methanol=10:1). Diethyl ether (10 ml) was added, and the mixture was stirred at room temperature for 2 hr. The precipitated solid was collected by filtration and dried to give the title compound as white crystals (581 mg).

### Example 5

### Production of 1-{1-[1-(2-carboxyphenylcarbamoyl)piperidin-4-yl]-5-cyclopropyl-1H-pyrazole-4-carbonyl}-[(S)-3-(2-trifluoromethylphenyl)]pyrrolidine

1-[5-Cyclopropyl-1-(piperidin-4-yl)-1H-pyrazole-4-carbonyl]-[(S)-3-(2-trifluoromethylphenyl)]pyrrolidine hydrochloride (703 mg) of Example 2 was suspended in chloroform (7 ml), and triethylamine (0.23 ml) was added. Under ice-cooling, 2-methoxycarbonylphenylisocyanate (292 mg) was added, and the mixture was stirred at room temperature for 2 hr. The mixture was concentrated under reduced pressure, and the obtained residue was purified by silica gel chromatography (chloroform:methanol=30:1) to give 1-{5-cyclopropyl-1-[1-(2-methoxycarbonylphenylcarbamoyl)piperidin-4-yl]-1H-pyrazole-4-carbonyl}-[(S)-3-(2-trifluoromethylphenyl)]pyrrolidine (992 mg) as a white amorphous solid.
The obtained 1-{5-cyclopropyl-1-[1-(2-methoxycarbonylphenylcarbamoyl)piperidin-4-yl]-1H-pyrazole-4-carbonyl}-[(S)-3-(2-trifluoromethylphenyl)]pyrrolidine (244 mg) was dissolved in tetrahydrofuran (0.4 ml) and methanol (0.2 ml), 4N aqueous sodium hydroxide solution (0.4 ml) was added, and the mixture was stirred at 60°C for 4 hrs. The mixture was concentrated under reduced pressure, and the obtained residue was dissolved in water. Under ice-cooling, the mixture was acidified with 2N hydrochloric acid and extracted with chloroform. The chloroform layer was dried over anhydrous sodium sulfate, and concentrated under reduced pressure. n-Hexane was added to the residue to allow crystal precipitation. The obtained crystal was washed with n-hexane and dried under reduced pressure to give the title compound as a white amorphous solid (181 mg).

### Example 6

### Production of 1-{5-cyclopropyl-1-[1-(2-hydroxymethylphenylcarbamoyl)piperidin-4-yl]-1H-pyrazole-4-carbonyl}-[(S)-3-(2-trifluoromethylphenyl)]pyrrolidine

1-{5-Cyclopropyl-1-[1-(2-methoxycarbonylphenylcarbamoyl)piperidin-4-yl]-1H-pyrazole-4-carbonyl}-[(S)-3-(2-trifluoromethylphenyl)]pyrrolidine (213 mg) obtained in Example 5 was dissolved in tetrahydrofuran (2 ml) and ethanol (2 ml), and lithium chloride (30 mg) and sodium borohydride (26 mg) were added under ice-cooling. The mixture was stirred overnight at room temperature. The precipitated insoluble material was collected by filtration, and washed with chloroform. The filtrate and washing were combined, and the mixture was washed with water and saturated aqueous sodium chloride solution, dried over anhydrous sodium sulfate, and concentrated under reduced pressure. The obtained residue was purified by silica gel column chromatography (chloroform:methanol=30:1) to give the title compound as a white amorphous solid (109 mg).

### Example 7

### Production of 1-{5-cyclopropyl-1-[1-(1-hydroxymethylcyclopropylcarbamoyl)piperidin-4-yl]-1H-pyrazole-4-carbonyl}-[(S)-3-(2-trifluoromethylphenyl)]pyrrolidine

By a method similar to that of Example 5, the title compound was obtained as a white amorphous solid (530 mg) from 1-[5-cyclopropyl-1-(piperidin-4-yl)-1H-pyrazole-4-carbonyl] [(S)-3-(2-trifluoromethylphenyl)]pyrrolidine hydrochloride (1.07 g) of Example 2.

### Example 8

### Production of 1-{5-cyclopropyl-1-[1-(2-hydroxyethylcarbamoyl)piperidin-4-yl]-1H-pyrazole-4-carbonyl}-[(S)-3-(2-trifluoromethylphenyl)]pyrrolidine

By a method similar to that of Example 5, the title compound was obtained as a white amorphous solid (202 mg) from 1-[5-cyclopropyl-1-(piperidin-4-yl)-1H-pyrazole-4-carbonyl]-[(S)-3-(2-trifluoromethylphenyl)]pyrrolidine hydrochloride (206 mg) of Example 2.

### Example 9

### Production of 1-{5-cyclopropyl-1-[1-(2-hydroxy-1,1-dimethylethylcarbamoyl)piperidin-4-yl]-1H-pyrazole-4-carbonyl}-[(S)-3-(2-trifluoromethylphenyl)]pyrrolidine

By a method similar to that of Example 5, the title compound was obtained as a white amorphous solid (341 mg) from 1-[5-cyclopropyl-1-(piperidin-4-yl)-1H-pyrazole-4-carbonyl]-[(S)-3-(2-trifluoromethylphenyl)]pyrrolidine hydrochloride (350 mg) of Example 2.

### Example 10

### Production of 1-{1-[1-(2-acetylaminoethylcarbamoyl)piperidin-4-yl]-5-cyclopropyl-1H-pyrazole-4-carbonyl}-[(S)-3-(2-trifluoromethylphenyl)]pyrrolidine

### Step 1

### Production of 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine

To 1-[5-cyclopropyl-1-(piperidin-4-yl)-1H-pyrazole-4-carbonyl]-[(S)-3-(2-trifluoromethylphenyl)]pyrrolidine hydrochloride (5.04 g) of Example 2 was added chloroform (50 ml), N,N-diisopropylethylamine (4.68 ml) and 4-nitrophenyl chloroformate (2.39 g) were added under ice-cooling, and the mixture was stirred at room temperature for 1 hr. The mixture was concentrated under reduced pressure, acidified with 1N hydrochloric acid, and extracted with ethyl acetate. The ethyl acetate layer was washed with water and brine, dried over anhydrous magnesium sulfate, and concentrated under reduced pressure. The obtained residue was purified by silica gel column chromatography (chloroform:methanol=20:1) to give the title compound as a pale-yellow amorphous form (5.41 g).

### Step 2

### Production of 1-{1-[1-(2-acetylaminoethylcarbamoyl)piperidin-4-yl]-5-cyclopropyl-1H-pyrazole-4-carbonyl}-[(S)-3-(2-trifluoromethylphenyl)]pyrrolidine

1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg) produced in the previous step was dissolved in N-methylpyrrolidone (1 ml), and N-(2-aminoethyl)-acetamide (96 µl) was added. The mixture was stirred overnight at 80°C. After allowing to cool, 10% aqueous potassium carbonate solution was added, and the mixture was extracted with ethyl acetate. The ethyl acetate layer was washed with water, 1N hydrochloric acid and water, dried over anhydrous magnesium sulfate, and concentrated under reduced pressure. The obtained residue was purified by silica gel column chromatography (chloroform:methanol=15:1) to give the title compound as a white amorphous form (65 mg).

### Example 11

### Production of 1-{1-[1-(2-aminoethylcarbamoyl)piperidin-4-yl]-5-cyclopropyl-1H-pyrazole-4-carbonyl}-[(S)-3-(2-trifluoromethylphenyl)]pyrrolidine hydrochloride

1-{5-Cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg) produced in Example 10, Step 1 and t-butyl (2-aminoethyl)carbamate (160 mg) were dissolved in acetonitrile (1 ml), and the mixture was heated under reflux overnight. The mixture was diluted with chloroform, and the mixture was washed with water, saturated aqueous potassium hydrogen carbonate solution, water and brine, and dried over anhydrous magnesium sulfate. The mixture was concentrated under reduced pressure, and the obtained residue was purified by silica gel column chromatography (chloroform:methanol=10:1). The obtained amorphous solid was dissolved in ethyl acetate (1 ml), and 4N hydrogen chloride ethyl acetate solution (4 ml) was added. The precipitated solid was collected by filtration and dried to give the title compound as a white amorphous solid (111 mg).

### Example 12

### Production of 1-{5-cyclopropyl-1-[1-(1,1-dioxothiomorpholine-4-carbonyl)piperidin-4-yl]-1H-pyrazole-4-carbonyl}-[(S)-3-(2-trifluoromethylphenyl)]pyrrolidine

1-{5-Cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (250 mg) produced in Example 10, Step 1 and thiomorpholine (0.13 ml) were dissolved in N-methylpyrrolidone (1 ml), and the mixture was stirred overnight at 100°C. After allowing to cool, the mixture was diluted with diethyl ether, washed with water, 5% aqueous potassium carbonate solution and brine, and dried over anhydrous sodium sulfate. The mixture was concentrated under reduced pressure, and the obtained residue was purified by silica gel column chromatography (ethyl acetate:methanol=30:1) to give 1-{5-cyclopropyl-1-[1-(thiomorpholine-4-carbonyl)piperidin-4-yl]-1H-pyrazole-4-carbonyl}-[(S)-3-(2-trifluoromethylphenyl)]pyrrolidine as a white amorphous solid (224 mg). This was dissolved in chloroform (4 ml), and m-chloroperbenzoic acid (319 mg) was added under ice-cooling. The mixture was stirred for 3 hr while allowing it to gradually return to room temperature. Saturated aqueous sodium thiosulfate solution and saturated aqueous sodium hydrogen carbonate solution were added, and the mixture was extracted with chloroform. The chloroform layer was washed with brine and dried over anhydrous sodium sulfate. The mixture was concentrated under reduced pressure, and the obtained residue was purified by silica gel column chromatography (ethyl acetate:methanol=30:1) to give the title compound as a white amorphous solid (198 mg).

### Example 13

### Production of 1-{5-cyclopropyl-1-[1-(2-dimethylaminoethylcarbamoyl)piperidin-4-yl]-1H-pyrazole-4-carbonyl}-[(S)-3-(2-trifluoromethylphenyl)]pyrrolidine hydrochloride

By a method similar to that of Example 10, the title compound was obtained as a yellow amorphous solid (130 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 14

### Production of 1-{5-cyclopropyl-1-[1-(4-hydroxypiperidine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a white amorphous solid (176 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 15

### Production of 1-{1-[1-(azetidine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 10, the title compound was obtained as a white amorphous solid (150 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 16

### Production of 1-{5-cyclopropyl-1-[1-(3-hydroxypyrrolidine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a white amorphous solid (175 mg) from 1-{5-cyclopropyl-1-[1-(4-nitrophenoxycarbonyl)piperidin-4-y1]-1H-pyrazole-4-carbonyl}-[(S)-3-(2-trifluoromethylphenyl)]pyrrolidine (200 mg).

### Example 17

### Production of 1-{5-cyclopropyl-1-[1-(2-piperidin-1-yl-ethylcarbamoyl)piperidin-4-yl]-1H-pyrazole-4-carbonyl}-[(S)-3-(2-trifluoromethylphenyl)]pyrrolidine hydrochloride

By a method similar to that of Example 10, the title compound was obtained as a pale-yellow amorphous solid (170 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 18

### Production of 1-{5-cyclopropyl-1-[1-(4,4-difluoropiperidine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a pale-yellow amorphous solid (132 mg) from 1-{5-cyclopropyl-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 19

### Production of 1-{5-cyclopropyl-1-[1-(3,3-difluoropyrrolidine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a pale-yellow amorphous solid (152 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 20

### Production of 1-{5-cyclopropyl-1-[1-(3-hydroxyazetidine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a white amorphous solid (158 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 21

### Production of 1-(5-cyclopropyl-1-{1-[(R)-3-hydroxypyrrolidine-1-carbonyl]piperidin-4-yl}-1H-pyrazole-4-carbonyl)-[(S)-3-(2-trifluoromethylphenyl)]pyrrolidine

By a method similar to that of Example 10, the title compound was obtained as a white amorphous solid (171 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 22

### Production of 1-(5-cyclopropyl-l-{1-[(S)-3-hydroxypyrrolidine-1-carbonyl]piperidin-4-yl}-1H-pyrazole-4-carbonyl)-[(S)-3-(2-trifluoromethylphenyl)]pyrrolidine

By a method similar to that of Example 10, the title compound was obtained as a white amorphous solid (172 mg) from 1-{5-cyclopropyl-1-[1-(4-nitrophenaxycarbonyl)piperidin-4-yl]-1H-pyrazole-4-carbonyl}-[(S)-3-(2-trifluoromethylphenyl)]pyrrolidine (200 mg).

### Example 23

### Production of 1-(5-cyclopropyl-1-{1-[(S)-2-hydroxy-1-methylethylcarbamoyl]piperidin-4-yl}-1H-pyrazole-4-carbonyl)-[(S)-3-(2-trifluoromethylphenyl)]pyrrolidine

By a method similar to that of Example 10, the title compound was obtained as a yellow amorphous solid (60 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 24

### Production of 1-{5-cyclopropyl-1-[1-(4-hydroxymethylpiperidine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a white amorphous solid (94 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (150 mg).

### Example 25

### Production of 1-{1-[1-(4-carboxypiperidine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 10, the title compound was obtained as a white amorphous solid (65 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (172 mg).

### Example 26

### Production of 1-{5-cyclopropyl-1-[1-(3-oxopiperazine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a white amorphous solid (143 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 27

### Production of 1-(5-cyclopropyl-1-{1-[(S)-2-hydroxymethylpyrrolidine-1-carbonyl]piperidin-4-yl}-1H-pyrazole-4-carbonyl)-[(S)-3-(2-trifluoromethylphenyl)]pyrrolidine

By a method similar to that of Example 10, the title compound was obtained as a yellow amorphous solid (150 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 28

### Production of 1-{5-cyclopropyl-1-[1-(3-hydroxymethylazetidine-1-carbonyl)piperidin-4-yl]-1H-pyrazole-4-carbonyl}-[(S)-3-(2-trifluoromethylphenyl)]pyrrolidine

By a method similar to that of Example 10, the title compound was obtained as a yellow amorphous solid (178 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 29

### Production of 1-{5-cyclopropyl-l-[1-(4-methylpiperazine-1-carbonyl)piperidin-4-yl]-1H-pyrazole-4-carbonyl}-[(S)-3-(2-trifluoromethylphenyl)]pyrrolidine hydrochloride

By a method similar to that of Example 10, the title compound was obtained as a white amorphous solid (66 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (150 mg).

### Example 30

### Production of 1-{5-cyclopropyl-1-[1-(4-isopropylpiperazine-1-carbonyl)piperidin-4-yl]-1H-pyrazole-4-carbonyl}-[(S)-3-(2-trifluoromethylphenyl)]pyrrolidine hydrochloride

By a method similar to that of Example 10, the title compound was obtained as a white amorphous solid (50 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (150 mg).

### Example 31

### Production of 1-{1-[1-(4-acetylpiperazine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 10, the title compound was obtained as a white amorphous solid (56 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (150 mg).

### Example 32

### Production of 1-(5-cyclopropyl-1-{1-[(R)-3-hydroxypiperidine-1-carbonyl]piperidin-4-yl}-1H-pyrazole-4-carbonyl)-[(S)-3-(2-trifluoromethylphenyl)]pyrrolidine

By a method similar to that of Examples 10, the title compound was obtained as a yellow amorphous solid (170 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (200 mg).

### Example 33

### Production of 2-{1-[1-(4-carbamoylpiperidine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 10, the title compound was obtained as a white amorphous solid (108 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (150 mg).

### Example 34

### Production of 1-{1-[1-(3-carbamoylazetidine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 10, the title compound was obtained as a white amorphous solid (93 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (171 mg).

### Example 35

### Production of 1-{1-[1-(4-aminopiperidine-1-carbonyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 11, the title compound was obtained as a white amorphous solid (302 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (478 mg).

### Example 36

### Production of 1-{1-[1-(3-aminopyrrolidine-1-carbonyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 11, the title compound was obtained as a pale-brown yellow amorphous solid (635 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (478 mg).

### Example 37

### Production of 1-{5-cyclopropyl-1-[1-(piperidin-4-yl-carbamoyl)piperidin-4-yl]-1H-pyrazole-4-carbonyl}-[(S)-3-(2-trifluoromethylphenyl)]pyrrolidine hydrochloride

By a method similar to that of Example 11, the title compound was obtained as a white amorphous solid (59 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (478 mg).

### Example 38

### Production of 1-{5-cyclopropyl-1-[1-(4-dimethylaminopiperidine-1-carbonyl)piperidin-4-yl]-1H-pyrazole-4-carbonyl}-[(S)-3-(2-trifluoromethylphenyl)]pyrrolidine hydrochloride

By a method similar to that of Example 12, the title compound was obtained as a white amorphous solid (74 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (478 mg).

### Example 39

### Production of 1-{1-[1-(4-acetylaminopiperidine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 12, the title compound was obtained as a white amorphous solid (100 mg) from 1-{5-cyciopropyl-1-[1-(4-nitrophenoxycarbonyl)piperidin-4-yl]-1H-pyrazole-4-carbonyl}-[(S)-3-(2-trifluoromethylphenyl)]pyrrolidine (478 mg).

### Example 40

### Production of 1-{1-[1-(3-acetylaminopyrrolidine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 12, the title compound was obtained as a white amorphous solid (127 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (478 mg).

### Example 41

### Production of 1-{5-cyclopropyl-1-[1-(3-dimethylaminopyrrolidine-1-carbonyl)piperidin-4-yl]-1H-pyrazole-4-carbonyl}-[(S)-3-(2-trifluoromethylphenyl)]pyrrolidine hydrochloride

By a method similar to that of Example 12, the title compound was obtained as a pale-brown yellow amorphous solid (141 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (478 mg).

### Example 42

### Production of 1-{5-cyclopropyl-1-[1-(1-methylpiperidin-4-yl-carbamoyl)piperidin-4-yl]-1H-pyrazole-4-carbonyl}-[(S)-3-(2-trifluoromethylphenyl)]pyrrolidine hydrochloride

By a method similar to that of Example 12, the title compound was obtained as a white amorphous solid (116 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (181 mg).

### Example 43

### Production of 1-{1-[1-(1-acetylpiperidin-4-yl-carbamoyl)piperidin-4-yl]-5-cyclopropyl-1H-pyrazole-4-carbonyl}-[(S)-3-(2-trifluoromethylphenyl)]pyrrolidine

By a method similar to that of Example 12, the title compound was obtained as a white amorphous solid (41 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (181 mg).

### Example 44

### Production of 1-{5-cyclopropyl-1-[1-(4-oxopiperidine-1-carbonyl)piperidin-4-yl]-1H-pyrazole-4-carbonyl}-[(S)-3-(2-trifluoromethylphenyl)]pyrrolidine

By a method similar to that of Example 12, the title compound was obtained as a white amorphous solid (155 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (540 mg).

### Example 45

### Production of 1-{1-[1-(3-acetylaminoazetidine-1-carbonyl)piperidin-4-yl]-5-cyclopropyl-1H-pyrazole-4-carbonyl}-[(S)-3-(2-trifluoromethylphenyl)]pyrrolidine

By a method similar to that of Example 12, the title compound was obtained as a white amorphous solid (68 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (90 mg).

### Example 46

### Production of 1-{5-cyclopropyl-1-[1-(3-oxopyrrolidine-1-carbonyl)piperidin-4-yl]-1H-pyrazole-4-carbonyl}-[(S)-3-(2-trifluoromethylphenyl)]pyrrolidine

By a method similar to that of Example 12, the title compound was obtained as a white amorphous solid (155 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (300 mg).

### Example 47

### Production of 1-{5-cyclopropyl-1-[1-(2,2,2-trifluoroethylcarbamoyl)piperidin-4-yl]-1H-pyrazole-4-carbonyl}-[(S)-3-(2-trifluoromethylphenyl)]pyrrolidine

### Step 1

### Production of 4-nitrophenyl (2,2,2-trifluoroethyl)carbamate

2,2,2-Trifluoroethylamine (392 mg) was dissolved in chloroform (4 ml), and pyridine (0.35 ml) and 4-nitrophenyl chloroformate (2.39 g) were added under ice-cooling. The mixture was stirred at room temperature for 1 hr. The mixture was concentrated under reduced pressure, water was added to the obtained residue, and the mixture was extracted with ethyl acetate. The ethyl acetate layer was washed with 1N hydrochloric acid and water, dried over anhydrous magnesium sulfate, and concentrated under reduced pressure. Diisopropyl ether was added to the obtained residue, and the precipitated solid was collected by filtration and dried to give the title compound as a white solid (501 mg).

### Step 2

### Production of 1-{5-cyclopropyl-1-[1-(2,2,2-trifluoroethylcarbamoyl)piperidin-4-yl]-1H-pyrazole-4-carbonyl}-[(S)-3-(2-trifluoromethylphenyl)]pyrrolidine

4-Nitrophenyl (2,2,2-trifluoroethyl)carbamate (113 mg) produced in the previous step and 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2 were dissolved in pyridine (1 ml), and the mixture was stirred at 80°C for 2 hr. After allowing to cool, toluene was added and the mixture was concentrated under reduced pressure. 10% Aqueous potassium carbonate solution was added, and the mixture was extracted with ethyl acetate. The ethyl acetate layer was washed with 10% aqueous potassium carbonate solution, water, 1N hydrochloric acid and water, dried over anhydrous magnesium sulfate, and concentrated under reduced pressure. The obtained residue was purified by silica gel column chromatography (chloroform:methanol=15:1) to give the title compound as a pale-yellow amorphous form (162 mg).

### Example 48

### Production of 1-{5-cyclopropyl-1-[1-(3,3,4,4-tetrafluoropyrrolidine-1-carbonyl)piperidin-4-yl]-1H-pyrazole-4-carbonyl}-[(S)-3-(2-trifluoromethylphenyl)]pyrrolidine

By a method similar to that of Example 47, the title compound was obtained as a pale-yellow amorphous solid (138 mg) from 1-[5-cyclopropyl-1-(piperidin-4-yl)-1H-pyrazole-4-carbonyl]-[(S)-3-(2-trifluoromethylphenyl)]pyrrolidine hydrochloride (468 mg) of Example 2.

### Example 49

### Production of 1-(5-cyclopropyl-1-{1-[(S)-1-hydroxymethyl-2-methylpropylcarbamoyl]piperidin-4-yl}-1H-pyrazole-4-carbonyl)-[(S)-3-(2-trifluoromethylphenyl)]pyrrolidine

By a method similar to that of Example 47, the title compound was obtained as a white amorphous solid (166 mg) from 1-[5-cyclopropyl-1-(piperidin-4-yl)-1H-pyrazole-4-carbonyl]-[(S)-3-(2-trifluoromethylphenyl)]pyrrolidine hydrochloride (250 mg) of Example 2.

### Example 50

### Production of 1-(1-{1-[(S)-1-benzyl-2-hydroxyethylcarbamoyl]piperidin-4-yl}-5-cyclopropyl-1H-pyrazole-4-carbonyl)-[(S)-3-(2-trifluoromethylphenyl)]pyrrolidine

The title compound (140 mg) was obtained as a white amorphous solid from 1-[5-cyclopropyl-1-(piperidin-4-yl)-1H-pyrazole-4-carbonyl]-[(S)-3-(2-trifluoromethylphenyl)]pyrrolidine hydrochloride (300 mg) of Example 2 in the same manner as in Example 47.

### Example 51

### Production of 1-(5-cyclopropyl-1-{1-[(S)-2-hydroxy-1-phenylethylcarbamoyl]piperidin-4-yl}-1H-pyrazole-4-carbonyl)-[(S)-3-(2-trifluoromethylphenyl)]pyrrolidine

By a method similar to that of Example 47, the title compound was obtained as a white amorphous solid (170 mg) from 1-[5-cyclopropyl-1-(piperidin-4-yl)-1H-pyrazole-4-carbonyl]-[(S)-3-(2-trifluoromethylphenyl)]pyrrolidine hydrochloride (300 mg) of Example 2.

### Example 52

### Production of 1-(5-cyclopropyl-1-{1-[(S)-1-hydroxymethyl-3-methylbutylcarbamoyl]piperidin-4-yl}-1H-pyrazole-4-carbonyl)-[(S)-3-(2-trifluoromethylphenyl)]pyrrolidine

By a method similar to that of Example 47, the title compound was obtained as a yellow amorphous solid (256 mg) from 1-[5-cyclopropyl-1-(piperidin-4-yl)-1H-pyrazole-4-carbonyl]-[(S)-3-(2-trifluoromethylphenyl)]pyrrolidine hydrochloride (250 mg) of Example 2.

### Example 53

### Production of 1-{5-cyclopropyl-1-[1-(2-hydroxyphenylcarbamoyl)piperidin-4-yl]-1H-pyrazole-4-carbonyl}-[(S)-3-(2-trifluoromethylphenyl)]pyrrolidine

By a method similar to that of Example 47, the title compound was obtained as a white amorphous solid (125 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 54

### Production of 1-{5-cyclopropyl-1-[1-(1-hydroxymethylcyclopentylcarbamoyl)piperidin-4-yl]-1H-pyrazole-4-carbonyl}-[(S)-3-(2-trifluoromethylphenyl)]pyrrolidine

By a method similar to that of Example 47, the title compound was obtained as a white amorphous solid (92 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 55

### Production of 1-[1-(1-benzenesulfonylaminocarbonylpiperidin-4-yl)-5-cyclopropyl-1H-pyrazole-4-carbonyl]-[(S)-3-(2-trifluoromethylphenyl)]pyrrolidine

Chloroform (3 ml) was added to 1-[5-cyclopropyl-1-(piperidin-4-yl)-1H-pyrazole-4-carbonyl]-[(S)-3-(2-trifluoromethylphenyl)]pyrrolidine hydrochloride (250 mg) of Example 2, triethylamine (81 µl) and benzenesulfonylisocyanate (78 µl) were added and the mixture was stirred for 2 hr. The mixture was concentrated under reduced pressure, water was added to the obtained residue, and the mixture was extracted with chloroform. The chloroform layer was washed with brine, dried over anhydrous magnesium sulfate, and concentrated under reduced pressure. The obtained residue was purified by silica gel column chromatography (chloroform:methanol=10:1). The obtained solid was recrystallized from ethyl acetate to give the title compound as a pale-yellow solid (40 mg).

### Example 56

### Production of 1-[5-cyclopropyl-1-(1-methanesulfonylaminocarbonylpiperidin-4-yl)-1H-pyrazole-4-carbonyl]-[(S)-3-(2-trifluoromethylphenyl)]pyrrolidine

By a method similar to that of Example 55, the title compound was obtained as a pale-yellow amorphous solid (200 mg) from 1-[5-cyclopropyl-1-(piperidin-4-yl)-1H-pyrazole-4-carbonyl]-[(S)-3-(2-trifluoromethylphenyl)]pyrrolidine hydrochloride (500 mg) of Example 2.

### Example 57

### Production of 1-[5-cyclopropyl-1-(1-methoxycarbonylpiperidin-4-yl)-1H-pyrazole-4-carbonyl]-[(S)-3-(2-trifluoromethylphenyl)]pyrrolidine

1-[5-Cyclopropyl-1-(piperidin-4-yl)-1H-pyrazole-4-carbonyl]-[(S)-3-(2-trifluoromethylphenyl)]pyrrolidine hydrochloride (200 mg) of Example 2 was suspended in chloroform (2.0 ml), and potassium carbonate (177 mg) was added under ice-cooling. Methyl chloroformate (49.4 µl) was added, and the mixture was allowed to return to room temperature and stirred for 1.5 hr. Methyl chloroformate (49.4 µl) was further added, and the mixture was stirred at room temperature for 15.5 hr. A saturated aqueous sodium hydrogen carbonate solution was added to the reaction mixture, and the mixture was extracted 3 times with ethyl acetate. The organic layer was washed with water and brine, dried over anhydrous sodium sulfate, and concentrated under reduced pressure. The obtained residue was purified by silica gel column chromatography (chloroform:methanol=30:1) to give the title compound as a white amorphous solid (210 mg).

### Example 58

### Production of 1-{5-cyclopropyl-1-[1-(2-hydroxyethoxycarbonyl)piperidin-4-yl]-1H-pyrazole-4-carbonyl}-[(S)-3-(2-trifluoromethylphenyl)]pyrrolidine

By a method similar to that of Example 57, the title compound was obtained as a white amorphous solid (214 mg) from 1-[5-cyclopropyl-1-(piperidin-4-yl)-1H-pyrazole-4-carbonyl]-[(S)3-(2-trifluoromethylphenyl)]pyrrolidine hydrochloride (300 mg) of Example 2.

### Example 59

### Production of 1-{5-cyclopropyl-1-[1-(2-dimethylaminoethoxycarbonyl)piperidin-4-yl]-1H-pyrazole-4-carbonyl}-[(S)-3-(2-trifluoromethylphenyl)]pyrrolidine hydrochloride

By a method similar to that of Example 10, the title compound was obtained as a white amorphous solid (166 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (300 mg).

### Example 60

### Production of 1-{5-cyclopropyl-1-[1-(2-piperidin-1-yl-ethoxycarbonyl)piperidin-4-yl]-1H-pyrazole-4-carbonyl}-[(S)-3-(2-trifluoromethylphenyl)]pyrrolidine hydrochloride

By a method similar to that of Example 10, the title compound was obtained as a white amorphous solid (219 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (300 mg).

### Example 61

### Production of 1-{5-cyclopropyl-1-[1-(piperidin-4-yl-oxycarbonyl)piperidin-4-yl]-1H-pyrazole-4-carbonyl}-[(S)-3-(2-trifluoromethylphenyl)]pyrrolidine hydrochloride

By a method similar to that of Example 11, the title compound was obtained as a pale-yellow amorphous solid (53 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (102 mg).

### Example 62

### Production of 1-{5-cyclopropyl-1-[1-(1-methylpiperidin-4-yl-oxycarbonyl)piperidin-4-yl]-1H-pyrazole-4-carbonyl}-[(S)-3-(2-trifluoromethylphenyl)]pyrrolidine hydrochloride

By a method similar to that of Example 12, the title compound was obtained as a white amorphous solid (64 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (124 mg).

### Example 63

### Production of 1-{1-[1-(1-acetylpiperidin-4-yl-oxycarbonyl)piperidin-4-yl]-5-cyclopropyl-1H-pyrazole-4-carbonyl}-[(S)-3-(2-trifluoromethylphenyl)]pyrrolidine

By a method similar to that of Example 12, the title compound was obtained as a white amorphous solid (65 mg) from 1-{5-cyclopropyl-1-[1-(4-nitrophenoxycarbonyl)piperidin-4-yl]-1H-pyrazole-4-carbonyl}-[(S)-3-(2-trifluoromethylphenyl)]pyrrolidine (122 mg).

### Example 64

### Production of 1-[1-(1-cyclopropanecarbonylpiperidin-4-yl)-5-cyclopropyl-1H-pyrazole-4-carbonyl]-[(S)-3-(2-trlfluoromethylphenyl)]pyrrolidine

Chloroform (5 ml) and triethylamine (0.13 ml) were added to 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2 and cyclopropanecarboxylic chloride (0.047 ml) was added under ice-cooling. After stirring overnight at room temperature, the solvent was removed under reduced pressure, water was added to the obtained residue, and the mixture was extracted with ethyl acetate. The ethyl acetate layer was washed with saturated aqueous sodium hydrogen carbonate solution and brine, and dried over anhydrous sodium sulfate. The mixture was concentrated under reduced pressure, and the obtained residue was purified by silica gel column chromatography (ethyl acetate:methanol=10:1) to give the title compound as a white amorphous solid (170 mg).

### Example 65

### Production of 1-{5-cyclopropyl-1-[1-(2-hydroxyacetyl)piperidin-4-yl]-1H-pyrazole-4-carbonyl}-[(S)-3-(2-trifluoromethylphenyl)]pyrrolidine

Chloroform (5 ml) and triethylamine (0.16 ml) were added to 1-[5-cyclopropyl-1-(piperidin-4-yl)-1H-pyrazole-4-carbonyl]-[(S)-3-(2-trifluoromethylphenyl)]pyrrolidine hydrochloride (250 mg) of Example 2 and acetoxyacetyl chloride (0.069 ml) was added under ice-cooling. After stirring overnight at room temperature, the mixture was concentrated under reduced pressure, water was added to the obtained residue, and the mixture was extracted with ethyl acetate. The ethyl acetate layer was washed with saturated aqueous sodium hydrogen carbonate solution and brine, and dried over anhydrous sodium sulfate. The mixture was concentrated under reduced pressure, and the obtained residue was purified by silica gel column chromatography (ethyl acetate:methanol=10:1) The obtained amorphous solid was dissolved in tetrahydrofuran (2 ml), methanol (2 ml) and water (4 ml), and lithium hydroxide monohydrate (103 mg) was added. The mixture was stirred at 60°C for 4 hrs. The mixture was concentrated under reduced pressure, 5% aqueous potassium hydrogen sulfate was added to the obtained residue, and the mixture was extracted with ethyl acetate. The ethyl acetate layer was washed with water and brine, and dried over anhydrous sodium sulfate. The mixture was concentrated under reduced pressure, and the obtained residue was purified by silica gel column chromatography (ethyl acetate:methanol=20:1) to give the title compound as a white amorphous solid (30 mg).

### Example 66

### Production of 1-(5-cyclopropyl-1-{1-[1-(4-fluorophenyl)cyclopropanecarbonyl]piperidin-4-yl}-1H-pyrazole-4-carbonyl)-[(S)-3-(2-trifluoromethylphenyl)]pyrrolidine

By a method similar to that of Example 64, the title compound was obtained as a pale-yellow amorphous solid (193 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 67

### Production of 1-{5-cyclopropyl-1-[1-(2-dimethylaminoacetyl)piperidin-4-yl]-1H-pyrazole-4-carbonyl}-[(S)-3-(2-trifluoromethylphenyl)]pyrrolidine hydrochloride

By a method similar to that of Example 64, the title compound was obtained as a pale-yellow amorphous solid (217 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 68

### Production of 1-{1-[1-(2-acetylaminoacetyl)piperidin-4-yl]-5-cyclopropyl-1H-pyrazole-4-carbonyl}-[(S)-3-(2-trifluoromethylphenyl)]pyrrolidine

By a method similar to that of Example 64, the title compound was obtained as a pale-brown yellow amorphous solid (119 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 69

### Production of 1-{5-cyclopropyl-1-[1-(1-methylcyclopropanecarbonyl)piperidin-4-yl]-1H-pyrazole-4-carbonyl}-[(S)-3-(2-trifluoromethylphenyl)]pyrrolidine

By a method similar to that of Example 64, the title compound was obtained as a pale-yellow amorphous solid (178 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 70

### Production of 1-{1-[1-(2-acetylamino-2-methylpropionyl)piperidin-4-yl]-5-cyclopropyl-1H-pyrazole-4-carbonyl}-[(S)-3-(2-trifluoromethylphenyl)]pyrrolidine

By a method similar to that of Example 64, the title compound was obtained as a pale-brown yellow amorphous solid (94 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 71

### Production of 1-(1-{1-[(S)-2-acetylaminopropionyl]piperidin-4-yl}-5-cyclopropyl-1H-pyrazole-4-carbonyl)-[(S)-3-(2-trifluoromethylphenyl)]pyrrolidine

By a method similar to that of Example 64, the title compound was obtained as a white amorphous solid (183 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 72

### Production of 1-(1-{1-[(S)-2-acetylamino-3-methylbutyryl]piperidin-4-yl}-5-cyclopropyl-1H-pyrazole-4-carbonyl)-[(S)-3-(2-trifluoromethylphenyl)]pyrrolidine

By a method similar to that of Example 64, the title compound was obtained as a pale-yellow amorphous solid (212 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 73

### Production of 1-{5-cyclopropyl-1-[1-(3,3,3-trifluoropropionyl)piperidin-4-yl]-1H-pyrazole-4-carbonyl}-[(S)-3-(2-trifluoromethylphenyl)]pyrrolidine

By a method similar to that of Example 64, the title compound was obtained as a pale-yellow amorphous solid (151 mg) from 1-[5-cyclopropyl-1-(piperidin-4-yl)-1H-pyrazole-4-carbonyl]-[(S)-3-(2-trifluoromethylphenyl)]pyrrolidine hydrochloride (150 mg) of Example 2.

### Example 74

### Production of 1-(5-cyclopropyl-1-{1-[(S)-5-oxopyrrolidine-2-carbonyl]piperidin-4-yl}-1H-pyrazole-4-carbonyl)-[(S)-3-(2-trifluoromethylphenyl)]pyrrolidine

By a method similar to that of Example 64, the title compound was obtained as a white amorphous solid (127 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 75

### Production of 1-{1-[1-(3-acetylaminopropionyl)piperidin-4-yl]-5-cyclopropyl-1H-pyrazole-4-carbonyl}-[(S)-3-(2-trifluoromethylphenyl)]pyrrolidine

By a method similar to that of Example 64, the title compound was obtained as a pale-yellow amorphous solid (263 mg) from 1-[5-cyclopropyl-1-(piperidin-4-yl)-1H-pyrazole-4-carbonyl]-[(S)-3-(2-trifluoromethylphenyl)]pyrrolidine hydrochloride (355 mg) of Example 2.

### Example 76

### Production of 1-{5-cyclopropyl-1-[1-(3-hydroxy-2,2-dimethylpropionyl)piperidin-4-yl]-1H-pyrazole-4-carbonyl}-[(S)-3-(2-trifluoromethylphenyl)]pyrrolidine

By a method similar to that of Example 65, the title compound was obtained as a white amorphous solid (238 mg) from 1-[5-cyclopropyl-1-(piperidin-4-yl)-1H-pyrazole-4-carbonyl]-[(S)-3-(2-trifluoromethylphenyl)]pyrrolidine hydrochloride (250 mg) of Example 2.

### Example 77

### Production of 1-{1-[1-(2-aminoacetyl)piperidin--4-yl]-5-cyclopropyl-1H-pyrazole-4-carbonyl}-[(S)-3-(2-trifluoromethylphenyl)]pyrrolidine hydrochloride

By a method similar to that of Example 65, the title compound was obtained as a pale-yellow amorphous solid (219 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 78

### 1-{5-cyclopropyl-1-[1-(1-hydroxymethylcyclopropanecarbonyl)piperidin-4-yl]-1H-pyrazole-4-carbonyl}-[(S)-3-(2-trifluoromethylphenyl)]pyrrolidine

By a method similar to that of Example 65, the title compound was obtained as while crystals (168 mg) from 1-[5-cyclopropyl-1-(piperidin-4-yl)-1H-pyrazole-4-carbonyl]-[(S)-3-(2-trifluoromethylphenyl)]pyrrolidine hydrochloride (250 mg) of Example 2.

### Example 79

### Production of 1-{1-[1-(2-amino-2-methylpropionyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 65, the title compound was obtained as a pale-brown yellow amorphous solid (154 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 80

### Production of 1-{5-cyclopropyl-1-[1-(4-hydroxybutyryl)piperidin-4-yl]-1H-pyrazole-4-carbonyl}-[(S)-3-(2-trifluoromethylphenyl)]pyrrolidine

By a method similar to that of Example 65, the title compound was obtained as a white amorphous solid (166 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 81

### Production of 1-(5-cyclopropyl-1-{1-[(S)-pyrrolidine-2-carbonyl]piperidin-4-yl}-1H-pyrazole-4-carbonyl)-[(S)-3-(2-trifluoromethylphenyl)]pyrrolidine hydrochloride

By a method similar to that of Example 65, the title compound was obtained as a pale-yellow amorphous solid (569 mg) from 1-[5-cyclopropyl-1-(piperidin-4-yl)-1H-pyrazole-4-carbonyl]-[(S)-3-(2-trifluoromethylphenyl)]pyrrolidine hydrochloride (450 mg) of Example 2.

### Example 82

### Production of 1-(5-cyclopropyl-1-{1-[(S)-1-methylpyrrolidine-2-carbonyl]piperidin-4-yl}-1H-pyrazole-4-carbonyl)-[(S)-3-(2-trifluoromethylphenyl)]pyrrolidine hydrochloride

By a method similar to that of Example 65, the title compound was obtained as a pale-yellow amorphous solid (192 mg) from 1-[5-cyclopropyl-1-(piperidin-4-yl)-1H-pyrazole-4-carbonyl]-[(S)-3-(2-trifluoromethylphenyl)]pyrrolidine hydrochloride (450 mg) of Example 2.

### Example 83

### Production of 1-{1-[1-(3-aminopropionyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 65, the title compound was obtained as a pale-yellow amorphous solid (414 mg) from 1-[5-cyclopropyl-1-(piperidin-4-yl)-1H-pyrazole-4-carbonyl]-[(S)-3-(2-trifluoromethylphenyl)]pyrrolidine hydrochloride (355 mg) of Example 2.

### Example 84

### Production of 1-(1-{1-[(S)-2-amino-3-methylbutyryl]piperidin-4-yl}-5-cyclopropyl-1H-pyrazole-4-carbonyl)-[(S)-3-(2-trifluoromethylphenyl)]pyrrolidine hydrochloride

By a method similar to that of Example 65, the title compound was obtained as a pale-brown yellow amorphous solid (182 mg) from 1-[5-cyclopropyl-1-(piperidin-4-yl)-1H-pyrazole-4-carbonyl]-[(S)-3-(2-trifluoromethylphenyl)]pyrrolidine hydrochloride (150 mg) of Example 2.

### Example 85

### Production of 1-{5-cyclopropyl-1-[1-(2-methylaminoacetyl)piperidin-4-yl]-1H-pyrazole-4-carbonyl}-[(S)-3-(2-trifluoromethylphenyl)]pyrrolidine hydrochloride

By a method similar to that of Example 65, the title compound was obtained as a pale-brown yellow amorphous solid (183 mg) from 1-[5-cyclopropyl-1-(piperidin-4-yl)-1H-pyrazole-4-carbonyl]-[(S)-3-(2-trifluoromethylphenyl)]pyrrolidine hydrochloride (150 mg) of Example 2.

### Example 86

### Production of 1-{5-cyclopropyl-1-[1-(piperidine-4-carbonyl)piperidin-4-yl]-1H-pyrazole-4-carbonyl}-[(S)-3-(2-trifluoromethylphenyl)]pyrrolidine hydrochloride

By a method similar to that of Example 65, the title compound was obtained as a pale-yellow amorphous solid (478 mg) from 1-[5-cyclopropyl-1-(piperidin-4-yl)-1H-pyrazole-4-carbonyl]-[(S)-3-(2-trifluoromethylphenyl)]pyrrolidine hydrochloride (404 mg) of Example 2.

### Example 87

### Production of 1-{5-cyclopropyl-1-[1-(2-isobutyrylaminoacetyl)piperidin-4-yl]-1H-pyrazole-4-carbonyl}-[(S)-3-(2-trifluoromethylphenyl)]pyrrolidine

By a method similar to that of Example 65, the title compound was obtained as a pale-brown yellow amorphous solid (214 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 88

### Production of 1-{1-[1-(2-cyclopropanecarbonylaminoacetyl)piperidin-4-yl]-5-cyclopropyl-1H-pyrazole-4-carbonyl}-[(S)-3-(2-trifluoromethylphenyl)]pyrrolidine

By a method similar to that of Example 65, the title compound was obtained as a pale-yellow amorphous solid (190 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 89

### Production of 1-(1-{1-[(S)-1-acetylpyrrolidine-2-carbonyl]piperidin-4-yl}-5-cyclopropyl-1H-pyrazole-4-carbonyl)-[(S)-3-(2-trifluoromethylphenyl)]pyrrolidine

By a method similar to that of Example 65, the title compound was obtained as a pale-yellow amorphous solid (189 mg) from 1-[5-cyclopropyl-1-(piperidin-4-yl)-1H-pyrazole-4-carbonyl]-[(S)-3-(2-trifluoromethylphenyl)]pyrrolidine hydrochloride (450 mg) of Example 2.

### Example 90

### Production of 1-{5-cyclopropyl-1-[1-(2-methanesulfonylaminoacetyl)piperidin-4-yl]-1H-pyrazole-4-carbonyl}-[(S)-3-(2-trifluoromethylphenyl)]pyrrolidine

By a method similar to that of Example 65, the title compound was obtained as a white amorphous solid (134 mg) from 1-[5-cyclopropyl-1-(piperidin-4-yl)-1H-pyrazole-4-carbonyl]-[(S)-3-(2-trifluoromethylphenyl)]pyrrolidine hydrochloride (152 mg) of Example 2.

### Example 91

### Production of 1-{1-[1-(1-acetylpiperidine-4-carbonyl)piperidin-4-yl]-5-cyclopropyl-1H-pyrazole-4-carbonyl}-[(S)-3-(2-trifluoromethylphenyl)]pyrrolidine

By a method similar to that of Example 65, the title compound was obtained as a pale-yellow amorphous solid (145 mg) from 1-[5-cyclopropyl-1-(piperidin-4-yl)-1H-pyrazole-4-carbonyl]-[(S)-3-(2-trifluoromethylphenyl)]pyrrolidine hydrochloride (404 mg) of Example 2.

### Example 92

### Production of 1-{5-cyclopropyl-1-[1-(1-methylpiperidine-4-carbonyl)piperidin-4-yl]-1H-pyrazole-4-carbonyl}-[(S)-3-(2-trifluoromethylphenyl)]pyrrolidine hydrochloride

By a method similar to that of Example 65, the title compound was obtained as a pale-brown yellow amorphous solid (151 mg) from 1-[5-cyclopropyl-1-(piperidin-4-yl)-1H-pyrazole-4-carbonyl]-[(S)-3-(2-trifluoromethylphenyl)]pyrrolidine hydrochloride (404 mg) of Example 2.

### Example 93

### Production of 1-{1-[1-(3-carbamoylpropionyl)piperidin-4-yl]-5-cyclopropyl-1H-pyrazole-4-carbonyl}-[(S)-3-(2-trifluoromethylphenyl)]pyrrolidine

By a method similar to that of Example 65, the title compound was obtained as a white amorphous solid (61 mg) from 1-[5-cyclopropyl-1-(piperidin-4-yl)-1H-pyrazole-4-carbanyl] [(S)-3-(2-trifluoromethylphenyl)]pyrrolidine hydrochloride (200 mg) of Example 2.

### Example 94

### Production of 1-[1-(1-carbamoylmethylpiperidin-4-yl)-5-cyclopropyl-1H-pyrazole-4-carbonyl]-[(S)-3-(2-trifluoromethylphenyl)]pyrrolidine hydrochloride

1-[5-Cyclopropyl-1-(piperidin-4-yl)-1H-pyrazole-4-carbonyl]-[(S)-3-(2-trifluoromethylphenyl)]pyrrolidine hydrochloride (200 mg) of Example 2 was dissolved in N,N-dimethylformamide (2.5 ml), and potassium carbonate (93 mg) and 2-bromoacetamide (50.7 mg) were added. The mixture was stirred at room temperature for 2 hr. Water was added to the reaction mixture, and the mixture was extracted 3 times with ethyl acetate. The organic layer was washed with water and brine, dried over anhydrous sodium sulfate, and concentrated under reduced pressure. The obtained residue was purified by silica gel column chromatography (chloroform:methanol=20:1) to give 1-[1-(1-carbamoylmethylpiperidin-4-yl)-5-cyclopropyl-1H-pyrazole-4-carbonyl]-[(S)-3-(2-trifluoromethylphenyl)]pyrrolidine (167 mg) as a white amorphous solid.
1-[1-(1-Carbamoylmethylpiperidin-4-yl)-5-cyclopropyl-1H-pyrazole-4-carbonyl]-[(S)-3-(2-trifluoromethylphenyl)]pyrrolidine (154 mg) obtained in the previous step was dissolved in ethyl acetate (2 ml), and 4N hydrogen chloride ethyl acetate solution (159 µl) was added. The mixture was stirred for 10 min. The precipitated crystals were collected by filtration and dried to give the title compound as a white amorphous solid (109 mg).

### Example 95

### Production of 1-[5-cyclopropyl-1-(1-methylcarbamoylmethylpiperidin-4-yl)-1H-pyrazole-4-carbonyl]-[(S)-3-(2-trifluoromethylphenyl)]pyrrolidine hydrochloride

1-[5-cyclopropyl-1-(piperidin-4-yl)-1H-pyrazole-4-carbonyl]-[(S)-3-(2-trifluoromethylphenyl)]pyrrolidine hydrochloride (2.5 g) of Example 2 was dissolved in N,N-dimethylformamide (20 ml), and potassium carbonate (1.62 g) and ethyl bromoacetate (0.98 g) were added. The mixture was stirred at room temperature for 1 hr. Water was added to the reaction mixture, and the mixture was extracted twice with ethyl acetate. The organic layer was washed with water and brine, dried over anhydrous sodium sulfate, and concentrated under reduced pressure. The obtained residue was purified by silica gel column chromatography (chloroform:methanol=25:1) to give 1-{5-cyclopropyl-1-[1-(ethoxycarbonylmethyl)piperidin-4-yl]-1H-pyrazole-4-carbonyl}-[(S)-3-(2-trifluoromethylphenyl)]pyrrolidine (2.79 g) as a white amorphous solid.
1-{5-Cyclopropyl-1-[1-(ethoxycarbonylmethyl)piperidin-4-yl]-1H-pyrazole-4-carbonyl}-[(S)-3-(2-trifluoromethylphenyl)]pyrrolidine (1.4 g) obtained in the previous step was dissolved in tetrahydrofuran (5.5 ml) and methanol (2.7 ml), and 4N aqueous sodium hydroxide solution (1.4 ml) was added. The mixture was stirred at room temperature for 1 hr. The reaction mixture was concentrated under reduced pressure, and the obtained residue was dissolved in water. The aqueous layer was washed twice with diethyl ether, adjusted to pH 5-6 with 2N hydrochloric acid under ice-cooling, and extracted 3 times with chloroform. The chloroform layer was dried over anhydrous magnesium sulfate, concentrated under reduced pressure, and n-hexane was added to the obtained residue. The precipitated solid was collected by filtration, and dried to give 1-[1-(1-carboxymethylpiperidin-4-yl)-5-cyclopropyl-1H-pyrazole-4-carbonyl]-[(S)-3-(2-trifluoromethylphenyl)]pyrrolidine (1.17 g) as a white solid.
N,N-Dimethylformamide (3.0 ml) was added to 1-[1-(1-carboxymethylpiperidin-4-yl)-5-cyclopropyl-1H-pyrazole-4-carbonyl]-[(S)-3-(2-trifluoromethylphenyl)]pyrrolidine (150 mg) obtained in the previous step, 1-hydroxybenzotriazole (61 mg) and 1-ethyl-3-(3-dimethylaminopropyl)-carbodiimide hydrochloride (76 mg) were further added, and the mixture was stirred at room temperature for 1 hr. 40% Aqueous methylamine solution (106 µl) was added, and the mixture was stirred for 5 hr, 40% methylamine aqueous solution (318 µl) was further added, and the mixture was stirred at room temperature for 13 hr. Water was added to the reaction mixture, and the mixture was extracted with chloroform. The organic layer was washed with saturated aqueous sodium hydrogen carbonate solution, water and brine, dried over anhydrous magnesium sulfate, and concentrated under reduced pressure. The obtained residue was purified by silica gel column chromatography (chloroform:methanol=20:1). The obtained amorphous solid was dissolved in ethyl acetate (2.0 ml), 4N hydrogen chloride ethyl acetate solution (100 µl) was added, and the mixture was stirred for 10 min. The precipitated crystals were collected by filtration and dried to give the title compound as a white amorphous solid (16.9 mg).

### Example 96

### Production of 1-{1-[1-(1-carbamoyl-1-methylethyl)piperidin-4-yl]-5-cyclopropyl-1H-pyrazole-4-carbonyl}-[(S)-3-(2-trifluoromethylphenyl)]pyrrolidine hydrochloride

Cumylamine (1.1 g) and triethylamine (1.2 ml) were dissolved in chloroform (10 ml), and a solution of 2-bromoisobutyryl bromide (1.0 ml) in chloroform (2 ml) was added under ice-cooling over 5 min. The mixture was stirred at room temperature for 15 min. The mixture was concentrated under reduced pressure, ethyl acetate was added to the obtained residue, and the mixture was washed with water and brine, dried over anhydrous magnesium sulfate, and concentrated under reduced pressure. The obtained white solid was washed with diisopropyl ether, and dried to give 2-bromo-2-methyl-N-(1-methyl-1-phenylethyl)prûpylroüide as a white solid (1.73 g).
1-[5-Cyclopropyl-1-(piperidin-4-yl)-1H-pyrazole-4-carbonyl]-[(S)-3-(2-trifluoromethylphenyl)]pyrrolidine hydrochloride of Example 2 was dissolved in tetrahydrofuran (5.5 ml), and sodium hydride (60%, oil, 104 mg) was added. The mixture was stirred at 60°C for 1 hr and at room temperature for 5 min. 2-Bromo-2-methyl-N-(1-methyl-1-phenylethyl)-propylamide (329 mg) produced in the previous step was added, and the mixture was stirred at 60°C for 22 hr. After allowing to cool, water was added to the reaction mixture, and the mixture was extracted with ethyl acetate. The ethyl acetate layer was washed with brine, dried over anhydrous magnesium sulfate, and concentrated under reduced pressure. The obtained residue was purified by silica gel column chromatography (chloroform:methanol=20:1) to give 1-(5-cyclopropyl-1-{1-[1-methyl-1-(1-methyl-1-phenylethylcarbamoyl)ethyl]piperidin-4-yl}-1H-pyrazole-4-carbonyl)-[(S)-3-(2-trifluoromethylphenyl)]pyrrolidine as a pale-yellow amorphous form (78 mg).
1-(5-Cyclopropyl-1-{1-[1-methyl-1-(1-methyl-1-phenylethylcarbamoyl)ethyl]piperidin-4-yl}-1H-pyrazole-4-carbonyl)-[(S)-3-(2-trifluoromethylphenyl)]pyrrolidine produced in the previous step was dissolved in trifluoroacetic acid (2 ml), and the mixture was stirred at 80°C for 8 hr. Trifluoroacetic acid (1 ml) was further added and the mixture was further stirred at 80°C for 8 hr. After allowing to cool, the mixture was concentrated under reduced pressure, and the obtained residue was dissolved in ethyl acetate. The ethyl acetate layer was washed with saturated aqueous sodium hydrogen carbonate solution and brine, dried over anhydrous magnesium sulfate, and concentrated under reduced pressure. The obtained residue was purified by silica gel column chromatography (chloroform:acetone=4:5→ chloroform:methanol=10:1). The obtained residue was dissolved in ethyl acetate (1 ml), and 4N hydrogen chloride ethyl acetate solution (1 ml) and diethyl ether (1.5 ml) were added. The obtained solid was collected by filtration and dried to give the title compound as a pale-yellow solid (36 mg).

### Example 97

### Production of 1-{1-[1-(2-carbamoylethyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 94, the title compound was obtained as a white amorphous solid (149 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 98

### Production of 1-[5-cyclopropyl-1-(1-cyclopropylmethylpiperidin-4-yl)-1H-pyrazole-4-carbonyl]-[(S)-3-(2-trifluoromethylphenyl)]pyrrolidine hydrochloride

By a method similar to that of Example 94, the title compound was obtained as while crystals (133 mg) from 1-[5-cyclopropyl-1-(piperidin-4-yl)-1H-pyrazole-4-carbonyl]-[(S)-3-(2-trifluoromethylphenyl)]pyrrolidine hydrochloride (150 mg) of Example 2.

### Example 99

### Production of 1-[5-cyclopropyl-1-(1-cyclopropylpiperidin-4-yl)-1H-pyrazole-4-carbonyl]-[(S)-3-(2-trifluoromethylphenyl)]pyrrolidine hydrochloride

By a method similar to that of Example 94, the title compound was obtained as a pale-brown yellow amorphous solid (144 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 100

### Production of 1-[5-cyclopropyl-1-(1-dimethylcarbamoylmethylpiperidin-4-yl)-1H-pyrazole-4-carbonyl]-[(S)-3-(2-trifluoromethylphenyl)]pyrrolidine hydrochloride

By a method similar to that of Example 95, the title compound was obtained as a white amorphous solid (20 mg) from 1-[5-cyclopropyl-1-(piperidin-4-yl)-1H-pyrazole-4-carbonyl]-[(S)-3-(2-trifluoromethylphenyl)]pyrrolidine hydrochloride (2.5 g) of Example 2.

### Example 101

### Production of 1-[1-(1-carboxymethylpiperidin-4-yl)-5-cyclopropyl-1H-pyrazole-4-carbonyl]-[(S)-3-(2-trifluoromethylphenyl)]pyrrolidine hydrochloride

The title compound was obtained as a white amorphous solid (24 mg) from 1-[1-(1-carboxymethylpiperidin-4-yl)-5-cyclopropyl-1H-pyrazole-4-carbonyl]-[(S)-3-(2-trifluoromethylphenyl)]pyrrolidine (112 mg) produced in Example 95.

### Example 102

### Production of 1-[1-(1-carboxyethylpiperidin-4-yl)-5-cyclopropyl-1H-pyrazole-4-carbonyl]-[(S)-3-(2-trifluoromethylphenyl)]pyrrolidine hydrochloride

By a method similar to that of Example 95, the title compound was obtained as a pale-yellow amorphous solid (295 mg) from 1-[5-cyclopropyl-1-(piperidin-4-yl)-1H-pyrazole-4-carbonyl]-[(S)-3-(2-trifluoromethylphenyl)]pyrrolidine hydrochloride (350 mg) of Example 2.

### Example 103

### Production of 1-{1-[1-(1-carbamoylethyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 95, the title compound was obtained as a pale-yellow amorphous solid (146 mg) from 1-[5-cyclopropyl-1-(piperidin-4-yl)-1H-pyrazole-4-carbonyl]-[(S)-3-(2-trifluoromethylphenyl)]pyrrolidine hydrochloride (350 mg) of Example 2.

### Example 104

### Production of 1-{1-[1-(2-carboxy-2-methylpropyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 95, the title compound was obtained as a pale-yellow amorphous solid (71 mg) from 1-[5-cyclopropyl-1-(piperidin-4-yl)-1H-pyrazole-4-carbonyl]-[(S)-3-(2-trifluoromethylphenyl)]pyrrolidine hydrochloride (400 mg) of Example 2.

### Example 105

### Production of 1-{1-[1-(2-carbamoyl-2-methylpropyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 95, the title compound was obtained as a white amorphous solid (52 mg) from 1-[5-cyclopropyl-1-(piperidin-4-yl)-1H-pyrazole-4-carbonyl]-[(S)-3-(2-trifluoromethylphenyl)]pyrrolidine hydrochloride (400 mg) of Example 2.

### Example 106

### Production of 1-{1-[1-(1-carbamoylcyclopropylmethyl)piperidin-4-yl]-5-cyclopropyl-1H-pyrazole-4-carbonyl}-[(S)-3-(2-trifluoromethylphenyl)]pyrrolidine hydrochloride

By a method similar to that of Example 95, the title compound was obtained as a white amorphous solid (82 mg) from 1-[5-cyclopropyl-1-(piperidin-4-yl)-1H-pyrazole-4-carbonyl]-[(S)-3-(2-trifluoromethylphenyl)]pyrrolidine hydrochloride (400 mg) of Example 2.

### Example 107

### Production of 1-(5-cyclopropyl-1-{1-[1-(2-hydroxyethylcarbamoyl)cyclopropylmethyl]piperidin-4-yl}-1H-pyrazole-4-carbonyl)-[(S)-3-(2-trifluoromethylphenyl)]pyrrolidine hydrochloride

By a method similar to that of Example 95, the title compound was obtained as a white amorphous solid (84 mg) from 1-[5-cyclopropyl-1-(piperidin-4-yl)-1H-pyrazole-4-carbonyl]-[(S)-3-(2-trifiuoromethylphenyl)]pyrrolidine hydrochloride (400 mg) of Example 2.

### Example 108

### Production of 1-[5-cyclopropyl-1-(1-trifluoromethanesulfonylpiperidin-4-yl)-1H-pyrazole-4-carbonyl]-[(S)-3-(2-trifluoromethylphenyl)]pyrrolidine

1-[5-Cyclopropyl-1-(piperidin-4-yl)-1H-pyrazole-4-carbonyl]-[(S)-3-(2-trifluoromethylphenyl)]pyrrolidine hydrochloride (200 mg) of Example 2 was dissolved in methylene chloride (2.0 ml), and under an argon atmosphere, the solution was cooled to -78°C, triethylamine (178 µl) and trifluoromethanesulfonic acid anhydride (78.8 µl) were added, and the mixture was stirred for 2.5 hr. Trifluoromethanesulfonic acid anhydride (78.8 µl) was further added and the mixture was stirred for 2 hr. A saturated aqueous sodium hydrogen carbonate solution was added to the reaction mixture at -78°C, and the mixture was extracted with chloroform. The organic layer was washed with 1N hydrochloric acid, water and brine, dried over anhydrous sodium sulfate, and concentrated under reduced pressure. The obtained residue was purified by silica gel column chromatography (chloroform:methanol=20:1) to give the title compound as a yellow amorphous form (192 mg).

### Example 109

### Production of 1-{5-cyclopropyl-1-[1-(2,2,2-trifluoroethanesulfonyl)piperidin-4-yl]-1H-pyrazole-4-carbonyl}-[(S)-3-(2-trifluoromethylphenyl)]pyrrolidine

By a method similar to that of Example 108, the title compound was obtained as a yellow amorphous solid (29 mg) from 1-[5-cyclopropyl-1-(piperidin-4-yl)-1H-pyrazole-4-carbonyl]-[(S)-3-(2-trifluoromethylphenyl)]pyrrolidine hydrochloride (200 mg) of Example 2.

### Example 110

### Production of 1-{1-[1-(1-cyanoiminoethyl)piperidin-4-yl]-5-cyclopropyl-1H-pyrazole-4-carbonyl}-[(S)-3-(2-trifluoromethylphenyl)]pyrrolidine

1-[5-Cyclopropyl-1-(piperidin-4-yl)-1H-pyrazole-4-carbonyl]-[(S)-3-(2-trifluoromethylphenyl)]pyrrolidine hydrochloride of Example 2 (300 mg) was dissolved in chloroform (5 ml), and triethylamine (97 µl) and methyl N-cyanoacetimidate (66 µl) were added. After stirring at room temperature for 30 min and at 45°C for 1.5 hr, triethylamine (97 µl) and methyl N-cyanoacetimidate (66 µl) were further added, and the mixture was stirred at 45°C for 2 hr. The mixture was concentrated under reduced pressure, and the obtained residue was purified by silica gel column chromatography (chloroform:methanol=15:1) to give the title compound as a pale-yellow amorphous form (214 mg).

### Example 111

### Production of 1-(1-{1-[cyanoimino(methylamino)methyl]piperidin-4-yl}-5-cyclopropyl-1H-pyrazole-4-carbonyl)-[(S)-3-(2-trifluoromethylphenyl)]pyrrolidine

### Step 1

### Production of 1-(1-{1-[cyanoimino(phenoxy)methyl]piperidin-4-yl}-5-cyclopropyl-1H-pyrazole-4-carbonyl)-[(S)-3-(2-trifluoromethylphenyl)]pyrrolidine

Chloroform (5 ml) was added to 1-[5-cyclopropyl-1-(piperidin-4-yl)-1H-pyrazole-4-carbonyl]-[(S)-3-(2-trifluoromethylphenyl)]pyrrolidine hydrochloride of Example 2 (500 mg) and triethylamine (0.162 ml) and diphenyl N-cyanocarbonimidate (284 mg) were added. The mixture was stirred for 1.5 hr. The mixture was concentrated under reduced pressure, and the obtained residue was purified by silica gel column chromatography (chloroform:acetone=1:1→2:3→1:2) to give the title compound as a pale-yellow amorphous form (575 mg).

### Step 2

### Production of 1-(1-{1-[cyanoimino(methylamino)methyl]piperidin-4-yl}-5-cyclopropyl-1H-pyrazole-4-carbonyl)-[(S)-3-(2-trifluoromethylphenyl)]pyrrolidine

1-(1-{1-[Cyanoimino(phenoxy)-methyl]piperidin-4-yl}-5-cyclopropyl-1H-pyrazole-4-carbonyl)-[(S)-3-(2-trifluoromethylphenyl)]pyrrolidine (575 mg) produced in the previous step was dissolved in chloroform (10 ml), methylamine hydrochloride (81 mg) and triethylamine (0.31 ml) were added, and the mixture was stirred at room temperature for 30 min and at 55°C for 5 hr. Methylamine hydrochloride (81 mg) and triethylamine (2 ml) further added and the mixture was stirred at 55°C for 1 hr and at 60°C for 30 min. Methylamine hydrochloride (162 mg) was further added and the mixture was stirred at 60°C for 2.5 hr. After allowing to cool, the mixture was concentrated under reduced pressure, and the obtained residue was dissolved in chloroform, and the mixture was washed with water and brine, dried over anhydrous magnesium sulfate, and concentrated under reduced pressure. The obtained residue was purified by silica gel column chromatography (chloroform:methanol=10:1) to give the title compound as a white amorphous solid (242 mg).

### Example 112

### Production of 1-{1-[1-(N-cyanocarbamimidoyl)piperidin-4-yl]-5-cyclopropyl-1H-pyrazole-4-carbonyl}-[(S)-3-(2-trifluoromethylphenyl)]pyrrolidine

By a method similar to that of Example 111, the title compound was obtained as a pale-yellow amorphous solid (249 mg) from 1-[5-cyclopropyl-1-(piperidin-4-yl)-1H-pyrazole-4-carbonyl]-[(S)-3-(2-trifluoromethylphenyl)]pyrrolidine hydrochloride (302 mg) of Example 2.

### Example 113

### Production of 4-(4-{5-cyclopropyl-4-[3-(2-trifluoromethylphenyl)pyrrolidine-1-carbonyl]pyrazol-1-yl}piperidin-1-yl)benzoic acid

### Step 1

### Production of tert-butyl 1-[1-(4-ethoxycarbonylphenyl)piperidin-4-yl]-5-cyclopropyl-1H-pyrazole-4-carboxylate

tert-Butyl 2-cyclopropylcarbonyl-3-dimethylaminoacrylate (1.12 g) produced by a method similar to that of Example 1, Step 1 and ethyl 4-(4-hydrazinopiperidin-1-yl)benzoate hydrochloride (692 mg) produced by a known method were suspended in ethanol (15 ml). Triethylamine (0.81 ml) was added to the obtained suspension, and the mixture was refluxed overnight. The reaction mixture was concentrated under reduced pressure, water was added to the obtained residue, and the mixture was extracted with ethyl acetate. The ethyl acetate layer was washed with water and brine, dried over anhydrous magnesium sulfate, and concentrated under reduced pressure. The obtained residue was purified by silica gel column chromatography (n-hexane:ethyl acetate=3:1) to give the title compound as a yellow oil (673 mg).

### Step 2

### Production of 1-[1-(4-ethoxycarbonylphenyl)piperidin-4-yl]-5-cyclopropyl-1H-pyrazole-4-carboxylic acid

tert-Butyl 1-[1-(4-ethoxycarbonylphenyl)piperidin-4-yl]-5-cyclopropyl-1H-pyrazole-4-carboxylate (673 mg) produced in the previous step was dissolved in chloroform (4 ml), trifluoroacetic acid (4 ml) was added under ice-cooling, and the mixture was stirred at 40°C for 2 hr. The reaction mixture was concentrated under reduced pressure, water was added to the obtained residue, and the mixture was extracted with ethyl acetate. The ethyl acetate layer was washed with water and brine, dried over anhydrous magnesium sulfate, and concentrated under reduced pressure. To the obtained residue were added diethyl ether and n-hexane, and the precipitated solid was collected by filtration and dried to give the title compound as a white solid (556 mg).

### Step 3

### Production of 5-cyclopropyl-1-{1-[1-(4-ethoxycarbonylphenyl)piperidin-4-yl]-1H-pyrazole-4-carbonyl}-{3-(2-trifluoromethylphenyl)}pyrrolidine

5-Cyclopropyl-1-[1-(4-ethoxycarbonylphenyl)piperidin-4-yl]-1H-pyrazole-4-carboxy acid (550 mg) produced in the previous step, 1-hydroxybenzotriazole (285 mg) and 1-ethyl-3-(3-dimethylaminopropyl)-carbodiimide hydrochloride (357 mg) were dissolved in N,N-dimethylformamide (8 ml). 3-(2-Trifluoromethylphenyl)pyrrolidine (308 mg) was added to the obtained solution and the mixture was stirred overnight at room temperature. Water was added to the reaction mixture, and the mixture was extracted with ethyl acetate. The ethyl acetate layer was washed with saturated aqueous sodium hydrogen carbonate solution and brine, dried over anhydrous magnesium sulfate, and concentrated under reduced pressure. The obtained residue was purified by silica gel column chromatography (ethyl acetate) to give the title compound as a white amorphous solid (757 mg).

### Step 4

### Production of 4-(4-{5-cyclopropyl-4-[3-(2-trifluoromethylphenyl)pyrrolidine-1-carbonyl]pyrazol-1-yl}piperidin-1-yl)benzoic acid

1--{1-[1-(4-Ethoxycarbonylphenyl)piperidin-4-yl]-5-cyclopropyl-1H-pyrazole-4-carbonyl}-{3-(2-trifluoromethylphenyl)}pyrrolidine (720 mg) produced in the previous step was dissolved in tetrahydrofuran (3 ml) and methanol (1.5 ml), and 4N aqueous sodium hydroxide solution (1.2 ml) was added. The mixture was stirred at room temperature for 4 hr. The reaction mixture was concentrated under reduced pressure, and the obtained residue was dissolved in water. The aqueous layer was washed with diethyl ether, acidified with 2N hydrochloric acid under ice-cooling, and extracted with ethyl acetate. The ethyl acetate layer was washed with water and brine, dried over anhydrous magnesium sulfate, and concentrated under reduced pressure. Diethyl ether:n-hexane (1:1) was added to the obtained residue and the precipitated solid was collected by filtration and dried to give the title compound as a white solid (600 mg).

### Example 114

### Production of 3-(4-{5-cyclopropyl-4-[3-(2-trifluoromethylphenyl)pyrrolidine-1-carbonyl]pyrazol-1-yl}piperidin-1-yl)benzoic acid

1-[5-Cyclopropyl-1-(piperidin-4-yl)-1H-pyrazole-4-carbonyl]-3-(2-trifluoromethylphenyl)pyrrolidine (195 mg) produced by a method similar to that of Example 1 and ethyl 3-iodobenzoate (142 mg) were dissolved in 1,4-dioxane (1 ml) and tert-butanol (1 ml), and tris(dibenzylideneacetone)dipalladium (14.2 mg), 2-dicyclohexylphosphino-2'-(N,N-dimethylamino)biphenyl (24.3 mg) and cesium carbonate (235 mg) were added. The mixture was heated under reflux overnight. After allowing to cool, the insoluble material was filtered through celite, and the filtrate was concentrated under reduced pressure. A saturated aqueous sodium hydrogen carbonate solution was added to the obtained residue, and the mixture was extracted with ethyl acetate. The ethyl acetate layer was washed with brine, dried over anhydrous sodium sulfate, and concentrated under reduced pressure. The obtained residue was purified by silica gel column chromatography (n-hexane:acetone=1:1) to give ethyl 3-(4-{5-cyclopropyl-4-[3-(2-trifluoromethylphenyl)pyrrolidine-1-carbonyl]pyrazol-1-yl}piperidin-1-yl)benzoate as a white amorphous solid (179 mg).
Ethyl 3-(4-{5-cyclopropyl-4-[3-(2-trifluoromethylphenyl)pyrrolidine-1-carbonyl]pyrazol-1-yl}piperidin-1-yl)benzoate (179 mg) produced in the previous step was dissolved in tetrahydrofuran (1 ml) and ethanol (1 ml), and 4N aqueous sodium hydroxide solution (1 ml) was added. The mixture was heated under reflux for 2 hr. The reaction mixture was concentrated under reduced pressure, and the obtained residue was dissolved in water, and acidified with 2N hydrochloric acid under ice-cooling. The precipitated solid was collected by filtration, washed with water and dried under reduced pressure to give the title compound as a white solid (165 mg).

### Example 115

### Production of 5-(4-{5-cyclopropyl-4-[3-(2-trifluoromethylphenyl)pyrrolidine-1-carbonyl]pyrazol-1-yl}piperidin-1-yl)thiophene-2-carboxylic acid

1-[5-Cyclopropyl-1-(piperidin-4-yl)-1H-pyrazole-4-carbonyl]-3-(2-trifluoromethylphenyl)pyrrolidine (220 mg) produced by a method similar to that of Example 1 and ethyl 5-bromothiophene-2-carboxylate (137 mg) were dissolved in toluene (4 ml), and palladium acetate (13 mg), 2,2'-bis(diphenylphosphino)-1,1'-binaphthyl (36 mg) and cesium carbonate (265 mg) were added. The mixture was heated under reflux for 6 hr. After allowing to cool, the insoluble material was filtered through celite, and the filtrate was concentrated under reduced pressure. A saturated aqueous sodium hydrogen carbonate solution was added to the obtained residue, and the mixture was extracted with ethyl acetate. The ethyl acetate layer was washed with brine, dried over anhydrous sodium sulfate, and concentrated under reduced pressure. The obtained residue was purified by silica gel column chromatography (n-hexane:acetone=1:1) to give ethyl 5-(4-{5-cyclopropyl-4-[3-(2-trifluoromethylphenyl)pyrrolidine-1-carbonyl]pyrazol-1-yl}piperidin-1-yl)thiophene-2-carboxylate (162 mg) as a white amorphous solid.
The obtained ethyl 5-(4-{5-cyclopropyl-4-[3-(2-trifluoromethylphenyl)pyrrolidine-1-carbonyl]pyrazol-1-yl}piperidin-1-yl)thiophene-2-carboxylate (162 mg) was dissolved in tetrahydrofuran (1 ml) and ethanol (1 ml), and a 4N aqueous sodium hydroxide solution (1 ml) was added. The mixture was heated under reflux for 3 hr. The reaction mixture was concentrated under reduced pressure, and the obtained residue was dissolved in water, and acidified with 2N hydrochloric acid under ice-cooling. The precipitated solid was collected by filtration, washed with water and dried under reduced pressure. The obtained solid was purified by silica gel column chromatography (chloroform:acetone=6:1), and recrystallized from ethyl acetate to give the title compound as a pale-green solid (28 mg).

### Example 116

### Production of 2-(4-{5-cyclopropyl-4-[3-(2-trifluoromethylphenyl)pyrrolidine-1-carbonyl]pyrazol-1-yl}piperidin-1-yl)thiazole-4-carboxylic acid

### Step 1

### Production of 4-{5-cyclopropyl-4-[3-(2-trifluoromethylphenyl)pyrrolidine-1-carbonyl]pyrazol-1-yl}piperidine-1-thiocarboxamide

1-[5-Cyclopropyl-1-(piperidin-4-yl)-1H-pyrazole-4-carbonyl]-3-(2-trifluoromethylphenyl)pyrrolidine (420 mg) produced by a method similar to that of Example 1 was dissolved in chloroform (10 ml), and triethylamine (0.139 ml) and 9-fluorenylmethoxycarbonylisothiocyanate (285 mg) were added at room temperature. The mixture was stirred for 1 hr. The reaction mixture was concentrated under reduced pressure, and the obtained residue was purified by silica gel column chromatography (chloroform:methanol=50:1). The obtained purified product was dissolved in N,N-dimethylformamide (7 ml), and piperidine (0.7 ml) was added at room temperature. The mixture was stirred for 1 hr and concentrated under reduced pressure. The residue was dissolved in ethyl acetate, and the mixture was washed with water and brine, dried over anhydrous sodium sulfate, and concentrated under reduced pressure. Diethyl ether was added to the obtained residue, and the precipitated solid was collected by filtration, washed with water and dried under reduced pressure to give the title compound as a white solid (287 mg).

### Step 2

### Production of 2-(4-{5-cyclopropyl-4-[3-(2-trifluoromethylphenyl)pyrrolidine-1-carbonyl]pyrazol-1-yl}piperidin-1-yl)thiazole-4-carboxylic acid

4-{5-Cyclopropyl-4-[3-(2-trifluoromethylphenyl)pyrrolidine-1-carbonyl]pyrazol-1-yl}piperidine-1-thiocarboxamide (280 mg) produced in the previous step was suspended in ethanol (5 ml), and ethyl bromopyruvate (0.09 ml) was added to the obtained suspension. The mixture was refluxed for 2 hr. The reaction mixture was concentrated under reduced pressure, water was added to the obtained residue, and the mixture was extracted with chloroform. The chloroform layer was washed with saturated aqueous sodium hydrogen carbonate solution and brine, dried over anhydrous sodium sulfate, and concentrated under reduced pressure. The obtained residue was purified by silica gel column chromatography (chloroform:methanol=20:1) to give ethyl 2-(4-{5-cyclopropyl-4-[3-(2-trifluoromethylphenyl)pyrrolidine-1-carbonyl]pyrazol-1-yl}piperidin-1-yl)thiazole-4-carboxylate (361 mg) as a white amorphous solid.
The obtained ethyl 2-(4-{5-cyclopropyl-4-[3-(2-trifluoromethylphenyl)pyrrolidine-1-carbonyl]pyrazol-1-yl}piperidin-1-yl)thiazole-4-carboxylate (355 mg) was dissolved in tetrahydrofuran (0.6 ml) and methanol (0.3 ml), and 4N aqueous sodium hydroxide solution (0.57 ml) was added at room temperature. The mixture was stirred for 3 hr. The reaction mixture was concentrated under reduced pressure, and a 1N aqueous sodium hydroxide solution was added to the obtained residue, and the mixture was washed with diethyl ether. The aqueous layer was acidified with 2N hydrochloric acid, and the precipitated solid was collected by filtration, washed with water and dried under reduced pressure to give the title compound as a white solid (272 mg).

### Example 117

### Production of 1-{5-cyclopropyl-1-[1-(5-methyl-4H-[1,2,4]triazol-3-yl)piperidin-4-yl]-1H-pyrazole-4-carbonyl}-[(S)-3-(2-trifluoromethylphenyl)]pyrrolidine hydrochloride

### Step 1

### Production of methyl 4-{5-cyclopropyl-4-[(S)-3-(2-trifluoromethylphenyl)pyrrolidine-1-carbonyl]pyrazol-1-yl}piperidine-1-imidothiocarboxylate hydroiodide

4-{5-Cyclopropyl-4-[(S)-3-(2-trifluoromethylphenyl)pyrrolidine-1-carbonyl]pyrazol-1-yl}piperidine-1-thiocarboxamide (500 mg) produced by a method similar to that of Example 116, Step 1 was dissolved in chloroform (5 ml), and methyl iodide (0.1 ml) was added. The mixture was stirred overnight at room temperature. The mixture was concentrated under reduced pressure, diethyl ether was added to the obtained residue, and the precipitated solid was collected by filtration and dried to give the title compound as a pale-yellow solid (654 mg).

### Step 2

### Production of 1-{5-cyclopropyl-1-[1-(5-methyl-4H-[1,2,4]triazol-3-yl)piperidin-4-yl]-1H-pyrazole-4-carbonyl}-[(S)-3-(2-trifluoromethylphenyl)]pyrrolidine hydrochloride

Methyl 4-{5-cyclopropyl-4-[(S)-3-(2-trifluoromethylphenyl)pyrrolidine-1-carbonyl]pyrazol-1-yl}piperidine-1-imidothiocarboxylate hydroiodide (317 mg) produced in the previous step, acetic acid hydrazide (50 mg) and sodium acetate (43 mg) were suspended in dioxane (2 ml) and water (0.4 ml), and the mixture was heated under reflux overnight. The solvent was removed under reduced pressure, water was added to the obtained residue, and the mixture was extracted with ethyl acetate. The ethyl acetate layer was washed with brine and dried over anhydrous magnesium sulfate. The mixture was concentrated under reduced pressure, and the obtained residue was purified by silica gel column chromatography (chloroform:methanol=10:1). The obtained amorphous solid was dissolved in ethyl acetate (1 ml), and a 4N hydrogen chloride ethyl acetate solution (0.4 ml) was added. The precipitated solid was collected by filtration and dried to give the title compound as white crystals (72 mg).

### Example 118

### Production of 1-{5-cyclopropyl-1-[1-(5-cyclopropyl-4H-[1,2,4]triazol-3-yl)piperidin-4-yl]-1H-pyrazole-4-carbonyl}-[(S)-3-(2-trifluoromethylphenyl)]pyrrolidine hydrochloride

By a method similar to that of Example 117, the title compound was obtained as while crystals (253 mg) from methyl 4-{5-cyclopropyl-4-[(S)-3-(2-trifluoromethylphenyl)pyrrolidine-1-carbonyl]pyrazol-1-yl}piperidine-1-imidothiocarboxylate hydroiodide (475 mg).

### Example 119

### Production of 1-{5-cyclopropyl-1-[l-(5-hydroxymethyl-4H-[1,2,4]triazol-3-yl)piperidin-4-yl]-1H-pyrazole-4-carbonyl}-[(S)-3-(2-trifluoromethylphenyl)]pyrrolidine hydrochloride

By a method similar to that of Example 117, the title compound was obtained as while crystals (25 mg) from methyl 4-{5-cyclopropyl-4-[(S)-3-(2-trifluoromethylphenyl)pyrrolidine-1-carbonyl]pyrazol-1-yl}piperidine-1-imidothiocarboxylate hydroiodide (317 mg).

### Example 120

### Production of 1-{5-cyclopropyl-1-[1-(5-trifluoromethyl-4H-[1,2,4]triazol-3-yl)piperidin-4-yl]-1H-pyrazole-4-carbonyl}-[(S)-3-(2-trifluoromethylphenyl)]pyrrolidine hydrochloride

By a method similar to that of Example 117, the title compound was obtained as while crystals (130 mg) from methyl 4-{5-cyclopropyl-4-[(S)-3-(2-trifluoromethylphenyl)pyrrolidine-1-carbonyl]pyrazol-1-yl}piperidine-1-imidothiocarboxylate hydroiodide (317 mg).

### Example 121

### Production of 1-{5-cyclopropyl-1-[1-(2-fluorophenylcarbamoyl)piperidin-4-yl]-1H-pyrazole-4-carbonyl}-[(R)-3-(2-trifluoromethylphenyl)]pyrrolidine

### Step 1

### Production of methyl 5-cyclopropyl-1-(piperidin-4-yl)-1H-pyrazole-4-carboxylate

Methyl 1-(1-benzyloxycarbonylpiperidin-4-yl)-5-cyclopropyl-1H-pyrazole-4-carboxylate (15.9 g) produced in Example 1, Step 2 was dissolved in methanol (12 ml), and palladium carbon (1.5 g) was added. The mixture was stirred overnight under a hydrogen atmosphere. The catalyst was filtered off, and the filtrate was concentrated under reduced pressure. n-Hexane was added to the obtained residue, and filtration gave the title compound as a white amorphous solid (9.66 g).

### Step 2

### Production of methyl 5-cyclopropyl-1-[1-(2-fluorophenylcarbamoyl)piperidin-4-yl]-1H-pyrazole-4-carboxylate

Methyl 5-cyclopropyl-1-(piperidin-4-yl)-1H-pyrazole-4-carboxylate (2.82 g) produced in the previous step was dissolved in chloroform (28 ml) and triethylamine (1.58 ml) was added. 2-Fluorophenylisocyanate (1.4 ml) was added to the obtained mixture under ice-cooling and the mixture was stirred at room temperature for 1 hr. The reaction mixture was concentrated under reduced pressure, and the obtained residue was purified by silica gel column chromatography (n-hexane:ethyl acetate=2:3) to give the title compound as a white amorphous solid (4.23 g).

### Step 3

### Production of 5-cyclopropyl-1-[1-(2-fluorophenylcarbamoyl)piperidin-4-yl]-1H-pyrazole-4-carboxylic acid

Methyl 5-cyclopropyl-1-[1-(2-fluorophenylcarbamoyl)piperidin-4-yl]-1H-pyrazole-4-carboxylate (4.23 g) produced in the previous step was dissolved in tetrahydrofuran (11 ml) and methanol (5 ml), and a 4N aqueous sodium hydroxide solution (11 ml) was added. The mixture was stirred at 60°C for 6 hr. The reaction mixture was allowed to cool and concentrated under reduced pressure. The obtained residue was dissolved in water. The aqueous layer was washed with diethyl ether, and acidified with concentrated hydrochloric acid under ice-cooling. The precipitated solid was collected by filtration, washed with water and n-hexane and dried under reduced pressure to give the title compound as a white solid (3.89 g).

### Step 4

### Production of 1-{5-cyclopropyl-1-[1-(2-fluorophenylcarbamoyl)piperidin-4-yl]-1H-pyrazole-4-carbonyl}-{(R)-3-(2-trifluoromethylphenyl)}pyrrolidine

5-Cyclopropyl-1-[1-(2-fluorophenylcarbamoyl)piperidin-4-yl]-1H-pyrazole-4-carboxylic acid (101 mg) produced in the previous step, 1-hydroxybenzotriazole (46 mg) and 1-ethyl-3-(3-dimethylaminopropyl)-carbodiimide hydrochloride (57 mg) were dissolved in N,N-dimethylformamide (15 ml). (R)-3-(2-Trifluoromethylphenyl)pyrrolidine (65 mg)* and 4-dimethylaminopyridine (37 mg) were added to the obtained solution and the mixture was stirred overnight at room temperature. Water was added to the reaction mixture, and the mixture was extracted with ethyl acetate. The ethyl acetate layer was washed with saturated aqueous sodium hydrogen carbonate solution and brine, dried over anhydrous magnesium sulfate, and concentrated under reduced pressure. The obtained residue was purified by silica gel column chromatography (chloroform:methanol=20:1) to give the title compound as a white amorphous solid (136 mg).
[α]²⁵D=+10.9 (c=0.495, EtOH)
*(R)-3-(2-Trifluoromethylphenyl)pyrrolidine was produced by a method similar to the method of Example 2, Step 1 to Step 4 and using (R)-4-isopropyl-5,5-diphenyloxazolidin-2-one.
[α]²⁵D=-21.0 (c=0.500, EtOH)

### Example 122

### Production of 1-{5-cyclopropyl-1-[1-(1-methylcyclopropylcarbamoyl)piperidin-4-yl]-1H-pyrazole-4-carbonyl}-[(R)-3-(2-trifluoromethylphenyl)]pyrrolidine

### Step 1

### Production of methyl 5-cyclopropyl-1-[1-(1-methylcyclopropylcarbamoyl)piperidin-4-yl]-1H-pyrazole-4-carboxylate

1-Methylcyclopropane-1-carboxylic acid (1.81 g), diphenylphosphoryl azide (4.67 ml) and triethylamine (2.77 ml) were dissolved in toluene (30 ml), and the mixture was heated under reflux for 1 hr to produce isocyanate.
Methyl 5-cyclopropyl-1-(piperidin-4-yl)-1H-pyrazole-4-carboxylate (1.50 g) produced in Example 121, Step 1 and triethylamine (0.84 ml) were dissolved in tetrahydrofuran (15 ml) and the toluene solution of isocyanate prepared in advance was added until the starting material, (methyl 1-(piperidin-4-yl)-5-cyclopropyl-1H-pyrazole-4-carboxylate), disappeared. The reaction mixture was concentrated under reduced pressure, water was added to the obtained residue, and the mixture was extracted with chloroform. The chloroform layer was washed with saturated aqueous sodium hydrogen carbonate solution and brine, and dried over anhydrous magnesium sulfate. The obtained residue was purified by silica gel column chromatography (chloroform:methanol=15:1) to give the title compound as a white amorphous solid (1.92 g).

### Step 2

### Production of 5-cyclopropyl-1-[1-(1-methylcyclopropylcarbamoyl)piperidin-4-yl]-1H-pyrazole-4-carboxylic acid

Methyl 5-cyclopropyl-1-[1-(1-methylcyclopropylcarbamoyl)piperidin-4-yl]-1H-pyrazole-4-carboxylate (1.92 g) produced in the previous step was dissolved in tetrahydrofuran (20 ml) and methanol (10 ml), and 4N aqueous sodium hydroxide solution (15 ml) was added. The mixture was stirred at 60°C for 6 hr. The reaction mixture was allowed to cool and concentrated under reduced pressure. The obtained residue was dissolved in water and the aqueous layer was washed with diethyl ether, acidified with concentrated hydrochloric acid under ice-cooling and extracted with chloroform. The chloroform layer was washed with brine, dried over anhydrous magnesium sulfate, and concentrated under reduced pressure. Ethyl acetate was added to the obtained residue, and the precipitated solid was collected by filtration and dried to give the title compound as a white solid (1.68 g).

### Step 3

### Production of 1-{5-cyclopropyl-1-[1-(1-methylcyclopropylcarbamoyl)piperidin-4-yl]-1H-pyrazole-4-carbonyl}-[(R)-3-(2-trifluoromethylphenyl)]pyrrolidine

5-Cyclopropyl-1-[1-(1-methylcyclopropylcarbamoyl)piperidin-4-yl]-1H-pyrazole-4-carboxylic acid (91 mg) produced in the previous step, 1-hydroxybenzotriazole (46 mg) and 1-ethyl-3-(3-dimethylaminopropyl)-carbodiimide hydrochloride (57 mg) were dissolved in N,N-dimethylformamide (1.5 ml). (R)-3-(2-Trifluoromethylphenyl)pyrrolidine (65 mg) and 4-dimethylaminopyridine (37 mg) were added to the obtained solution and the mixture was stirred overnight at room temperature. Water was added to the reaction mixture, and the mixture was extracted with ethyl acetate. The ethyl acetate layer was washed with saturated aqueous sodium hydrogen carbonate solution and brine, dried over anhydrous magnesium sulfate, and concentrated under reduced pressure. The obtained residue was purified by silica gel column chromatography (chloroform:methanol=15:1) to give the title compound as a white amorphous solid (123 mg).
[α]²⁵D=+13.1 (c=0.495, EtOH)

### Example 123

### Production of 1-{5-cyclopropyl-1-[1-(1-isopropylcarbamoyl)piperidin-4-yl]-1H-pyrazole-4-carbonyl}-[(R)-3-(2-trifluoromethylphenyl)]pyrrolidine

### Step 1

### Production of methyl 5-cyclopropyl-1-[1-(1-isopropylcarbamoyl)piperidin-4-yl]-1H-pyrazole-4-carboxylate

Methyl 5-cyclopropyl-1-(piperidin-4-yl)-1H-pyrazole-4-carboxylate (1.87 g) produced in Example 121, Step 1 was dissolved in chloroform (20 ml), and triethylamine (1.05 ml) was added. Isopropylisocyanate (0.7 ml) was added to the obtained mixture under ice-cooling and the mixture was stirred at room temperature for 1 hr. The reaction mixture was concentrated under reduced pressure, and the obtained residue was purified by silica gel column chromatography (chloroform:methanol=15:1) to give the title compound as a white amorphous solid (2.00 g).

### Step 2

### Production of 5-cyclopropyl-1-[1-(1-isopropylcarbamoyl)piperidin-4-yl]-1H-pyrazole-4-carboxylic acid

Methyl 5-cyclopropyl-1-[1-(1-isopropylcarbamoyl)piperidin-4-yl]-1H-pyrazole-4-carboxylate (2.00 g) produced in the previous step was dissolved in tetrahydrofuran (6 ml) and methanol (3 ml), and 4N aqueous sodium hydroxide solution (6 ml) was added. The mixture was stirred at 60°C for 4.5 hr. The reaction mixture was allowed to cool and concentrated under reduced pressure. The obtained residue was dissolved in water and the aqueous layer was washed with diethyl ether, and acidified with concentrated hydrochloric acid under ice-cooling. The mixture was extracted with chloroform. The chloroform layer was washed with brine, dried over anhydrous magnesium sulfate, and concentrated under reduced pressure. n-Hexane was added to the obtained residue, and the precipitated solid was collected by filtration and dried to give the title compound as a white solid (1.58 g).

### Step 3

### Production of 1-{5-cyclopropyl-1-[1-(1-isopropylcarbamoyl)piperidin-4-yl]-1H-pyrazole-4-carbonyl}-[(R)-3-(2-trifluoromethylphenyl)]pyrrolidine

5-Cyclopropyl-1-[1-(1-isopropylcarbamoyl)piperidin-4-yl]-1H-pyrazole-4-carboxylic acid (88 mg) produced in the previous step, 1-hydroxybenzotriazole (46 mg) and 1-ethyl-3-(3 dimethylaminopropyl)-carbodiimide hydrochloride (57 mg) were dissolved in N,N-dimethylformamide (1.5 ml). (R)-3-(2-Trifluoromethylphenyl)pyrrolidine (65 mg) and 4-dimethylaminopyridine (37 mg) were added to the obtained solution and the mixture was stirred overnight at room temperature. Water was added to the reaction mixture, and the mixture was extracted with ethyl acetate. The ethyl acetate layer was washed with saturated aqueous sodium hydrogen carbonate solution and brine, dried over anhydrous magnesium sulfate, and concentrated under reduced pressure. The obtained residue was purified by silica gel column chromatography (chloroform:methanol=15:1) to give the title compound as a white amorphous solid (120 mg).
[α]²⁵D=+14.2 (c=0.500, EtOH)

### Example 124

### Production of 1-{5-cyclopropyl-1-[1-(2-fluorophenylcarbamoyl)piperidin-4-yl]-1H-pyrazole-4-carbonyl}-[(S)-3-(2-trifluoromethylphenyl)]pyrrolidine

By a method similar to that of Example 121, Step 4, the title compound was obtained as a white amorphous solid (127 mg) from 5-cyclopropyl-1-[1-(2-fluorophenylcarbamoyl)piperidin-4-yl]-1H-pyrazole-4-carboxylic acid (123 mg) and (S)-3-(2-trifluoromethylphenyl)pyrrolidine (78 mg).
[α]²⁵D=-10.8 (c=0.510, EtOH)

### Example 125

### Production of 1-{5-cyclopropyl-1-[1-(1-isopropylcarbamoyl)piperidin-4-yl]-1H-pyrazole-4-carbonyl}-[(S)-3-(2-trifluoromethylphenyl)]pyrrolidine

By a method similar to that of Example 123, Step 3, the title compound was obtained as a white amorphous solid (123 mg) from 5-cyclopropyl-1-[1-(1-isopropylcarbamoyl)piperidin-4-yl]-1H-pyrazole-4-carboxylic acid (107 mg) and (s) -3- (2-trifluoromethylphenyl)pyrrolidine (79 mg).
[α]²⁵D=-13.2 (c=0.515, EtOH)

### Example 126

### Production of 1-{5-cyclopropyl-1-[1-(1-methylcyclopropylcarbamoyl)piperidin-4-yl]-1H-pyrazole-4-carbonyl}-[(S)-3-(2-trifluoromethylphenyl)]pyrrolidine

By a method similar to that of Example 122, Step 3, the title compound was obtained as a white amorphous solid (149 mg) from 5-cyclopropyl-1-[1-(1-methylcyclopropylcarbamoyl)piperidin-4-yl]-1H-pyrazole-4-carboxylic acid (107 mg) and (S)-3-(2-trifluoromethylphenyl)pyrrolidine (76 mg).
[α]²⁵D=-13.6 (c=0.500, EtOH)

### Example 127

### Production of (-)-3-(4-fluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride

### Step 1

### Production of ethyl cyano-(4-fluorophenyl)acetate

Toluene (40 ml) was added to 4-fluorophenylacetonitrile (15.0 g) and diethyl carbonate (66.9 ml) and sodium ethoxide (8.30 g) were further added. The mixture was stirred for 2 hr while evaporating the solvent at 110°C to 130°C. After allowing to cool, the reaction mixture was poured into water, and acetic acid (13 ml) was added. The mixture was stirred for 5 min and extracted 3 times with ethyl acetate. The organic layer was washed with water and brine, dried over anhydrous sodium sulfate and concentrated under reduced pressure to give the title compound as a pale-yellow oil (25.0 g).

### Step 2

### Production of diethyl 2-cyano-2-(4-fluorophenyl)succinate

Tetrahydrofuran (40 ml) was added to sodium hydride (60%, oil, 2.12 g), and a solution of ethyl cyano-(4-fluorophenyl) acetate (10.0 g) produced in the previous step in tetrahydrofuran (40 ml) was added dropwise under ice-cooling. The mixture was stirred for 40 min and ethyl bromoacetate (6.4 ml) was added under ice-cooling. The mixture was stirred at room temperature for 3 hrs. The reaction mixture was concentrated under reduced pressure, water was added to the residue, and the mixture was extracted 3 times with ethyl acetate. The organic layer was washed with water and brine, dried over anhydrous sodium sulfate, and concentrated under reduced pressure. The obtained residue was purified by silica gel column chromatography (n-hexane:ethyl acetate=5:1) to give the title compound as a pale-yellow oil (14.7 g).

### Step 3

### Production of ethyl 3-(4-fluorophenyl)-5-oxopyrrolidine-3-carboxylate

A solution of diethyl 2-cyano-2-(4-fluorophenyl)succinate (7.0 g) produced in the previous step in ethanol (50 ml) was added to Raney nickel (7.0 g), and the mixture was stirred overnight under a hydrogen atmosphere. The reaction mixture was filtered through celite, and the filtrate and washing were combined and concentrated under reduced pressure. The obtained residue was purified by silica gel column chromatography (chloroform:methanol=25:1) and a mixed solvent of diethyl ether and n-hexane was added to the obtained solid residue. The residue was collected by filtration and dried to give the title compound as white crystals (3.96 g).

### Step 4

### Production of ethyl 1-benzyl-3-(4-fluorophenyl)-5-oxopyrrolidine-3-carboxylate

Tetrahydrofuran (25 ml) was added to sodium hydride (60%, oil, 1.63 g), and a solution of ethyl 3-(4-fluorophenyl)-5-oxopyrrolidine-3-carboxylate (10.3 g) produced in the previous step in tetrahydrofuran (30 ml)/N,N-dimethylformamide (10 ml) was added dropwise under ice-cooling. The mixture was stirred for 10 min. Under ice-cooling, benzyl bromide (4.86 ml) was added. The mixture was stirred at the same temperature for 30 min and at room temperature for 3.5 hr. The reaction mixture was concentrated under reduced pressure, water was added to the residue, and the mixture was extracted 3 times with ethyl acetate. The organic layer was washed with water and brine, dried over anhydrous sodium sulfate, and concentrated under reduced pressure. The obtained residue was purified by silica gel column chromatography (n-hexane:ethyl acetate=2:1) to give the title compound as a pale-yellow oil (12.1 g).

### Step 5

### Production of 1-benzyl-3-(4-fluorophenyl)-3-hydroxymethylpyrrolidine

Tetrahydrofuran (20 ml) was added to lithium aluminum hydride (1.16 g) and a solution of ethyl 1-benzyl-3-(4-fluorophenyl)-5-oxopyrrolidine-3-carboxylate (4.16 g) produced in the previous step in tetrahydrofuran (20 ml) was added dropwise under ice-cooling. The mixture was stirred under reflux for 3.5 hr. After allowing to cool, water (0.9 ml), 4N aqueous sodium hydroxide solution (0.9 ml) and water (2.7 ml) were successively added under ice-cooling, and diethyl ether and magnesium sulfate were further added. The mixture was stirred for 20 min and filtered through celite. The filtrate and washing were combined and concentrated under reduced pressure to give the title compound as a pale-yellow oil (3.18 g).

### Step 6

### Production of 3-(4-fluorophenyl)-3-hydroxymethylpyrrolidine

To a solution of 1-benzyl-3-(4-fluorophenyl)-3-hydroxymethylpyrrolidine (3.18 g) produced in the previous step in methanol (30 ml) was added palladium hydroxide (300 mg), and the mixture was stirred overnight under a hydrogen atmosphere (3 atm). The reaction mixture was filtered through celite, and the filtrate and washing were combined and concentrated under reduced pressure to give the title compound as a pale-yellow oil (2.30 g).

### Step 7

### Production of tert-butyl 3-(4-fluorophenyl)-3-hydroxymethylpyrrolidine-1-carboxylate

To 3-(4-fluorophenyl)-3-hydroxymethylpyrrolidine (2.30 g) produced in the previous step were added tetrahydrofuran (40 ml) and triethylamine (5.7 ml), and di-tert-butyl dicarbonate (4.60 g) was further added under ice-cooling. The mixture was stirred overnight at room temperature. The reaction mixture was concentrated under reduced pressure, and ethyl acetate was added. The mixture was washed with water and brine, dried over anhydrous magnesium sulfate, and concentrated under reduced pressure. The obtained residue was purified by silica gel column chromatography (n-hexane:ethyl acetate=2:1) to give the title compound as a colorless oil (2.70 g).

### Step 8

### Production of (-)-3-(4-fluorophenyl)-3-hydroxymethylpyrrolidine

To tert-butyl 3-(4-fluorophenyl)-3-hydroxymethylpyrrolidine-1-carboxylate (1.02 g) produced in the previous step were added chloroform (10 ml), triethylamine (962 µl) and 4-dimethylaminopyridine (421 mg), and (S)-(+)-3,3,3-trifluoro-2-methoxy-2-phenylpropionyl chloride (969 µl) was further added under ice-cooling. The mixture was stirred at room temperature for 1 hr. The reaction mixture was concentrated under reduced pressure, water was added to the residue, and the mixture was extracted 3 times with ethyl acetate. The organic layer was washed with aqueous potassium hydrogen sulfate solution, water, aqueous sodium hydrogen carbonate solution and brine, dried over anhydrous sodium sulfate, and concentrated under reduced pressure. The obtained residue was purified by silica gel column chromatography (n-hexane:diethyl ether=5:3) to give less polar diastereomer as a colorless oil (871 mg). In addition, a more polar diastereomer was obtained as a colorless oil (781 mg).
The colorless oil (871 mg) of the less polar diastereomer was dissolved in tetrahydrofuran (4 ml) and methanol (2 ml), and 4N aqueous sodium hydroxide solution (2.6 ml) was added. The mixture was stirred at 60°C for 40 min. The reaction mixture was concentrated under reduced pressure and water was added to the residue. The mixture was extracted 3 times with diethyl ether. The organic layer was washed with brine, dried over anhydrous sodium sulfate, and concentrated under reduced pressure to give a colorless oil (481 mg).
Methanol (3 ml) and a 4N hydrogen chloride ethyl acetate solution (3.3 ml) were added to the obtained colorless oil (481 mg) and the mixture was stirred overnight at room temperature. The reaction mixture was concentrated under reduced pressure and water was added to the residue. The mixture was washed with diethyl ether, and the aqueous layer was alkalified with a 2N aqueous sodium hydroxide solution. The mixture was extracted 5 times with chloroform. The organic layer was washed with brine, dried over anhydrous sodium sulfate, concentrated under reduced pressure to give the title compound as a colorless oil (312 mg).
[α]²⁵D=-9.70 (c=0.598, EtOH)

### Production of (+)-3-(4-fluorophenyl)-3-hydroxymethylpyrrolidine

By a method similar to the method for the production of (-)-3-(4-fluorophenyl)-3-hydroxymethylpyrrolidine and using a colorless oil (781 mg) of a more polar diastereomer, the title compound was obtained as a colorless oil (251 mg).
[α]²⁵D=+10.37 (c=0.588, EtOH)

### Step 9

### Production of tert-butyl 5-(1-methylcyclopropyl)-1-(1-pyrimidin-2-ylpiperidin-4-yl)-1H-pyrazole-4-carboxylate

tert-Butyl 5-(1-methylcyclopropyl)-1-(piperidin-4-yl)-1H-pyrazole-4-carboxylate (1.15 g) produced by a method similar to that of Example 121, Step 1 and 2-chloropyrimidine (700 mg) were dissolved in 1,4-dioxane (12 ml), and palladium acetate (85 mg), 2,2'-bis(diphenylphosphino)-1,1'-binaphthyl (352 mg) and cesium carbonate (1.84 g) were added. The mixture was heated under reflux for 3 hr. After allowing to cool, the insoluble material was filtered through celite, and the filtrate was concentrated under reduced pressure. A saturated aqueous sodium hydrogen carbonate solution was added to the obtained residue, and the mixture was extracted with ethyl acetate. The ethyl acetate layer was washed with brine, dried over anhydrous sodium sulfate, and concentrated under reduced pressure. The obtained residue was purified by silica gel column chromatography (n-hexane:ethyl acetate=2:1) to give the title compound as a white amorphous solid (922 mg).

### Step 10

### Production of 5-(1-methylcyclopropyl)-1-(1-pyrimidin-2-ylpiperidin-4-yl)-1H-pyrazole-4-carboxylic acid hydrochloride

tert-Butyl 5-(1-methylcyclopropyl)-1-(1-pyrimidin-2-ylpiperidin-4-yl)-1H-pyrazole-4-carboxylate (922 mg) produced in the previous step was dissolved in chloroform (2 ml), and trifluoroacetic acid (4 ml) was added. The mixture was stirred overnight at room temperature. The reaction mixture was concentrated under reduced pressure, and the obtained crystal was dissolved in tetrahydrofuran. A 4N hydrogen chloride ethyl acetate solution (2 ml) was added, and the mixture was concentrated under reduced pressure. The obtained crystalline residue was slurry washed with ethyl acetate (10 ml) for 1 hr to give the title compound as a white solid (800 mg).

### Step 11

### Production of (-)-3-(4-fluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride

5-(1-Methylcyclopropyl)-1-(1-pyrimidin-2-ylpiperidin-4-yl)-1H-pyrazole-4-carboxylic acid hydrochloride (150 mg) produced in the previous step, 1-hydroxybenzotriazole (81 mg) and 1-ethyl-3-(3-dimethylaminopropyl)-carbodiimide hydrochloride (102 mg) were dissolved in N,N-dimethylformamide (3 ml). (-)-3-(4-Fluorophenyl)-3-hydroxymethylpyrrolidine (88 mg) produced in Step 8, triethylamine (0.057 ml) and 4-dimethylaminopyridine (50 mg) were added to the obtained solution, and the mixture was stirred overnight at room temperature. Water was added to the reaction mixture, and the mixture was extracted with ethyl acetate. The ethyl acetate layer was washed with saturated aqueous sodium hydrogen carbonate solution and brine, dried over anhydrous magnesium sulfate, and concentrated under reduced pressure. The residue was purified by silica gel column chromatography (chloroform:methanol=20:1) and the obtained free base was dissolved in methanol (3 ml). A 4N hydrogen chloride 1,4-dioxane solution (0.2 ml) was added, and the mixture was concentrated under reduced pressure. The obtained crystalline residue was washed in the slurry with ethyl acetate (3 ml) for 15 min to give the title compound as white crystals (203 mg).
[α]²⁵D=-21.3 (c=0.506, MeOH)

### Example 128

### Production of (+)-3-(4-fluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride

By a method similar to that of Example 127, Step 11, the title compound was obtained as white crystals (198 mg) from 5-(1-methylcyclopropyl)-1-(1-pyrimidin-2-ylpiperidin-4-yl)-1H-pyrazole-4-carboxylic acid hydrochloride (150 mg) and (+)-3-(4-fluorophenyl)-3-hydroxymethylpyrrolidine (88 mg) produced in Example 127, Step 8.
[α]²⁵D=+26.0 (c=0.508, MeOH)

### Example 129

### Production of 1-[5-cyclopropyl-1-(1-pyrazin-2-ylpiperidin-4-yl)-1H-pyrazole-4-carbonyl]-[(S)-3-(2-trifluoromethylphenyl)]pyrrolidine hydrochloride

The title compound was obtained as a yellow amorphous solid (165 mg) from 5-cyclopropyl-1-(1-pyrazin-2-ylpiperidin-4-yl)-1H-pyrazole-4-carboxylic acid hydrochloride (281 mg) produced by a method similar to that of Example 127.

### Example 130

### Production of 1-[1-(trans-4-carbamoylcyclohexyl)-5-cyclopropyl-1H-pyrazole-4-carbonyl]-[(S)-3-(2-trifluoromethylphenyl)]pyrrolidine

### Step 1

### Production of ethyl trans-4-(2-tert-butoxycarbonylhydrazino)cyclohexanecarboxylate

Ethyl 4-cyclohexanonecarboxylate (8.0 g) and tert-butyl carbazate (6.2 g) were dissolved in chloroform (150 ml), and acetic acid (5.4 ml) and sodium triacetoxyborohydride (30 g) were added under ice-cooling. The mixture was allowed to gradually return to room temperature, and the mixture was stirred for 7 hr. The reaction mixture was poured into saturated aqueous sodium hydrogen carbonate solution, and the mixture was extracted with ethyl acetate. The organic layer was washed with water and brine, dried over anhydrous magnesium sulfate, and concentrated under reduced pressure. The obtained residue was purified by silica gel column chromatography (n-hexane:ethyl acetate=4:1) to give the title compound as a white solid (4.63 g).

### Step 2

### Production of ethyl trans-4-hydrazinocyclohexanecarboxylate hydrochloride

Ethyl trans-4-(2-tert-butoxycarbonylhydrazino)cyclohexanecarboxylate (4.63 g) produced in the previous step was dissolved in ethanol (30 ml), and a 4N hydrogen chloride ethyl acetate solution (82 ml) was added. The mixture was stirred for 5.5 hr and concentrated under reduced pressure, and diethyl ether was added to the obtained residue. The residue was collected by filtration and dried to give the title compound as a white powder (3.86 g).

### Step 3

### Production of tert-butyl 1-(trans-4-ethoxycarbonylcyclohexane)-5-cyclopropyl-1H-pyrazole-4-carboxylate

Ethyl trans-4-hydrazinocyclohexanecarboxylate hydrochloride (3.80 g) obtained in the previous step and tert-butyl 2-cyclopropylcarbonyl-3-dimethylaminoacrylate (3.71 g) synthesized by a method similar to that of Example 1, Step 1 were dissolved in ethanol (50 ml), and triethylamine (4.32 ml) was added. The mixture was stirred under reflux for 1 hr. After allowing to cool, the mixture was concentrated under reduced pressure, and water was added to the residue. The mixture was extracted 3 times with ethyl acetate. The organic layer was washed with water and brine, dried over anhydrous magnesium sulfate, and concentrated under reduced pressure. The obtained residue was purified by silica gel column chromatography (n-hexane:ethyl acetate=5:1) to give the title compound as a yellow liquid (4.80 g).

### Step 4

### Production of 1-(trans-4-ethoxycarbonylcyclohexane)-5-cyclopropyl-1H-pyrazole-4-carboxylic acid

To a solution of tert-butyl 1-(trans-4-ethoxycarbonylcyclohexane)-5-cyclopropyl-1H-pyrazole-4-carboxylate (4.80 g) obtained in the previous step in chloroform (14 ml) was added trifluoroacetic acid (14 ml), and the mixture was heated to 40°C and stirred for 1.5 hr. After allowing to cool, the mixture was concentrated under reduced pressure and azeotroped with toluene. Ethyl acetate was added to the residue to allow crystal precipitation. The mixture was once concentrated under reduced pressure, n-hexane was added to the residue, and the crystals were collected by filtration and dried to give the title compound as a white powder (3.09 g)

### Step 5

### Production of 1-{5-cyclopropyl-1-(trans-4-ethoxycarbonylcyclohexyl)-1H-pyrazole-4-carbonyl}-[(S)-3-(2-trifluoromethylphenyl)]pyrrolidine

1-(trans-4-Ethoxycarbonylcyclohexane)-5-cyclopropyl-1H-pyrazole-4-carboxylic acid (1.50 g) produced in the previous step and (S)-3-(2-trifluoromethylphenyl)pyrrolidine (1.16 g) were dissolved in N,N-dimethylformamide (15 ml), and 1-hydroxybenzotriazole (825 mg), 1-ethyl-3-(3-dimethylaminopropyl)-carbodiimide hydrochloride (1.03 g) and 4-dimethylaminopyridine (658 mg) were added. The mixture was stirred at room temperature for 14 hrs. Ethyl acetate was added to the reaction mixture and the mixture was washed twice with water. The organic layer was washed with 1N hydrochloric acid, water, saturated aqueous sodium hydrogen carbonate solution, water and brine, dried over anhydrous sodium sulfate, and concentrated under reduced pressure. The obtained residue was purified by silica gel column chromatography (chloroform:methanol=20:1) to give the title compound as a brown amorphous (2.34 g).

### Step 6

### Production of 1-[1-(trans-4-carboxycyclohexyl)-5-cyclopropyl-1H-pyrazole-4-carbonyl]-[(S)-3-(2-trifluoromethylphenyl)]pyrrolidine

1-{5-Cyclopropyl-1-(trans-4-ethoxycarbonylcyclohexyl)-1H-pyrazole-4-carbonyl}-[(S)-3-(2-trifluoromethylphenyl)]pyrrolidine (2.34 g) produced in the previous step was dissolved in tetrahydrofuran (12 ml) and methanol (6 ml), and a solution of lithium hydroxide monohydrate (973 mg) in water (12 ml) was added. The mixture was stirred at 45°C for 1 hr. The mixture was concentrated under reduced pressure, and water was added to the obtained residue. The mixture was washed twice with diethyl ether, and the aqueous layer was acidified with 2N hydrochloric acid and extracted 3 times with ethyl acetate. The organic layer was washed with water and brine, dried over anhydrous magnesium sulfate, and concentrated under reduced pressure. Ethyl acetate and n-hexane were added to the obtained residue, and the precipitated solid was collected by filtration and dried to give the title compound as a milky white solid (2.13 g).

### Step 7

### Production of 1-[1-(trans-4-carbamoylcyclohexyl)-5-cyclopropyl-1H-pyrazole-4-carbonyl]-[(S)-3-(2-trifluoromethylphenyl)]pyrrolidine

1-[1-(trans-4-Carboxycyclohexyl)-5-cyclopropyl-1H-pyrazole-4-carbonyl]-[(S)-3-(2-trifluoromethylphenyl)]pyrrolidine (300 mg) produced in the previous step was dissolved in N,N-dimethylformamide (3.5 ml), and 1-hydroxybenzotriazole (106 mg), 1-ethyl-3-(3-dimethylaminopropyl)-carbodiimide hydrochloride (133 mg), ammonium chloride (80 mg) and triethylamine (0.105 ml) were added. The mixture was stirred at room temperature for 15 hr. Ethyl acetate was added to the reaction mixture, and the mixture was washed successively with water, 1N hydrochloric acid, water, saturated aqueous sodium hydrogen carbonate solution, water and brine, dried over anhydrous sodium sulfate, and concentrated under reduced pressure. Diethyl ether was added to the obtained solid residue and the mixture was stirred for 10 min. The solid was collected by filtration and dried to give the title compound as a white amorphous solid (171 mg).

### Example 131

### Production of 1-[5-cyclopropyl-1-(trans-4-ureidocyclohexyl)-1H-pyrazole-4-carbonyl]-[(S)-3-(2-trifluoromethylphenyl)]pyrrolidine

Toluene (3.0 ml) was added to 1-[1-(trans-4-carboxycyclohexyl)-5-cyclopropyl-1H-pyrazole-4-carbonyl]-[(S)-3-(2-trifluoromethylphenyl)]pyrrolidine (200 mg) produced in Example 130, Step 6, and triethylamine (76 µl) and diphenylphosphonic azide (118 µl) were further added under an argon atmosphere. The mixture was heated under reflux for 2 hr. After allowing to cool, the toluene solution was added dropwise to a solution of 28% aqueous ammonia (10 ml) and ethyl acetate (4 ml) under ice-cooling, and the mixture was stirred at the same temperature for 30 min. Water was added to the reaction mixture, and the mixture was extracted 3 times with chloroform. The organic layer was washed with water and brine, dried over anhydrous sodium sulfate, and concentrated under reduced pressure. The obtained residue was purified by silica gel column chromatography (chloroform:methanol=10:1). Diethyl ether was added to the obtained solid residue and the mixture was stirred for 10 min. The solid was collected by filtration and dried to give the title compound as a white amorphous solid (135 mg).

### Example 132

### Production of 1-{5-cyclopropyl-1-[trans-4-(1H-tetrazol-5-yl)cyclohexyl]-1H-pyrazole-4-carbonyl}-[(S)-3-(2-trifluoromethylphenyl)]pyrrolidine

### Step 1

### Production of 1-[1-(trans-4-cyanocyclohexyl)-5-cyclopropyl-1H-pyrazole-4-carbonyl]-[(S)-3-(2-trifluoromethylphenyl)]pyrrolidine

1-[1-(trans-4-Carbamoylcyclohexyl)-5-cyclopropyl-1H-pyrazole-4-carbonyl]-[(S)-3-(2-trifluoromethylphenyl)]pyrrolidine (514 mg) produced in Example 130, Step 7 was dissolved in tetrahydrofuran (6 ml), and pyridine (180 µl) and trifluoromethanesulfonic acid anhydride (220 µl) were added. The mixture was stirred at room temperature for 1 hr. Pyridine (180 µl) and trifluoromethanesulfonic acid anhydride (220 µl) were further added, and the mixture was stirred at room temperature for 1 hr. The mixture was concentrated under reduced pressure, and 1N aqueous potassium hydrogen sulfate solution was added. The mixture was extracted with ethyl acetate. The ethyl acetate layer was washed with water, dried over anhydrous magnesium sulfate, and concentrated under reduced pressure. The obtained residue was purified by chromatography (chloroform:methanol=15:1) to give the title compound as a pale-yellow amorphous form (207 mg).

### Step 2

### Production of 1-{5-cyclopropyl-1-[trans-4-(1H-tetrazol-5-yl)cyclohexyl]-1H-pyrazole-4-carbonyl}-[(S)-3-(2-trifluoromethylphenyl)]pyrrolidine

1-[1-(trans-4-Cyanocyclohexyl)-5-cyclopropyl-1H-pyrazole-4-carbonyl]-[(S)-3-(2-trifluoromethylphenyl)]pyrrolidine (207 mg) produced in the previous step was dissolved in 1,4-dioxane (2.5 ml), and azidotributyltin (188 mg) was added. The mixture was heated under reflux overnight. Azidotributyltin (94 mg) was further added and the mixture was heated under reflux for 4 hr. The mixture was concentrated under reduced pressure, 1N hydrochloric acid was added, and the mixture was extracted twice with chloroform. The chloroform layer was dried over anhydrous magnesium sulfate, and concentrated under reduced pressure. The obtained residue was purified by silica gel column chromatography (chloroform:methanol=25:2) and concentrated under reduced pressure. Diisopropyl ether was added to the obtained residue, and the precipitated solid was collected by filtration, and dried to give the title compound as a white amorphous solid (122 mg).

### Example 133

### Production of 1-{5-(1-methylcyclopropyl)-1-[1-(4-trifluoromethylphenylcarbamoyl)piperidin-4-yl]-1H-pyrazole-4-carbonyl}-3-(pyridin-3-yl)pyrrolidine

By a method similar to that of Example 3, the title compound was obtained from the corresponding starting compound.

### Example 134

### Production of 1-{1-[1-(2-fluorophenylcarbamoyl)piperidin-4-yl]-5-(1-methoxycyclopropyl)-1H-pyrazole-4-carbonyl}-3-(2-trifluoromethylphenyl)pyrrolidine

By a method similar to that of Example 3, the title compound was obtained from the corresponding starting compound.

### Example 135

### Production of 1-{1-[1-(2-fluorophenylcarbamoyl)piperidin-4-yl]-5-(1-methylcyclopropyl)-1H-pyrazole-4-carbonyl}-3-phenyl-3-trifluoromethylpyrrolidine

By a method similar to that of Example 3, the title compound was obtained from the corresponding starting compound.

### Example 136

### Production of 3-(2-chlorophenyl)-1-{5-cyclopropyl-1-[1-(2,4-difluorophenylcarbamoyl)piperidin-4-yl]-1H-pyrazole-4-carbonyl}pyrrolidine

By a method similar to that of Example 3, the title compound was obtained from the corresponding starting compound.

### Example 137

### Production of 3-(2-chloropyridin-3-yl)-1-{5-cyclopropyl-1-[1-(2-fluorophenylcarbamoyl)piperidin-4-yl]-1H-pyrazole-4-carbonyl}pyrrolidine

By a method similar to that of Example 3, the title compound was obtained from the corresponding starting compound.

### Example 138

### Production of 1-{1-[1-(2-fluorophenylcarbamoyl)piperidin-4-yl]-5-(2,2,3,3-tetramethylcyclopropyl)-1H-pyrazole-4-carbonyl}-3-(pyridin-3-yl)pyrrolidine

By a method similar to that of Example 3, the title compound was obtained from the corresponding starting compound.

### Example 139

### Production of 1-{5-cyclohexyl-1-[1-(2-fluorophenylcarbamoyl)piperidin-4-yl]-1H-pyrazole-4-carbonyl}-3-(pyridin-3-yl)pyrrolidine

By a method similar to that of Example 3, the title compound was obtained from the corresponding starting compound.

### Example 140

### Production of 1-{5-cyclopropyl-1-[1-(2-fluorophenylcarbamoyl)piperidin-4-yl]-1H-pyrazole-4-carbonyl}-3-(2-trifluoromethoxyphenyl)pyrrolidine

By a method similar to that of Example 3, the title compound was obtained from the corresponding starting compound.

### Example 141

### Production of 1-{5-cyclopropyl-1-[1-(4-trifluoromethylphenylcarbamoyl)piperidin-4-yl]-1H-pyrazole-4-carbonyl}-3-(2-trifluoromethylphenyl)pyrrolidine

By a method similar to that of Example 3, the title compound was obtained from the corresponding starting compound.

### Example 142

### Production of 1-(5-cyclopropyl-1-{1-[((S)-1-carboxy-2-methylpropyl)methylcarbamoyl]piperidin-4-yl}-1H-pyrazole-4-carbonyl)-3-(2-trifluoromethylphenyl)pyrrolidine

By a method similar to that of Example 12, the title compound was obtained from the corresponding starting compound.

### Example 143

### Production of 1-{5-cyclopropyl-1-[1-(2-fluorophenylcarbamoyl)piperidin-4-yl]-1H-pyrazole-4-carbonyl}-3-methoxy-3-(2-trifluoromethylphenyl)pyrrolidine

By a method similar to that of Example 3, the title compound was obtained from the corresponding starting compound.

### Example 144

### Production of 1-{1-[1-(2-fluorophenylcarbamoyl)piperidin-4-yl]-5-(1-hydroxymethylcyclopropyl)-1H-pyrazole-4-carbonyl}-3-(2-trifluoromethylphenyl)pyrrolidine

By a method similar to that of Example 3, the title compound was obtained from the corresponding starting compound.

### Example 145

### Production of 1-{5-cyclopropyl-1-[1-(thiazol-2-ylcarbamoyl)piperidin-4-yl]-1H-pyrazole-4-carbonyl}-3-(2-trifluoromethylphenyl)pyrrolidine

By a method similar to that of Example 10, the title compound was obtained from the corresponding starting compound.

### Example 146

### Production of 1-{1-[1-(isopropoxycarbamoyl)piperidin-4-yl]-5-(1-methylcyclopropyl)-1H-pyrazole-4-carbonyl}-3-(2-trifluoromethylphenyl)pyrrolidine

By a method similar to that of Example 10, the title compound was obtained from the corresponding starting compound.

### Example 147

### Production of 1-{5-cyclopropyl-1-[1-(4-fluorobenzylcarbamoyl)piperidin-4-yl]-1H-pyrazole-4-carbonyl}-3-(2-trifluoromethylphenyl)pyrrolidine

By a method similar to that of Example 10, the title compound was obtained from the corresponding starting compound.

### Example 148

### Production of 1-{1-[1-(2,3-dihydroindole-1-carbonyl)piperidin-4-yl]-5-(1-methylcyclopropyl)-1H-pyrazole-4-carbonyl}-3-(pyridin-3-yl)pyrrolidine

By a method similar to that of Example 10, the title compound was obtained from the corresponding starting compound.

### Example 149

### Production of 1-{1-[1-(2,3-dihydro[1,4]oxazine-4-carbonyl)piperidin-4-yl]-5-(1-methylcyclopropyl)-1H-pyrazole-4-carbonyl}-3-(pyridin-3-yl)pyrrolidine

By a method similar to that of Example 10, the title compound was obtained from the corresponding starting compound.

### Example 150

### Production of 1-{5-cyclopropyl-1-[1-(2,6-dichloropyridin-3-ylcarbamoyl)piperidin-4-yl]-1H-pyrazole-4-carbonyl}-3-(2-trifluoromethylphenyl)pyrrolidine

By a method similar to that of Example 10, the title compound was obtained from the corresponding starting compound.

### Example 151

### Production of 1-{5-(1-methylcyclopropyl)-1-[1-(2,3,4,5-tetrahydrobenzo[b]azepine-1-carbonyl)piperidin-4-yl]-1H-pyrazole-4-carbonyl}-3-(pyridin-3-yl)pyrrolidine

By a method similar to that of Example 10, the title compound was obtained from the corresponding starting compound.

### Example 152

### Production of ethyl 4-(4-{5-cyclopropyl-4-[3-(2-trifluoromethylphenyl)pyrrolidine-1-carbonyl]pyrazol-1-yl}piperidin-1-yl)benzoate

By a method similar to that of Example 114, the title compound was obtained from the corresponding starting compound.

### Example 153

### Production of 1-[1-(1-benzyloxycarbonylpiperidin-4-yl)-5-cyclopropyl-1H-pyrazole-4-carbonyl]-3-(pyridin-3-yl)pyrrolidine

By a method similar to that of Example 1, Step 4, the title compound was obtained.

### Example 154

### Production of 1-{1-[1-(3-carboxy-3-methyl-butyryl)piperidin-4-yl]-5-cyclopropyl-1H-pyrazole-4-carbonyl}-3-(2-trifluoromethylphenyl)pyrrolidine

By a method similar to that of Example 65, the title compound was obtained from the corresponding starting compound.

### Example 155

### Production of 1-{5-cyclopropyl-1-[1-(2-phenoxyacetyl)piperidin-4-yl]-1H-pyrazole-4-carbonyl}-3-(pyridin-3-yl)pyrrolidine

By a method similar to that of Example 64, the title compound was obtained from the corresponding starting compound.

### Example 156

### Production of 1-{1-[1-(3-chlorobenzoyl)piperidin-4-yl]-5-cyclopropyl-1H-pyrazole-4-carbonyl}-3-(pyridin-3-yl)pyrrolidine

By a method similar to that of Example 64, the title compound was obtained from the corresponding starting compound.

### Example 157

### Production of 1-{1-[1-(2-chlorobenzoyl)piperidin-4-yl]-5-cyclopropyl-1H-pyrazole-4-carbonyl}-3-(pyridin-3-yl)pyrrolidine

By a method similar to that of Example 64, the title compound was obtained from the corresponding starting compound.

### Example 158

### Production of 1-[5-cyclopropyl-1-(1-oxalylpiperidin-4-yl)-1H-pyrazole-4-carbonyl]-3-(2-trifluoromethylphenyl)pyrrolidine

By a method similar to that of Example 65, the title compound was obtained from the corresponding starting compound.

### Example 159

### Production of 1-{1-[1-(4-chlorobenzoyl)piperidin-4-yl]-5-cyclopropyl-1H-pyrazole-4-carbonyl}-3-(pyridin-3-yl)pyrrolidine

By a method similar to that of Example 64, the title compound was obtained from the corresponding starting compound.

### Example 160

### Production of 1-(5-cyclopropyl-1-{1-[2-(4-fluorophenyl)acetyl]piperidin-4-yl}-1H-pyrazole-4-carbonyl)-3-(pyridin-3-yl)pyrrolidine

By a method similar to that of Example 64, the title compound was obtained from the corresponding starting compound.

### Example 161

### Production of 1-{5-cyclopropyl-1-[1-(3-trifluoromethylbenzoyl)piperidin-4-yl]-1H-pyrazole-4-carbonyl}-3-(pyridin-3-yl)pyrrolidine

By a method similar to that of Example 64, the title compound was obtained from the corresponding starting compound.

### Example 162

### Production of 1-{5-cyclopropyl-1-[1-(3-methoxybenzoyl)piperidin-4-yl]-1H-pyrazole-4-carbonyl}-3-(pyridin-3-yl)pyrrolidine

By a method similar to that of Example 64, the title compound was obtained from the corresponding starting compound.

### Example 163

### Production of 1-{1-[1-(4-fluorobenzyl)piperidin-4-yl]-5-(1-methylcyclopropyl)-1H-pyrazole-4-carbonyl}-3-(2-trifluoromethylphenyl)pyrrolidine hydrochloride

By a method similar to that of Example 94, the title compound was obtained from the corresponding starting compound.

### Example 164

### Production of 1-[1-(1-benzenesulfonylpiperidin-4-yl)-5-cyclopropyl-1H-pyrazole-4-carbonyl]-3-(pyridin-3-yl)pyrrolidine

By a method similar to that of Example 108, the title compound was obtained from the corresponding starting compound.

### Example 165

### Production of 1-{1-[1-(4-carbamoylphenyl)piperidin-4-yl]-5-cyclopropyl-1H-pyrazole-4-carbonyl}-3-(2-trifluoromethylphenyl)pyrrolidine

By a method similar to that of Example 113, the title compound was obtained from the corresponding starting compound.

### Example 166

### Production of 1-{5-cyclopropyl-1-[1-(4-hydroxymethylphenyl)piperidin-4-yl]-1H-pyrazole-4-carbonyl}-3-(2-trifluoromethylphenyl)pyrrolidine

By a method similar to that of Example 113, the title compound was obtained from the corresponding starting compound.

### Example 167

### Production of 1-{1-[1-(5-carbamoyl-pyridin-2-yl)piperidin-4-yl]-5-cyclopropyl-1H-pyrazole-4-carbonyl}-3-(2-trifluoromethylphenyl)pyrrolidine

By a method similar to that of Example 113, the title compound was obtained from the corresponding starting compound.

### Example 168

### Production of 1-{1-[1-(4-aminophenyl)piperidin-4-yl]-5-cyclopropyl-1H-pyrazole-4-carbonyl}-3-(2-trifluoromethylphenyl)pyrrolidine

By a method similar to that of Example 113, the title compound was obtained from the corresponding starting compound.

### Example 169

### Production of 1-(5-cyclopropyl-1-{1-[4-(2-oxooxazolidin-3-yl)phenyl]piperidin-4-yl}-1H-pyrazole-4-carbonyl)-3-(2-trifluoromethylphenyl)pyrrolidine

By a method similar to that of Example 113, the title compound was obtained from the corresponding starting compound.

### Example 170

### Production of 1-(5-cyclopropyl-1-{1-[4-(3-methoxyureido)phenyl]piperidin-4-yl}-1H-pyrazole-4-carbonyl)-3-(2-trifluoromethylphenyl)pyrrolidine

By a method similar to that of Example 113, the title compound was obtained from the corresponding starting compound.

### Example 171

### Production of 1-{5-cyclopropyl-1-[1-(4-methanesulfonylaminophenyl)piperidin-4-yl]-1H-pyrazole-4-carbonyl}-3-(2-trifluoromethylphenyl)pyrrolidine

By a method similar to that of Example 113, the title compound was obtained from the corresponding starting compound.

### Example 172

### Production of 1-[1-(1-ethoxycarbamoylpiperidin-4-yl)-5-(1-methylcyclopropyl)-1H-pyrazole-4-carbonyl]-3-(2-trifluoromethylphenyl)pyrrolidine

By a method similar to that of Example 10, the title compound was obtained from the corresponding starting compound.

### Example 173

### Production of 1-[1-(5-chloropyridin-2-yl)azetidin-3-yl]-5-cyclopropyl-1H-pyrazole-4-carbonyl]-3-(2-trifluoromethylphenyl)pyrrolidine

By a method similar to that of Example 113, the title compound was obtained from the corresponding starting compound.

### Example 174

### Production of 1-{1-[1-(5-chloropyridin-2-yl)piperidin-4-yl]-5-(1-methylcyclopropyl)-1H-pyrazole-4-carbonyl}-3-methoxymethyl-3-phenylpyrrolidine

By a method similar to that of Example 113, the title compound was obtained from the corresponding starting compound.

### Example 175

### Production of 1-{1-[1-(5-cyanopyridin-2-yl)piperidin-4-yl]-5-cyclopropyl-1H-pyrazole-4-carbonyl}-3-(2-trifluoromethylphenyl)pyrrolidine

By a method similar to that of Example 113, the title compound was obtained from the corresponding starting compound.

### Example 176

### Production of 3-(2-acetoxyethyl)-3-(4-fluorophenyl)-1-{5-(1-methylcyclopropyl)-1-[1-(pyrimidin-2-yl)piperidin-4-yl]-1H-pyrazole-4-carbonyl}pyrrolidine hydrochloride

By a method similar to that of Example 113, the title compound was obtained from the corresponding starting compound.

### Example 177

### Production of {(3S*,4R*)-3-methyl-1-{5-(1-methylcyclopropyl)-1-[1-(pyrimidin-2-yl)piperidin-4-yl]-1H-pyrazole-4-carbonyl}-4-phenylpyrrolidine hydrochloride

By a method similar to that of Example 113, the title compound was obtained from the corresponding starting compound.

### Example 178

### Production of methyl 6-(4-{5-cyclopropyl-4-[3-(2-trifluoromethylphenyl)pyrrolidine-1-carbonyl]pyrazol-1-yl}piperidin-1-yl)nicotinate

By a method similar to that of Example 113, the title compound was obtained from the corresponding starting compound.

### Example 179

### Production of methyl 2-{1-[5-(1-methylcyclopropyl)-1-(1-pyrimidin-2-ylpiperidin-4-yl)-1H-pyrazole-4-carbonyl]pyrrolidin-3-yl}benzoate

By a method similar to that of Example 113, the title compound was obtained from the corresponding starting compound.

### Example 180

### Production of 3-(2-hydroxymethylphenyl)-1-{5-(1-methylcyclopropyl)-1-[1-(pyrimidin-2-yl)piperidin-4-yl]-1H-pyrazole-4-carbonyl}pyrrolidine hydrochloride

By a method similar to that of Example 113, the title compound was obtained from the corresponding starting compound.

### Example 181

### Production of 1-[5-cyclopropyl-1-(1-pyridin-2-ylpiperidin-4-yl)-1H-pyrazole-4-carbonyl]-3-hydroxy-3-(pyridin-3-yl)pyrrolidine

By a method similar to that of Example 113, the title compound was obtained from the corresponding starting compound.

### Example 182

### Production of 1-{1-[1-(4-acetylaminophenyl)piperidin-4-yl]-5-cyclopropyl-1H-pyrazole-4-carbonyl}-3-(2-trifluoromethylphenyl)pyrrolidine

By a method similar to that of Example 114, the title compound was obtained from the corresponding starting compound.

### Example 183

### Production of Sodium 4-(4-{5-cyclopropyl-4-[3-(2-trifluoromethylphenyl)pyrrolidine-1-carbonyl]pyrazol-1-yl}piperidin-1-yl)-3-fluorobenzoate

By a method similar to that of Example 114, the title compound was obtained from the corresponding starting compound.

### Example 184

### Production of 3-(2-cyanophenyl)-1-{5-cyclopropyl-1-[1-(2-fluorophenylcarbamoyl)piperidin-4-yl]-1H-pyrazole-4-carbonyl}pyrrolidine

By a method similar to that of Example 121, the title compound was obtained from the corresponding starting compound.

### Example 185

### Production of 3-hydroxymethyl-1-{1-[1-(4-methoxypyrimidin-2-yl)piperidin-4-yl]-5-(1-methylcyclopropyl)-1H-pyrazole-4-carbonyl}-3-phenylpyrrolidine hydrochloride

By a method similar to that of Example 127, the title compound was obtained from the corresponding starting compound.

### Example 186

### Production of 3-(3,5-difluorophenyl)-3-hydroxymethyl-1-{5-(1-methylcyclopropyl)-1-[1-(pyrimidin-2-yl)piperidin-4-yl]-1H-pyrazole-4-carbonyl}pyrrolidine hydrochloride

By a method similar to that of Example 127, the title compound was obtained from the corresponding starting compound.

### Example 187

### Production of 1-{1-[1-(3-chloropyridin-2-yl)piperidin-4-yl]-5-cyclopropyl-1H-pyrazole-4-carbonyl}-3-(pyridin-3-yl)pyrrolidine

By a method similar to that of Example 113, the title compound was obtained from the corresponding starting compound.

### Example 188

### Production of 1-{5-cyclopropyl-1-[1-(2-fluorophenylcarbamoyl)piperidin-4-yl]-1H-pyrazole-4-carbonyl}-3-(thiazol-2-yl)pyrrolidine

By a method similar to that of Example 121, the title compound was obtained from the corresponding starting compound.

### Example 189

### Production of (S)-2-[(trans-4-{5-cyclopropyl-4-[3-(2-trifluoromethylphenyl)pyrrolidine-1-carbonyl]pyrazol-1-yl}cyclohexanecarbonyl)methylamino]-3-methylbutyric acid

By a method similar to that of Example 130, the title compound was obtained from the corresponding starting compound.

### Example 190

### Production of 1-{5-(1-methylcyclopropyl)-1-[trans-4-(2-fluorophenylcarbamoyl)cyclohexyl]-1H-pyrazole-4-carbonyl}-3-(2-trifluoromethylphenyl)pyrrolidine

By a method similar to that of Example 130, the title compound was obtained from the corresponding starting compound.

### Example 191

### Production of cis-4-{5-(1-methylcyclopropyl)-4-[3-(2-trifluoromethylphenyl)pyrrolidine-1-carbonyl]pyrazol-1-yl}cyclohexanecarboxylic acid

By a method similar to that of Example 130, the title compound was obtained from the corresponding starting compound.

The structural formula and NMR data of the compound of each Example are shown in the following Table 1-1 to Table 1-30.

**[Table 1-1]**

| Example No. | Structural formula | NMR |
|---|---|---|
| 1 | 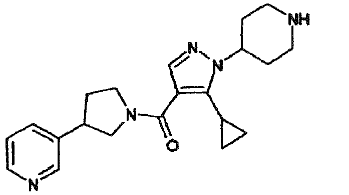 | ¹H-NMR (400MHz, CDCl₃) δ: 0.63-0.77 (2H, m), 0.97-1.11 (2H, m), 1.70-1.82 (1H, m), 1.96-2.67 (8H, m), 2.83-2.97 (2H, m), 3.33-3.53 (3H, m), 3.55-4.17 (3H, m), 4.50-4.64 (1H, m), 7.23-7.32 (1H, m), 7.48-7.64 (2H, m), 8.46-8.61 (2H, m) |
| 2 | 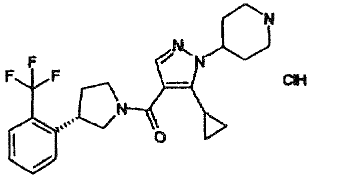 | ¹H-NMR (400MHz, DMSO-D₆) δ : 0.59-0.75 (2H, m), 0.94-1.07 (2H, m), 1.86-2.36 (8H, m), 3.00-3.21 (2H, m), 3.30-3.93 (6H, m), 4.69-4.88 (1H, m), 7.44-7.64 (2H, m), 7.66-7.78 (3H, m), 8.83 (1H, brs), 9.22 (1H, brs) |
| 3 | 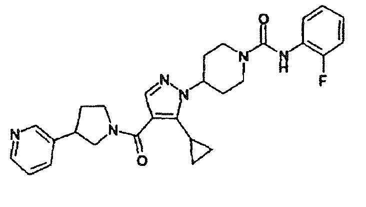 | ¹H-NMR (400MHz, CDCl₃) δ: 0.65-0.83 (2H, m), 0.99-1.15 (2H, m), 1.73-1.84 (1H, m), 1.90-2.50 (6H, m), 3.02-3.20 (2H, m), 3.36-3.55 (1.5H, m), 3.56-3.80 (2H, m), 3.83-3.99 (1H, m), 4.06-4.17 (0.5H, m), 4.18-4.36 (2H, m), 4.54-4.71 (1H, m), 6.71 (1H, brs), 6.93-7.00 (1H, m), 7.02-7.14 (2H, m), 7.24-7.32 (1H, m), 7.50-7.62 (2H, m), 8.02-8.09 (1H, m), 8.47-8.59 (2H, m) |
| 4 | 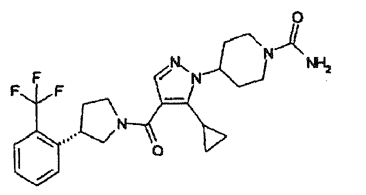 | ¹H-NMR (400MHz, CDCl₃) δ: 0.69-0.84 (2H, m), 1.03-1.14 (2H, m), 1.54-2.47 (7H, m), 2.94-3.14 (2H, m), 3.40-4.20 (7H, m), 4.47-4.67 (2H, m), 7.32-7.72 (6H, m) |
| 5 | 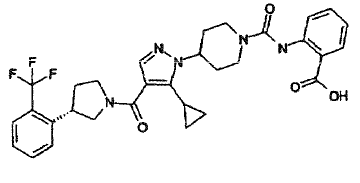 | ¹H-NMR (400MHz, DMSO-D₆) δ : 0.60-0.75 (2H, m), 0.92-1.07 (2H, m), 1.81-2.36 (6H, m), 3.04-3.23 (2H, m), 3.44-3.93 (6H, m), 4.10-4.27 (2H, m), 4.65-4.83 (1H, m), 6.98-7.07 (1H, m), 7.39-7.61 (3H, m), 7.63-7.79 (3H, m), 7.92-8.02 (1H, m), 8.37-8.40 (1H, m), 11.0-11.2 (1H, m), 13.6 (1H, brs) |
| 6 | 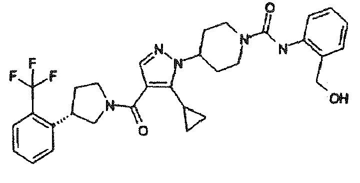 | ¹H-NMR (400MHz, CDCl₃) δ: 0.68-0.85 (2H, m), 1.01-1.14 (2H, m), 1.70-2.44 (7H, m), 2.72-2.86 (1H, m), 2.97-3.13 (2H, m), 3.41-4.09 (5H, m), 4.20-4.35 (2H, m), 4.51-4.75 (3H, m), 6.97-7.04 (1H, m), 7.11-7.19 (1H, m), 7.28-7.60 (5H, m), 7.64-7.72 (1H, m), 7.92-7.98 (1H, m), 8.13-8.23 (1H, m) |

**[Table 1-2]**

| Example No. | Structural formula | NMR |
|---|---|---|
| 7 | 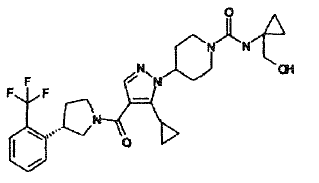 | ¹H-NMR (400MHz, CDCl₃) δ: 0.61-0.94 (6H, m), 0.98-1.17 (2H, m), 1.64-1.86 (3H, m), 1.93-2.23 (3H, m), 2.24-2.48 (1 H, m), 2.79-2.99 (2H, m), 3.32-4.22 (9H, m), 4.38-4.63 (1H, m), 5.64-5.87 (1H, m), 7.29-7.60 (4H, m), 7.60-7.71 (1H, m) |
| 8 | 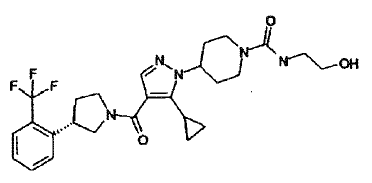 | ¹H-NMR (400MHz, CDCl₃) δ: 0.66-0.82 (2H, m), 0.99-1.12 (2H, m), 1.20-1.34 (1H, m) 1.67-1.88 (4H, m), 1.95-2.21 (3H, m), 2.23-2.46 (1H, m), 2.82-3.02 (2H, m), 3.28-3.48 (3H, m), 3.52-3.99 (5H, m), 3.99-4.22 (2H, m), 4.41-4.63 (1H, m), 5.16-5.33 (1H, m), 7.28-7.71 (5H, m) |
| 9 | 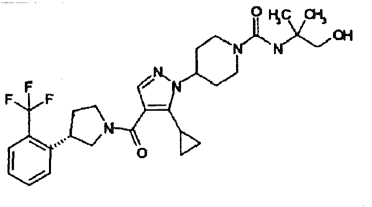 | ¹H-NMR (400MHz, CDCl₃) δ: 0.64-0.87 (2H, m), 0.98-1.17 (2H, m), 1.31 (3H, s), 1.32 (3H, s), 1.66-2.25 (6H, m), 2.26-2.47 (1H, m), 2.83-3.02 (2H, m), 3.39-4.20 (9H, m), 4.43-4.64 (1 H, m), 4.67-4.84 (1H, m), 5.00-5.46 (1H, brs), 7.32-7.40 (1H, m), 7.41-7.60 (3H, m), 7.61-7.72 (1 H, m) |
| 10 | 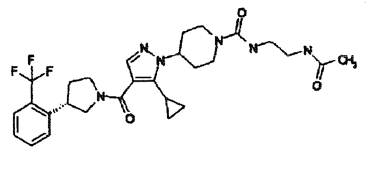 | ¹H-NMR (400MHz, CDCl₃) δ: 0.67-0.82 (2H, m), 1.00-1.11 (2H, m), 1.70-1.83 (1H, m), 1.83-1.95 (3H, m), 1.99 (3H, br s), 2.04-2.22 (3H, m), 2.27-2.43 (1H, m), 2.86-3.01 (2H, m), 3.33-3.43 (4H, m), 3.54-4.18 (6H, m), 4.44-4.63 (1H, m), 5.42-5.53 (1H, m), 6.33-6.41 (1H, m), 7.32-7.71 (5H, m) |
| 11 | 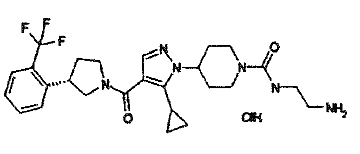 | ¹H-NMR (400MHz, DMSO-D₆) δ : 0.58-0.74 (2H, m),0.91-1.05 (2H, m), 1.78-2.34 (7H, m), 2.78-2.99 (5H, m), 3.22-3.94 (6H, m), 4.06-4.21 (2H, m), 4.54-4.72 (1H, m), 6.46-6.95 (1H, m), 7.42-7.77 (5H, m), 7.88-8.03 (3H, m) |
| 12 | 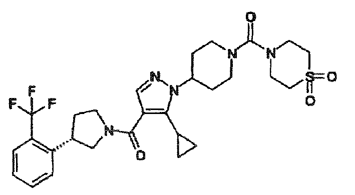 | ¹H-N (400MHz, CDCl₃) δ: 0.69-0.86 (2H, m), 1.01-1.16 (2H, m), 1.71-1.86 (1H, m), 1.89-2.46 (6H, m), 2.91-3.15 (6H, m), 3.40-4.14 (11H, m), 4.47-4.67 (1H, m), 7.32-7.73 (5H, m) |
| 13 | 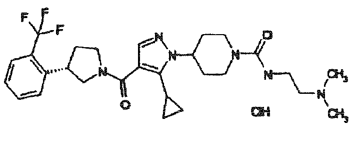 | ¹H-NMR (400MHz, DMSO-D₆) δ : 0.63-0.86 (2H, m), 1.00-1.15 (2H, m), 1.58-2.49 (15H, m), 2.84-3.03 (2H, m), 3.20-3.39 (2H, m), 3.41-4.25 (7H, m), 4.44-4.65 (1H, m), 5.13-5.29 (1H, m), 7.31-7.73 (5H, m) |

**[Table 1-3]**

| Example No. | Structural formula. | NMR |
|---|---|---|
| 14 | 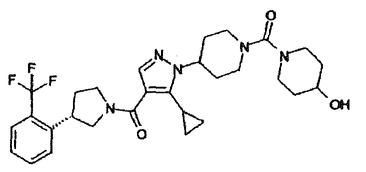 | ¹H-NMR (400MHz, CDCl₃) δ: 0.71-0.82 (2H, m), 1.03-1.12 (2H, m), 1.46-1.60 (3H, m), 1.72-1.97 (5H, m), 2.00-2.44 (4H, m), 2.80-3.07 (4H, m), 3.42-4.10 (10H, m), 4.43-4.61 (1H, m), 7.32-7.71 (5H, m) |
| 1 5 | 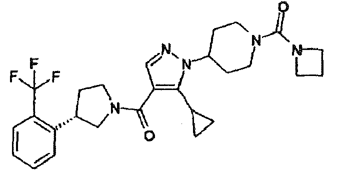 | ¹H-NMR (400MHz, CDCl₃) δ: 0.70-0.83 (2H, m),1.02-1.14 (2H, m), 1.71-2.45 (9H, m), 2.81-2.96 (2H, m), 3.42-4.22 (11H, m), 4.45-4.63 (1H, m), 7.32-7.72 (5H, m) |
| 1 6 | 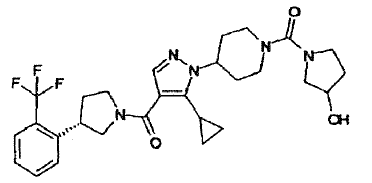 | ¹H-NMR (400MHz, CDCl₃) δ: 0.66-0.85 (2H, m), 0.99-1.17 (2H, m), 1.71-2.45 (10H, m), 2.79-3.00 (2H, m), 3.27-4.11 (11H. m), 4.39-4.63 (2H, m), 7.32-7.73 (5H, m) |
| 1 7 | 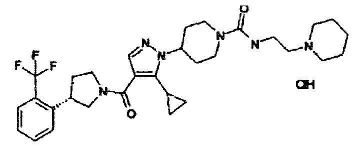 | ¹H-NMR (400MHz, DMSO-D₆) δ : 0.58-0.75 (2H, m),0.92-1.06 (2H, m), 1.32-1.47 (1H, m), 1.63-1.98 (6H, m), 2.02-2.39 (2H, m), 2.78-3.00 (4H, m), 3.02-3.16 (2H, m), 3.38-3.54 (4H, m), 3.54-3.78 (3H, m), 3.80-4.55 (8H, m), 4.55-4.72 (1H, m), 7.01-7.20 (1H. brs), 7.41-7.60 (2H, m), 7.65-7.78 (3H, m), 9.99-10.16 (1H, brs) |
| 1 8 | 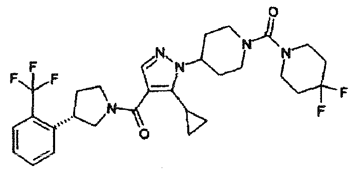 | ¹H-NMR (400MHz, CDCl₃) δ: 0.70-0.86 (2H, m), 1.01-1.15 (2H, m), 1.71-2.45 (11H, m), 2.83-3.01 (2H, m), 3.33-3.51 (4H, m), 3.57-4.11 (7H, m), 4.45-4.62 (1H, m), 7.31-7.73 (5H, m) |
| 1 9 | 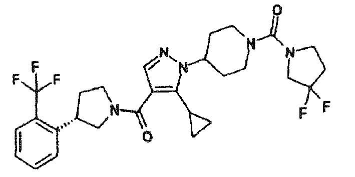 | ¹H-NMR (400MHz, CDCl₃) δ : 0.68-0.86 (2H, m), 1.02-1.15 (2H. m), 1.70-2. (9H, m), 2.83-3.01 (2H, m), 3.41-4.12 (11H, m), 4.46-4.65 (1H. m), 7.31-7.73 (5H, m) |
| 20 | 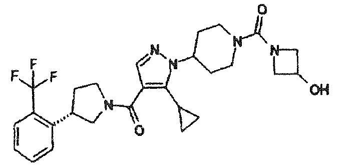 | ¹H-NMR (400MHz. CDCl₃) δ : 0.69-0.84 (2H, m), 1.00-1.16 (2H, m), 1.71-2.26 (6H, m), 2.27-2.45 (1H, m), 2.50-2.59 (1H, m), 2.81-3.00 (2H, m), 3.42-4.12 (9H, m), 4.46-4.68 (2H, m), 7.31-7.72 (5H, m) |

**[Table 1-4]**

| Example No. | Structural formula | NMR |
|---|---|---|
| 21 | 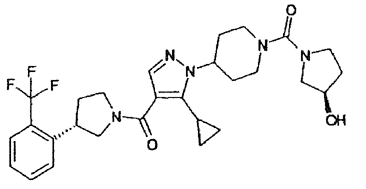 | ¹H-NMR (400MHz, CDCl₃) δ: 0.68-0.84 (2H, m), 0.99-1.15 (2H, m), 1.71-2.44 (10H, m), 2.78-2.99 (2H, m), 3.26-4.12 (11H, m), 4.42-4.62 (2H, m), 7.31-7.72 (5H, m) |
| 22 | 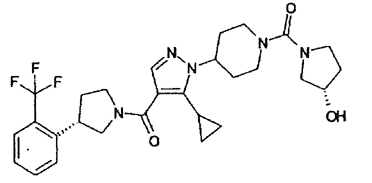 | ¹H-NMR (400MHz. CDCl₃) δ: 0.69-0.85 (2H, m), 0.99-1.16 (2H, m), 1.68-2.47 (10H, m), 2.76-2.99 (2H, m), 3.27-4.11 (11H, m). 4.41-4.62 (2H, m). 7.32-7.73 (5H, m) |
| 23 | 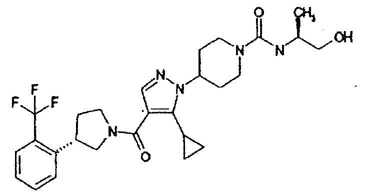 | ¹H-NMR (400MHz, CDCl₃) δ: 0.65-0.81 (2H, m), 1.05-1.15 (2H, m), 1.71-2.45 (7H. m), 1.94-2.44 (6H, m), 2.85-3.05 (2H, m), 3.35-4.20 (9H, m). 4.45-4.65 (1H, m), 4.65-4.75 (1H, m). 7.30-8.30 (5H, m) |
| 24 | 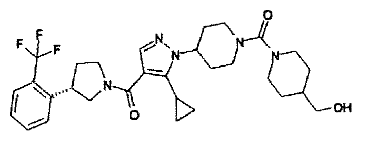 | ¹H-NMR (400MHz, CDCl₃) δ: 0.67-0.83 (2H, m), 1.02-1.13 (2H, m), 1.15-1.32 (3H, m), 1.35-1.46 (1H, m), 1.63-2.45 (9H, m), 2.73-2.98 (4H, m), 3.40-4.12 (11H, m), 4.42-4.63 (1H, m), 7.29-7.74 (5H, m) |
| 25 | 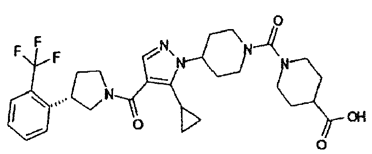 | ¹H-NMR (400MHz. DMSO-D₆) δ : 0.59-0.74 (2H, m), 0.91-1.06 (2H, m), 1.42-1.63 (2H, m), 1.72-2.46 (10H, m), 2.75-3.03 (4H, m), 3.43-3.96 (9H, m), 4.48-4.72 (1H, m), 7.38-7.83 (5H, m), 12.1-12.4 (1 H, m) |
| 26 | 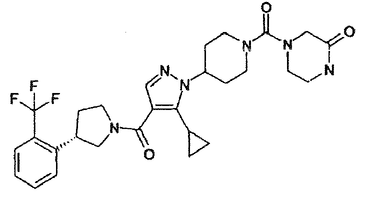 | ¹H-NMR (400MHz, CDCl₃) δ: 0.66-0.85 (2H, m), 0.97-1.17 (2H, m), 1.68-2.46 (7H, m), 2.84-3.08 (2H, m), 3.36-4.13 (13H, m), 4.44-4.67 (1H, m), 6.54-6.71 (1H. m). 7.30-7.73 (5H, m) |
| 27 | 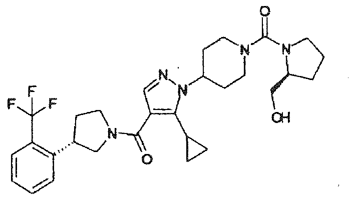 | ¹H-NMR (400MHz, CDCl₃) δ: 0.67-0.84 (2H, m), 1.00-1.15 (2H, m). 1.43-1.59 (1H, m), 1.61-2.45 (10H, m), 2.78-3.01 (2H, m), 3.32-4.10 (11H, m), 4.18-4.31 (1H, m), 4.45-4.63 (1H, m). 7.31-7.72 (5H, m) |

**[Table 1-5]**

| Example No. | Structural formula | NMR |
|---|---|---|
| 28 | 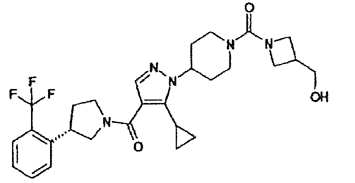 | ¹H-NMR (400MHz, CDCl₃) δ: 0.64-0.83 (2H, m), 0.96-1.16 (2H, m), 1.70-2.29 (6H, m), 2.29-2.39 (1H, m), 2.67-2.69 (1H, m), 2.82-2.97 (2H, m), 3.38-4.18 (13H, m), 4.41-4.65 (1H, m), 7.30-7.74 (5H, m) |
| 29 | 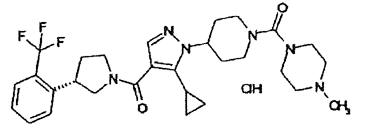 | ¹H-NMR (400MHz, DMSO-D₆) δ: 0.59-0.73 (2H, m), 0.94-1.06 (2H, m), 1.79-2.34 (7H, m), 2.74-2.82 (3H, m), 2.89-3.93 (17H, m), 4.55-4.74 (1H, m), 7.42-7.80 (5H, m), 10.4-10.6 (1 H, m) |
| 30 | 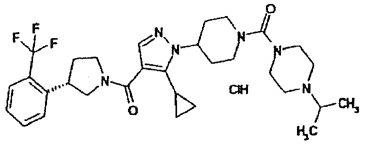 | ¹H-NMR (400MHz, DMSO-D₆) δ : 0.59-0.75 (2H, m), 0.92-1.06 (2H, m), 1.22-1.35 (6H, m), 1.77-2.46 (7H, m), 2.85-3.11 (5H, m), 3.21-3.96 (13H, m), 4.52-4.74 (1H, m), 7.42-7.82 (5H, m), 10.3-10.5 (1H, m) |
| 31 | 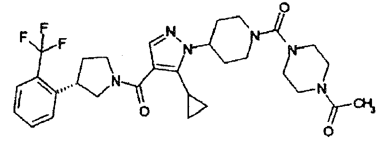 | ¹H-NMR (400MHz, CDCl₃) δ: 0.69-0.85 (2H, m), 1.02-1.16 (2H, m), 1.71-2.47 (10H, m). 2.87-3.03 (2H, m), 3.20-4.15 (15H, m), 4.45-4.66 (1H, m), 7.31-7.73 (5H, m) |
| 32 | 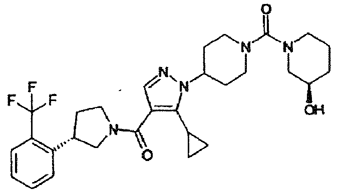 | ¹H-NMR (400MHz, CDCl₃) δ: 0.62-0.84 (2H, m), 0.97-1.15 (2H, m), 1.37-1.55 (1H, m), 1.55-1.96 (6H, m), 1.99-2.46 (5H, m), 2.77-3.03 (1H, m), 3.15-4.12 (12H, m), 4.38-4.65 (1H, m), 7.30-7.74 (5H, m) |
| 33 | 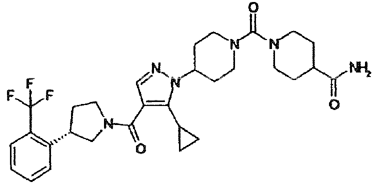 | ¹H-NMR (400MHz, CDCl₃) δ: 0.68-0.85 (2H, m), 1.01-1.14 (2H, m), 1.62-1.99 (7H, m), 1.99-2.47 (5H, m), 2.74-3.00 (4H, m), 3.41-3.53 (0.6H, m), 3.56-4.11 (8.4H, m), 4.43-4.61 (1H, m), 5.44 (1H, brs), 5. (1H, brs), 7.32-7.38 (1H, m), 7.39-7.60 (3H, m), 7.62-7.71 (1H, m) |
| 34 | 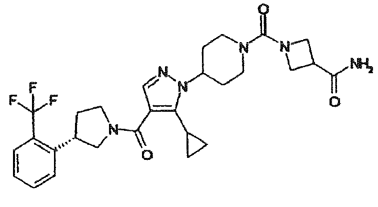 | ¹H-NMR (400MHz, CDCl₃) δ: 0.69-0.83 (2H, m), 0.99-1.15 (2H, m), 1.67-1.96 (3H, m), 1.98-2.24 (3H, m), 2.25-2.44 (1H, m), 2.78-3.00 (2H, m), 3.20-3.39 (1H, m), 3.40-3.53 (0.5H, m), 3.53-4.09 (6.5H, m), 4.10-4.25 (4H, m), 4.40-4.66 (1H, m), 5.59 (1H, brs), 5.77 (1H, brs), 7.31-7.39 (1H, m), 7.40-7.60 (3H, m), 7.62-7.71 (1H, m) |

**[Table 1-6]**

| Example No. | Structural formula | NMR |
|---|---|---|
| 35 | 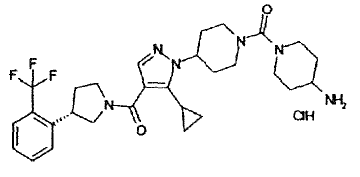 | ¹H-NMR (400MHz, DMSO-D₆) δ : 0.59-0.72 (2H, m), 0.94-1.06 (2H, m), 1.39-1.56 (2H, m), 1.78-2.01 (6H, m), 2.05-2.34 (2H, m), 2.72-3.01 (4H, m), 324-3.27 (1H, m), 3.43-4.01 (9H, m), 4.53-4.70 (1H, m), 7.43-7.60 (2H, m), 7.66-7.77 (3H, m), 8.33 (3H, brs) |
| 36 | 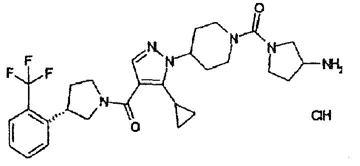 | ¹H-NMR (400MHz, DMSO-D₆) δ : 0.55-0.73 (2H, m), 0.89-1.08 (2H, m), 1.69-2.38 (10H, m), 2.77-3.02 (2H, m), 3.12-4.15 (11H, m), 4.52-4.73 (1H, m), 7.41-7.77 (3H, m), 8.18-8.40 (3H, m), 8.42-8.61 (1H. m), 9.36-9.63 (1H, m) |
| 37 | 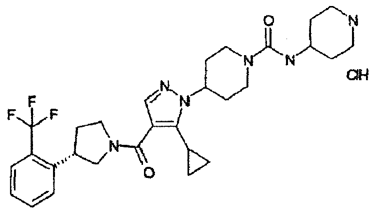 | ¹H-NMR (400MHz, DMSO-D₆) δ : 0.58-0.73 (2H, m), 0.92-1.04 (2H, m), 1.55-1.70 (2H, m), 1.74-1.98 (7H, m), 2.02-2.36 (2H, m), 2.73-3.02 (4H, m), 3.19-3.33 (2H, m), 3.45-4.32 (8H, m), 4.53-4.72 (1H, m), 6.44-6.62 (1H, m), 7.42-7.79 (5H, m), 8.62 (1H, br s), 8.72 (1H, br s) |
| 38 | 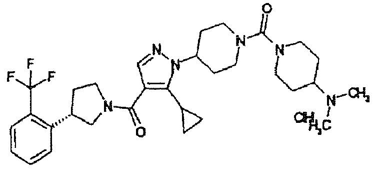 | ¹H-NMR (400MHz, DMSO-D₆) δ : 0.58-0.74 (2H, m). 0.92-1.06 (2H, m). 1.47-1.68 (2H, m), 1.78-2.56 (8H, m). 2.62-3.03 (4H, m), 2.77 (6H, s), 3.24-3.94 (10H. m), 4.54-4.70 (1H, m), 7.42-7.62 (2H, m), 7.63-7.79 (3H, m), 10.2-10.4 (1H, m) |
| 39 | 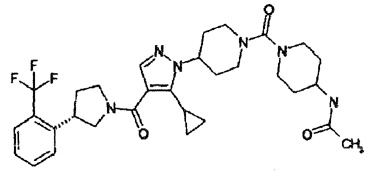 | ¹H-NMR (400MHz, CDCl₃) δ: 0.70-0.84 (2H, m), 1.02-1.14 (2H, m), 1.31-1.46 (2H, m), 1.71-2.45 (9H, m), 1.97 (3H, s), 2.81-3.00 (4H, m), 3.42-4.10 (10H, m), 4.44-4.61 (1H, m), 5.29-5.43 (1H, m), 7.33-7.72 (5H, m) |
| 40 | 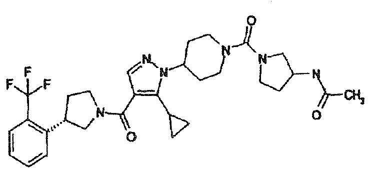 | ¹H-NMR (400MHz. CDCl₃) δ: 0.69-0.83 (2H, m), 1.00-1.13 (2H, m), 1.68-2.46 (13H, m), 2.81-3.00 (2H, m), 328-4.10 (10H. m), 4.36-4.63 (2H, m), 5.61-5.72 (1H, m), 7.31-7.70 (5H, m) |
| 41 | 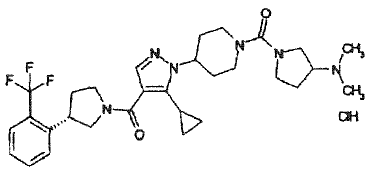 | ¹H-NMR (400MHz, DMSO-D₆) δ : 0.58-0.71 (2H, m). 0.92-1.03 (2H, m), 1.76-2.36 (8H, m), 2.70-2.80 (6H, brs), 2.81-3.03 (2H, m), 3.24-3.92 (13H, m), 4.52-4.71 (1H, m), 7.41-7.77 (5H, m), 10.76-10.91 (1H, brs) |

**[Table 1-7]**

| Example No. | Structural formula | NMR |
|---|---|---|
| 42 | 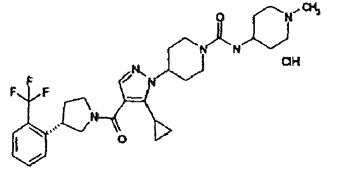 | ¹H-NMR (400MHz, DMSO-D₆) δ : 0.58-0.74 (2H, m), 0.92-1.05 (2H, m), 1.60-2.00 (9H, m), 2.03-2.38 (2H, m), 2.65-3.93 (15H, m), 4.04-4.24 (2H, m), 4.51-4.73 (1H, m), 6.28-6.65 (1H, m), 7.41-7.81 (5H, m), 9.88 (1H, brs) |
| 43 | 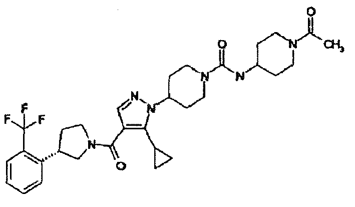 | ¹H-NMR (400MHz, CDCl₃) δ: 0.70-0.83 (2H, m), 1.02-1.15 (2H, m), 1.22-1.38 (2H, m), 1.70-2.26 (11H, m), 2.28-2.46 (1H, m), 2.67-2.79 (1H, m), 2.86-3.04 (2H, m), 3.10-3.24 (1H, m), 3.40-4.21 (9H, m), 4.32-4.45 (1H, m), 4.47-4.64 (2H, m), 7.32-7.72 (5H, m) |
| 44 | 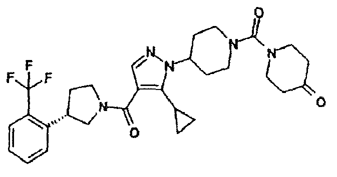 | ¹H-NMR (400MHz, CDCl₃) δ: 0.70-0.85 (2H, m), 1.02-1.18 (2H, m), 1.70-2.57 (11H, m), 2.91-3.08 (2H, m), 3.38-4.13 (11H, m), 4.74-4.66 (1H, m), 7.32-7.76 (5H, m) |
| 45 | 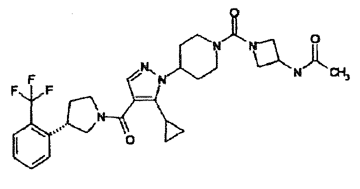 | ¹H-NMR (400MHz. CDCl₃) δ: 0.65-0.85 (2H, m), 0.96-1.17 (2H, m), 1.65-1.95 (3H, m), 1.96-2.22 (3H, m), 1.99 (3H, s), 2.25-2.48 (1H, m), 2.81-3.02 (2H, m), 3.38-3.5 (0.6H, m), 3.54-4.14 (8.4H, m), 4.19-4.37 (2H, m), 4.43-4.72 (2H, m), 6.24-6.40 (1H, m), 7.32-7.40 (1H, m), 7.41-7.61 (3H, m), 7.62-7.70 (1H, m) |
| 46 | 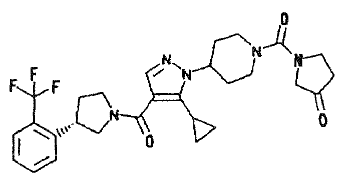 | ¹H-NMR (400MHz, CDCl₃) δ: 0.69-0.88 (2H, m), 1.02-1.17 (2H, m), 1.71-1.87 (1H, m), 1.88-2.45 (6H, m), 2.48-2.60 (2H, m), 2.90-3.06 (2H, m), 3.43-4.11 (11H, m), 4.49-4.66 (1H, m), 7.33-7.72 (5H, m) |
| 47 | 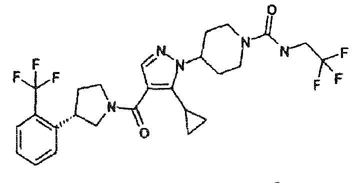 | ¹H-NMR (400MHz, CDCl₃) δ: 0.69-0.81 (2H, m), 1.01-1.12 (2H, m), 1.71-1.82 (1H, m), 1.83-1.96 (3H, m), 1.99-2.25 (3H, m), 2.26-2.43 (1 H, m), 2.93-3.08 (2H, m), 3.41-4.00 (6H, m), 4.00-4.20 (2H, m), 4.49-4.64 (1H, m), 4.85-4.97 (1 H, m), 7.30-7.70 (5H, m) |
| 48 | 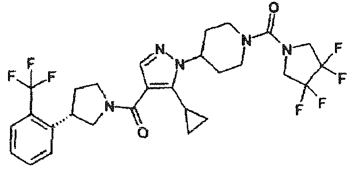 | ¹H-NMR (400MHz. CDCl₃) δ: 0.70-0.86 (2H, m), 1.01-1.17 (2H, m), 1.71-2.45 (7H, m), 2.86-3.06 (2H, m), 3.42-4.12 (11H, m), 4.49-4.67 (1H, m), 7.31-7.73 (5H, m) |

**[Table 1-8]**

| Example No. | Structural formula | NMR |
|---|---|---|
| 49 | 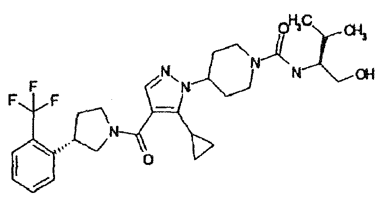 | ¹H-NMR (400MHz. CDCl₃) δ: 0.71-0.83 (2H, m), 0.92-1.02 (6H, m), 1.02-1.13 (2H, m), 1.22-1.33 (2H, m), 1.71-1.98 (4H, m), 1.99-2.45 (4H, m), 2.88-3.08 (2H, m), 3.14-3.28 (1H, brs), 3.38-4.24 (8H, m), 4.47-4.73 (2H, m), 7.32-7.72 (5H, m) |
| 50 | 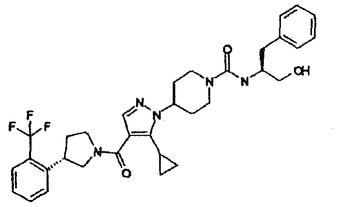 | ¹H-NMR (400MHz, CDCl₃) δ: 0.63-0.81 (2H, m), 0.96-1.13 (2H, m), 1.60-1.81 (3H, m), 1.90-2.21 (3H, m), 2.26-2.46 (1H, m), 2.72-2.98 (4H, m), 3.38-4.13 (9H, m), 4.37-4.59 (1H, m), 4.94-5.04 (1H, m), 7.16-7.73 (10H, m) |
| 51 | 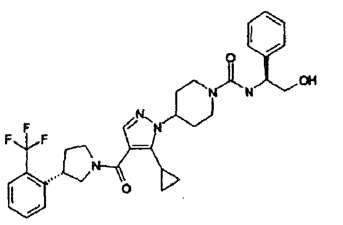 | ¹H-NMR (400MHz. CDCl₃) δ: 0.68-0.82 (2H, m), 1.00-1.12 (2H, m), 1.69-1.85 (3H, m), 1.96-2.22 (3H, m), 2.25-2.45 (1H, m), 2.84-3.01 (2H, m), 3.39-3.50 (2H, m), 3.54-4.27 (7H, m), 4.41-4.61 (1H, m), 4.93-5.04 (1H, m), 5.38-5.55 (1H, m). 7.23-7.73 (10H, m) |
| 52 | 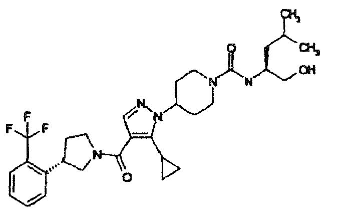 | ¹H-NMR (400MHz, CDCl₃) δ: 0.68-0.79 (2H, m), 0.89-0.99 (6H, m), 1.02-1.12 (2H, m), 1.31-1.43 (2H, m), 1.60-1.85 (4H, m), 2.00-2.20 (3H, m), 2.25-2.45 (1H, m), 2.81-3.00 (2H, m), 3.39-4.21 (10H, m), 4.42-4.60 (1H, m), 4.79-4.92 (1H, m), 7.30-7.73 (5H, m) |
| 53 | 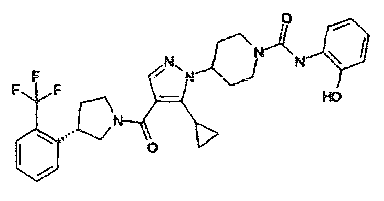 | ¹H-NMR (400MHz, CDCl₃) δ: 0.66-0.83 (2H, m), 1.00-1.14 (2H, m), 1.67-1.90 (3H, m), 1.98-2.27 (3H, m), 2.27-2.24 (1H, m), 2.98-3.14 (2H, m), 3.42-4.11 (5H, m), 4.22-4.40 (2H, m), 4.50-4. (1H, m), 6.78-6.88 (1H, m), 6.98-7.13 (3H, m), 7.14-7.21 (1H, m), 7.34-7.61 (4H, m), 7.64-7.73 (1H, m), 9.24-9.36 (1H, m) |
| 54 | 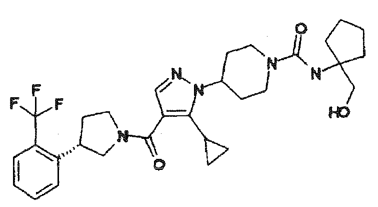 | ¹H-NMR (400MHz, CDCl₃) δ: 0.67-0.85 (2H, m), 0.97-1.16 (2H, m), 1.51-2.46 (15H, m), 2.84-3.07 (2H, m), 3.42-4.19 (9H, m), 4.47-4.64 (1H, m), 4.65-4.75 (1H, m), 5.14-5.24 (1H, m), 7.31-7.73 (5H, m) |

**[Table 1-9]**

| Example No. | Structural formula | NMR |
|---|---|---|
| 55 | 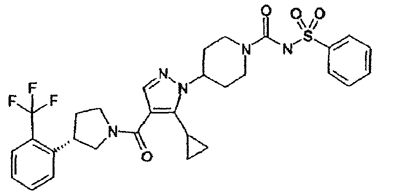 | ¹H-NMR (400MHz, CDCl₃) δ : 0.61-0.85 (2H, m), 0.95-1.18 (2H, m), 1.36-2.25 (7H, m), 2.25-2.44 (1H, m), 2.76-3.05 (2H, m), 3.38-3.47 (0.5H. m), 3.52-3.91 (3.5H, m), 3.95-4.31 (3H, m), 4.34-4.60 (1H, m), 7.29-7.73 (8H, m), 8.00-8.13 (2H, m) |
| 56 | 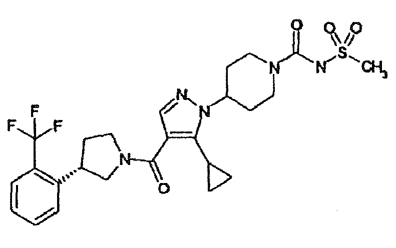 | ¹H-NMR (400MHz, CDCl₃) δ : 0.63-0.83 (2H, m), 0.98-1.16 (2H, m), 1.65-1.91 (3H, m), 1.92-2.23 (3H, m), 2.24-2.50 (1H, m), 2.80-3.05 (2H, m), 3.16-3.34 (3H, m), 3.39-3.51 (0.6H, m), 3.52-4.13 (5.4H, m), 4.19-4.43 (2H, m). 4.44-4.64 (1H, m), 7.30-7.39 (1 H, m), 7.41-7.60 (3H, m), 7.61-7.70 (1H, m) |
| 57 | 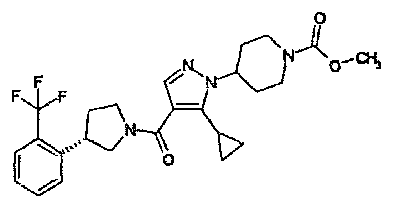 | ¹H-NMR (400MHz, CDCl₃) δ : 0.64-0.83 (2H, m), 0.98-1.15 (2H, m), 1.65-1.94 (3H, m), 1.94-2.25 (3H, m), 2.25-2.45 (1H, m), 2.77-3.05 (2H, m), 3.43-4.07 (8H, m), 4.11-4.45 (2H, m). 4.45-4.65 (1H, m), 7.30-7.72 (5H, m) |
| 58 | 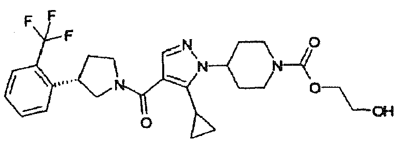 | ¹H-NMR (400MHz, DMSO-D₆) δ: 0.67-0.84 (2H, m), 1.03-1.15 (2H, m), 1.69-2.59 (8H, m), 2.86-3.11 (2H, m), 3.38-4.13 (7H, m), 4.19-4.44 (4H, m), 4.48-4.66 (1H, m), 7.30-7.74 (5H, m) |
| 59 | 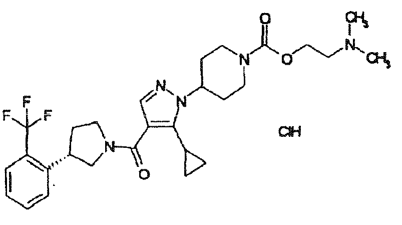 | ¹H-NMR (400MHz, CDCl₃) δ : 0.64-0.87 (2H, m), 0.99-1.18 (2H, m), 1.72-1.86 (1H, m), 1.87-1.99 (2H, m), 2.06-2.47 (4H, m), 2.63-3.23 (10H, m), 3.26-3.42 (2H, m), 3.43-4.13 (3H, m), 4.20-4.75 (5H, m), 7.33-7.39 (1H, m), 7.40-7.63 (3H, m), 7.64-7.69 (1H, m), 12.69 (1H, brs) |
| 60 | 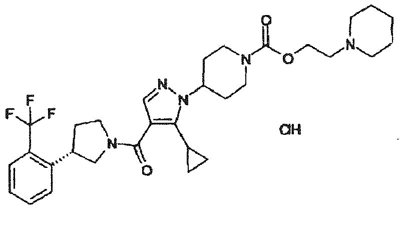 | ¹H-NMR (400MHz, CDCl₃) δ : 0.72-0.83 (2H, m), 1.07-1.23 (2H, m), 1.35-1.56 (1H, m), 1.79-200 (4H, m), 2.06-2.45 (6H, m), 2.68-3.20 (4H, m), 3.27-3.41 (2H, m), 3.43-4.08 (9H, m), 4.22-4.48 (2H, m), 4.58-4.82 (3H, m), 7.34-7.40 (1H, m), 7.44-7.50 (1H, m), 7.53-7.60 (1H, m), 7.65-7.72 (2H, m), 12. (1 H, brs) |

**[Table 1-10]**

| Example No. | Structural formula | NMR |
|---|---|---|
| 61 | 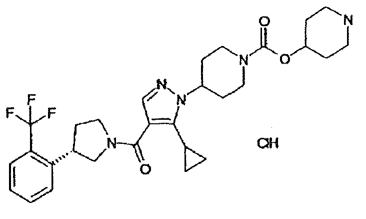 | ¹H-NMR (400MHz, CDCl₃) δ: 0.69-0.88 (2H, m), 1.03-1.21 (2H, m), 1.79-2.01 (3H, m), 2.01-2.32 (7H, m), 2.33-2.45 (1H, m), 2.81-3.12 (2H, m), 3.11-4.14 (9H, m), 4.15-4.49 (2H, m), 4.54-4.75 (1H, m), 4.92-5.09 (1H, m), 7.33-7.41 (1H, m), 7.44-7.61 (2H, m), 7.63-7.75 (2H, m), 9.45-9.81 (2H, m) |
| 62 | 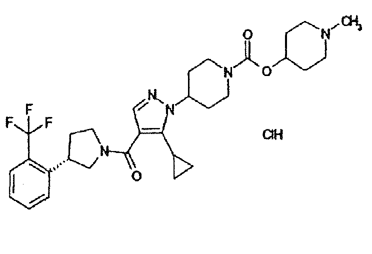 | ¹H-NMR (400MHz, CDCl₃) δ: 0.68-0.87 (2H, m), 1.04-1.21 (2H, m), 1.78-2.01 (3H, m), 2.03-2.49 (7H, m), 2.50-2.68 (1H, m), 2.72-3.14 (7H, m), 3.23-4.07 (7H, m), 4.17-4.44 (2H, m), 4.57-4.75 (1H, m), 4.76-4.89 (0.5H, m), 5.01-5.09 (0.5H, m), 7.33-7.41 (1H, m), 7.43-7.52 (1H, m), 7.52-7.61 (1H, m), 7.62-7.73 (2H, m), 12.38-12.70 (1H, m) |
| 63 | 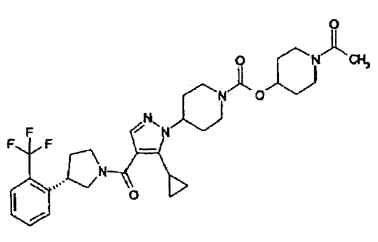 | ¹H-NMR (400MHz, CDCl₃) δ: 0.66-0.85 (2H, m), 0.99-1.16 (2H, m), 1.57-2.25 (13H, m), 2.26-2.46 (1H, m), 2.79-3.11 (2H, m), 3.27-3.54 (2.6H, m), 3.55-4.12 (6.4H, m). 4.14-4.66 (3H, m), 4.85-5.00 (1H, m), 7.31-7.40 (1H, m), 7.41-7.61 (3H, m), 7.62-7.71 (1H, m) |
| 64 | 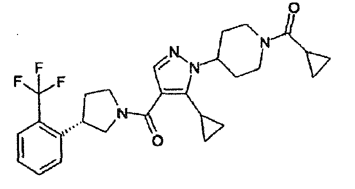 | ¹H-NMR (400MHz, CDCl₃) δ: 0.64-0.87 (4H, m), 0.91-1.18 (4H, m), 1.70-1.86 (2H, m), 1.88-2.48 (6H, m), 2.65-2.89 (1H, m), 3.13-3.39 (1H, m), 3.41-4.13 (5H, m), 4.27-4.89 (3H, m), 7.31-7.76 (5H, m) |
| 65 | 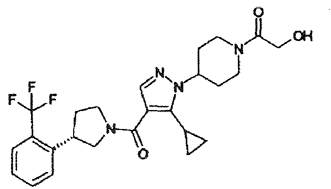 | ¹H-NMR (400MHz, CDCl₃) δ: 0.68-0.87 (2H, m), 1.01-1.16 (2H, m), 1.69-1.86 (1H m), 1.90-2.49 (6H, m), 2.85-3.02 (1H, m), 3.07-3.26 (1H, m). 3.40-4.12 (7H, m), 4.16-4.29 (2H, m), 4.54-4.82 (2H, m), 7.31-7.77 (5H, m) |
| 66 | 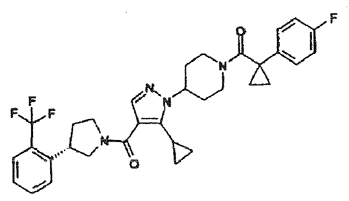 | ¹H-NMR (400MHz, CDCl₃) δ: 0.63-0.83 (2H, m), 0.98-1.10 (2H, m), 1.12-1.23 (2H, m), 1.38-1.51 (2H, m), 1.66-2.22 (6H, m), 2.27-2.45 (1H, m), 2.73-3.03 (2H, m), 3.37-4.11 (5H, m), 4.15-4.95 (3H, m), 6.94-7.73 (9H, m) |

**[Table 1-11]**

| Example No. | Structural formula | NMR |
|---|---|---|
| 67 | 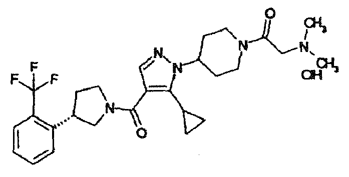 | ¹H-NMR (400MHz. DMSO-D₆) δ : 0.60-0.74 (2H, m), 0.92-1.06 (2H, m), 1.15-1.20 (1H, m), 1.75-2.36 (4H, m), 2.78-2.87 (6H, brs), 3.14-3.36 (1H, m), 3.40-3.92 (9H, m), 4.22-4.56 (3H, m), 4..86 (1H, m), 7.40-7.62 (2H, m), 7.64-7.77 (3H, m), 9.61-9.74 (1H, brs) |
| 68 | 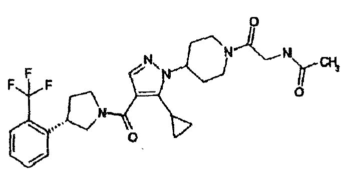 | ¹H-NMR (400MHz. CDCl₃) δ: 0.72-0.84 (2H, m), 1.04-1.16 (2H, m), 1.72-1.86 (1H, m), 1.90-2.28 (8H, m), 2.28-2.45 (1H, m), 2.81-2.96 (1H, m), 3.15-3.29 (1H, m), 3.42-3.51 (0.5H, m), 3.58-4.21 (7.5H, m), 4.58-4.79 (2H, m), 6.58-6.67 (1H, br s), 7.33-7.42 (1H, m), 7.42-7.62 (3H, m), 7.63-7.72 (1H, m) |
| 69 | 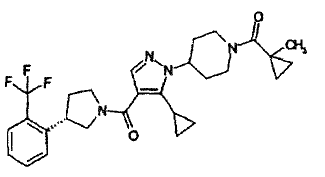 | ¹H-NMR (400MHz, CDCl₃) δ: 0.54-0.67 (2H, m), 0.71-0.86 (2H, m), 0.92-1.02 (2H, m), 1.04-1.14 (2H, m), 1.28-1.40 (3H, m), 1.72-1.86 (1H, m), 1.89-2.24 (5H, m), 2.27-2.46 (1H, m), 2.86-3.13 (2H, m), 3.41-4.12 (5H, m), 4.51-4.71 (3H, m), 7.32-7.75 (5H, m) |
| 70 | 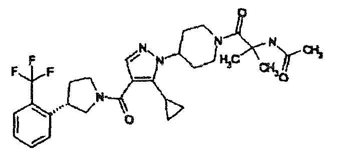 | ¹H-NMR (400MHz, CDCl₃) δ: 0.69-0.82 (2H, m), 1.01-1.12 (2H, m), 1.60-1.71 (7H, m), 1.71-2.45 (10H, m), 2.93-3.12 (1H, m), 3.37-4.10 (6H, m), 4.47-4.70 (2H, m), 6.70-6.79 (1 H, brs), 7.28-7.71 (5H, m) |
| 71 | 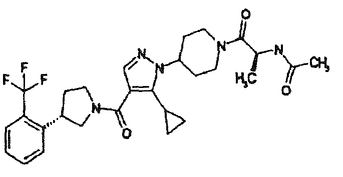 | ¹H-NMR (400MHz, CDCl₃) δ: 0.70-0.84 (2H, m), 1.02-1.13 (2H, m), 1.29-1.41 (4H, m), 1.71-1.84 (1H, m), 1.89-2.45 (7H, m), 2.76-2, (2H, m), 3.16-3.34 (2H, m), 3.39-4.15 (5H, m), 4.56-4.79 (2H, m), 4.85-4.99 (1H, m), 6.55-6.67 (1H, m). 7.31-7.70 (5H, m) |
| 72 | 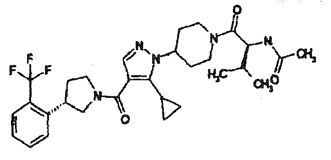 | ¹H-NMR (400MHz, CDCl₃) δ: 0.69-0.83 (2H, m), 0.84-1.03 (6H, m), 1.03-1.14 (2H, m), 1,22-1.34 (2H, m), 1.71-2.44 (9H, m), 2.76-2.92 (1H, m), 3.17-4.79 (9H, m), 4.84-4.95 (1H, m), 6.19-6.30 (1H, m), 7.31-7.71 (5H, m) |
| 73 | 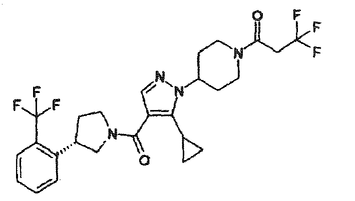 | ¹H-NMR (400MHz, CDCl₃) δ: 0.66-0.86 (2H, m), 0.99-1.18 (2H, m), 1.69-1.85 (1H, m), 1.89-2.45 (6H, m), 2.78-2.94 (1H, m), 3.19-3.39 (3H, m), 3.41-3.50 (0.6H, m), 3.56-4.10 (5.4H, m), 4.56-4.83 (2H, m), 7.32-7. (1H, m), 7.41-7.61 (3H, m), 7.62-7.71 (1H, m) |

**[Table 1-12]**

| Example No. | Structural formula | NMR |
|---|---|---|
| 74 | 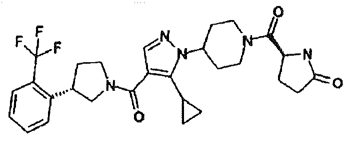 | ¹H-NMR (400MHz, CDCl₃) δ: 0.67-0.85 (2H, m), 1.03-1.15 (2H, m), 1.69-1.88 (1H, m), 1.91-2.61 (9H, m), 2.76-2.95 (1H, m), 3.18-4.11 (8H, m), 4.44-4.83 (3H, m), 5.76-5.93 (1 H, m), 7.32-7.75 (5H, m) |
| 75 | 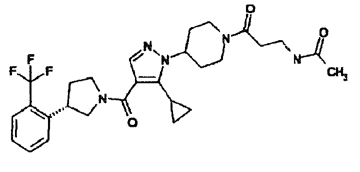 | ¹H-NMR (400MHz, CDCl₃) δ: 0.69-0.82 (2H, m), 1.02-1.13 (2H, m), 1.89-1.84 (1H, m), 1.85-2.01 (6H, m), 2.02-2.23 (2H, m), 2.26-2.46 (1H, m), 2.47-2.60 (2H, m), 2.71-2.84 (1H, m), 3.11-3.26 (1H, m), 3.41-4.10 (8H, m), 4.54-4.68 (1H, m), 4.69-4.82 (1 H, m), 6.32-6.40 (1H, brs), 7.31-7.70 (5H, m) |
| 76 | 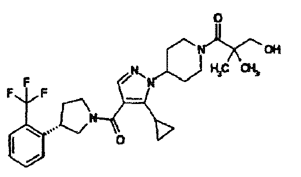 | ¹H-NMR (400MHz, CDCl₃) δ: 0.68-0.87 (2H, m), 1.02-1.18 (2H, m), 1.31 (3H, s), 1.33 (3H, s), 1.72-1.88 (1H, m), 1.90-2.48 (6H, m), 2.88-3.10 (2H, m), 3.42-4.15 (8H, m), 4.47-4.75 (3H, m), 7.32-7.74 (5H, m) |
| 77 | 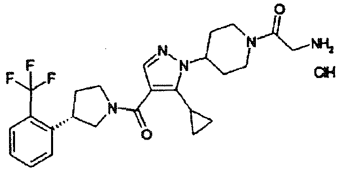 | ¹H-NMR (400MHz. DMSO-D₆) δ: 0.59-0.74 (2H, m), 0.93-1.05 (2H, m), 1.21-1.32 (1H, m), 1.72-2.34 (5H, m), 2.79-2.98 (1H, m), 3.13-3.34 (1H, m), 3.39-4.36 (9H, m), 4.38-4.55 (1H, m), 4.63-4.84 (1H, m), 7.40-7.60 (2H, m), 7.63-7.74 (3H, m), 8.80-8.18 (3H, brs) |
| 78 | 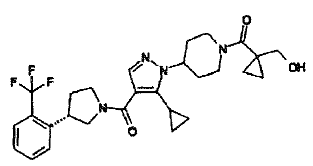 | ¹H-NMR (400MHz, CDCl₃) δ: 0.70-0.89 (4H, m), 0.97-1.16 (4H, m), 1.71-1.86 (1H, m), 1.88-2.24 (6H, m), 2.27-2.46 (1H, m), 2.90-3.11 (2H, m), 3.41-4.11 (7H, m), 4.54-4.74 (3H, m), 7.33-7.74 (5H, m) |
| 79 | 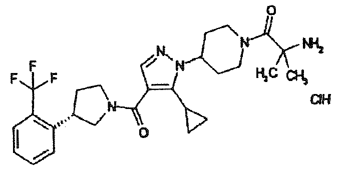 | ¹H-NMR (400MHz, DMSO-D₆) δ: 0.60-0.74 (2H, m), 0.94-1.03 (2H, m), 1.50-1.65 (7H, m), 1.74-2.38 (5H, m), 2.3.28 (2H, m), 3.37-3.90 (5H, m), 3.94-4.65 (3H, m), 4.67-4.89 (1H, m), 7.40-7.78 (5H, m), 8.12-8.32 (3H, |
| 80 | 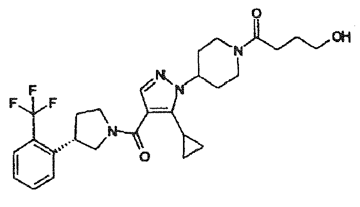 | ¹H-NMR (400MHz, CDCl₃) δ: 0.68-0.83 (2H, m), 1.02-1.14 (2H, m), 1.70-1.85 (1H, m), 1.85-2.46 H, m), 2.49-2. (2H, m), 2.69-2.85 H, m), 3.13-3. (1H, m), 3.41-3.50 (0.5H, m), 3.55-4.14 m), 4.53-4.70 (1H, m), 4.71-4.85 (1H, m), 7.31-7.40 (1H, m), 7.40-7.61 (3H, m), 7.62-7.73 (1H, m) |

**[Table 1-13]**

| Example No. | Structural formula | NMR |
|---|---|---|
| 81 | 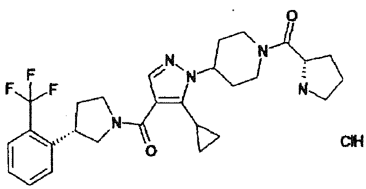 | ¹H-NMR (400MHz, DMSO-D₆) δ : 0.58-0.74 (2H, m), 0.93-1.10 (2H, m), 1.70-2.48 (12H, m), 2.83-3.03 (1H, m), 3.11-4.08 (9H, m), 4.41-4.85 (2H, m), 7.42-7.53 (2H, m), 7.65-7.78 (3H, m), 9.91-10.05 (1H, brs), 10.12-10.24 (1H, brs) |
| 82 | 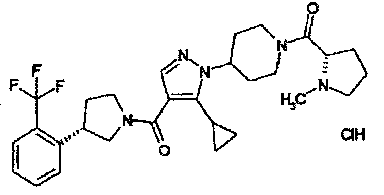 | ¹H-NMR (400MHz, DMSO-D₆) δ : 0.60-0.75 (2H, m), 0.92-1.03 (2H, m), 1.74-2.17 (11H, m), 2.18-2.35 (1H, m), 2.73-2.87 (3H, m), 2.88-3.90 (10H, m), 4.43-4.56 (1H, m), 4.60-4.85 (1H, m), 7.41-7.76 (5H, m), 9.53-9.70 (1H, brs) |
| 83 | 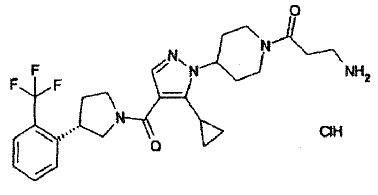 | ¹H-NMR (400MHz, DMSO-D₆) δ : 0.58-0.72 (2H, m), 0.93-1.04 (2H, m), 1.69-2.02 (5H, m), 2.03-2.16 (1H, m), 2.17-2.34 (1H, m), 2.64-2.87 (3H, m), 2.91-3.07 (2H, m), 3.16-3.32 (1H, m), 3.42-3.78 (6H, m). 3.80-3.99 (1H, m), 4.44-4.58 (1H, m), 7.42-7.94 (8H, m) |
| 84 | 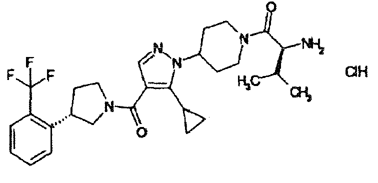 | ¹H-NMR (400MHz, DMSO-D₆) δ : 0.60-0.73 (2H, m), 0.87-1.10 (8H, m), 1.73-2.17 (8H, m), 2.18-2.34 (1H, m), 2.81-2.98 (1H, m), 3.21-3.37 (1H, m), 3.40-3.92 (4H, m). 4.04-4.19 (1H, m), 4.23-4.41 (1H, m), 4.46-4.61 (1H, m), 4.66-4.85 (1H, m), 7.43-7.61 (2H, m), 7.64-7.77 (3H, m), 8.07-8.24 (3H, m) |
| 85 | 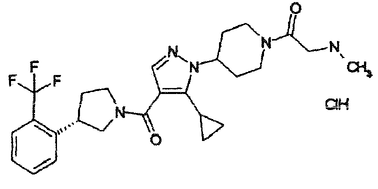 | ¹H-NMR (400MHz, DMSO-D₆) δ : 0.60-0.74 (2H, m), 0.92-1.06 (2H, m), 1.72-2.35 (8H, m), 2.53-2.82 (3H, m), 2.80-2.98 (1H, m), 3.18-3.34 (1H, m), 3.42-3.98 (5H, m), 3.98-4.23 (2H, m), 4.40-4.54 (1H, m), 4.66-4.82 (1H, m), 7.42-7.78 (5H, m), 8.77-9.02 (2H, m) |
| 86 | 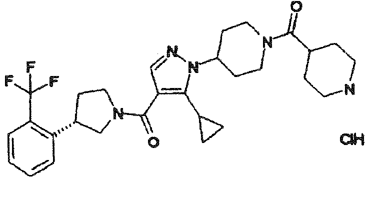 | ¹H-NMR (400MHz, DMSO-D₆) δ : 0.59-0.72 (2H, m), 0.93-1.02 (2H, m), 1.67-1.84 (6H, m), 1.85-2.00 (4H, m), 2.03-2.17 (1H, m), 2.18-2.33 (1H, m), 2.65-2.81 (1H, m), 2.84-3.08 (3H, m), 3.16-3.32 (4H, m), 3.52-3.90 (3H, m), 4.05-4.19 (1H, m), 4.44-4.57 (1H, m), 4.63-4.79 (1H, m), 7.40-7.75 (5H, m), 8.62-8.78 (1H, m), 9.04-9.16 (1H, m) |

**[Table 1-14]**

| Example No. | Structural formula | NMR |
|---|---|---|
| 87 | 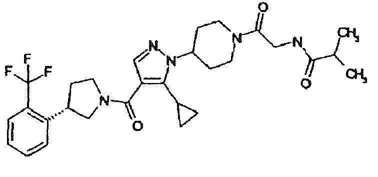 | ¹H-NMR (400MHz, CDCl₃) δ: 0.70-0.84 (2H, m), 1.03-1.12 (2H, m), 1.14-1.22 (7H, m), 1.66-2.24 (6H, m), 2.27-2.54 (2H, m), 2.79-2.96 (1H, m), 3.13-3.27 (1H, m), 3.39-3.50 (1H, m), 3.55-4.18 (6H, m), 4.53-4.79 (2H, m), 6.58-6.61 (1H, m), 7.31-7.71 (5H, m) |
| 88 | 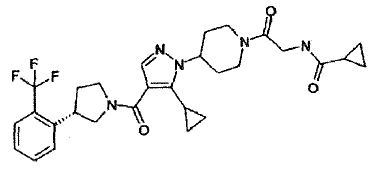 | ¹H-NMR (400MHz, CDCl₃) δ: 0.68-0.83 (4H, m), 0.92-1.01 (2H, m), 1.01-1.13 (2H, m), 1.45-1.54 (1H, m), 1.88-2.44 (8H, m), 2.80-2.95 (1H, m), 3.12-3.29 (1H, m), 3.41-3.50 (1H, m), 3.57-4.22 (6H, m), 4.56-4.79 (2H, m), 6.68-6.76 (1H, m), 7.31-7.70 (5H, m) |
| 89 | 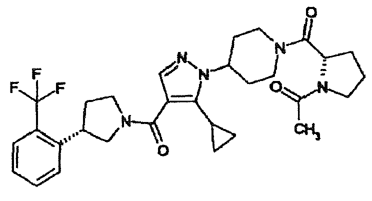 | ¹H-NMR (400MHz, CDCl₃ δ: 0.71-0.82 (2H, m), 1.03-1.14 (2H, m), 1.70-1.84 (1H, m), 1.85-2.25 (12H, m), 225-2.42 (1H, m), 2.75-2.95 (1H, m), 3.18-4.21 (9H, m), 4.54-4.78 (2H, m), 4.87-4.97 (1H, m), 7.32-7.71 (5H, m) |
| 90 | 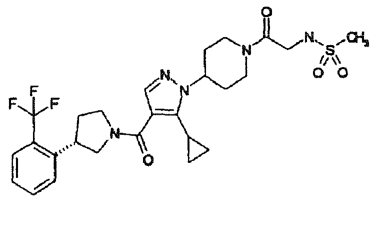 | ¹H-NMR (400MHz, CDCl₃) δ: 0.68-0.85 (2H, m), 1.02-1.16 (2H, m), 1.70-1.86 (1H, m), 1.89-2.45 (6H, m), 2.83-2.96 (1H, m), 2.96-3.03 (3H, m), 3.16-3.31 (1H, m), 3.41-3.50 (0.6H, m), 3.56-4.12 (7.4H, m), 4.57-4.77 (2H, m), 5.35-5.44 (1H, m), 7.32-7.40 (1H, m), 7.41-7.61 (3H, m), 7.62-7.71 (1 H, m) |
| 91 | 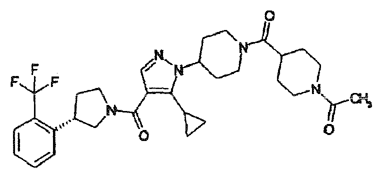 | ¹H-NMR (400MHz, CDCl₃) δ: 0.71-0.81 (2H, m), 1.04-1.12 (2H, m), 1.62-2. (11H, m), 2.09 (3H, brs), 2.66-2.82 (3H, m), 3.05-3.17 (1H, m), 3.18-3.31 (1H, m), 3.39-3.50 (1H, m), 3.55-3.99 (5H, m), 4.00-4.15 (1H, m), 4.51-4.70 (2H, m), 4.71-4.84 (1H, m), 7.31-7.70 (5H, m) |
| 92 | 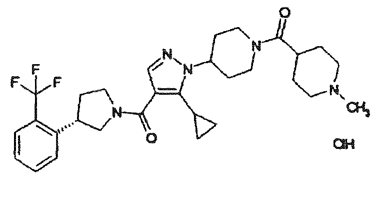 | ¹H-NMR (400MHz, DMSO-D₆) δ : 0.59-0.72 (2H, m), 0.93-1.05 (2H, m), 1.67-2.02 (11H, m), 2.04-2.16 (1H, m), 2.18-2.34 (1H, m), 2.66-2.75 (3H, bars), 2.86-3.01 (2H, m), 3.09-3.32 (1H, m), 3.36-3.91 (6H, m), 3.99-4.16 (1H, m), 4.44-4.58 (2H, m), 4.63-4.80 (1H, m), 7.43-7.76 (5H, m), 10.21-10. (1H, m) |

**[Table 1-15]**

| Example No. | Structural formula | NMR |
|---|---|---|
| 93 | 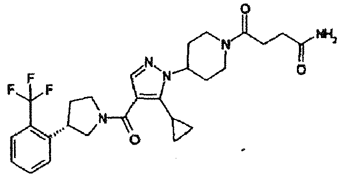 | ¹H-NMR (400MHz, CDCl₃) δ: 0.70-0.84 (2H, m), 1.03-1.12 (2H, m), 1.14-1.22 (7H, m), 1.66-2.24 (6H, m), 2.27-2.54 (2H, m), 2.79-2.96 (1H, m), 3.13-3.27 (1H, m), 3.39-3.50 (1H, m), 3.55-4.18 (6H, m), 4.53-4.79 (2H, m), 6.58-6.61 (1H, m), 7.31-7.71 (5H, m) |
| 94 | 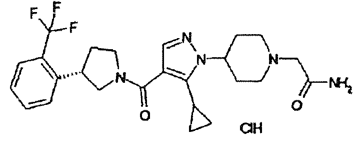 | ¹H-NMR (400MHz, DMSO-D₆) δ : 0.57-0.73 (2H, m), 0.90-1.05 (2H, m), 1.82-2.53 (8H, m), 3.23-3.79 (6H, m), 3.81-4.24 (4H, m), 4.63-4.97 (1 H, m), 7.39-7.77 (6H, m), 7.98-8.17 (1H, m), 9.99-10.27 (1 H, m) |
| 95 | 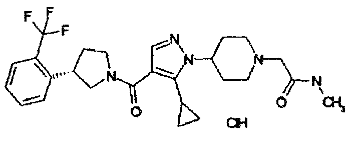 | ¹H-NMR (400MHz, DMSO-D₆) δ : 0.59-0.75 (2H, m), 0.91-1.07 (2H, m), 1.82-2.46 (8H, m), 2.64-2.76 (3H, m), 3.21-3.99 (10H, m). 4.61-4.82 (1H, m), 7.41-7.79 (5H, m), 8.48-8.69 (1H, m), 9.99-10.24 (1H, m) |
| 96 | 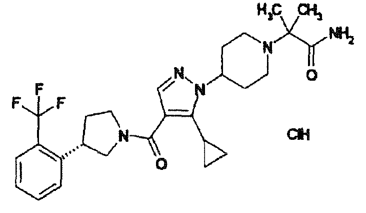 | ¹H-NMR (400MHz. CDCl₃) δ: 0.61-0.82 (2H, m), 1.02-1.18 (2H, m), 1.69 (6H, s), 1.70-1.84 (1H, m), 2.05-2.23 (3H, m), 2.29-2.46 (1H, m), 3.03-4.07 (11H, m), 5.02 (1H, brs), 5.74 (1H, brs), 7.34-7.41 (1H, m), 7.44-7.50 (1H, m), 7.51-7.60 (2H, m), 7.65-7.70 (1H, m), 9.13 (1H, brs). 11.80 (1H, brs) |
| 97 | 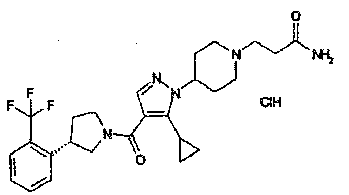 | ¹H-NMR (400MHz, DMSO-D₆) δ : 0.58-0.77 (2H, m), 0.90-1.06 (2H, m), 1.83-1.98 (1H, m), 2.04-2.74 (6H, m), 3.07-3.96 (13H, m), 4.64-4.97 (1H, m), 7.20-7.85 (7H, m), 10.09-10.49 (1H, m) |
| 98 | 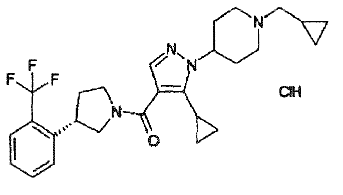 | ¹H-NMR (400MHz, DMSO-D₆) δ : 0.30-0.49 (2H, m), 0.56-0.76 (4H, m), 0.88-1.05 (2H, m), 1.07-1.22 (1H, m), 1.81-1.98 (1H, m), 1.99-2.38 (6H, m), 2.88-3.04 (2H, m), 3.09-3.26 (2H, m), 3.41-3.95 (7H, m), 4.58-4.86 (1H, m), 7.34-7.85 (5H, m), 10.17-10.53 (1H, m) |
| 99 | 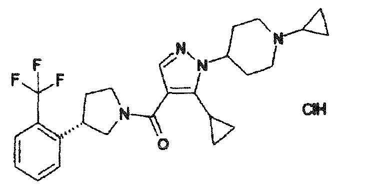 | ¹H-NMR (400MHz, DMSO-D₆) δ : 0.60-0.74 (2H, m), 0.75-0.85 (2H, m), 0.93-1.04 (2H, m), 1.12-1.23 (2H, m), 1.86-2.54 (7H, m), 2.71-2.82 (1H, m). 3.28-3.92 (9H, m), 4.66-4.83 (1H, m), 7.42-7.77 (5H, m), 10.60-10.79 (1H, m) |

**[Table 1-16]**

| Example No. | Structural formula | NMR |
|---|---|---|
| 100 | 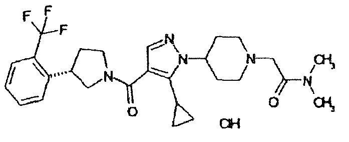 | ¹H-NMR (400MHz, DMSO-D₆) δ : 0.57-0.75 (2H, m), 0.91-1.05 (2H, m), 1.82-2.53 (7H, m), 2.85-3.01 (6H, m), 3.18-4.44 (11H, m), 4.62-4.83 (1H, m), 7.41-7.78 (5H, m), 9.66-9.97 (1H, br) |
| 101 | 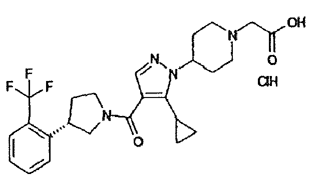 | ¹H-NMR (400MHz, DMSO-D₆) δ : 0.59-0.75 (2H, m), 0.91-1.06 (2H, m), 1.84-2.00 (1H, m), 2.03-2.45 (6H, m), 3.15-3.95 (9H, m), 4.06-4.23 (2H, m), 4.63-4.83 (1H, m), 7.41-7.80 (5H, m) |
| 102 | 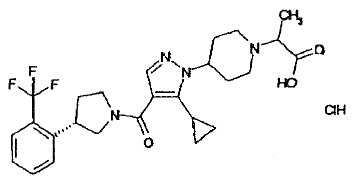 | ¹H-NMR (400MHz, DMSO-D₆) δ : 0.59-0.75 (2H, m), 0.91-1.06 (2H, m), 1.46-1.62 (4H, m), 1.84-2.60 (7H, m), 3.23-3.94 (7H, m), 4.16-4.32 (1H, m), 4.51-5.06 (2H, m), 7.40-7.79 (6H, m), 10.11-10.42 (1H, brs) |
| 103 | 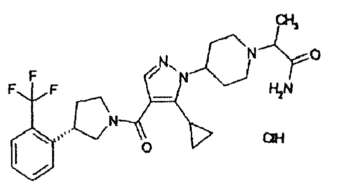 | ¹H-NMR (400MHz, DMSO-D₆) δ : 0.60-0.73 (2H, m), 0.92-1.04 (2H, m), 1.43-1.54 (3H, m), 2.00-2.58 (8H, m), 3.14-4.17 (9H, m), 4.68-4.85 (1H, m), 7.42-7.79 (6H, m), 8.08-8.20 (1H, m), 10.09 (1H, brs) |
| 104 | 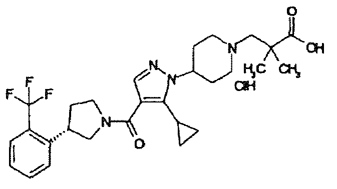 | ¹H-NMR (400MHz, DMSO-D₆) δ : 0.61-0.75 (2H, m), 0.84-1.06 (2H, m), 1.22-1.38 (6H, m), 1.82-2.62 (7H, m), 3.13-3.96 (11H, m), 4.694.95 (1H, m), 7.38-7.82 (6H, m), 9.23-9.88 (1H, m) |
| 105 | 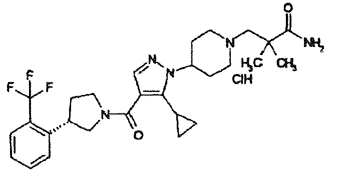 | ¹H-NMR (400MHz, DMSO-D₆) δ : 0.59-0.75 (2H, m), 0.93-1.06 (2H, m), 1.22-1.38 (6H, m), 1.83-1.98 (1H, m), 1.99-2.62 (6H, m), 3.14-3.94 (11H, m), 4.69-4.93 (1H, m), 7.27-7.80 (7H, m), 9.05-9.52 (1H, m) |
| 106 | 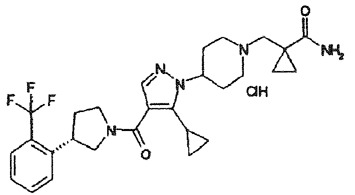 | ¹H-NMR (400MHz, DMSO-D₆) δ : 0.56-0.77 (2H, m), 0.91-1.07 (2H, m), 1.15-1.39 (4H, m), 1.82-2.62 (7H, m), 3.13-4.03 (11H, m), 4.63-4.97 (1H, m), 7.06-7.32 (2H, m), 7.40-7.81 (5H, m), 9.76-10. (1H, m) |

**[Table 1-17]**

| Example No. | I Structural formula | NMR |
|---|---|---|
| 107 | 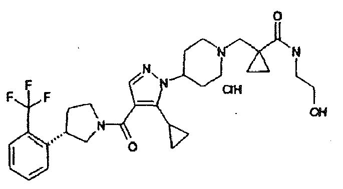 | ¹H-NMR (400MHz, DMSO-D₆) δ : 0.61-0.74 (2H, m), 0.93-1.04 (2H, m), 1.13-1.21 (2H, m), 1.25-1.34 (2H, m), 1.85-1.98 (1H, m), 2.01-2.54 (6H, m), 3.08-3.92 (16H, m), 4.64-4.84 (1H, m), 7.43-7.78 (6H, m), 9.57-9.92 (1H, m) |
| 108 | 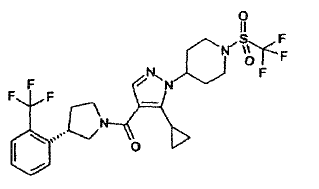 | ¹H-NMR (400MHz, CDCl₃) δ: 0.67-0.82 (2H, m), 1.01-1.14 (2H, m), 1.66-1.83 (1H, m), 1.94-2.44 (6H, m), 3.18-3.49 (2H, m), 3.54-4.20 (7H, m), 4.53-4.70 (1H, m), 7.28-7.72 (5H, m) |
| 109 | 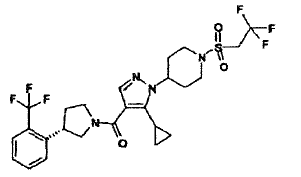 | ¹H-NMR (400MHz, CDCl₃) δ: 0.65-0.83 (2H, m), 0.99-1.11 (2H, m), 1.68-1.83 (1H, m), 1.91-2.45 (6H, m). 3.04-3.20 (2H, m), 3.38-4.11 (9H, m), 4.47-4.66 (1H, m), 7.72-8.35 (5H, m) |
| 110 | 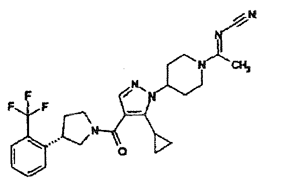 | ¹H-NMR (400MHz, CDCl₃) δ: 0.64-0.88 (2H, m), 0.98-1.21 (2H, m), 1.66-1.88 (1H, m), 1.89-2.54 (9H, m), 2.94-3.17 (1H, m), 3.26-3.53 (1.5H, m), 3.54-4.21 (5.5H, m), 4.58-4.93 (2H, m), 7.29-7.75 (5H, m) |
| 111 | 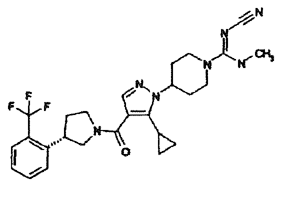 | ¹H-NMR (400 CDCl₃) δ : 0.62-0.83 (2H, m), 0.97-1.17 (2H, m), 1.63-1.95 (3H, m), 1.96-2.24 (3H, m), 2.25-2.47 (1H, m), 2.97-3.19 (5H, m), 3.40-3.49 (0.5H, m), 3.55-4.26 (6.5H, m), 4.46-4.69 (1H, m), 5.54-5.76 (1H, m), 7.30-7.60 (4H, m), 7.61-7.73 (1H, m) |
| 112 | 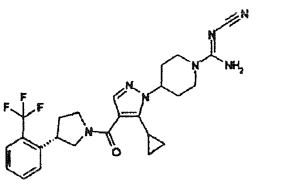 | ¹H-NMR (400MHz, CDCl₃) δ: 0.60-0.81 (2H, m), 0.98-1.16 (2H, m), 1.62-1.82 (3H, m), 1.93-2.25 (3H, m), 2.26-2.47 (1H, m), 2.90-3.08 (2H, m), 3.35-3.50 (0.5H, m), 3.53-4.09 (4.5H, m), 4.17-4.34 (2H, m), 4.45-4.65 (1H, m), 5.94-6.14 (2H, m), 7.29-7.61 (4H, m), 7.62-7.74 (1H, m) |
| 113 | 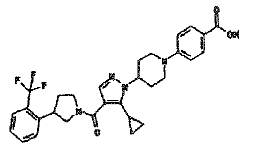 | ¹H-NMR (400MHz, CDCl₃) δ: 0.71-0.87 (2H, m), 1.03-1.18 (2H, m), 1.71-2.47 (7H, m), 3.00-3.18 (2H, m), 3.42-4.17 (7H, m), 4.56-4.75 (1H, m), 6.85-6.97 (2H, m), 7.32-7.71 (5H, m), 7.92-8.00 (2H, m) |

**[Table 1-18]**

| Example No. | Structural formula | NMR |
|---|---|---|
| 114 | 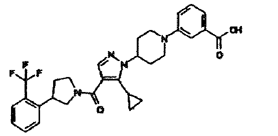 | ¹H-NMR (400MHz, CDCl₃) δ: 0.70-0.87 (2H, m), 1.02-1.19 (2H, m), 1.74-2.50 (7H, m), 2.81-3.19 (2H, m), 3.44-4.14 (7H, m), 4.48-4.80 (1H, m), 7.07-7.85 (9H, m) |
| 115 | 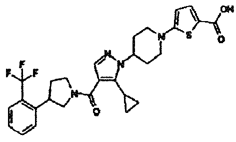 | ¹H-NMR (400MHz, DMSO-D₆) δ : 0.59-0.77 (2H, m), 0.92-1.48 (2H, m), 1.87-2.36 (7H, m), 3.08-3.94 (9H, m), 4.60-4.81 (1H, m), 6.16-6.29 (1 H, m), 7.38-7.80 (6H, m), 12.25 (1H, bs) |
| 116 | 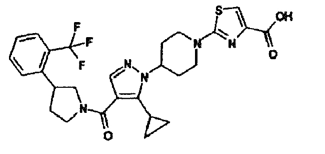 | ¹H-NMR (300MHz, DMSO-D₆) δ : 0.62-0.73 (2H, m), 0.98-1.04 (2H, m), 1.87-2.33 (7H, m), 3.18-3.32 (3H, m), 3.44-3.91 (4H, m), 3.97-4.10 (2H, m), 4.66-4.80 (1H, m), 7.41-7.60 (2H, m), 7.63-7.78 (4H, m), 12.6 (1 H, brs) |
| 117 | 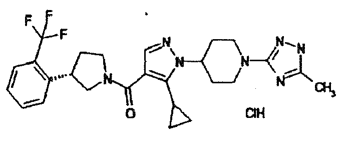 | ¹H-NMR (400MHz, DMSO-D₆) δ : 0.59-0.77 (2H, m), 0.93-1.08 (2H, m), 1.88-2.39 (10H, m), 3.21-4.12 (11H, m), 4.64-4.84 (1H, m), 7.41-7.80 (5H, m) |
| 118 | 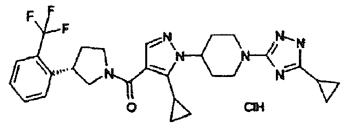 | ¹H-NMR (400MHz. DMSO-D₆) δ : 0.60-0.75 (2H, m), 0.89-1.09 (6H, m), 1.86-2.36 (8H, m), 3.11-4.11 (11H, m), 4.61-4.82 (1H, m), 7.41-7.80 (5H, m) |
| 119 | 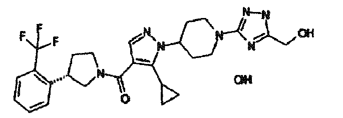 | ¹H-NMR (400MHz, DMSO-D₆) δ : 0.60-0.76 (2H, m), 0.92-1.06 (2H, m), 1.88-2.38 (8H, m), 3.15-4.15 (10H, m), 4.42-4.53 (2H, m), 4.63-4.82 (1H, m), 7.39-7.79 (6H, m) |
| 120 | 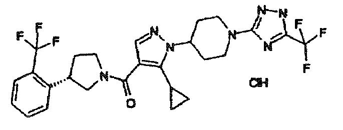 | ¹H-NMR (400MHz, DMSO-D₆) δ : 0.58-0.77 (2H, m), 0.92-1.06 (2H, m), 1.85-2.41 (7H, m), 3.11-4.10 (9H, m), 4.31-5.07 (2H, m), 7.41-7.79 (5H, m), 13.3-13.5 (1H, m) |
| 121 | 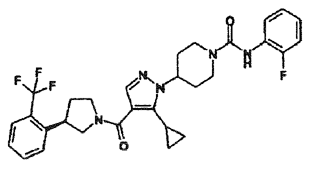 | ¹H-NMR (400MHz, CDCl₃) δ : 0.72-0.87 (2H, m), 1.05-1.15 (2H, m), 1.49-2.48 (7H, m), 3.05-3.22 (2H, m), 3.40-4.14 (5H, m), 4.20-4.36 (2H, m), 4.52-4.73 (1H, m), 6.60-6.71 (1H, m), 6.94-7.16 (3H, m), 7.33-7.72 (5H, m), 8.14 1H, m) |
| 122 | 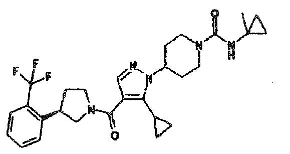 | ¹H-NMR (400MHz, CDCl₃) δ : 0.57-0.66 (2H, m), 0.66-0.84 (4H, m), 1.01-1.13 (2H, m), 1.32-1.45 (3H, m), 1.55-2.48 (7H, m), 2.81-3.00 (2H, m), 3.41-4.15 (5H, m), 4.44-4.63 (2H, m), 4.85-4.96 (1H, m), 7.33-7.71 (6H, m) |

**[Table 1-19]**

| Example No. | Structural formula | NMR |
|---|---|---|
| 123 | 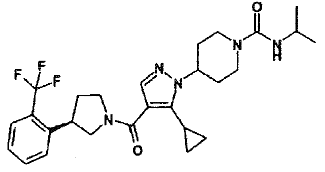 | ¹H-NMR (400MHz, CDCl₃) δ: 0.71-0.82 (2H, m), 1.02-1.13 (2H, m), 1.13-1.22 (6H, m), 1.55-2.47 (8H, m), 2.84-3.01 (2H, m), 3.41-4.34 (9H, m), 4.45-4.63 (1H, m), 7.30-7.71 (6H, m) |
| 124 | 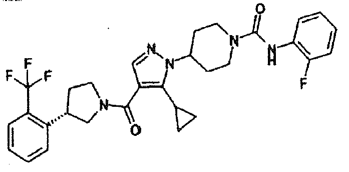 | ¹H-NMR (400MHz, CDCl₃) δ: 0.72-0.87 (2H, m), 1.04-1.17 (2H, m), 1.53-2.46 (7H, m), 3.05-3.21 (2H, m), 3.42-4.11 (5H, m), 4.20-4.35 (2H, m), 4.55-4.71 (1H, m), 6.62-6.70 (1H, m), 6.93-7.16 (3H, m), 7.33-7.72 (5H, m), 8.04-8.14 (1H, m) |
| 125 | 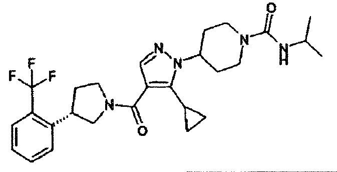 | ¹H-NMR (400MHz, CDCl₃) δ: 0.70-0.84 (2H, m), 1.03-1.13 (2H, m), 1.13-1.21 (6H, m), 1.55-2.47 (8H, m), 2.84-3.01 (2H, m), 3.41-4.31 (9H, m), 4.45-4.63 (1H, m), 7.33-7.72 (6H, m) |
| 126 | 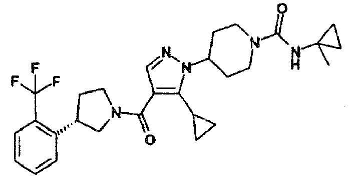 | ¹H-NMR (400MHz, CDCl₃) δ: 0.58-0.66 (2H, m), 0.66-0.83 (4H, m), 1.02-1.11 (2H, m), 1.36-1.43 (3H, m), 1.53-2.46 (7H, m), 2.83-2.99 (2H, m), 3.41-4.15 (5H, m), 4.45-4.61 (2H, m). 4.85-4.92 (1H, m), 7.32-7.72 (6H, m) |
| 127 | 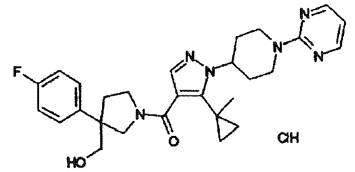 | ¹H-NMR (400MHz, DMSO-D₆) δ : 0.56-0.86 (4H, m), 1.32-1.43 (3H, m), 1.85-2.12 (6H. m), 2.28-2.39 (1H, m), 3.09-3.26 (2H, m), 3.31-4.94 (6H, m), 4.74-4.91 (3H, m), 6.30 (1H, bs), 6.69-6.75 (1H, m), 7.07-7.37 (4H, m), 7.53-7.62 (1H, m), 8.40-8.50 (2H, m) |
| 128 | 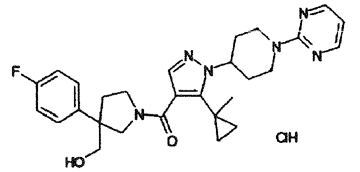 | ¹H-NMR (400MHz, DMSO-D₆) δ : 0.56-8.88 (4H, m), 1.32-1.48 (3H, m), 1.85-2.12 (6H, m), 2.28-2.39 (1H, m), 3.09-3.24 (2H, m), 3.30-4.95 (6H, m), 4.73-4.93 (3H, m), 5.81 (1H, bs), 6.64-6.73 (1 H, m), 7.06-7.40 (4H, m), 7.50-7.67 (1H, m), 8.39-8.49 (2H, m) |
| 129 | 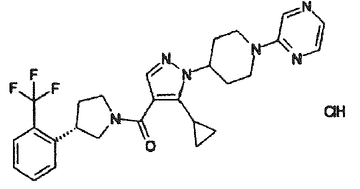 | ¹H-MMR (400MHz, DMSO-D₆) δ : 0.66-0.69 (2H, m), 0.99-1.01 (2H, m), 1.91-2.33 (7H, m), 3.11-3.14 (2H, m), 3.46-3.89 (5H, m), 4.49-4.52 (2H, m), 4.75-4.77 (1H, m), 7.48-7.74 (6H, m), 7.85-7.90 (1H, m), 8.17 (1H, brs). 8.43-8.45 (1 H, m) |

**[Table 1-20]**

| Example No. | Structural formula | NMR |
|---|---|---|
| 130 | 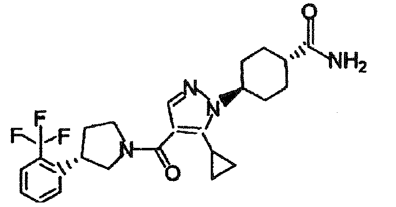 | ¹H-NMR (400MHz, CDCl₃) δ : 0.65-0.79 (2H, m), 0.94-1.10 (2H, m), 1.63-2.44 (12H, m), 3.38-4.10 (5H, m), 4.32-4.51 (1H, m), 5.33 (1H, br), 5.51 (1H, br), 7.27-7.70 (5H, m) |
| 131 | 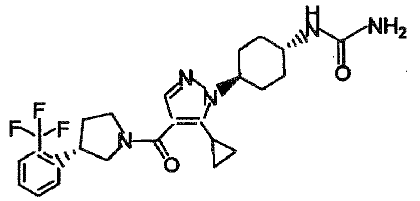 | ¹H-NMR (400MHz, CDCl₃) δ: 0.62-0.82 (2H, m), 0.95-1.10 (2H, m), 1.23-1.39 (2H, m), 1.65-2.45 (8H, m), 3.40-4.10 (6H, m), 4.26-4.60 (4H, m), 7.27-7.71 (6H, m) |
| 132 | 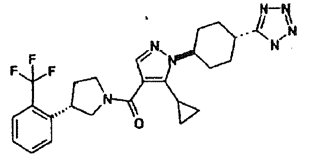 | ¹H-NMR (400MHz, CDCl₃) δ: 0.67-0.79 (2H, m), 0.84-0.92 (2H, m), 1.08-1.15 (2H, m), 1.22-1.35 (3H, m), 1.73-2.49 (8H, m), 3.05-3.19 (1H, m), 3.39-3.55 (1H, m), 3.61-4.17 (3H, m), 4.46-4.61 (1H, m), 7.33-7.71 (5H, m) |
| 133 | 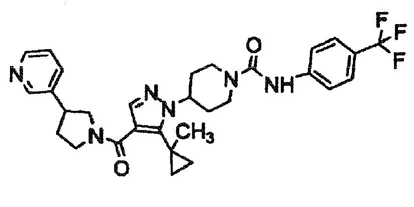 | ¹H-NMR (400MHz, CDCl₃) δ : 0.71-0.95 (4H, m), 1.38-1.47 (3H, m), 1.69-1.88 (2H, m), 1.99-2.29 (3H, m), 2.34-2.50 (1H, m), 2.94-3.12 (2H, m), 3.36-4.18 (5H, m), 4.25-4.40 (2H, m), 4.54-4.72 (1H, m), 6.99-7.33 (2H, m), 7.44-7.65 (6H, m), 8.44-8.61 (2H, m) |
| 134 | 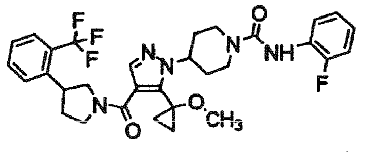 | ¹H-NMR (400MHz, CDCl₃) δ: 0.89-1.05 (2H, m), 1.22-1.34 (2H, m), 1.94-2.44 (6H, m), 3.05-3.21 (2H, m), 3.28-3.39 (3H, m), 3.42-4.11 (5H, m), 4.20-4.35 (2H, m), 4.77-4.93 (1H, m), 6.62-6.71 (1H, m), 6.93-7.18 (3H, m), 7.32-7.72 (5H, m), 8.05-8.14 (1H. m) |
| 135 | 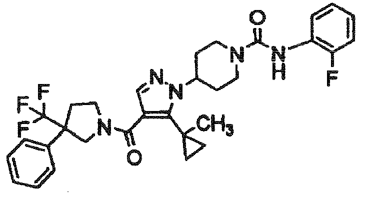 | ¹H-NMR (400MHz, CDCl₃) δ: 0.57-0.95 (4H, m), 1.34-1.54 (3H, m), 1.83-2.08 (2H, m), 2.18-2.41 (2H, m), 2.45-2.61 (1H, m), 2.73-2.92 (1H, m), 3.02-3.24 (2H, m), 3.51-4.86 (7H, m), 6.60-6.78 (1H, m), 6.93-7.18 (3H, m), 7.20-7.46 (5H, m), 7.46-7.57 (1H, m), 8.01-8.15 (1H, m) |

**[Table 1-21]**

| Example No. | Structural formula | NMR |
|---|---|---|
| 136 | 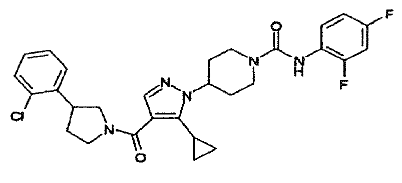 | ¹H-NMR (400MHz, CDCl₃) δ: 0.71-0.81 (2H, m), 1.00-1.14 (2H, m), 1.71-1.85 (1H, m), 1.93-2.43 (6H, m), 3.05-3.19 (2H, m), 3.40-4.10 (5H, m), 4.20-4.33 (2H, m), 4.55-4.69 (1H, m), 6.48-6.57 (1H, m), 6.81-6.92 (2H, m), 7.17-7.45 (4H, m), 7.50-7.60 (1H, m), 7.94-8.03 (1H, m) |
| 137 | 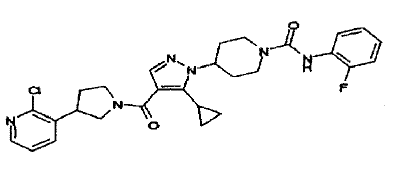 | H-NMR (400MHz, CDCl₃) δ: 0.70-0.83 (2H, m), 1.01-1.14 (2H, m), 1.75-1.86 (1H, m), 1.94-2.36 (6H, m), 2.37-2.49 (1H, m), 3.07-3.20 (2H, m), 3.40-4.14 (4H, m), 4.22 4.34 (2H, m), 4.57-4.71 (1H, m), 6.61-6.70 (1H, m), 6.93-7.03 (1H, m), 7.03-7.17 (3H, m), 7.51-7.70 (2H, m), 8.02-8.15 (1H, m), 8.28-8.37 (1H, brs) |
| 138 | 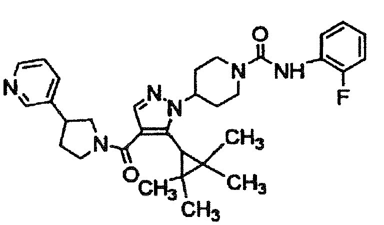 | ¹H-NMR (CDCl₃) δ: 0.94 (6H, s), 1.33 (6H, s), 2.03-2.11 (4H, m), 2.36-2.39 (2H, m), 3.07-3.09 (2H, m), 3.37-3.76 (4H, m), 3.89-3.96 (1H, m), 4.28-.4.32 (4H, m), 6.65 (1H, s), 7.01-7.08 (3H, m), 7.24-7.32 (1H, m), 7.53-7.63 (2H, m), 8.07-8.09 (1H, m), 8.53-8.57 (2H, m) |
| 139 | 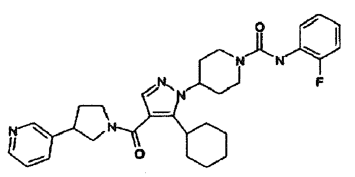 | ¹H-NMR (300MHz, CDCl₃) δ: 1.25-1.48 (3H, m), 1.69-2.48 (13H, m), 2.85-3.19 (3H, m), 3.33-4.41 (8H, m), 6.59-6.69 (1H, m), 6.89-7.14 (3H, m), 7.23-7.36 (1H, m), 7.43-7.68 (2H, m), 8.05 (1H, t, J = 8.1 Hz), 8.44-8.59 (2H, m) |
| 140 | 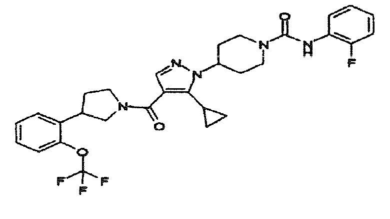 | ¹H-NMR (400MHz, CDCl₃) δ: 0.67-0.83 (2H, m), 1.01-1.15 (2H, m), 1.72-1.86 (1H, m), 1.92-2.43 (6H, m), 3.03-3.20 (2H, m), 3.36-4.11 (5H, m), 4.20-4.34 (2H, m), 4.55-4.71 (1H, m), 6.66 (1H, br s), 6.93-7.16 (3H, m), 7.20-7.42 (4H, m), 7.48-7.63 (1H, m), 8.03-8.14 (1H, m) |
| 141 | 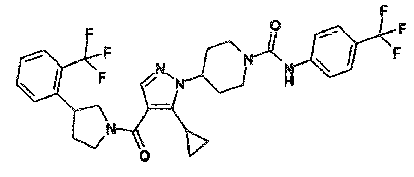 | ¹H-NMR (400Hz, CDCl₃) δ: 0.66-0.80 (2H, m), 1.00-1.12 (2H, m), 1.58-1.82 (3H, m), 1.97-2.23 (3H, m), 2.27-2.47 (1H, m), 2.90-3.06 (2H, m), 3.40-4.11 (5H, m), 4.24-4.41 (2H, m), 4..62 (1H, m), 7.32-7.73 (10H, m) |

**[Table 1-22]**

| Example No. | Structural formula | NMR |
|---|---|---|
| 142 | 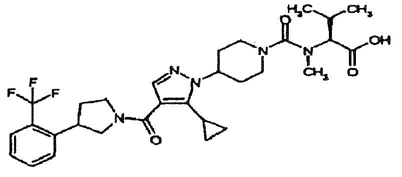 | ¹H-NMR (400MHz, DMSO-D₆) δ: 0.57-0.74 (2H, m), 0.81-1.03 (8H, m), 1.79-2.36 (8H, m), 2.82-2.90 (8H, m), 3.44-3.92 (9H, m), 4.50-4.70 (1H, s), 7.42-7.53 (2H, m), 7.55-7.61 (1H, m), 7.66-7.78 (2H, m) |
| 143 | 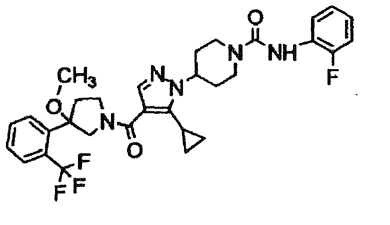 | ¹H-NMR (400MHz, CDCl₃) δ: 0.62-0.84 (2H, m), 0.91-1.17 (2H, m), 1.67-1.88 (1H, m), 1.91-2.08 (2H, m), 2.14-2.42 (2.5H, m), 2.43-2.56 (0.5H, m), 2.57-2.72 (1H, m), 2.88-3.21 (5H, m), 3.45-3.61 (0.6H, m), 3.64-3.91 (2H, m), 3.98-4.13 (1H, m), 4.19-4.36 (2.4H, m), 4.53-4.72 (1H, m), 6.63-6.79 (1H, m), 6.89-7.18 (3H, m), 7.40-7.64 (4H, m), 7.75-7.93 (1H, m), 8.00-8.13 (1H, m) |
| 144 | 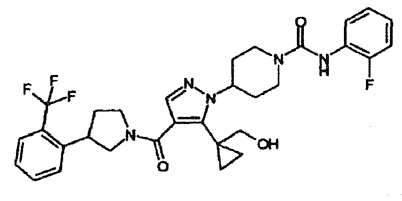 | ¹H-NMR (400MHz, CDCl₃) δ: 0.68-0.97 (2H, m), 0.97-1.35 (2H, m), 1.85-2.54 (6H, m), 2.87-3.21 (2H, m), 3.21-4.14 (7H, m), 4.16-4.46 (2H, m), 4.51-4.79 (1H, m), 5.98-6.35 (1H, m), 6.56-6.78 (1H, m), 6.85-7.20 (3H, m), 7.20-7.79 (5H, m), 7.95-8.16 (1H, m) |
| 145 | 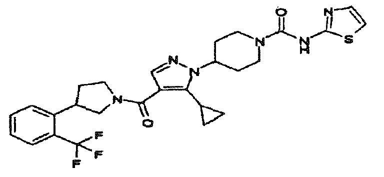 | ¹H-NMR (400MHz, CDCl₃) δ: 0.68-0.84 (2H, m), 1.01-1.16 (2H, m), 1.68-1.85 (1H, m). 1.88-2.46 (6H, m), 3.02-3.19 (2H, m), 3.40-4.11 (5H, m), 4.24-4.41 (2H, m), 4.53-4.71 (1H, m), 6.82-6.91 (1H, m), 7.30-7.72 (7H, m) |
| 146 | 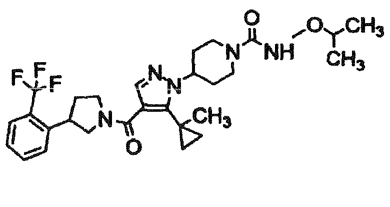 | ¹H-NMR (400MHz, CDCl₃) δ: 0.71-0.99 (4H, m), 1.15-1.31 (6H, m), 1.35-1.52 (3H, m), 1.73-1.98 (2H, m), 1.98-2.47 (4H, m), 2.86-3.08 (2H, m), 3.42-4.11 (6H, m), 4.12-4.31 (2H, m), 4.53-4.72 (1H, m), 7.08-7.21 (1H, m), 7.30-7.40 (1H, m), 7.41-7.61 (3H, m), 7.61-7.74 (1H, m) |
| 147 | 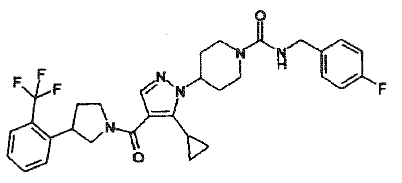 | ¹H-NMR (300MHz, CDCl₃) δ: 0.64-0.81 (2H, m), 0.96-1.13 (2H, m), 1.55-1.93 (3H, m), 1.95-2.44 (4H, m), 2.84-3.02 (2H, m), 3.36-4.22 (7H, m), 4.30-4.41 (2H, m), 4.43-4.62 (1H, m), 4.83-4.99 (1H, m), 6.91-7.05 (2H, m), 7.17-7.71 (7H, m) |

**[Table 1-23]**

| Example No. | Structural formula | NMR |
|---|---|---|
| 148 | 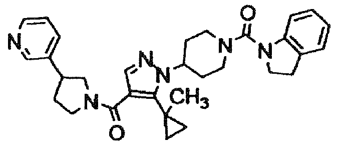 | ¹H-NMR (400MHz, CDCl₃) δ: 0.73-0.84 (2H, m), 0.85-0.96 (2H, m), 1.45 (3H, s), 1.84-1.97 (2H, m), 2.01-2.20 (1H, m), 2.26-2.49 (3H, m), 2.97-3.12 (4H, m), 3.37-4.19 (9H, m), 4.61-4.75 (1H, m), 6.86-6.95 (1H, m), 7.08-7.22 (3H, m), 7.25-7.33 (1H, m), 7.48-7.65 (2H, m), 8.46-8.63 (2H, m) |
| 149 | 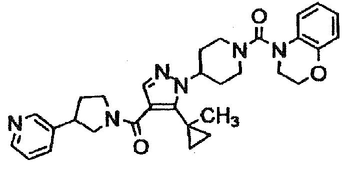 | ¹H-NMR (400MHz, CDCl₃) δ: 0.70-0.82 (2H, m), 0.82-0.96 (2H, m), 1.42 (3H, s), 1.79-1.97 (2H, m), 1.98-2.19 (4H, m), 293-3.08 (2H, m), 3.33-3.82 (5.5H, m), 3.84-4.01 (1H, m), 4.04-4.23 (2.5H, m), 4.26-4.40 (2H, m), 4.55-4.73 (1H, m), 6.81-6.97 (3H, m), 7.11-7.22 (1H, m), 7.23-7.35 (1H, m), 7.46-7.67 (2H, m), 8.44-8.62 (2H, m) |
| 150 | 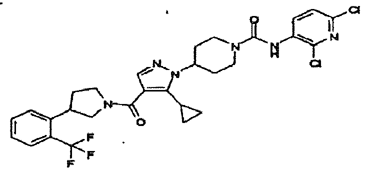 | ¹H-NMR (400MHz, CDCl₃) δ: 0.68-0.85 (2H, m), 1.02-1.18 (2H, m), 1.72-1.87 (1H, m), 1.94-2.46 (6H, m), 3.08-3.26 (2H, m), 3.41-4.11 (5H, m), 4.18-4.33 (2H, m), 4.57-4.75 (1H, m), 6.97-7.07 (1H, m), 7.23-7.61 (5H, m), 7.64-7.72 (1H, m), 8.53-8.60 (1H, m) |
| 151 | 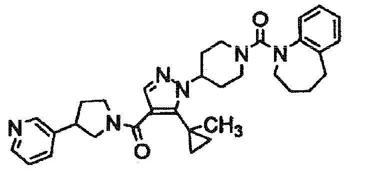 | ¹H-NMR (400MHz, CDCl₃) δ: 0.61-0.89 (4H, m), 1.36 (3H, s), 1.55-1.83 (6H, m), 1.89-2.17 (3H, m), 2.32-2.47 (1H, m), 2.59-2.82 (4H, m), 3.33-4.18 (9H, m), 4.38-4.52 (1H, m), 7.03-7.14 (2H, m), 7.16-7.32 (3H, m), 7.43-7.64 (2H, m), 8.44-8.61 (2H, m) |
| 152 | 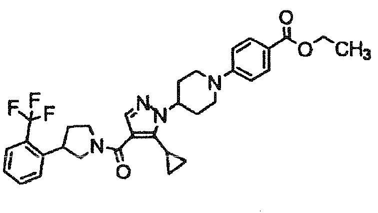 | H-NMR (400MHz, CDCl₃) δ: 0.71-0.85 (2H, m), 1.03-1.16 (2H, m), 1.33-1.42 (3H, m), 1.73-1.88 (1H, m), 1.93-2.22 (3H, m), 2.25-2.45 (3H, m), 2.96-3.13 (2H, m), 3.41-4.11 (7H, m), 4.28.-4.39 (2H, m), 4.52-4.71 (1H, m), 6.85-6.96 (2H, m), 7.32-7.40 (1H, m), 7.41-7.61 (3H, m), 7.63-7.71 (1H, m), 7.89-7.99 (2H, m) |
| 153 | 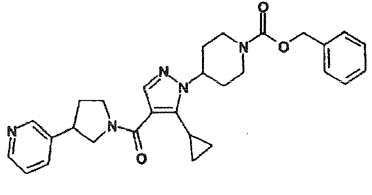 | ¹H-NMR (400MHz, CDCl₃) δ: 0.65-0.79 (2H, m), 1.00-1.09 (2H, m), 1.70-1.97 (3H, m), 2.00-2.26 (3H, m), 2.31-2.47 (1H, m), 2.86-3.05 (2H, m), 3.35-3.53 (1.5H, m), 3.56-3.77 (2H, m), 3.84-3.94 (1H, m), 4.08-4.16 (0.5H, m), 425-4.61 (3H, m), 5.14 (2H, s), 7.23-7.40 (6H, m), 7.49-7.61 (2H, m), 8.47-8.57 (2H, m) |

**[Table 1-24]**

| Example No. | Structural formula | NMR |
|---|---|---|
| 154 | 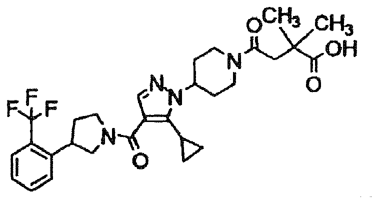 | ¹H-NMR (400MHz, CDCl₃) δ: 0.61-0.86 (2H, m), 0.98-1.18 (2H, m), 1.23-1.41 (6H. m), 1.67-1.86 (1H, m), 1.87-2.47 (6H, m), 2.52-2.88 (3H, m), 3.06-3.32 (1H, m), 3.35-4.15 (6H, m), 4.49-4.85 (2H, m), 7.28-7.73 (5H, m) |
| 155 | 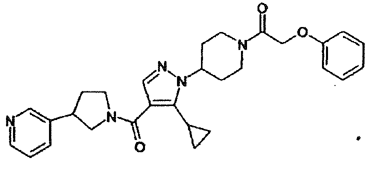 | ¹H-NMR (400MHz, CDCl₃) δ: 0.62-0.83 (2H, m), 0.97-1.14 (2H, m), 1.66-2.50 (8H, m), 2.78-2.95 (1H, m), 3.16-3.34 (1H, m), 3.37-3.54 (1H, m), 3.57-3.79 (2H, m), 3.83-3.97 (1H, m), 4.09-4.29 (1 H, m), 4.54-4.80 (4H, m), 6.89-7.04 (3H, m), 7.21-7.38 (3H, m), 7.48-7.68 (2H, m), 8.42-8.70 (2H, m) |
| 156 | 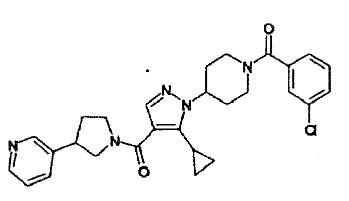 | ¹H-NMR (400MHz, CDCl₃) δ: 0.65-0.82 (2H, m), 1.00-1.13 (2H, m). 1.67-2.30 (9H, m), 2.32-2.51 (1H, m), 2.72-3.34 (2H, m), 3.36-4.03 (4H, m), 4.56-4.99 (1H, m), 7.20-7.46 (5H, m), 7.49-7.65 (2H, m), 8.42-8.62 (2H, m) |
| 157 | 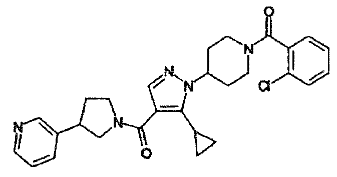 | ¹H-NMR (400MHz, CDCl₃) δ: 0.64-0.85 (2H, m), 0.98-1.13 (2H, m), 1.57-1.92 (3H, m), 1.96-2.50 (5H, m), 2.90-3.54 (3H, m), 3.56-3.81 (3H, m), 3.83-3.98 (1H, m), 4.53-4.73 (1H, m), 4.85-5.04 (1H, m), 7.21-7.47 (5H, m), 7.49-7.65 (2H, m), 8.45-8.62 (2H, m) |
| 158 | 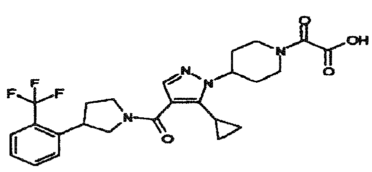 | ¹H-NMR (400MHz, CDCl₃) δ: 0.62-1.19 (4H, m), 1.62-2.53 (7H, m), 2.70-2.92 (1H, m), 3.08-3.29 (1H, m), 3.33-4.21 (6H, m), 4.51-4.75 (2H, m), 7.31-7.62 (4H, m), 7.63-7.73 (1H, m) |
| 159 | 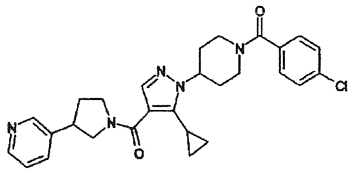 | ¹H-NMR (400MHz, CDCl₃) δ: 0.64-0.82 (2H, m), 0.98-1.12 (2H, m), 1.65-2.32 (9H, m), 2.34-2.50 (1 H, m), 3.08 (2H, brs), 3.34-4.00 (4H, m), 4.56-4.95 (1H, m), 7.23-7.32 (1H, m), 7.35-7.44 (4H, m), 7.48-7.65 (2H, m), 8.45-8.61 (2H, m) |
| 160 | 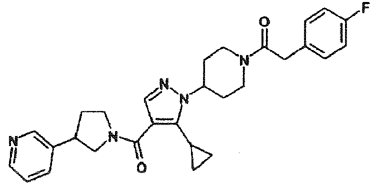 | ¹H-NMR (400MHz, CDCl₃) δ: 0.59-0.83 (2H, m), 0.95-1.14 (2H, m), 1.62-2.20 (6H, m), 2.30-2.50 (1H, m), 2.69-2.91 (1H, m), 3.06-3.27 (1H, m), 3.33-4.20 (6H, m), 3.73 (2H, s), 4.50-4.66 (1H, m), 4.71-4.85 (1H, m). 6.93-7.10 (2H, m), 7.15-7.34 (3H, m). 7.45-7.64 (2H, m), 8.41-8.62 (2H, m) |

**[Table 1-25]**

| Example No. | Structural formula | NMR |
|---|---|---|
| 161 | 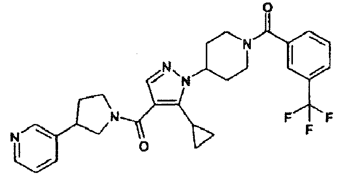 | ¹H-NMR (400MHz, CDCl₃) δ: 0.65-0.81 (2H, m), 0.99-1.13 (2H, m), 1.72-2.50 (9H, m), 2.85-3.33 (2H, m), 3.35-3.78 (3H, m), 3.81-4.96 (3H, m), 7.22-7.33 (1H, m), 7.47-7.77 (6H, m), 8.44-8.63 (2H, m) |
| 162 | 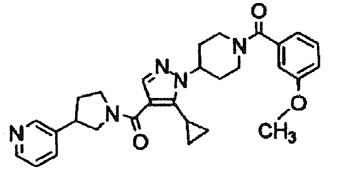 | ¹H-NMR (400MHz, CDCl₃) δ: 0.64-0.82 (2H, m), 1.00-1.12 (2H, m), 1.71-2.32 (8H, m), 2.33-2.51 (1H, m), 2.85-3.28 (2H, m), 3.32-4.05 (5H, m), 3.83 (3H, s), 4.57-4.99 (1H, m), 6.90-7.04 (3H, m), 7.24-7.38 (2H, m), 7.50-7.69 (2H, m), 8.53 (2H, brs) |
| 163 | 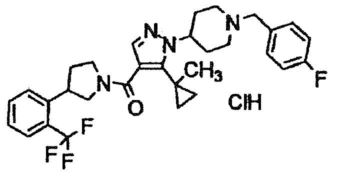 | ¹H-NMR (400MHz, DMSO-D₆) δ: 0.68-0.86 (4H, m), 1.29-1.38 (3H, m), 1.99-2.17 (2H, m), 2.18-2.66 (5H, m), 3.14-3.32 (2H, m), 3.39-4.50 (8H, m), 4.68-4.83 (1H, m), 7.30-7.40 (2H, m), 7.43-7.53 (1H, m), 7.60-7.82 (6H, m), 10.55-10.76 (1H, m) |
| 164 | 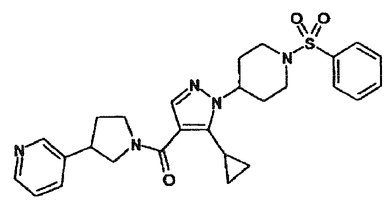 | ¹H-NMR (400MHz, CDCl₃) δ: 0.59-0.74 (2H, m), 0.93-1.04 (2H, m), 1.61-1.73 (1H, m), 1.88-2.16 (3H, m), 2.25-2.59 (5H, m), 3.34-3.76 (3.5H, m), 3.83-4.01 (3H, m), 4.08-4.15 (0.5H, m), 4.27-4.39 (1H, m), 7.23-7.31 (1H, m), 7.49-7.67 (5H, m), 7.79-7.84 (2H, m), 8.46-8.58 (2H, m) |
| 165 | 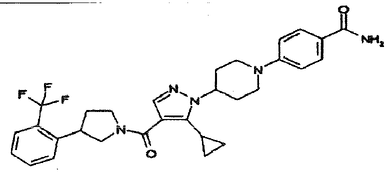 | H-NMR (400MHz, CDCl₃) δ: 0.67-0.86 (2H, m), 1.00-1.19 (2H, m). 1.73-1.88 (1H, m), 1.92-2.21 (3H, m), 2.24-2.47 (3H, m), 2.92-3.12 (2H, m), 3.40-4.12 (7H, m), 4.51-4.69 (1H, m), 5.74 (2H, br s), 6.87-6.99 (2H, m), 7.31-7.40 (1H, m). 7.41-7.61 (3H, m), 7.63-7.78 (3H, m) |
| 166 | 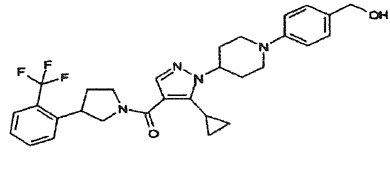 | ¹H-NMR (400MHz, CDCl₃) δ: 0.69-0.85 (2H, m), 1.01-1.16 (2H, m), 1.51-1.67 (1H, m), 1.75-1.89 (1H, m), 1.91-2.23 (3H, m), 2.26-2.49 (3H, m), 2.83-3.00 (2H, m), 3.40-4.12 (7H, m), 4.44-4.68 (3H, m), 6.88-7.04 (2H, m), 7.23-7.31 (2H, m), 7.32-7.40 (1H, m), 7.41-7.62 (3H, m), 7.63-7.72 (1H, m) |

**[Table 1-26]**

| Example No. | Structural formula | NMR |
|---|---|---|
| 167 | 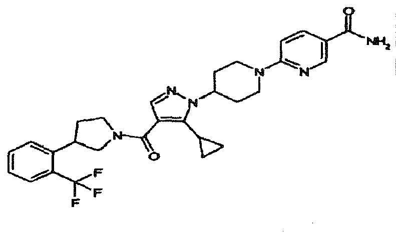 | ¹H-NMR (400MHz, CDCl₃) δ : 0.73-0.86 (2H, m), 1.06-1.12 (2H, m), 1.76-1.88 (1H, m), 1.94-2.08 (2H, m), 2.11-2.44 (5H, m), 3.05-3.19 (2H, m), 3.42-3.51 (0.5H, m), 3.59-4.10 (3.5H, m), 4.56-4.78 (3H, m), 5.40-5.94 (2H, brs), 6.67-6.74 (1H, m), 7.12-7.22 (1H, m), 7.31-7.40 (1H, m), 7.41-7.60 (2H, m), 7.64-7.73 (1H, m), 7.91-7.99 (1H, m), 8.56-8.63 (1H, m) |
| 168 | 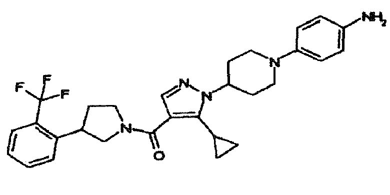 | ¹H-NMR (400MHz, CDCl₃) δ : 0.71-0.85 (2H, m), 1.02-1.14 (2H, m), 1.73-1.88 (1H, m). 1.90-2.22 (3H, m), 2.27-2.51 (3H, m), 2.71-2.88 (2H, m), 3.35-4.11 (9H, m), 4.38-4.56 (1H, m), 6.60-6.71 (2H, m). 6.78-6.91 (2H, m). 7.31-7.40 (1H, m), 7.41-7.62 (3H, m), 7.63-7.71 (1H, m) |
| 169 | 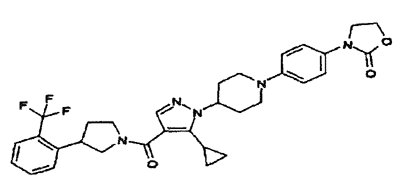 | ¹H-NMR (400MHz, CDCl₃) δ : 0.71-0.88 (2H, m), 1.02-1.16 (2H, m), 1.73-1.88 (1H, m), 1.91-2.19 (3H, m), 2.27-2.50 (3H, m), 2.81-3.00 (2H, m), 3.40-4.17 (9H, m), 4.39-4.63 (3H, m), 6.92-7.04 (2H, m), 7.31-7.72 (7H, m) |
| 170 | 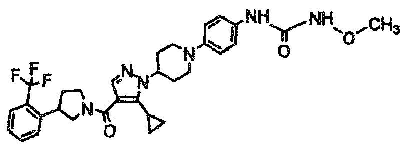 | ¹H-NMR (400MHz, CDCl₃) δ : 0.70-0.86 (2H, m), 1.01-1.16 (2H, m), 1.72-1.88 (1H, m), 1.91-2.23 (3H, m), 2.26-2.51 (3H, m), 2.76-2.98 (2H, m), 3.42-4.11 (10H, m), 4.42-4.62 (1H, m), 6.89-7.00 (2H, m), 7.16 (1H, s), 7.31-7.73 (8H, m) |
| 171 | 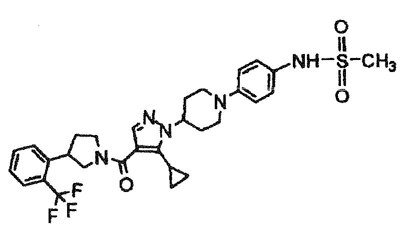 | ¹H-NMR (400MHz, CDCl₃) δ : 0.70-0.86 (2H, m), 1.00-1.16 (2H, m), 1.72-1.88 (1H, m), 1.92-2.23 (3H, m), 2.26-2.48 (3H, m), 2.84-3.02 (5H, m), 3.38-4.12 (7H, m), 4.45-4.65 (1H, m), 6.25-6.38 (1H, m), 6.88-6.99 (2H, m), 7.11-7.22 (2H, m), 7.32-7.40 (1H, m), 7.41-7.62 (3H. m), 7.63-7.71 (1H, m) |

**[Table 1-27]**

| Example No. | Structural formula | NMR |
|---|---|---|
| 172 | 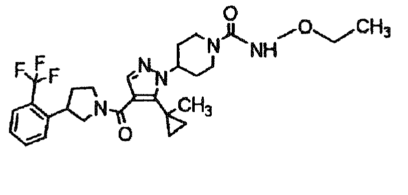 | ¹H-NMR (400MHz, CDCl₃) δ : 0.69-1.01 (4H, m), 1.16-1.35 (3H, m), 1.35-1.53 (3H, m), 1.71-1.97 (2H, m), 1.97-2.49 (4H, m), 2.86-3.09 (2H, m), 3.41-4.31 (9H, m), 4.53-4.75 (1H, m), 7.17-7.30 (1 H, m), 7.30-7.40 (1H, m), 7.41-7.60 (3H, m), 7.61-7.74 (1H, m) |
| 173 | 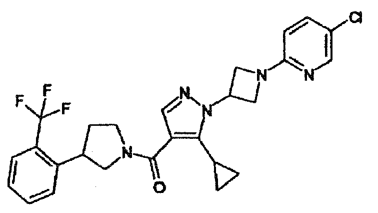 | ¹H-NMR (400MHz, CDCl₃) δ : 0.61-0.87 (2H, m), 1.00-1.19 (2H, m), 1.57-1.87 (1H, m), 1.93-2.25 (1H, m), 2.25-2.48 (1H, m), 3.31-4.15 (5H, m), 4.35-4.59 (4H, m), 5.44-5.66 (1H, m), 6.26-6.40 (1H, m), 7.30-7.76 (6H, m), 8.02-8.17 (1H, m) |
| 174 | 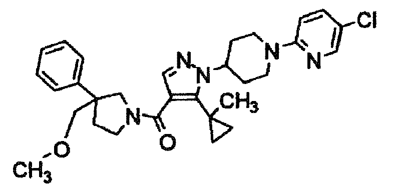 | ¹H-NMR (400MHz, CDCl₃) δ : 0.69-0.93 (4H, m), 1.41-1.52 (3H, m), 1.83-2.01 (2H, m), 2.13-2.51 (4H, m), 2.97-3.11 (2H, m), 3.16-4.12 (9H, m), 4.41-4.54 (2H, m), 4.65-4.78 (1H, m), 6.62-6.71 (1H, m), 7.12-7.55 (7H, m), 8.10-8.16 (1H, m) |
| 175 | 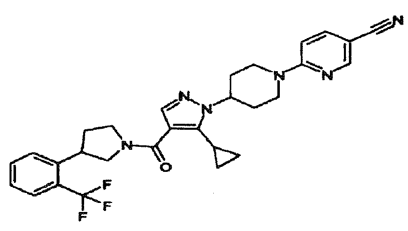 | ¹H-NMR (400MHz, CDCl₃) δ : 0.72-0.85 (2H, m), 1.04-1.16 (2H, m), 1.73-1.88 (1H, m), 1.94-2.09 (2H, m), 2.10-2.45 (5H, m), 3.07-3.22 (2H, m), 3.42-3.51 (0.5H, m), 3.57-4.10 (3.5H, m), 4.52-4.80 (3H, m), 6.62-6.71 (1H, m), 7.32-7.41 (1H, m), 7.41-7.72 (5H, m), 8.38-8.45 (1H, m) |
| 176 | 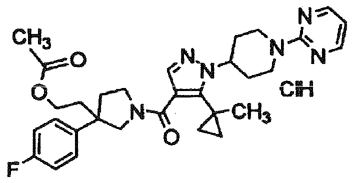 | ¹H-NMR (400MHz, DMSO-D₆) δ: 0.46-0.86 (4H, m), 1.07-1.24 (2H, m), 1.27-1.41 (3H, m), 1.73-2.07 (7H, m). 2.15-2.30 (2H, m), 3.06-4.08 (8H, m), 4.72-4.88 (3H, m), 6.66-6.74 (1H, m), 7.08-7.61 (5H, m), 8.38-8.48 (2H, m) |
| 177 | 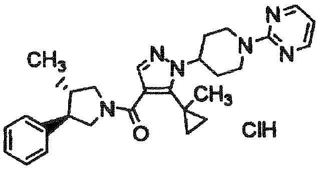 | ¹H-NMR (400MHz, DMSO-D₆) δ: 0.70-0.97 (7H, m), 1.35-1.42 (3H, m), 1.86-2.03 (4H, m), 2.25-2.46 (1H, m), 2.84-2.95 (1H, m), 3.07-3.24 (3H, m), 3.39-3.57 (1H, m), 3.67-3.77 (1H, m), 3.81-3.94 (1H, m), 4.75-4.88 (3H, m), 6.64-6.72 (1H, m), 7.20-7.41 (5H, m), 7.53-7.61 (1H, m), 8.39-8.44 (2H, m) |

**[Table 1-28]**

| Example No. | Structural formula | NMR |
|---|---|---|
| 178 | 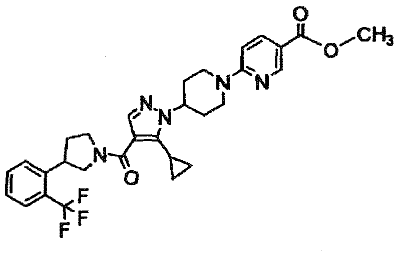 | ¹H-NMR (400MHz, CDCl₃) δ : 0.74-0.86 (2H, m), 1.05-1.15 (2H, m), 1.76-1.88 (1H, m), 1.93-2.09 (3H, m), 2.11-2.46 (4H, m), 3.04-3.19 (2H, m), 3.43-3.51 (0.5H, m), 3.59-4.09 (3.5H, m), 3.84-3.91 (3H, s), 4.58-4.78 (3H, m), 6.61-6.71 (1H, m), 7.32-7.61 (3.5H, m). 7.63-7.71 (1H, m), 7.98-8.08 (1.5H, m). 8.77-8.83 (1H, m) |
| 179 | 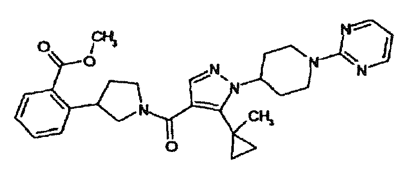 | ¹H-NMR (400MHz, CDCl₃) δ : 0.77-0.98 (4H, m), 1.44-1.53 (3H, m), 1.86-2.46 (6H, m). 2.99-3.15 (2H, m), 3.45-4.06 (7H, m), 4.18-4.41 (1H, m). 4.68-4.85 (1H, m), 4.94-5.09 (2H, m), 6.48-6.55 (1H, m), 7.26-7.56 (4H, m), 7.82-7.92 (1H, m), 8.31-8.37 (2H, m) |
| 180 | 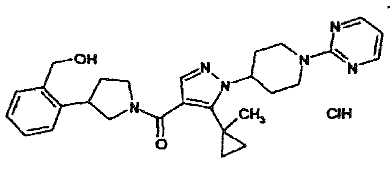 | ¹H-NMR (400MHz, DMSO-D₆) δ: 0.67-0.91 (4H, m), 1.29-1.44 (3H, m), 1.74-2.34 (7H. m). 3.02-4.18 (7H, m), 4.47-4.69 (2H, m), 4.77-4.91 (3H, m), 6.60-6.71 (1H, m), 7.14-7.69 (5H, m), 8.34-8.46 (2H, m) |
| 181 | 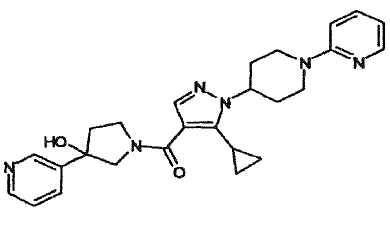 | ¹H-NMR (400MHz, CDCl₃) δ : 0.68-0.77 (2H, m), 0.99-1.15 (2H, m), 1.75-1.89 (1H, m), 1.92-2.05 (2H, m), 2.18-2.53 (4H, m), 2.96-3.09 (2H, m), 3.68-3.85 (2H, m), 3.87-4.04 (2H, m), 4.44-4.56 (2H, m). 4.58-4.72 (1H, m), 6.58-6.67 (1H, m), 6.68-6.76 (1H, m), 7.29-7.39 (1H, m), 7.45-7.53 (2H, m). 7.75-7.88 (1H, m), 8.17-8.23 (1H, m), 8.54-8.62 (1H, m), 8.69-8.83 |
| 182 | 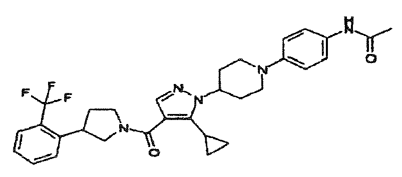 | ¹H-NMR (400MHz, CDCl₃) δ : 0.70-0.83 (2H, m), 1.01-1.15 (2H, m), 1.69-1.88 (1H, m), 1.90-2.21 (6H, m), 2.26-2.50 (3H, m), 2.77-3.00 (2H, m), 3.41-4.11 (7H, m), 4.40-4.62 (1H, m), 6.85-6.97 (2H, m), 7.31-7.71 (7H, m) |
| 183 | 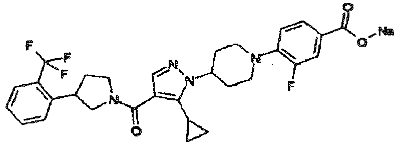 | ¹H-NMR (400MHz, DMSO-D₆) δ: 0.60-0.75 (2H, m), 0.94-1.86 (2H, m), 1.88-2.34 (7H, m), 2.79-2.98 (2H, m), 3.44-3.93 (7H, m), 4.50-4.68 (1H, m), 6.89-7.03 (1H, m), 7.43-7.80 (7H, m) |

**[Table 1-29]**

| Example No. | Structural formula | NMR |
|---|---|---|
| 184 | 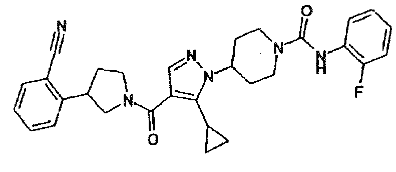 | ¹H-NMR (400MHz, CDCl₃) δ: 0.70-0.86 (2H, m), 1.05-1.16 (2H, m), 1.77-1.88 (1H, m), 1.94-2.53 (6H, m), 3.06-3.22 (2H, m), 3.45-4.16 (5H, m), 4.21-4.35 (2H, m), 4.57-4.73 (1H, m), 6.67 (1H, s), 6.95-7.17 (3H, m), 7.35-7.73 (5H, m), 8.05-8.13 (1H, m) |
| 185 | 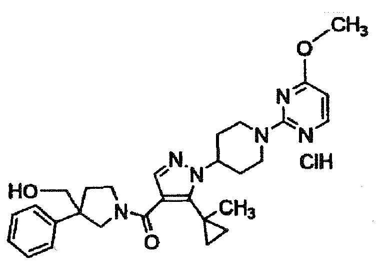 | ¹H-NMR (400MHz, DMSO-D₆) δ : 0.58-0.88 (4H, m), 1.29-1.44 (3H, m), 1.94-2.21 (5H, m), 2.28-2.41 (1H, m), 3.27-3.93 (8H, m), 3.95-4.05 (3H, m), 4.58-4.97 (3H, m), 5.42-6.26 (1H, m), 6.37-6.50 (1H, m), 7.14-7.41 (5H, m), 7.53-7.68 (1H, m), 8.11-8.23 (1 H, m) |
| 186 | 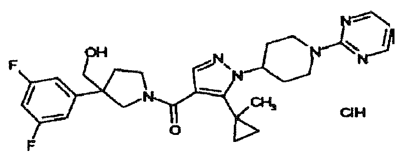 | ¹H-NMR (400MHz, DMSO-D₆) δ : 0.55-0.85 (4H, m), 1.32-1.41 (3H, m), 1.84-2.40 (7H, m), 3.06-3.23 (2H, m), 3.29-3.90 (5H, m), 4.72-4.88 (3H, m), 4.89-5.35 (2H, m), 6.66-6.72 (1H, m), 6.91-7.64 (4H, m), 8.39-8.44 (2H, m) |
| 187 | 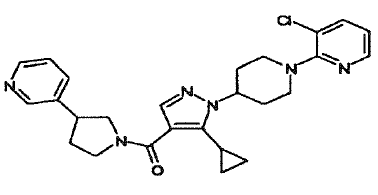 | ¹H-NMR (400MHz, CDCl₃) δ: 0.68-0.85 (2H, m), 1.01-1.13 (2H, m), 1.57-1.88 (2H, m), 1.93-2.20 (1H, m), 2.33-2.56 (3H, m), 2.94-3.07 (2H, m), 3.35-3.82 (4H, m), 3.86-4.21 (4H, m), 4.52-4.66 (1H, m), 6.82-6.88 (1H, m), 7.24-32 (3H, m), 7.55-7.64 (3H, m), 8.15-8.22 (1H, m), 8.47-8.61 (1H, m) |
| 188 | 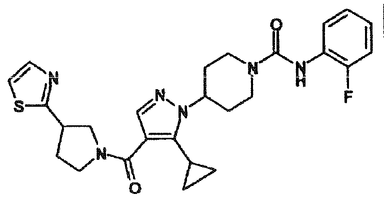 | ¹H-NMR (400MHz, CDCl₃) δ: 0.67-0.78 (2H, m), 0.96-1.11 (2H, m), 1.71-1.85 (1H, m), 1.92-2.09 (3H, m), 2.16-2.57 (4H, m), 3.04-3.21 (2H, m), 3.55-4.00 (4H, m), 4.19-4.34 (2H, m), 4.54-4.68 (1H, m), 6.61-6.70 (1H, m), 6.91-7.15 (3H, m), 7.23-1.31 (1H, m), 7.51-7.58 (1H, m), 7.67-7.76 (1H, m), 8.03-8.12 (1H, m) |

**[Table 1-30]**

| Example No. | Structural formula | NMR |
|---|---|---|
| 189 | 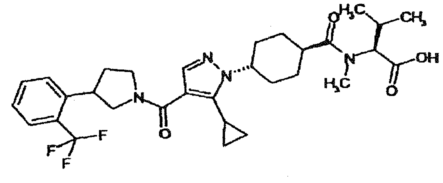 | ¹H-NMR (400MHz, DMSO-D₆) δ : 0.53-0.69 (2H, m), 0.71-0.88 (2H, m), 0.91-1.11 (6H, m), 1.43-2.39 (10H, m), 2.62-2.82 (2H, m), 2.92-3.06 (3H, m), 3.42-3.92 (6H, m), 4.10-4.74 (2H, m), 7.39-7.59 (2H, m), 7.63-7.81 (2H, m) |
| 190 | 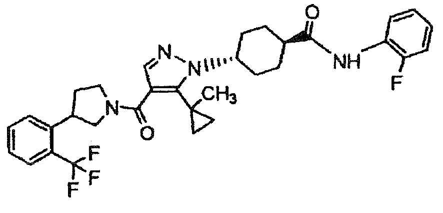 | H-NMR (400MHz, CDCl₃) δ: 0.73-0.97 (4H, m), 1.36-1.51 (3H, m), 1.77-1.95 (2H, m), 1.97-2.26 (7H, m), 2.29-2.49 (2H, m), 3.47-4.11 (5H, m), 4.46-4.64 (1H, m), 7.00-7.20 (3H, m), 7.32-7.72 (6H, m), 8.29-8.42 (1H, m) |
| 191 | 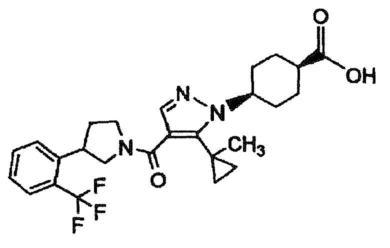 | ¹H-NMR (400MHz, CDCl₃) δ: 0.73-0.97 (4H, m), 1.38-1.49 (3H, m), 1.60-1.76 (1H, m), 1.77-1.90 (2H, m), 1.93-2.18 (2H, m), 2.19-2.56 (5H, m), 2.70-2.81 (1H, m), 3.47-4.09 (5H, m), 4.42-4.61 (1H, m), 7.31-7.72 (5H, m) |

In addition, the structural formula and NMR data of the compounds obtained in each step of Example 2-1 and step 7-4 of Example 2 are shown in the following Table 2.

**Table 2**

| Step | Structural formula | NMR |
|---|---|---|
| Example 2-1 Step 1 | 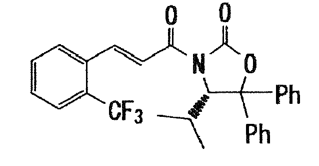 | ¹H-NMR (400MHz, CDCl₃) δ: 0.80-0.84 (3H, m), 0.93-0.97 (3H, m), 1.97-2.10 (1H, m), 5.53 (1 H, d, J = 3.2 Hz), 7.24-7.89 (15H, m), 8.13-8.23 (1 H, m) |
| Example 2-1 Step 2 | 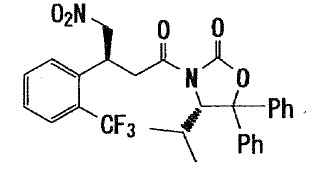 | ¹H-NMR (400MHz, CDCl₃) δ: 0.77 (3H, d, J = 6.8 Hz), 0.86 (3H, d, J = 6.8 Hz), 1.89-2.02 (1H, m), 3.29-3.51 (2H, m), 4.45-4.53 (1 H, m), 4.62-4.76 (2H, m), 5.31 (1 H, d, J = 3.3 Hz), 7.19-7.41 (13H, m), 7.63-7.68 (1 H, m) |
| Example 2-1 Step 3 | 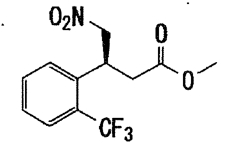 | ¹H-NMR (400MHz, CDCl₃) δ: 2.85 (2H, d, J = 7.2 Hz), 3.65 (3H, s), 4.38-4.48 (1 H, m), 4.76 (2H, d, J = 7.0 Hz), 7.33-7.78 (4H, m) |
| Example 2-1 Step 4 | 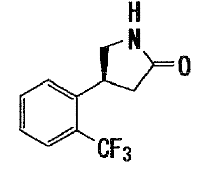 | ¹H-NMR (400MHz, CDCl₃) δ: 2.42-2.56 (1 H, m), 2.75-2.90 (1H, m), 3.36-3.49 (1 H, m), 3.78-3.89 (1 H, m), 4.05-4.21 (1H,m), 6.26-6.51 (1H, m), 7.31-7.45 (1 H, m), 7.52-7.62 (2H, m), 7.64-7.72 (1H, m) |
| Example 2-1 Step 5 | 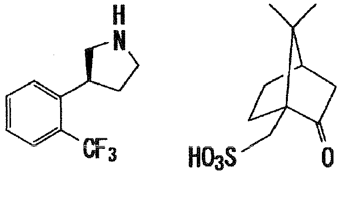 | ¹H-NMR (400MHz, CDCl₃) δ: 0.72-0.75 (3 H, m), 1.04 (3H, s), 1.21-1.38 (2H, m), 1.74-2.13 (4H, m), 2.17-2.47 (3H, m), 2.58-2.72 (1 H, m), 2.83-2.96 (1H, m), 3.11-3.43 (2H, m), 3.44-3.63 (2H, m), 3.64-3.77 (1H, m), 7.43-7.54 (1H, m), 7.66-7.84 (3H, m), 8.77-9.23 (2H, m) |
| Example 2-1 Step 7-4 | 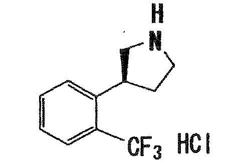 | ¹H-NMR (400MHz, CDCl₃) δ: 2.15-2.29 (1 H, m), 2.44-2.55 (1H, m), 3.33-3.49 (2H, m), 3.65-3.85 (2H, m), 3.87-4.00 (1H, m), 7.35-7.45 (1H, m), 7.58-7.76 (3H, m), 9.89-10.50 (2H, m) |

### Experimental Example 1: HSD-1 (hydroxysteroid dehydrogenase 1) activity inhibitory action in vitro

The HSD-1 activity inhibitory action was determined by quantitation of inhibition of conversion of cortisone to cortisol by SPA (scintillation proximity assay) system and using, as an enzyme source, human HSD-1 (hereinafter recombinant HSD-1) expressed using baculovirus system. Reagents (100 µl) were added to a 96 well plate (96 well Optiplates^{™}-96 (Packard)) to a final concentration shown below and the reaction was carried out at room temperature for 90 to 120 min. As a reaction mixture, 5-100 ng/well recombinant HSD-1, 500 µM NADPH and 10 nM ³H cortisone (American Radiolabeled Chemicals Inc., 50 Ci/mmol) were dissolved in PBS containing 0.1% BSA (Sigma), and the test substance was 2 µl of a compound solution (dissolved in DMSO). After the reaction, 50 µl of PBS (containing 0.1% BSA (Sigma)) containing 0.2 µg of anticortisol mouse monoclonal antibody (East Coast Biologics), 500 µg of anti-mouse antibody bonded PVT SPA beads (Amersham Biosciences) and 133 µM of carbenoxolone (Sigma) was added to the reaction mixture to stop the reaction. After completion of the reaction, the mixture was incubated at room temperature for not less than 2 hr, and the radioactivity was measured with Topcount (Packard). As the control, the value of the well added with 2 µl of DMSO instead of the test substance was used (0% inhibition) and, as the positive control, the value of the well added with carbenoxolone (final concentration 100 µM) instead of the test substance was used (100% inhibition). inhibition (%) of the test substance was calculated from ((value of control - value of test substance)/(value of control - value of positive control))×100(%). The IC₅₀ value was calculated from the inhibition rates of two points across the 50% inhibition. The obtained results are shown in the following Table 3-1 to Table 3-3.

**[Table 3-1]**

| Example No. | hHSD1 (IC₅₀(nM)) | Example No. | hHSD1 (IC₅₀(nM)) |
|---|---|---|---|
| 2 | ++ | 41 | ++ |
| 3 | ++ | 42 | ++ |
| 4 | ++ | 43 | ++ |
| 6 | ++ | 44 | ++ |
| 7 | ++ | 45 | ++ |
| 8 | ++ | 47 | ++ |
| 9 | ++ | 48 | ++ |
| 10 | ++ | 49 | ++ |
| 11 | ++ | 50 | ++ |
| 12 | ++ | 51 | ++ |
| 13 | ++ | 52 | ++ |
| 14 | ++ | 53 | ++ |
| 15 | ++ | 54 | ++ |
| 16 | ++ | 55 | ++ |
| 17 | ++ | 56 | ++ |
| 18 | ++ | 57 | ++ |
| 19 | ++ | 58 | ++ |
| 20 | ++ | 59 | ++ |
| 21 | ++ | 60 | ++ |
| 22 | ++ | 61 | ++ |
| 23 | ++ | 63 | ++ |
| 24 | ++ | 64 | ++ |
| 25 | ++ | 65 | ++ |
| 26 | ++ | 66 | ++ |
| 27 | ++ | 67 | ++ |
| 28 | ++ | 68 | ++ |
| 29 | ++ | 69 | ++ |
| 30 | ++ | 71 | ++ |
| 31 | ++ | 72 | ++ |
| 32 | ++ | 73 | ++ |
| 33 | ++ | 74 | ++ |
| 34 | ++ | 75 | ++ |
| 35 | ++ | 76 | ++ |
| 36 | ++ | 77 | ++ |
| 37 | ++ | 78 | ++ |
| 39 | ++ | 79 | ++ |
| 40 | ++ | 80 | ++ |

**[Table 3-2]**

| Example No. | hHSD1 (IC₅₀(nM)) | Example No. | hHSD1 (IC₅₀(nM)) |
|---|---|---|---|
| 83 | ++ | 130 | ++ |
| 84 | ++ | 131 | ++ |
| 85 | ++ | 132 | ++ |
| 87 | ++ | 133 | ++ |
| 88 | ++ | 134 | ++ |
| 89 | ++ | 135 | ++ |
| 90 | ++ | 136 | ++ |
| 91 | ++ | 137 | ++ |
| 93 | ++ | 138 | ++ |
| 94 | ++ | 139 | ++ |
| 95 | ++ | 140 | ++ |
| 96 | ++ | 141 | ++ |
| 97 | ++ | 142 | ++ |
| 99 | ++ | 145 | ++ |
| 100 | ++ | 146 | ++ |
| 103 | ++ | 147 | ++ |
| 105 | ++ | 148 | ++ |
| 106 | ++ | 149 | ++ |
| 107 | ++ | 150 | ++ |
| 108 | ++ | 151 | ++ |
| 109 | ++ | 154 | ++ |
| 110 | ++ | 155 | ++ |
| 111 | ++ | 156 | ++ |
| 112 | ++ | 157 | ++ |
| 113 | ++ | 158 | ++ |
| 114 | ++ | 165 | ++ |
| 115 | ++ | 166 | ++ |
| 116 | ++ | 167 | ++ |
| 117 | ++ | 168 | ++ |
| 118 | ++ | 169 | ++ |
| 119 | ++ | 170 | ++ |
| 120 | ++ | 171 | ++ |
| 124 | ++ | 172 | ++ |
| 125 | ++ | 173 | ++ |
| 126 | ++ | 174 | ++ |
| 128 | ++ | 175 | ++ |
| 129 | ++ | 176 | ++ |

**[Table 3-3]**

| Example No. | hHSD1 (IC₅₀(nM)) | Example No. | HHSD1 (IC₅₀(nM)) |
|---|---|---|---|
| 177 | ++ | 185 | ++ |
| 178 | ++ | 186 | ++ |
| 179 | ++ | 187 | ++ |
| 180 | ++ | 189 | ++ |
| 182 | ++ | 190 | ++ |
| 183 | ++ | 191 | ++ |
| 184 | ++ | | |

In the above-mentioned Tables, ++ means IC₅₀ value of not more than 30 nM.

### Industrial Applicability

The production method of the present invention can efficiently produce a compound represented by the above-mentioned formula [1'], which has a superior HSD1 inhibitory action, and therefore, is useful as a prophylactic or therapeutic agent for metabolic diseases such as diabetes, insulin resistance, diabetic complications, obesity, hyperlipidemia, hypertension, fatty liver and the like, and the like and a compound useful as a synthetic intermediate therefor, with superior achievability of asymmetric synthesis (i.e., superior selectivity), superior yield, superior safety and superior industrial workability at a low cost.
In addition, the compound of the present invention can be advantageously used as a synthetic intermediate for a compound represented by the above-mentioned formula [1'].

This application is based on application No. 2006-331146 filed in Japan and No. 60/876,239 filed in the US, the contents of which are incorporated hereinto by reference.

## Claims

1. A compound represented by the following formula [3]: 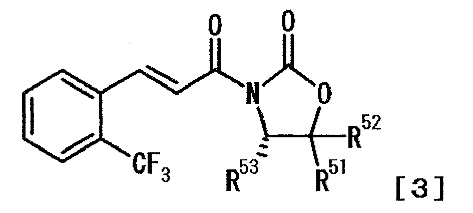 wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof.

2. The compound of claim 1, wherein both R⁵¹ and R⁵² are phenyl groups, or a salt thereof, or a solvate thereof.

3. The compound of claim 1 or 2, wherein R⁵³ is an isopropyl group, or a salt thereof, or a solvate thereof.

4. A compound represented by the following formula [4]: 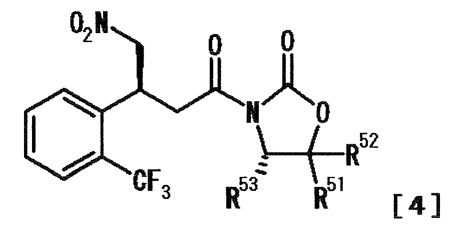 wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof.

5. The compound of claim 4, wherein both R⁵¹ and R⁵² are phenyl groups, or a salt thereof, or a solvate thereof.

6. The compound of claim 4 or 5, wherein R⁵³ is an isopropyl group, or a salt thereof, or a solvate thereof.

7. A compound represented by the following formula [6]: 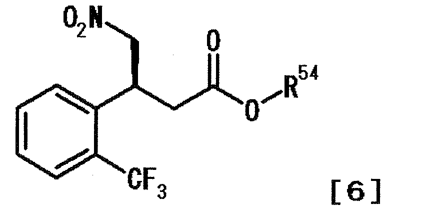 wherein R⁵⁴ is a C₁₋₆ alkyl group optionally substituted by one or more, same or different aryl groups,
or a solvate thereof.

8. A compound represented by the following formula [7]: 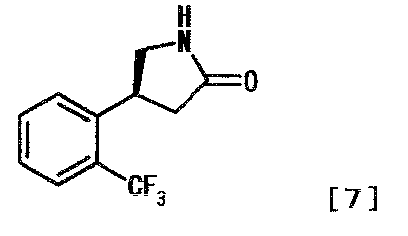 or a solvate thereof.

9. A compound represented by the following formula [8]: 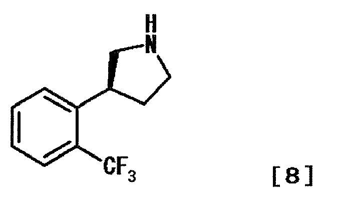 or a salt thereof, or a solvate thereof.

10. A production method of a compound represented by the following formula [3]: 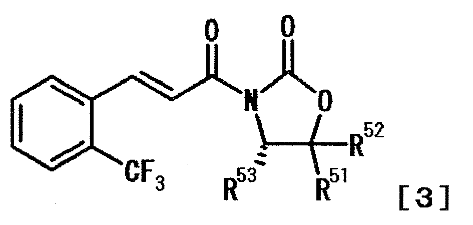 wherein R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group, or a salt thereof, or a solvate thereof, comprising reacting a compound represented by the following formula [15]: 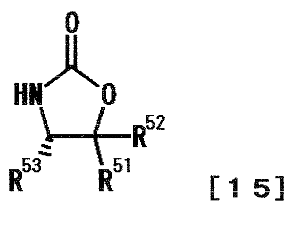 wherein R⁵¹, R⁵² and R⁵³ are as defined above, or a salt thereof, or a solvate thereof, with a compound represented by the following formula [2]: 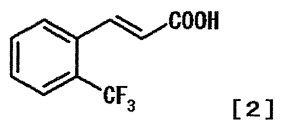 or a reactive derivative thereof, or a salt thereof, or a solvate thereof.

11. The production method of claim 10, wherein the reactive derivative of the compound represented by the formula [2] is a reactive derivative represented by the following formula [2a]: 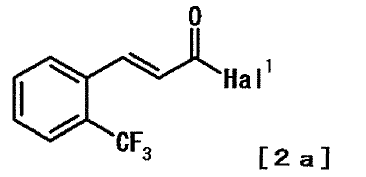 wherein Hal¹ is a halogen atom.

12. The production method of claim 11, wherein Hall is a chlorine atom.

13. The production method of any one of claims 10 to 12, wherein R⁵¹ and R⁵² are each a phenyl group.

14. The production method of any one of claims 10 to 13, wherein R⁵³ is an isopropyl group.

15. The production method of any one of claims 10 to 12, wherein the compound represented by the formula [3] is (S)-4-isopropyl-5,5-diphenyl-3-[(E)-3-(2-trifluoromethylphenyl)-acryloyl]-oxazolidin-2-one.

16. A production method of a compound represented by the following formula [4]: 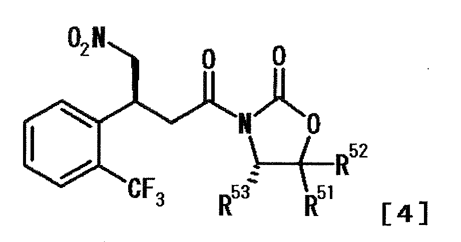 wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group, or a salt thereof, or a solvate thereof, comprising reacting a compound represented by the following formula [3]: 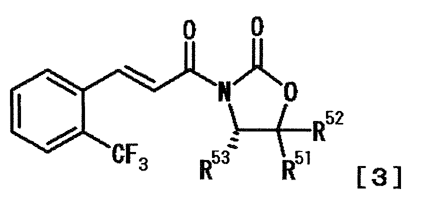
wherein R⁵¹, R⁵² and R⁵³ are as defined above,
or a salt thereof, or a solvate thereof, with nitromethane.

17. The production method of claim 16, wherein R⁵¹ and R⁵² are each a phenyl group.

18. The production method of claim 16 or 17, wherein R⁵³ is an isopropyl group.

19. A production method of a compound represented by the following formula [6]: 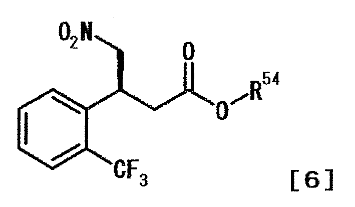 wherein R⁵⁴ is a C₁₋₆ alkyl group optionally substituted by one or more, same or different aryl groups, or a solvate thereof, comprising reacting a compound represented by the following formula [4]: 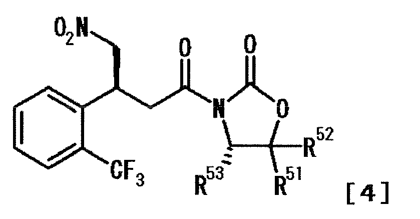 wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof, with a compound represented by the following formula [5]:
R⁵⁴-OH [5]
wherein R⁵⁴ is as defined above,
or a solvate thereof.

20. The production method of claim 19, wherein R⁵¹ and R⁵² are each a phenyl group.

21. The production method of claim 19 or 20, wherein R⁵³ is an isopropyl group.

22. A production method of a compound represented by the following formula [7]: 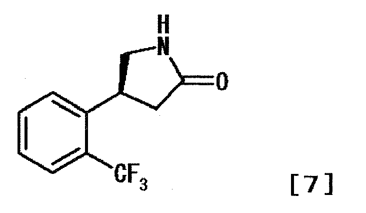 or a solvate thereof, comprising reducing a compound represented by the following formula [6]: 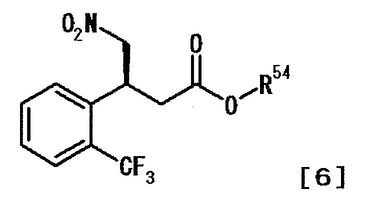 wherein R⁵⁴ is a C₁₋₆ alkyl group optionally substituted by one or more, same or different aryl groups, or a solvate thereof, and then cyclizing the compound.

23. A production method of a compound represented by the following formula [8]: 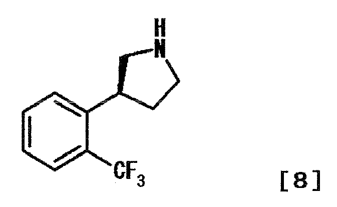 or a salt thereof, or a solvate thereof, comprising reducing a compound represented by the following formula [7]: 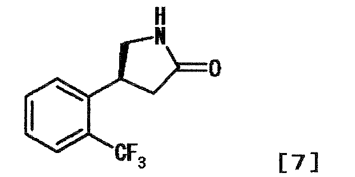 or a solvate thereof.

24. A method of producing a compound represented by the following formula [8]: 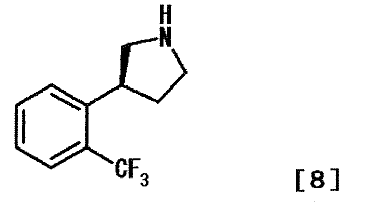 or a salt thereof, or a solvate thereof, from a compound represented by the following formula [2]: 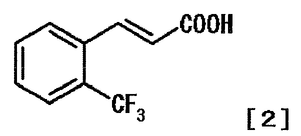 or a reactive derivative thereof, or a salt thereof, or a solvate thereof, comprising one or more steps selected from the following group A:
[group A]
1. a step of producing a compound represented by the following formula [3]: 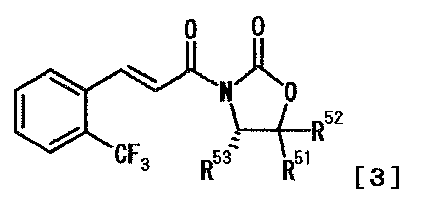 wherein R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group,
or a salt thereof, or a solvate thereof, comprising reacting a compound represented by the following formula [15]: 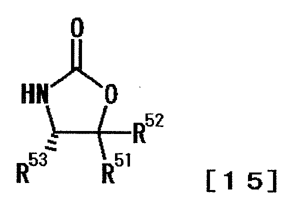 wherein R⁵¹, R⁵² and R⁵³ are as defined above, or a salt thereof, or a solvate thereof, with a compound represented by the following formula [2]: 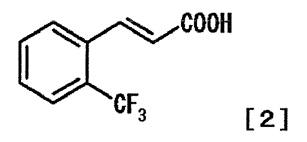 or a reactive derivative thereof, or a salt thereof, or a solvate thereof;
2. a step of producing a compound represented by the following formula [4]: 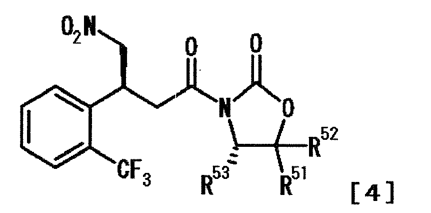 wherein R⁵¹, R⁵² and R⁵³ are as defined above,
or a salt thereof, or a solvate thereof, comprising reacting a compound represented by the above-mentioned formula [3] or a salt thereof, or a solvate thereof, with nitromethane;
3. a step of producing a compound represented by the following formula [6]: 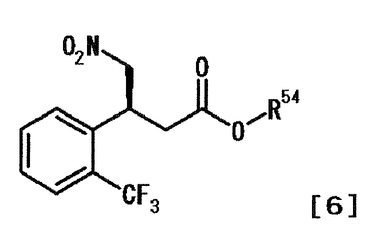 wherein R⁵⁴ is a C₁₋₆ alkyl group optionally substituted by one or more, same or different aryl groups, or a solvate thereof, comprising reacting a compound represented by the above-mentioned formula [4] or a salt thereof, or a solvate thereof, with a compound represented by the following formula [5]:
R⁵⁴-OH [5]
wherein R⁵⁴ is as defined above, or a solvate thereof;
4. a step of producing a compound represented by the following formula [7]: 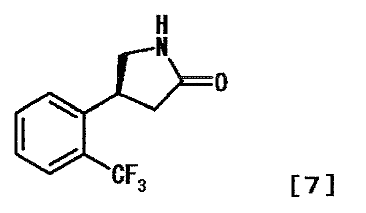 or a solvate thereof, comprising reducing a compound represented by the above-mentioned formula [6] or a solvate thereof, and then cyclizing the compound; and
5. a step of producing a compound represented by the following formula [8]: 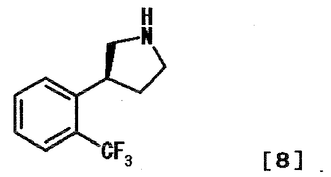 or a salt thereof, or a solvate thereof, comprising reducing a compound represented by the above-mentioned formula [7] or a solvate thereof.

25. The production method of claim 24, comprising all steps of group A.

26. The production method of claim 24 or 25, wherein R⁵¹ and R⁵² are each a phenyl group.

27. The production method of any one of claims 24 to 26, wherein R⁵³ is an isopropyl group.

28. A method of producing a compound represented by the following formula [9]: 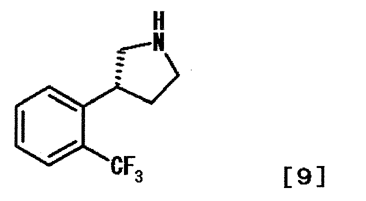 or a salt thereof, or a solvate thereof, from a compound represented by the following formula [2]: 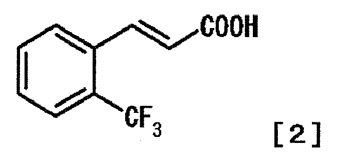 or a reactive derivative thereof, or a salt thereof, or a solvate thereof, comprising one or more steps selected from the following group B:
[group B]
1. a step of producing a compound represented by the following formula [11]: 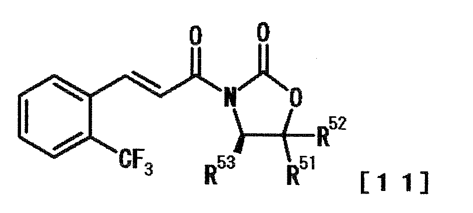 wherein
R⁵¹ and R⁵² are the same or different and each is
1) a hydrogen atom,
2) a C₁₋₆ alkyl group,
3) an aryl group, or
4) an unsaturated monocyclic 5-membered or 6-membered heterocyclic group; and
R⁵³ is
1) a C₁₋₆ alkyl group,
2) an aryl group, or
3) an unsaturated monocyclic 5-membered or 6-membered heterocyclic group, or a salt thereof, or a solvate thereof, comprising reacting a compound represented by the following formula [10]: 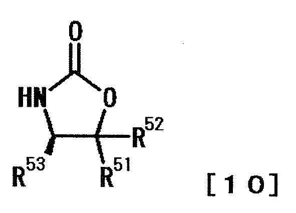
wherein R⁵¹, R⁵² and R⁵³ are as defined above, or a salt thereof, or a solvate thereof, with a compound represented by the following formula [2]: 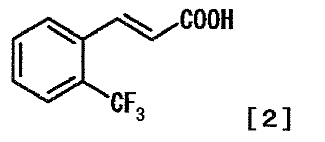 or a reactive derivative thereof, or a salt thereof, or a solvate thereof;
2. a step of producing a compound represented by the following formula [12]: 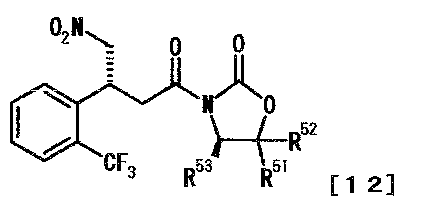 wherein R⁵¹, R⁵² and R⁵³ are as defined above, or a salt thereof, or a solvate thereof, comprising reacting a compound represented by the above-mentioned formula [11] or a salt thereof, or a solvate thereof, with nitromethane;
3. a step of producing a compound represented by the following formula [13]: 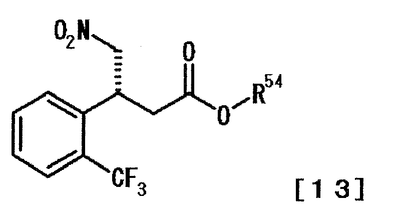 wherein R⁵⁴ is a C₁₋₆ alkyl group optionally substituted by one or more, same or different aryl groups, or a solvate thereof, comprising reacting a compound represented by the above-mentioned formula [12] or a salt thereof, or a solvate thereof, with a compound represented by the following formula [5]:
R⁵⁴-OH [5]
wherein R⁵⁴ is as defined above, or a solvate thereof;
4. a step of producing a compound represented by the following formula [14]: 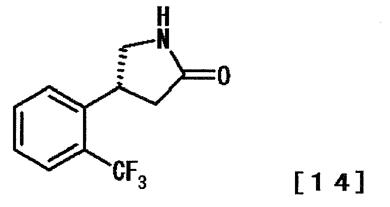 or a solvate thereof, comprising reducing a compound represented by the above-mentioned formula [13] or a solvate thereof, and then cyclizing the compound; and
5. a step of producing a compound represented by the following formula [9]: 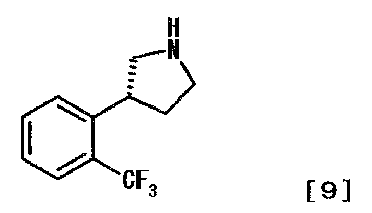 or a salt thereof, or a solvate thereof, comprising reducing a compound represented by the above-mentioned formula [14] or a solvate thereof.

29. The production method of claim 28, comprising all steps of group B.

30. The production method of claim 28 or 29, wherein R⁵¹ and R⁵² are each a phenyl group.

31. The production method of any one of claims 28 to 30, wherein R⁵³ is an isopropyl group.
